# EUROPEAN PATENT SPECIFICATION

(11) **EP 1 144 365 B1**
(45) Date of publication and mention of the grant of the patent: **17.03.2004**
(21) Application number: 99966584.7
(22) Date of filing: 20.12.1999
(51) Int. Cl.: C07C 235/00

(54) **INHIBITORS OF ALPHA-4 BETA-1 MEDIATED CELL ADHESION**
INHIBITOREN DER ALPHA-4 BETA-1 VERMITTELTEN ZELLADHÄSION
INHIBITEURS DE L'ADHESION CELLULAIRE INDUITE PAR ALPHA-4 BETA-1

(30) Priority: 22.12.1998 US 113501 P
(43) Date of publication of application: 17.10.2001
(73) Proprietor: TANABE SEIYAKU CO., LTD., Chuo-ku, Osaka-shi, Osaka 541-8505 (JP)
(72) Inventor: TEEGARDEN, Bradley, R., San Diego, CA 92131 (US); JAYAKUMAR, Honnappa, San Diego, CA 92129 (US); MATSUKI, Kenji, Hiki-gun, Saitama 355-0811 (JP); CHRUSCIEL, Robert, A., Portage, MI 49024 (US); FISHER, Jed, F., Kalamazoo, MI 49008 (US); TANIS, Steven, P., Kalamazoo, MI 49002 (US); THOMAS, Edward, W., Kalamazoo, MI 49008 (US); BLINN, James, R., Portage, MI 49024 (US)
(74) Representative: VOSSIUS & PARTNER
(86) International application number: PCT/US1999/030665
(87) International publication number: WO 2000/037429

(56) References cited:
- WO-A-96/22966
- WO-A-98/58902

## Description

The present invention relates to small molecules that are potent inhibitors of α₄β₁ mediated adhesion to either VCAM or CS-1 and which could be useful for the treatment of inflammatory diseases.

### Description of Related Art

The extracellular matrix (ECM) is the major component of connective tissue which provides structural integrity, and promotes cell migration and differentiation. As part of these functions, extracellular matrix molecules such as fibronectin, collagen, laminin, von Willebrand factor, thrombospondin, fibrinogen, and tenascin have been shown to support adhesion of cells *in vitro*. This adhesive interaction is critical for a number of biological processes including hemostasis, thrombosis, wound healing, tumor metastasis, immunity and inflammation.

Fibronectin (FN) is the prototype ECM molecule. The major cell attachment site in the fibronectin molecule has been reproduced synthetically with the amino acid sequence arginine-glycine-aspartic acid, or RGD using single letter nomenclature. Peptides containing the RGD sequence which either inhibit or promote cell adhesion have been described (US Patent Nos. 4,589,881; 4,661,111; 4,517,686; 4,683,291; 4,578,079; 4,614,517; and 4,792,525). Changes in the peptide as small as the exchange 'of alanine for' glycine or glutamic acid for aspartic acid, which constitute the addition of a single methyl or methylene group to the tripeptide, eliminates these activities (Pierschbacher et al., *Proc. Natl. Acad. Sci*. *USA* 81:5985 (1984)). Recently, a second FN cell binding domain has been identified within the alternatively spliced region of the A chain of the molecule, known as the connecting segment 1 (CS-1). The most active cell-binding site within this alternatively spliced region is composed of 25 amino acids where the carboxy terminus contains the sequence EILDVPST. The amino acid sequence EILDVPST forms a recognition motif on FN for cell surface receptors. (Wayner et al., *J. Cell Biol*. 109:1321 (1989); Guan et al., *Cell* 60:53 (1990)).

The receptors which' recognize these sites on FN belong to a gene superfamily called integrins which consist of heterodimeric complexes of non-covalently associated alpha and beta subunits. A common β subunit combines with unique α subunits to form an adhesion receptor of defined specificity. To date, 8 β subunits have been identified which can dimerize with 16 distinct α subunits forming 22 distinct integrins. The β₁ subfamily, also known as the VLA family (Very Late Activation Antigens), binds to ECM molecules such as FN, collagen and laminin. For reviews, see, Hynes, *Cell* 48:549 (1987); Hemler, Annu. *Rev. Immunol*. 8:365 (1990). Leukocyte interaction with FN at the two spatially separate binding domains is mediated by two distinct integrins. The RGD site is recognized by the integrin α₅β₁, while, EILDV is recognized by α₄β₁ (Pytela et al., *Cell* 40:191 (1985); Wayner et al., *J. Cell Biol*. 109:1321 (1989); Guan et al, *Cell* 60:53 (1990)).

Vascular endothelial cells form the interface between blood and tissues and control the passage of leukocytes as well as plasma fluid into tissues. A variety of signals 'generated at the 'site of inflammation can activate both endothelial cells as well as circulating leukocytes so that they become more adhesive to one another. Following this initial adhesion the leukocytes migrate into the tissues to perform host defense functions. Several adhesion molecules have been identified which are involved in leukocyte-endothelial interactions.

In the β₁ subfamily, in addition to binding to fibronectin, α₄β₁ interacts with a cytokine inducible protein on endothelial cells termed vascular cell adhesion molecule (VCAM). Further involved in the leukocyte-endothelial adhesion process is the β₂ integrin subfamily. β₂ integrins include CD11a/CD18, CD11b/CD18, and CD11c/CD18. In addition, the β₇ subunit associates with α₄ to form a unique α₄β₇ heterodimer which binds to FN, to VCAM, and to Mucosal Addressin Cell Adhesion Molecule-1 (MAdCAM) (Ruegg et al, *J. Cell.Biol.* 117:179 (1992); Andrew et al., *J. Immunol.* 153:3847 (1994); Briskin et al., *Nature* 363:461 (1993); Shyjan et al, *J. Immunol*. 156:2851 (1996)). α₄ integrins are widely expressed on different cell types including hematopoietic progenitors, lymphocytes, natural killer cells, monocytes, eosinophils, basophils, and mast cells (Helmer, M. E., *Annu. Rev. Immunol.* 8:365 (1990)). Other molecules on endothelial cells which bind to the leukocytes include ICAM-1, ICAM-2, E-selectin and P-selectin (Carlos and Harlan, *Immunol. Rev.* 114:1 (1990); Osborn, L., *Cell* 62:3 (1990); Springer T., *Nature* 346:425 (1990); Geng et al., *Nature* 347:757 (1990); Stoolman, *Cell* 56:907 (1989)).

A number of *in vitro* and *in vivo* studies indicate that α₄β₁ plays a critical role in the pathogenesis of a variety of diseases. Monoclonal antibodies directed against α₄ have been tested in a variety of disease models. Anti-α₄ antibodies block adhesion of lymphocytes to synovial endothelial cells; this adhesion plays a potential role in rheumatoid arthritis (van Dinther-Janssen et al, *J. Immunol*. 147:4207 (1991)). α₄ has also been implicated with respect to rheumatoid arthritis in separate studies (Laffon et al, *J. Clin. Invest.* 88:546 (1991); Morales-Ducret et al, *J. Immunol*. 149:1424 (1992)). A significant number of studies have evaluated the role of α₄ in allergy and asthma. For example, monoclonal antibodies to α₄ block adhesion of basophils and eosinophils to cytokine activated endothelial cells (Walsh et al, *J. Immunol.* 146:3419 (1991); Bochner et al, *J. Exp. Med.* 173:1553 (1991)). Monoclonal antibodies to α₄ were also effective in several lung antigen challenge models (Abraham et al, *J. Clin. Invest.* 93:776 (1994); Weg et al, *J. Exp. Med*. 177:561 (1993)). The cotton-top tamarin, which experiences spontaneous chronic colitis, showed a significant attenuation of their colitis when anti-α₄ antibody was administered (Podolsky et al, *J. Clin. Invest.* 92:372 (1993); Bell et al, *J. Immunol.* 151:4790 (1993)). In a rat and mouse model, autoimmune encephalomyelitis was blocked by anti-α₄ antibody (Yednock et al, *Nature* 356:63 (1992); Baron et al, *J. Exp. Med*. 177:57 (1993)). Anti-α₄ monoclonal antibodies also inhibit insulitis and delay the onset of diabetes in the non-obese diabetic mouse (Baron et al, *J. Clin. Invest.* 93:1700 (1994); Yang et al, Proc. *Natl. Acad.* Sci. USA 90:10494 (1993); Burkly et al, *Diabetes* 43:529 (1994)). α₄ is also implicated in atherosclerosis due to its endothelial expression during atherogenesis (Cybulsky et al, *Science* 251:788 (1991)). The migration of leukocytes to an inflammatory site can also be blocked by anti-α₄ antibodies. In addition to the blocking of migration, inhibitors of leukocyte endothelial adhesion may block the costimulatory signals mediated by integrins and thus inhibit overproduction of inflammatory cytokines. In a separate set of experiments not using anti-α₄ antibodies, the peptides GRDGSP or EILDV were tested against contact hypersensitivity response. The contact hypersensitivity response was found to be blocked by GRDGSP or EILDV suggesting that both α₄β₁ and α₅β₁ are involved in this inflammatory response.

Other ailments which may involve α₄β₁-mediated conditions include the inflammatory disorders rheumatoid arthritis, allergic disorders, asthma, spontaneous chronic colitis, insulitis, contact hypersensitivity response, atherosclerosis and autoimmune encephalomyelitis. These studies illustrate that small molecules that are potent inhibitors of α₄β₁ mediated adhesion to either VCAM-1 or CS-1 may be used as a form of treatment in numerous inflammatory diseases. However, these inflammatory conditions could be expanded to include adult respiratory distress syndrome, AIDS, cardiovascular diseases, thrombosis or harmful platelet aggregation, reocclusion following thrombolysis, allograft rejection, reperfusion injury, psoriasis, eczema, contact dermatitis and other skin inflammatory diseases, osteoporosis, osteoarthritis, atherosclerosis, neoplastic diseases including metastasis of neoplastic or cancerous growth, wound healing enhancement, treatment of certain eye diseases such as detaching retina, Type I diabetes, multiple sclerosis, systemic lupus erythematosus (SLE), inflammatory and immunoinflammatory conditions including ophthalmic inflammatory conditions and inflammatory bowel diseases, ulcerative colitis, regional enteritis and other autoimmune diseases. Accordingly, a compound which could inhibit these conditions is desirable.

### SUMMARY OF THE INVENTION

The present invention is directed to a compound of the following formula:

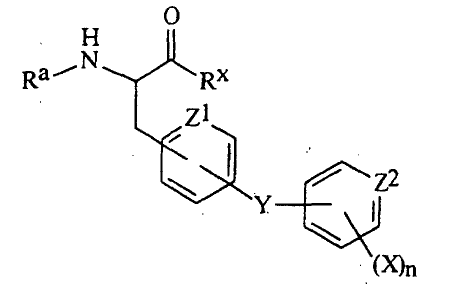

In the above formula, X is selected from the group consisting of halogen, CF₃, NO₂, OH, C₁₋₃ alkoxy, NH₂ and C₁₋₄ alkyl, both Z¹ and Z² are CH or N, n is 1, 2 or 3, Y is -OCH₂- or -NHC(=O)-, R^{x} is OH or C₁₋₆ alkoxy and R^{a} is selected from the group consisting of:

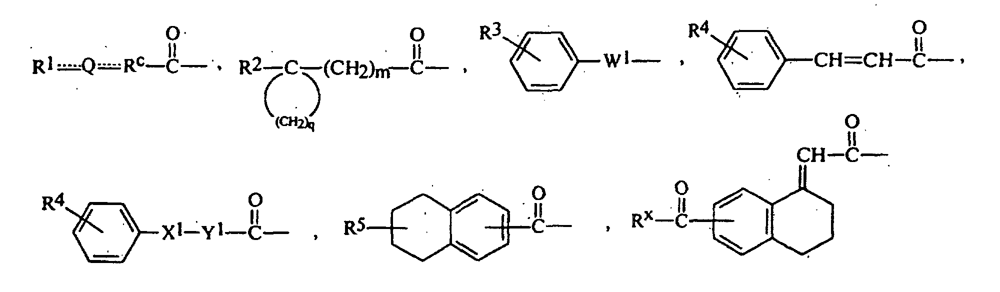

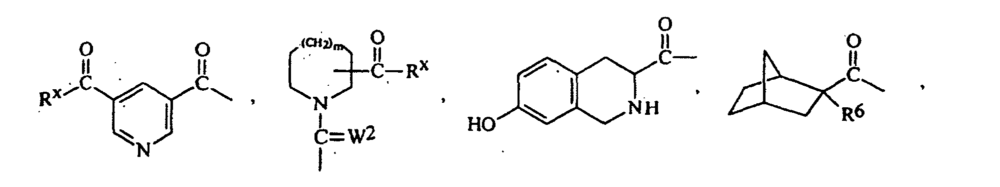

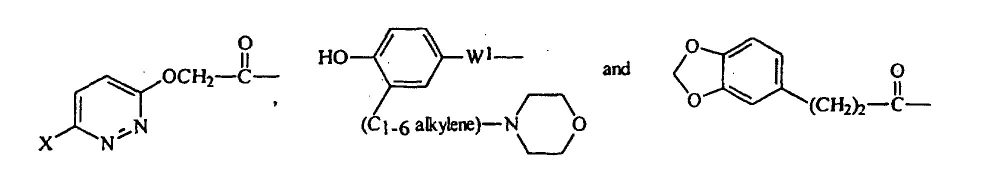

In the above formula, - - - - - represents a single or a double bond. Also, in the above formula, W¹ is selected from the group consisting of -CO-, -OCH₂CO-, -OCH(CH₃)CO-, -OC(CH₃)₂CO-, -SCH₂CO-, -SCH(CH₃)CO-, -SC(CH₃)₂CO-, -CH=CHCO-, -OCH(C₆H₅)CO- and -SCH(C₆H₅)CO-, W² is O or'S, q is 5 or 6, m is 0 or 1, X¹ is O, S or a bond, Y¹ is C₁₋₃ alkylene or -CH(C₆H₅), R^{c} is a bond, -CH₂- or =CH-, and Q is a ring of C₃₋₁₀ cycloalkane or C₃₋₁₀ cycloalkene, which ring is substituted by the group of R¹ and may further be substituted by 1 to 3 methyl groups with the proviso that Q is other than a ring of cyclopentane which is substituted by three methyl groups.

In the above formula, R¹ is selected from the group consisting of -H, -COR^{x}, -CH(OH)CH₃, -(C₂₋₇ alkenylene)COR^{x}, -NHCO(C₁₋₆ alkylene)COR^{x}, =CHCOR^{x}, -NHCO(C₁₋₆ alkylene)O(C₁₋₆ alkyl), -NHCO(C₂₋₇ alkenylene)COR^{x}, -NHCO(C₁₋₆ alkylene)CO(C₁₋₆ alkyl), -NHCONH(C₁₋₆ alkylene)COR^{x}, - C₂₋₇ alkanoyl, =CHCN, -(C₁₋₆ alkylene)COR^{x},

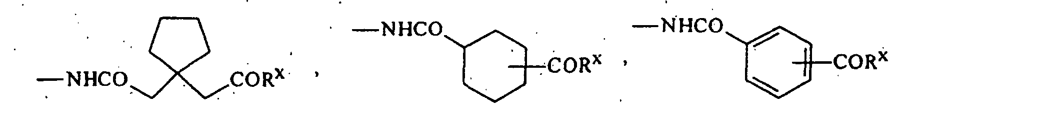

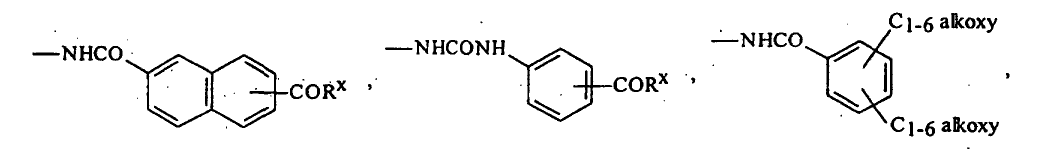

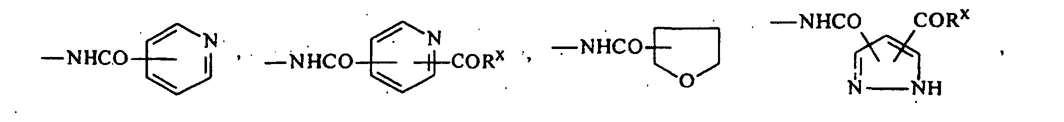

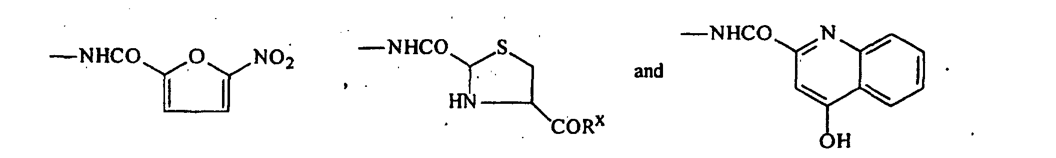

In the above formula, R² is selected from the group consisting of -CH₂COR^{x}, -NHCO(C₁₋₆ alkylene)COR^{x}, - NHCO(C₂₋₇ alkenylene)COR^{x}, -NHCO(C₁₋₆ alkylene)O(C₁₋₆ alkyl), -NHCONH(C₁₋₆ alkylene)COR^{x}, 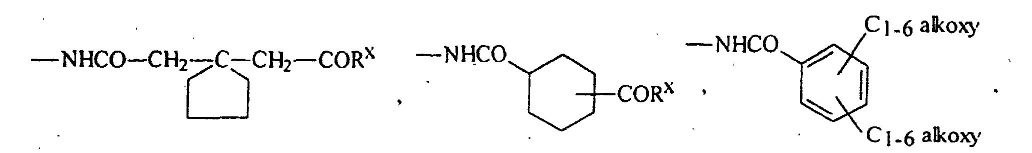 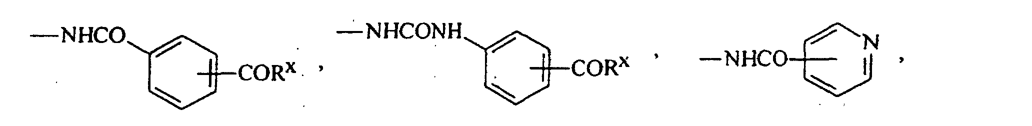 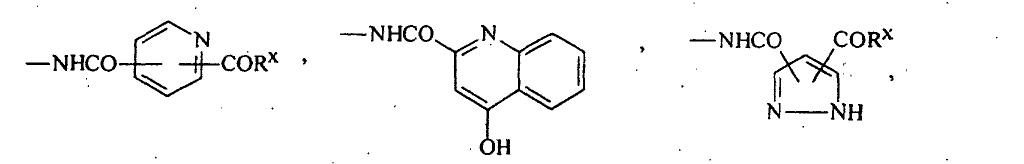 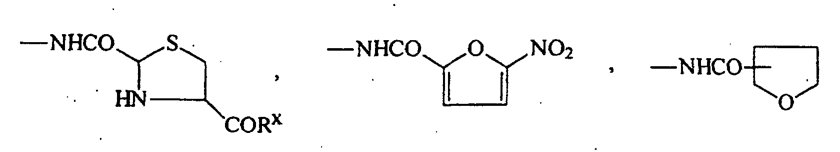 and

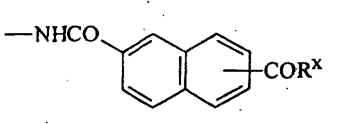

In the above formula, R³ is selected from the group consisting of -OH, -COR^{x}, -(C₂₋₇ alkenylene)COR^{x}, -NHCO(C₁₋₆ alkylene)COR^{x}, -NHCO(C₁₋₆ alkylene)O(C₁₋₆ alkyl), -NHCO(C₂₋₇ alkenylene)COR^{x}, -NHCO(C₁₋₆ alkylene)CO(C₁₋₆ alkyl), -NHCONH(C₁₋₆ alkylene)COR^{x}, -C₂₋₇ alkanoyl, 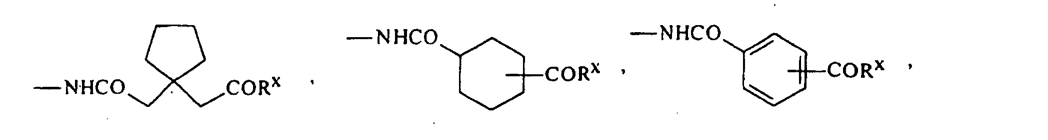 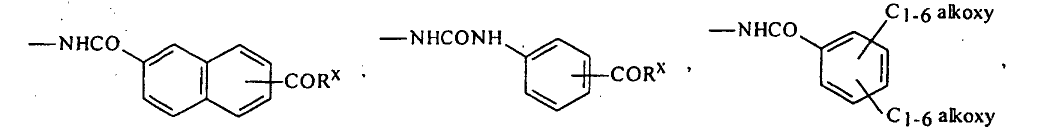 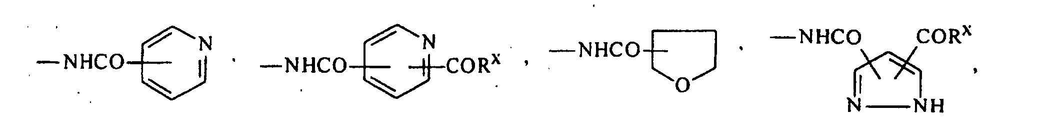 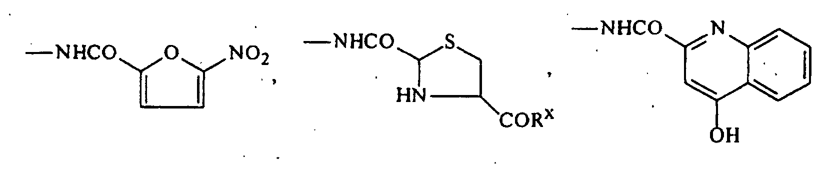 and

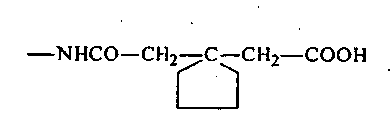

Lastly, in the above formula, R⁴ is selected from the group consisting of -COOH, -CH=CHCOOH and methylenedioxy, R⁵ is =CHCOR^{x} or =CHCN, and R⁶ is selected from the group consisting of

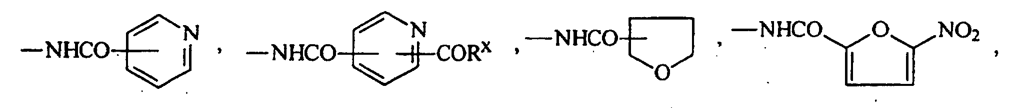

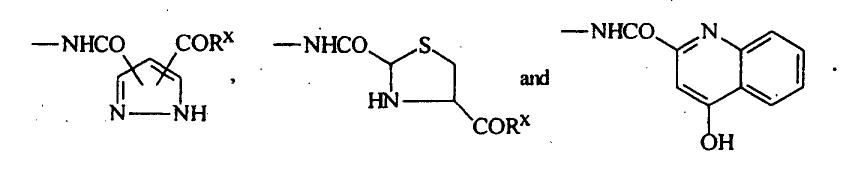

In another embodiment, the compound of the present invention is represented by the following formula:

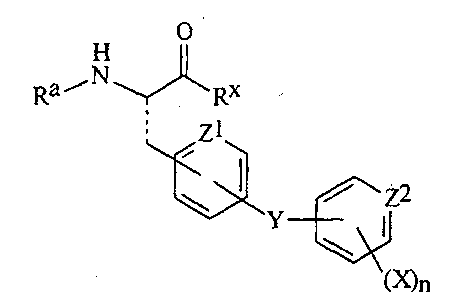

In a preferred embodiment of the present invention',' X is halogen, Y is -OCH₂- or -NHC(=O)-, both Z¹ and Z² are CH or N, R^{x} is OH or C₁₋₆ alkoxy, and R^{a} is selected from the group consisting of:

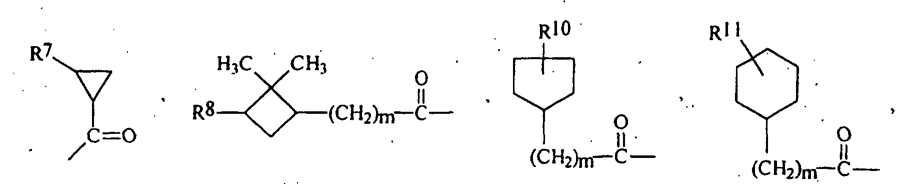

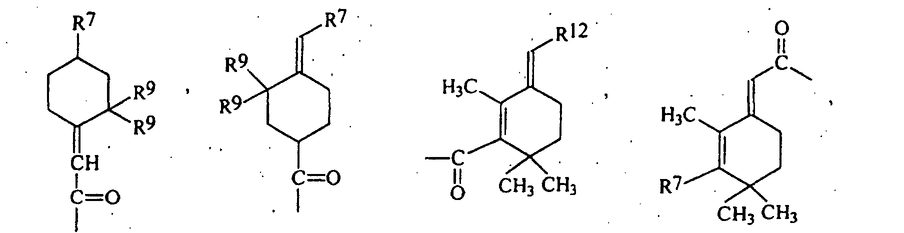

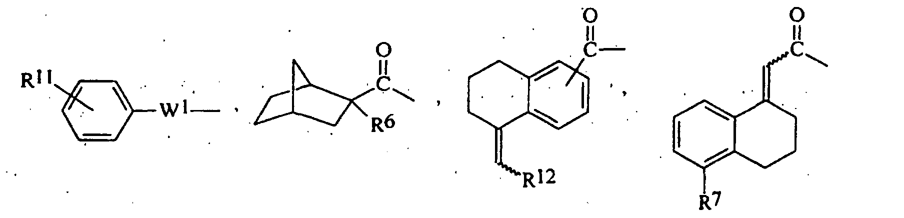

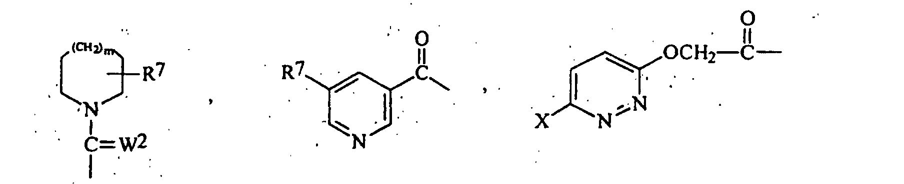

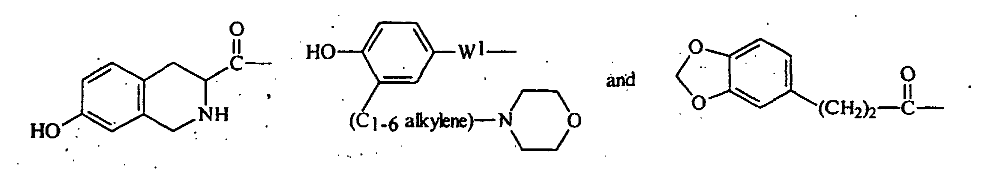

In the above preferred embodiment of the present invention, R⁶ is selected from the group consisting of:

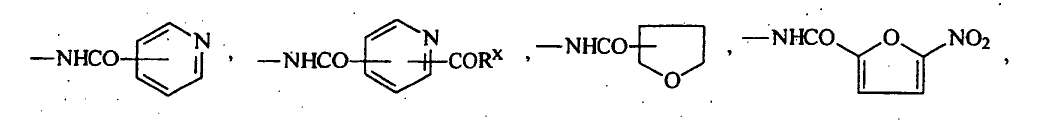

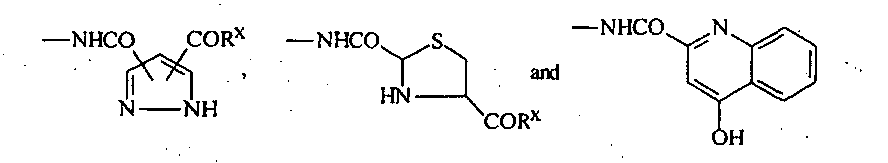

Also, in the preferred embodiment of the present invention, R⁷ is -COR^{x}, R⁸ is selected from the group consisting of hydrogen, -COR^{x}, -COCH₃ and -CH(OH)CH₃, R⁹ is hydrogen or methyl, R¹⁰ is -COR^{x} or -(C₁₋₆ alkylene)COR^{x}, and R¹¹ is selected from the group consisting of -COR^{x}, -(C₂₋₇ alkenylene)COR^{x}, -NHCO(C₁₋₆ alkylene)COR^{x}, -NHCO(C₁₋₆ alkylene)O(C₁₋₆ alkyl), -NHCO(C₂₋₇ alkenylene)COR^{x}, -NHCO(C₁₋₆ alkylene)CO(C₁₋₆ alkyl), -NHCONH(C₁₋₆ alkylene)COR^{x}, - C₂₋₇ alkanoyl, 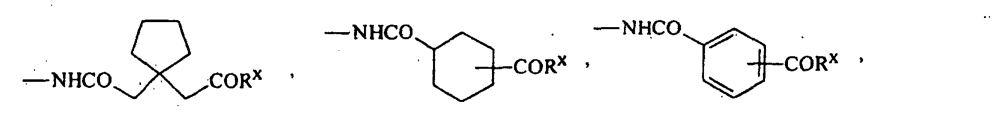 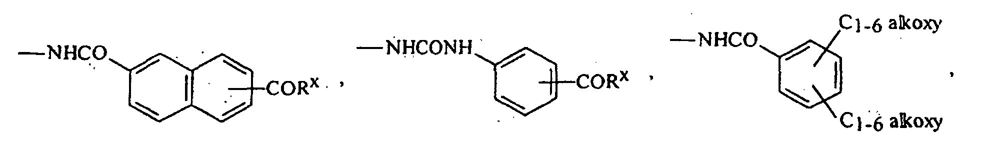 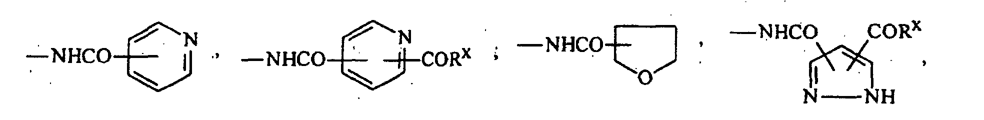 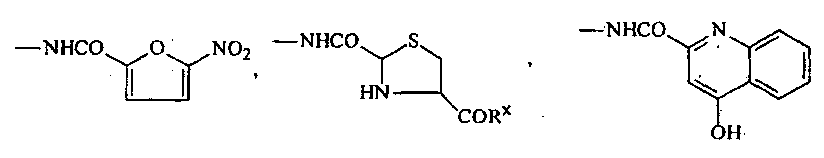 and

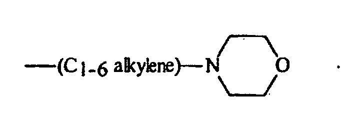

In the preferred embodiment of the present invention, R¹² is -COR^{x} or -CN, W¹ is selected from the group consisting of - CO-, -OCH₂CO-, -OCH(CH₃)CO-, -OC(CH₃)₂CO-, -SCH₂CO-, -SCH(CH₃)CO-, -SC(CH₃)₂CO-, -CH=CHCO-, -OCH(C₆H₅)CO- and - SCH(C₆H₅)CO-, W² is O or S, n is 1 or 2, and m is 0 or 1.

In another embodiment of the present invention, Y is - NHC(=O)-.

In another embodiment, the compound of the present invention is represented by the following formula:

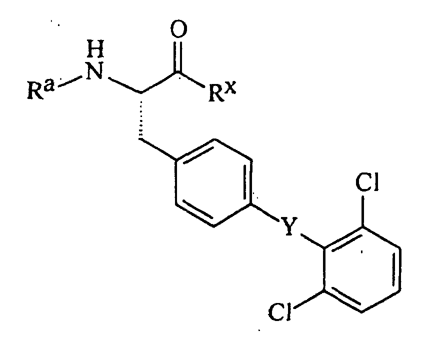

In another preferred embodiment of the present invention, R^{a} is selected from the group consisting of:

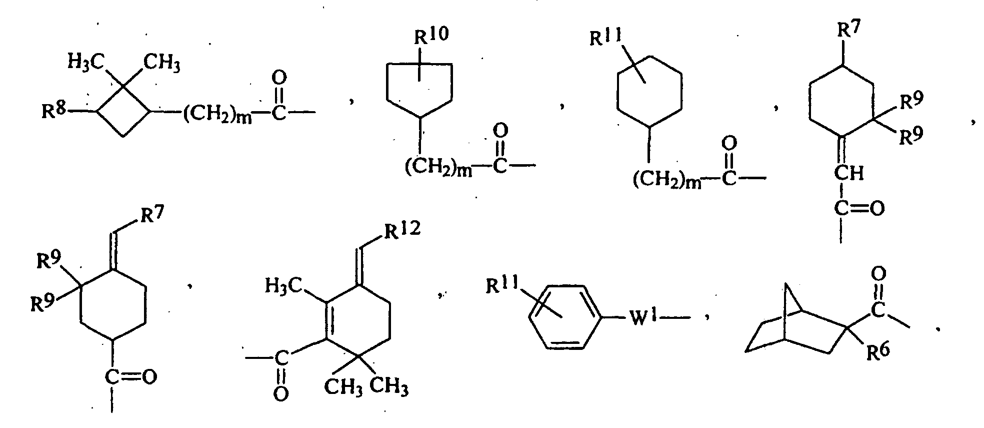

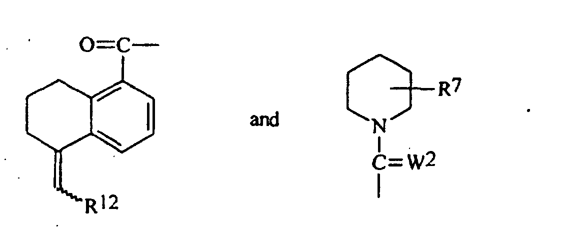

In another preferred embodiment of the present invention,
R^{a} is selected from the group consisting of: 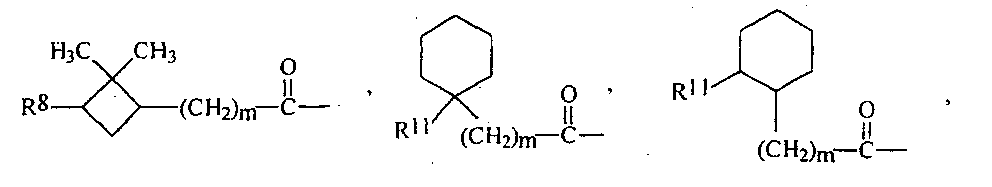 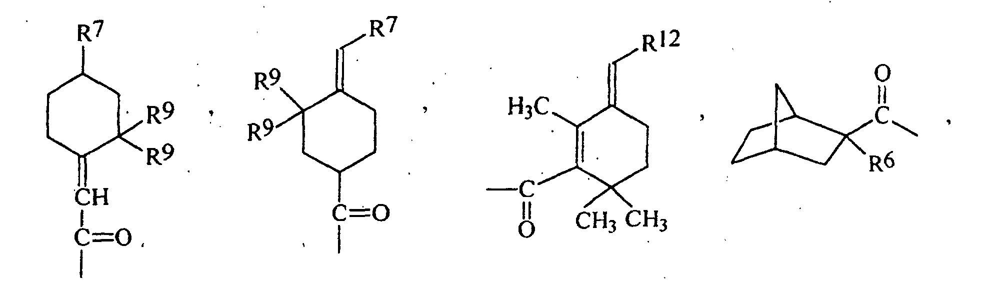 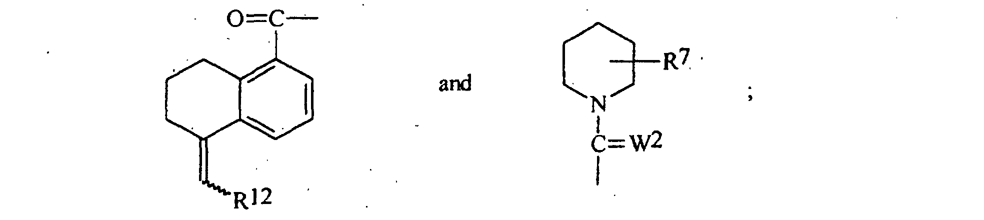 R⁶ is selected from the group consisting of: 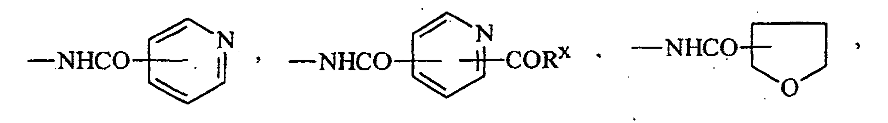 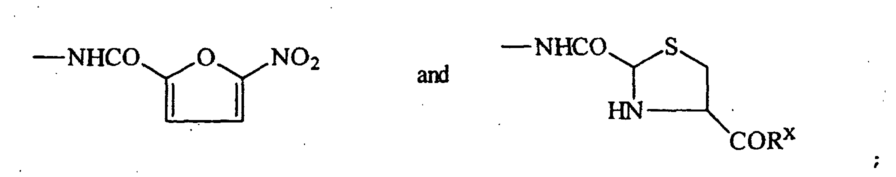 and
R¹¹ is selected from the group consisting of -NHCO(C₂₋₇ alkenylene)COR^{x}, -NHCO(C₁₋₆ alkylene)CO(C₁₋₆ alkyl),

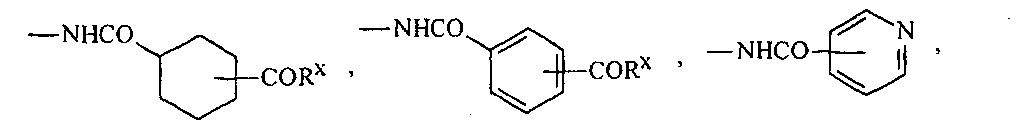

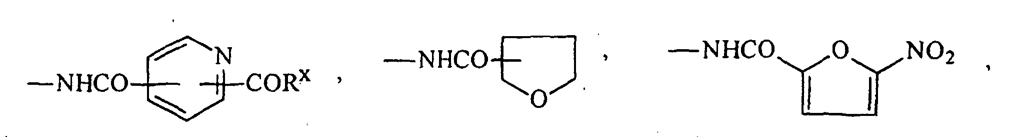

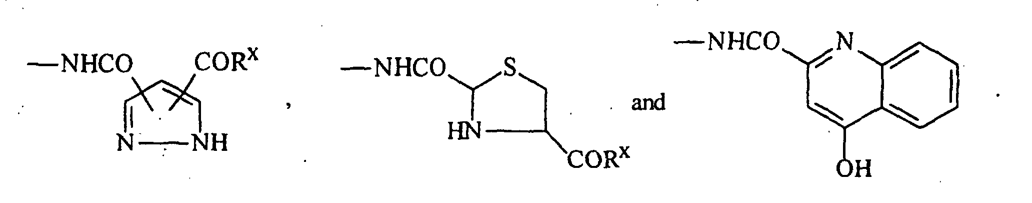

In a more preferred embodiment of the present invention, R^{a} is 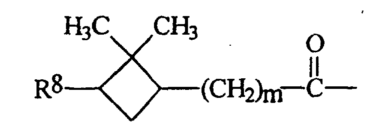 and R⁸ is COR^{x} or COCH₃.

In another more preferred embodiment of the present invention, R^{a} is 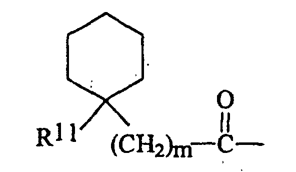 and R¹¹ is selected from the group consisting of -NHCO(C₁₋₆ alkylene)CO(C₁₋₆ alkyl), 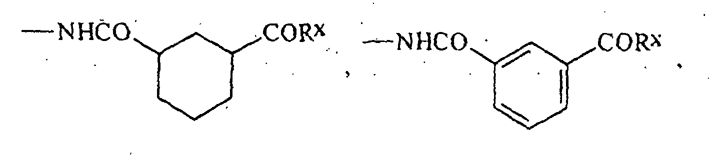 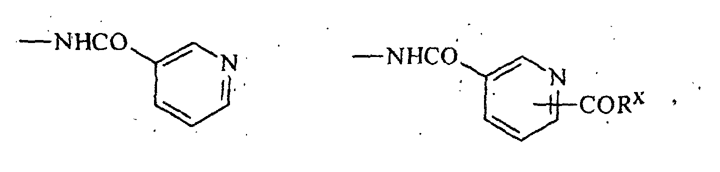 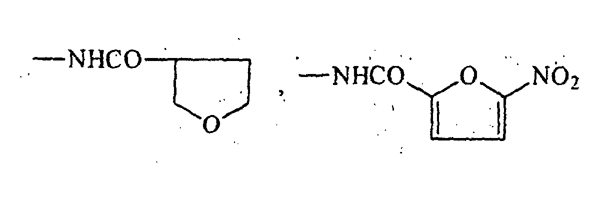 and

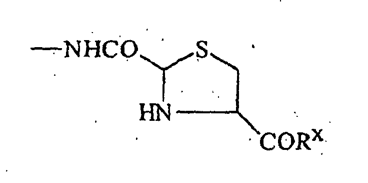

In another more preferred embodiment of the present invention, R^{a} is 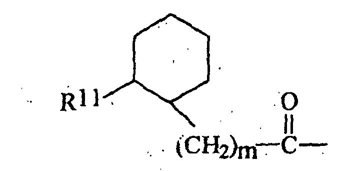 and R¹¹ is selected from the group consisting of

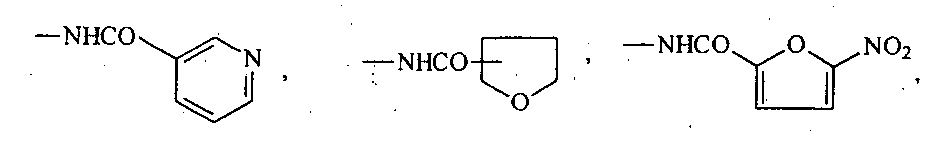

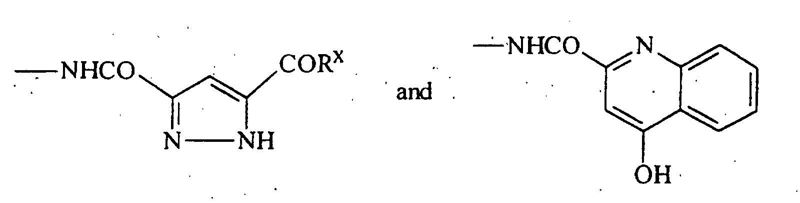

In another more preferred embodiment of the present invention, R^{a} is

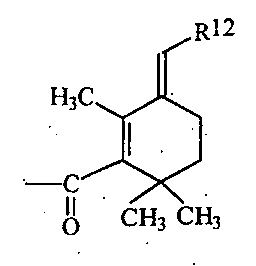

In another more preferred embodiment of the present invention, R^{a} is 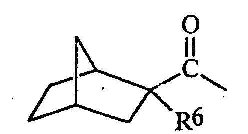 and R⁶ is selected from the group consisting of:

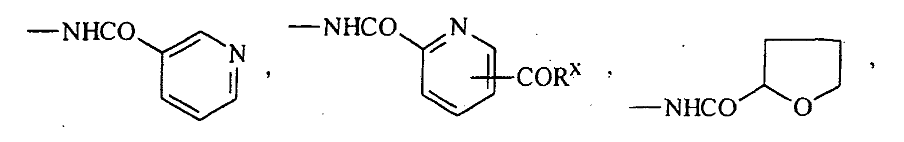

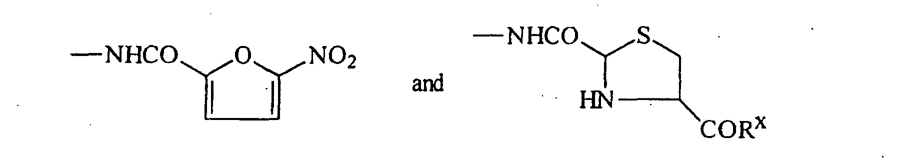

With respect to the present invention, alkyl, alkylene and alkoxy groups are intended to encompass both straight and branched chain moieties. For example, a C₁₋₆ alkyl group would encompass moieties including, but not limited to, methyl, ethyl, propyl, butyl, pentyl, hexyl, iso-propyl, and t-butyl.

The desired compounds of the present invention may exist in the form of optical isomers based on asymmetric carbon atoms thereof, and the present invention also includes these optical isomers and mixtures thereof.

In an embodiment of the present invention, the steric configuration of a bond need not be fixed. A bond may be of any acceptable configuration. Further, a compound may be a mixture with several different configurations of the same bond.

The desired compound of the present invention may be clinically used either in a free form or in the form of pharmaceutically acceptable salts thereof. Pharmaceutically acceptable salts include acid-addition salts with inorganic acid or organic acid (e.g., hydrochloride, sulfate, nitrate, hydrobromide, methanesulfonate, p-toluenesulfonate, acetate), salt with inorganic base, organic base or amino acid (e.g., triethylamine salt, a salt with lysine, an alkali metal salt, an alkali earth metal salt and the like).

The compound may also be formulated into a pharmaceutical composition comprising a therapeutically effective amount of the compound as defined above and a pharmaceutically acceptable carrier or diluent.

The compound can also be used for the preparation of a pharmaceutical composition for treating or preventing α₄β₁ adhesion mediated conditions in a mammal such as a human. This method may comprise administering to a mammal or a human patient an effective amount of the compound or composition as explained above.

This pharmaceutical composition can be used to treat such inflammatory conditions as rheumatoid arthritis, asthma, allergy conditions, adult respiratory distress syndrome, AIDS, cardiovascular diseases, thrombosis or harmful platelet aggregation, reocclusion following thrombolysis, allograft rejection, reperfusion injury, psoriasis, eczema, contact dermatitis and other skin inflammatory diseases, osteoporosis, osteoarthritis, atherosclerosis, neoplastic diseases including metastasis of neoplastic or cancerous growth, 'wound healing enhancement, treatment of certain eye diseases such as detaching retina, Type I diabetes, multiple sclerosis, systemic lupus erythematosus (SLE), inflammatory and immunoinflammatory conditions including' ophthalmic inflammatory conditions and inflammatory bowel diseases, ulcerative colitis, atherosclerosis, regional enteritis and other autoimmune diseases.

The desired compound of the present invention or pharmaceutically acceptable salts thereof may be administered either orally or parenterally, and it may be used as a suitable pharmaceutical preparation, for example, a tablet, a granule, a capsule, a powder, an injection, and an inhalation by a conventional process.

The dose of the desired compound of the present invention or a pharmaceutically acceptable salt thereof varies depending on an administration method, age, body weight, and state of a patient, but, in general, the daily dose is preferably about 0.1 to 100 mg/kg/day, particularly preferably 1 to 100 mg/kg/day.

### Preferred routes of administration for asthma:

It is preferred that the compound of the present invention be administered in the form of an Aerosol. However, other routes of administration include intravenous, oral, intramuscular, and subcutaneous.

In the case of aerosol administration, compositions containing the compounds of the present invention can be prepared to provide for an excellent means for administering in aerosol form for inhalation therapy. Accordingly, the present invention will provide for self-propelling compositions containing the compounds of the present invention.

Propellants employed should be non-toxic and have a vapor pressure suitable for the conditions under which administration occurs. These propellants can be fluorinated or fluorochlorinated lower saturated aliphatic hydrocarbons. The preferred propellants of this type are the halogenated alkanes containing not more than two carbon atoms and at least one fluorine atom. Illustrative of these are trichloromonofluoromethane, dichlorodifluoromethane, monochlorotrifluoromethane, dichloromonofluoromethane and 1,2-dichloro-1,1,2,2-tetrafluoroethane. These compounds are available from E.I, duPont de Nemours and Company under the trade name "Freon". These propellants may be employed singularly or in admixture.

In addition to the propellant, an organic solvent may also be employed. The organic solvent must be non-toxic and without undesirable effects on inhalation in the amount present in the aerosol produced. In addition, the solvent should be substantially anhydrous, completely miscible with the propellant or mixture of propellants employed and have a suitable boiling point. Examples of such solvents included non-toxic aliphatic alcohols such as ethanol; ethers such as ethyl ether and vinyl ether; ketones such as acetone; and suitable halogenated lower alkanes.

In addition to the organic solvent, the composition may also optionally contain a non-toxic hygroscopic glycol. The glycol must be substantially miscible with the organic solvent and the propellant employed. Satisfactory glycols include propylene glycol, triethylene glycol, glycerol, butylene glycol and hexylene glycol.

'The above indicated methods of administration and formulation of aerosol compositions should not be viewed as limiting. The compounds of the present invention can be formulated in anyway deemed suitable to one of ordinary skill in the art so as to obtain the desired effects.

### Pharmaceutical Compositions

As indicated previously, the compounds of formula (I) can be formulated into pharmaceutical compositions. In determining when a compound of formula (I) is indicated for the treatment of a given disease, the particular disease in question, its severity, as well as the age, sex, weight, and condition of the subject to be treated, must be taken into consideration and this perusal is to be determined by the skill of the attendant physician.

For medical use, the amount of a compound' of Formula (I) required to achieve a therapeutic 'effect will, of course, vary both with the particular compound, the route of administration, the patient under treatment, and the particular disorder or disease being treated. A suitable daily dose of a compound of Formula (I), or a pharmaceutically acceptable salt thereof, for a mammalian subject suffering from, or likely to suffer from, any condition as described hereinbefore is 0.1 mg to 100 mg of the compound of formula I, per kilogram body weight of the mammalian subject. In the case of systematic administration, the dose may be in the range of 0.5 to 500 mg of the compound per kilogram body weight, the most preferred dosage being 0.5 to 50 mg/kg of mammal body weight administered two to three times daily. In the case of topical administration, e.g., to the skin or eye, a suitable dose may be in the range of 0.1 µg to 100 µg of the compound per kilogram, typically about 0.1 µg/kg. In the case of oral dosing, a suitable dose of a compound of Formula (I), or a physiologically acceptable salt thereof, may be as specified in the preceding paragraph, but most preferably is from 1 mg to 10 mg of the compound per kilogram, the most preferred dosage being from 1 mg to 5 mg/kg of mammal body weight, for example, from 1 to 2 mg/kg. Most preferably, a unit dosage of an orally administrable composition encompassed by the present invention contains less than about 1.0 g of a formula (I) compound.

It is understood that formulation, both for human and veterinary use, of the present invention may be presented to the mammal by inhalation. To achieve therapeutic effect, the dose may be in the range of 0.5 to 500 mg of the compound, per kg body weight. The most preferred dosage being 0.5 to 50 mg/kg of mammal body weight administered two to three times daily.

It is understood that the ordinarily skilled physician or veterinarian will readily determine and prescribe the effective amount of a compound of Formula I to prevent or arrest the progress of the condition for which treatment is administered. In so proceeding, the physician or veterinarian could employ relatively low doses at first, subsequently increasing the dose until a maximum response is obtained.

The compounds and compositions of the present invention can be administered to patients suffering from a condition listed herein in an amount which is effective to fully or partially alleviate undesired symptoms of the condition. The symptoms may be caused by inappropriate cell adhesion mediated by α₄β₁ integrins. Such inappropriate cell adhesion would typically be expected to occur as a result of increased VCAM-1 and/or CS-1 expression on the surface of endothelial cells. Increased VCAM-1 and/or CS-1 expression can be due to a normal inflammation response or due to abnormal inflammatory states. In either case, an effective dose of a compound of the invention may reduce the increased cell adhesion due to increased VCAM-1 expression by endothelial cells. Reducing the adhesion observed in the disease state by 50% can be considered an effective reduction in adhesion. More preferably, a reduction in adhesion by 90%, is achieved. Most preferably adhesion mediated by VCAM-1/α₄β₁ and/or CS-1 interaction is abolished by an effective dose. Clinically, in some instances, effect of the compound can be observed or a decrease in white cell infiltration into tissues or a site of injury. To achieve a therapeutic effect, then, the compounds or compositions of the present invention are administered to provide a dose effective to reduce or eliminate inappropriate cell adhesion or to alleviate undesired symptoms.

While it is possible for an active ingredient to be administered alone, it is preferable to present it as a pharmaceutical formulation comprising a compound of Formula (I) and a pharmaceutically acceptable carrier thereof. Such formulations constitute a further feature of the present invention.

The formulations, both for human and veterinary medical use, of the present invention comprise an active ingredient of Formula (I), in association with a pharmaceutically acceptable carrier thereof and optionally other therapeutic ingredient(s) which are generally known to be effective in treating the disease or condition encountered. The carrier(s) must be "acceptable" in the sense of being compatible with the other ingredients of the formulations and not deleterious to the recipient thereof.

The formulations include those in a form suitable for oral, pulmonary, ophthalmic, rectal, parenteral (including subcutaneous, intramuscular, and intravenous), intra-articular, topical, nasal inhalation (e.g., with an aerosol) or buccal administration. Such formulation are understood to include long-acting formulations known in the art.

The formulations may conveniently be presented in unit dosage form and may be prepared by any of the methods well known in the art of pharmacy. All methods may include the step of bringing the active ingredient into association with the carrier which constitutes one or more accessory ingredients.
In general, the formulations are prepared by uniformly and intimately bringing the active ingredient into association with a liquid carrier or a finely divided solid carrier or both, and then, if necessary, shaping the product into the desired form.

Formulations of the present invention suitable for oral administration may be in the form of discrete units such as capsules, cachets, tablets, or lozenges, each containing a predetermined amount of the active ingredient'in the form of a powder or granules; in the form of a solution or suspension in an aqueous liquid. Formulations for other uses could involve a nonaqueous liquid; in the form of an oil-in-water emulsion or a water-in-oil emulsion; in the form of an aerosol; or in the form of a cream or ointment or impregnated into a transdermal patch for use in administering the active ingredient transdermally, to a patient in need thereof. The active ingredient of the present inventive compositions may also be administered to a patient in need thereof in the form of a bolus, electuary, or paste.

The practitioner is referred to "Remington: The Science and Practice of Pharmacy," 19th Edition, c. 1995 by the Philadelphia College of Pharmacy and Science, as a comprehensive tome on pharmaceutical preparations.

### Abbreviations

- DMF:: Dimethylformamide
- TFA:: Trifluoroacetic Acid
- HBTU:: O-Benzotriazol-1-yl-N,N,N',N'-tetramethyluronium hexahydrophosphate
- HOBT:: 1-Hydroxybenzotriazole
- DIEA:: Diisopropylethylamine
- THF:: Tetrahydrofuran
- BOC:: tert-Butoxycarbonyl

### Examples

The compounds of Examples 1-87 were prepared by the following Methods A, B, C, D, E, F, G, H, or I and could be purified by conventional chromatography on silica gel using a proper eluent such as CHCl₃, AcOEt, etc.

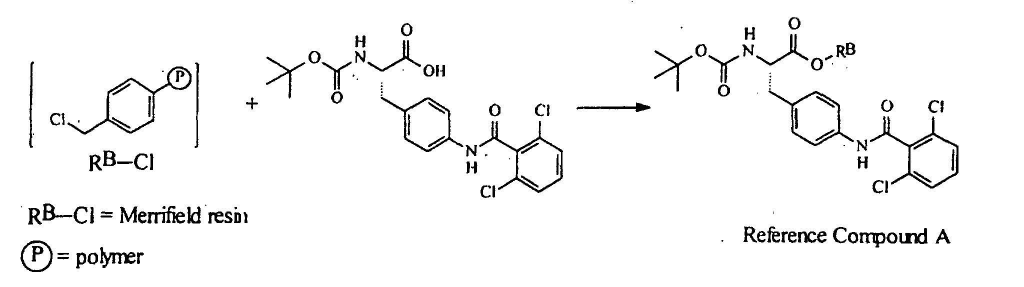

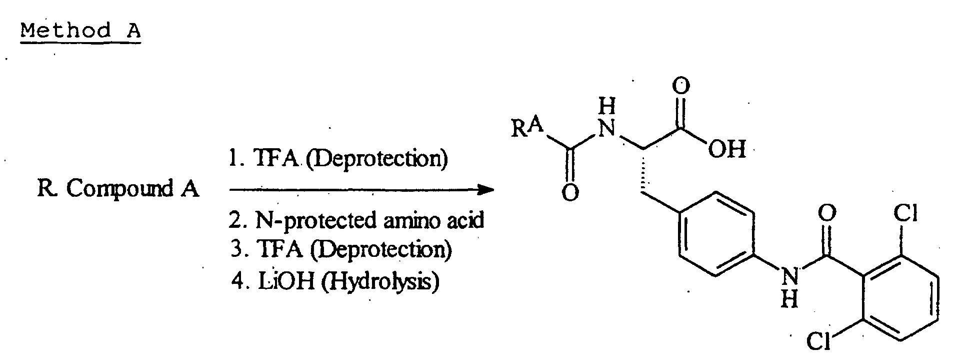

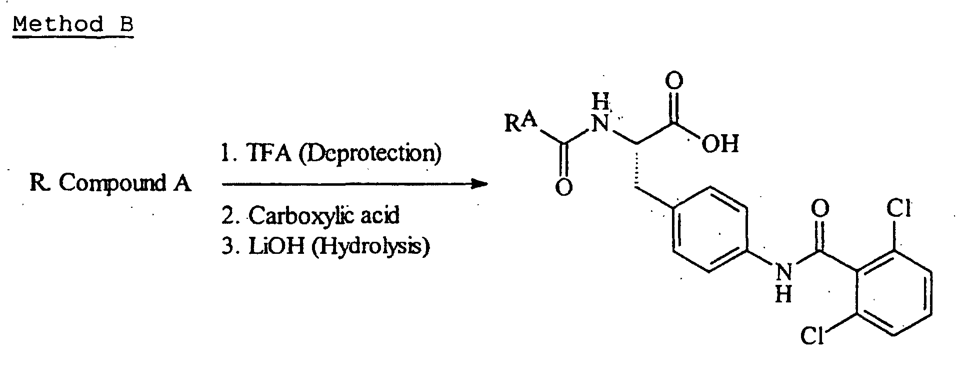

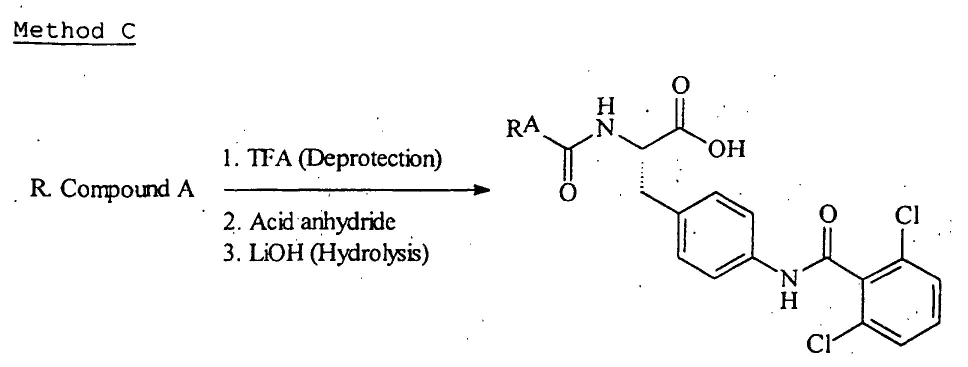

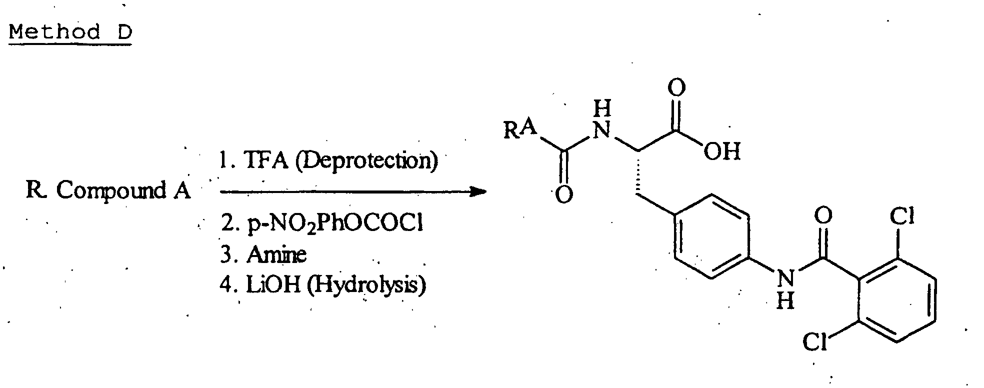

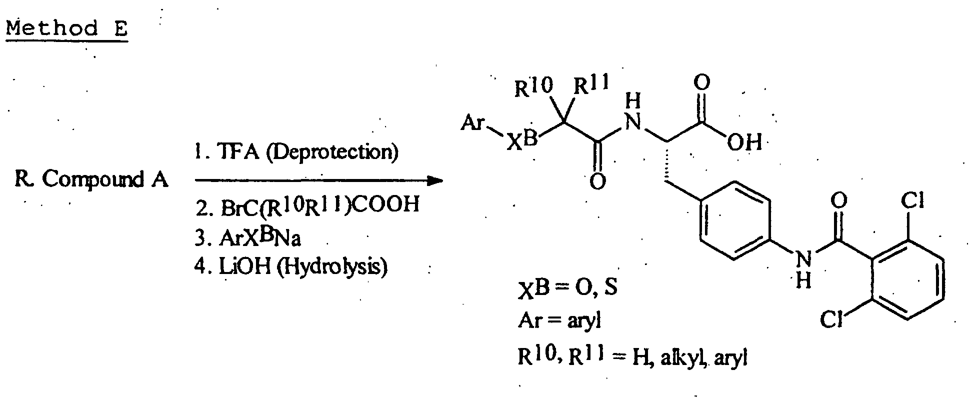

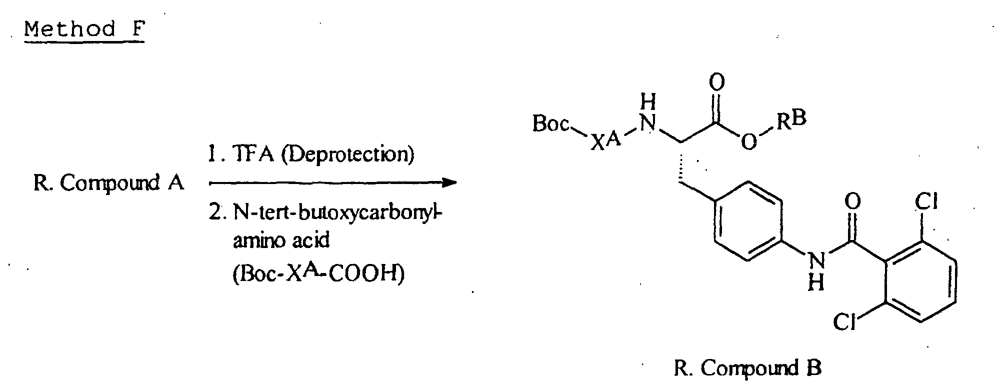

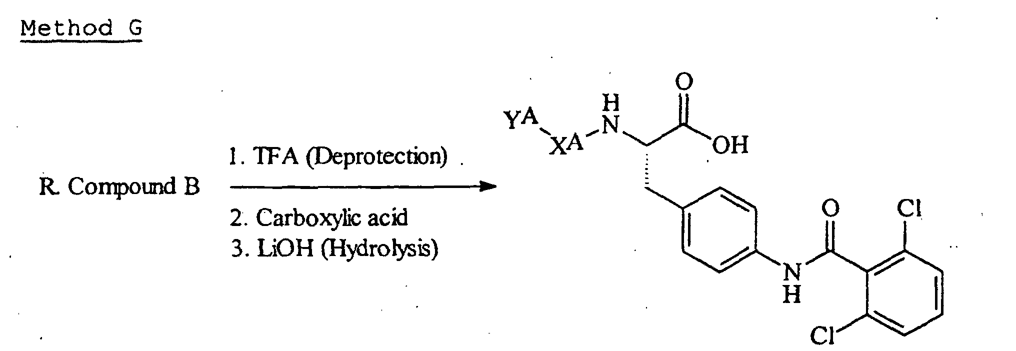

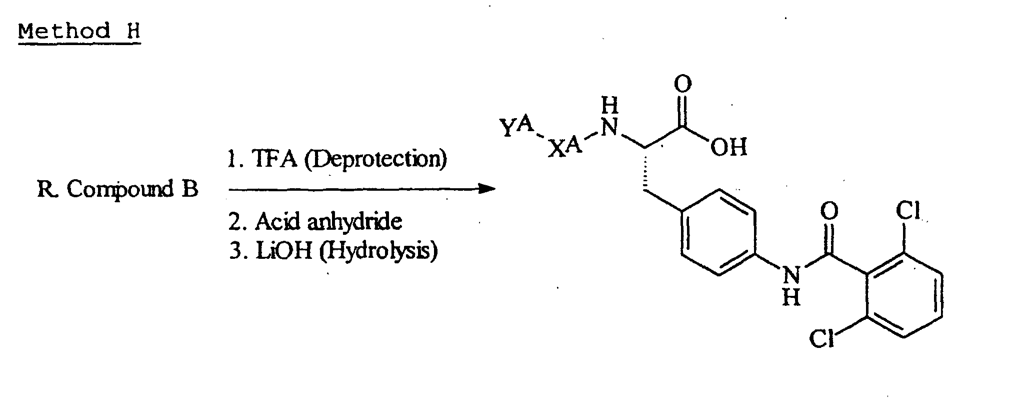

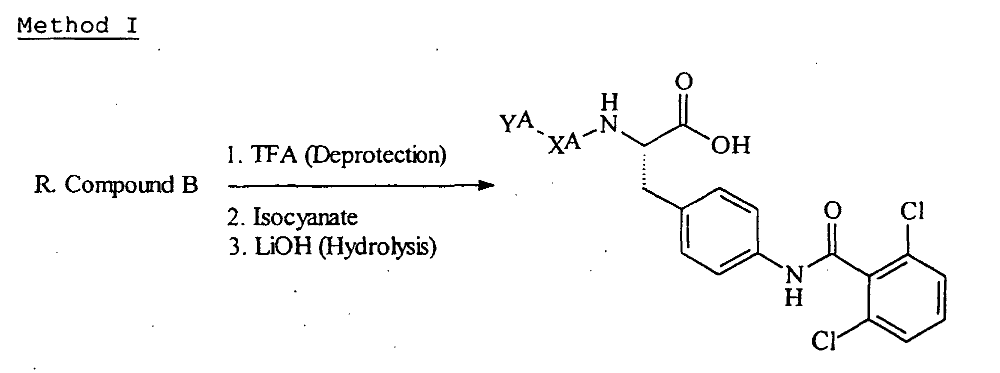

### Example 1: 4-[(2,6-Dichlorobenzoyl)amino]-N-[[(3S)-7-hydroxy-1,2,3,4-tetrahydro-3-isoquinolyl]carbonyl]-L-phenylalanine

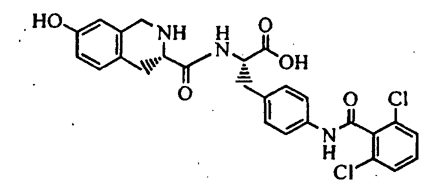

The above-titled compound was prepared by Method A (R^{A}: (3S)-2-(tert-butoxycarbonyl)-7-hydroxy-1,2,3,4-tetrahydro-3-isoquinolyl). **Reference Compound A** (300 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL) , CH₂Cl₂ (2 x 3 mL). The resin was swollen with DMF (2 x 3 mL). (3S)-2-(tert-butoxycarbonyl)-7-hydroxy-1,2,3,4-tetrahydro-3-isoquinolinecarboxylic acid (258 mg) in DMF (1.0 mL) was activated with 0.5 M HBTU/HOBT in DMF (1.6 mL) and DIEA (0.34 mL), and then added to the swollen resin. The mixture was stirred for 2 hr at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test (Kaiser et al., *Anal. Biochem.* **1970**, *34*, 594-598) on a small quantity of the resin is positive (blue) then repeat the coupling procedure until a negative result is obtained. After pretreating the resin with CH₂Cl₂ (2 x 3 mL), the swollen resin was then deprotected with 50% TFA/CH₂Cl₂ (3mL, 30min) to remove tert-butoxycarbonyl group. The resin was rinsed in the following order: CH₂Cl₂ (2 x 3 mL), CH₃OH (2 x 3 mL), CH₂Cl₂ (2 x 3 mL). The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 1: ESMS (m/z) 526 (M-H)⁻.

### Example 2: N-[(5-Carboxy-3-pyridinyl)carbonyl]-4-[(2,6-dichlorobenzoyl)amino]-L-phenylalanine

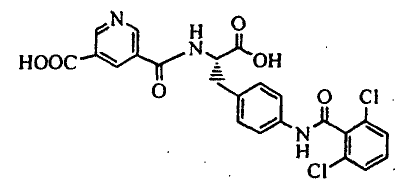

The above titled compound was prepared by Method B (R^{A} : 5-carboxy-3-piridinyl). **R. Compound A** (200 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). The resin was swollen with DMF (2 x 3 mL). 3,5-Pyridine dicarboxylic acid (138 mg) in DMF (1.0 mL) was activated with 0.5 M HBTU/HOBT in DMF (1.6 mL) and DIEA (0.34 mL). The preactivated 3,5-pyridine dicarboxylic acid was then added to the swollen resin and the mixture was stirred for 2 hr at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant.weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 2: ESMS (m/z) 501 (M-H)⁻.

### Examples 3, 4

The following compounds were prepared in a manner similar to the compound of Example 2:

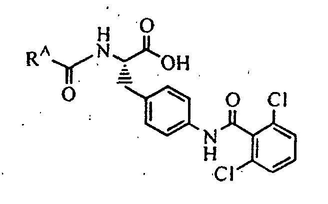

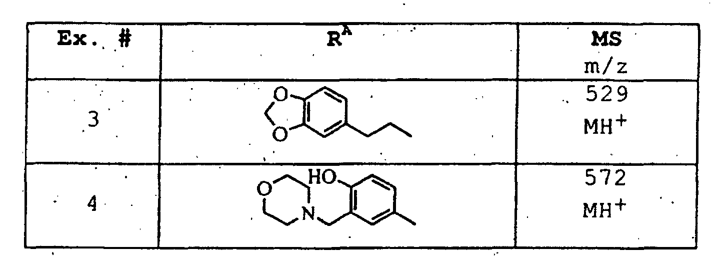

### Example 5: N-(2-Carboxybenzoyl)-4-[(2,6-dichlorobenzoyl)-amino]-L-phenylalanine

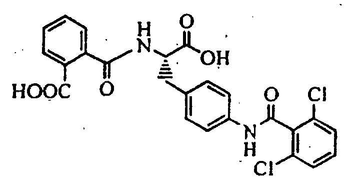

The above titled compound was prepared by the Method C (R^{A}: 2-carboxyphenyl). **R. Compound A** (150 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). Phthalic anhydride (80 mg, 0.54 mmol) in DMF (2.0 mL)/DIEA (0.15 mL) was added. The mixture was stirred for 3 days at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 5: ESMS (m/z) 499 (M-H)⁻.

### Examples 6-9

The compounds of the following Examples were prepared in the same way as the compound of Example 5:

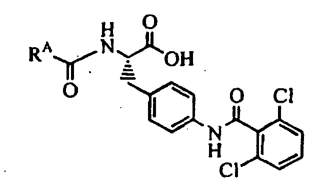

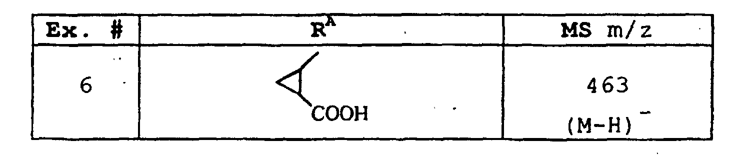

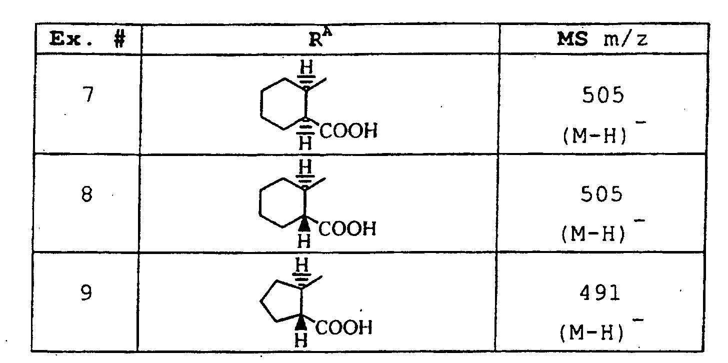

### Example 10: N-[[(2S)-2-Carboxy-1-pyrrolidinyl]carbonyl]-4-[(2,6-dichlorobenzoyl)amino]-L-phenylalanine

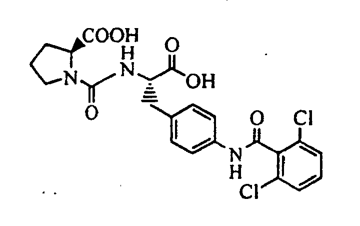

The above titled compound was prepared by the Method D (R^{A}: (2S)-2-carboxy-1-pyrrolodinyl). **R. Compound A** (100 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). The resin was then treated with *p*-nitrophenylchloroformate (130 mg) in THF/CH₂Cl₂ (1.5 mL) and DIEA (100 µL). This was shaken for 2 hr at room temperature. The resin was rinsed in the following order: CH₂Cl₂ (2 x 3 mL), CH₃OH (2 x 3 mL), CH₂Cl₂ (2 x 3 mL). The resulting resin was then treated with L-proline methyl ester (60 mg) in DMF (2 mL) and Et₃N (100 µL) and the mixture was stirred for 3 days at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 10: ESMS (m/z) 492 (M-H)⁻.

### Example 11. N-[[(2S)-2-Carboxy-1-piperidyl]carbonyl]-4-[2,6-dichlorobenzoyl)amino]-L-phenylalanine

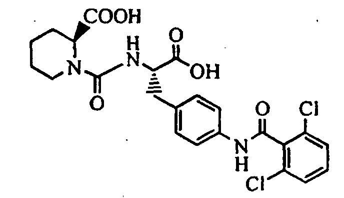

The above titled compound was prepared in a similar way as the compound of Example 10. ESMS(m/z): 506(M-H)⁻.

### Example 12: N-[(4-carboxyphenoxy)acetyl]-4-[(2,6-dichlorobenzoyl)amino]-L-phenylalanine

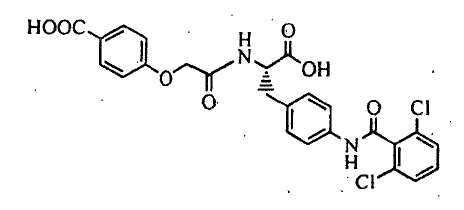

The above titled compound was prepared by the Method E (ArX^{B}C(R¹⁰R¹¹): (4-carboxyphenoxy)methyl). **R. Compound A** (100 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). Bromoacetic acid (85 mg) and diisopropyl-carbodiimide (80 mg) in DMF (2.0 mL) was added and the mixture was stirred for 1 hr at room temperature. The resin was filtered and washed in the following order: DMF (2 x 3 mL), CH₂Cl₂ (2 x 3 mL), DMF (2 x 3 mL), respectively. If a Kaiser test on a small quantity of the resin is positive (blue) then 'repeat the' coupling procedure until a negative result is obtained. Methyl *p*-hydroxybenzoate (91 mg) was pretreated with NaH (20 mg) in THF (0.5 mL) and DMF (2 mL) for 1.5 hr. The resulting phenoxide was added to the resin and the mixture was stirred for 2 hr at room temperature. The resin was then filtered and washed in the following order: DMF (2 x 3 mL), CH₂Cl₂ (2 x 3 mL), THF (2 x 3 mL), respectively. The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 12: ESMS (m/z) 529 (M-H)⁻.

### Examples 13-30:

The compounds of the following Examples were prepared in' a similar way as Example 12:

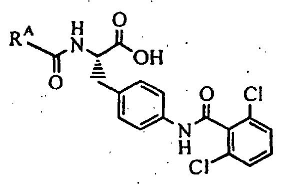

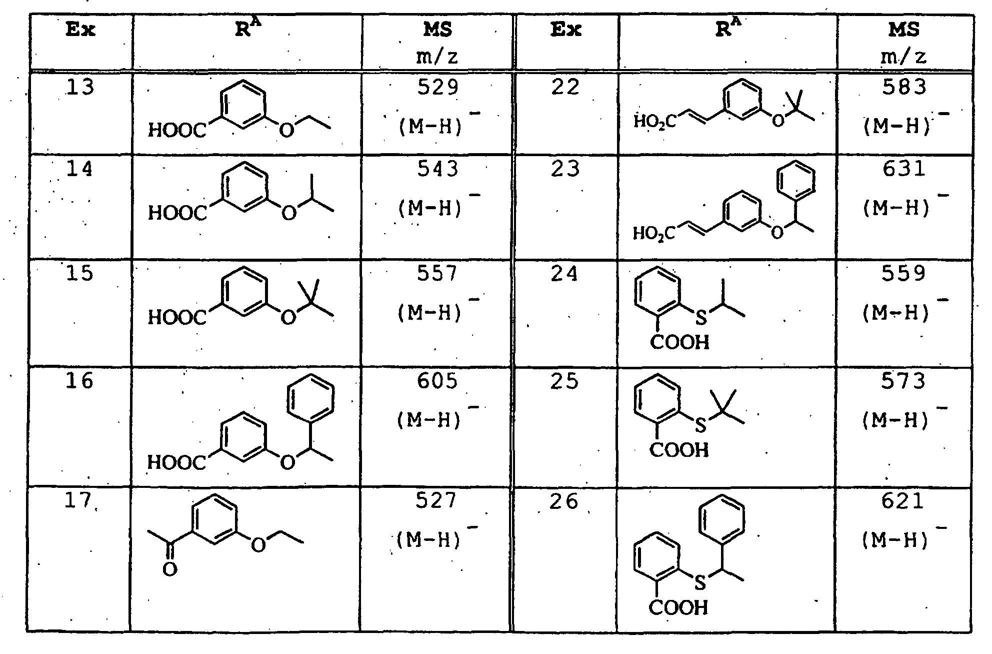

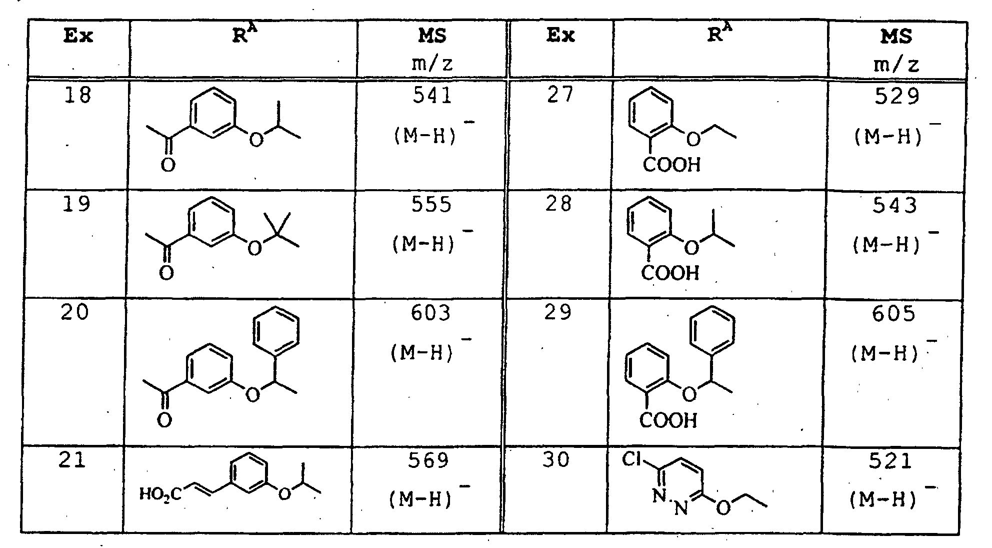

### Example 31: 4-[(2,6-Dichlorobenzoyl)amino]-N-[3-[N-(3-methoxy-1-oxopropyl)amino]benzoyl]-L-phenylalanine

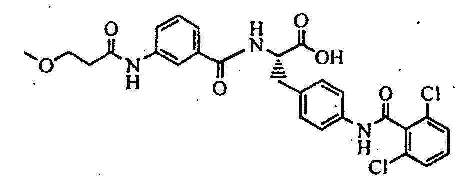

The above titled compound was prepared by the Method G (Y^{A}-X^{A}: 3-[N-(3-methoxy-1-oxopropyl)amino]benzoyl). **R. Compound B-1** (0.2 g) was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). The resin was swollen with DMF (2 x 3 mL). 3-methoxypropionic acid (81 mg) in DMF (1.0 mL) was activated with 0.5 M HBTU/HOBT in DMF (1.6 mL) and DIEA (0.34 mL). The preactivated 3-methoxy propionic acid was then added to the swollen resin and the mixture was stirred for 2 hr at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then THF (4 mL), CH₃OH (1.0 mL) and 2N LiOH (0.390 mL) were added. The mixture was stirred for 15 min and filtered to a clean and pre weighed test tube. The resin was next washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with water (2 x 5 mL) and dried *in vacuum* to furnish the compound of Example 31: ESMS (m/z) 556 (M-H)⁻.

### Example 32: N-[3-[N-[[(1-Carboxymethyl)cyclopentyl]acetyl]amino]benzoyl]-4-[(2,6-dichlorobenzoyl)amino]-L-phenylalanine

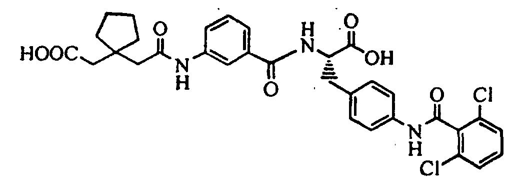

The above titled compound was prepared by the Method H (Y^{A}-X^{A}:(3-[N-[[(1-carboxymethyl)cyclopentyl]acetyl]amino]-benzoyl). **R. Compound B-1** (0.2 g) was pretreated with CH₂Cl₂ (2 x 3 mL). The swollen resin was then deprotected with 50% TFA/CH₂Cl₂ (3mL. 30min). The resin was rinsed in the following order: CH₂Cl₂ (2 x 3 mL), CH₃OH (2 x 3 mL), CH₂Cl₂ (2 x 3 mL). The resin was then swollen with DMF (3mL). 3,3-Tetramethyleneglutaric anhydride (130 mg) dissolved in DMF (4 mL) was added to the swollen resin and stirred at 50 °C for 2hr. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant weight. The cleavage of the resin (saponification) was achieved with LiOH/CH₃OH/THF (1:6:20). The resin bound material was placed in the polypropylene column and pretreated with THF (3 mL). Then to the swollen resin THF (3.9 mL), CH₃OH (1.0 mL) and 2N LiOH (0.39 mL) were added. The mixture was stirred for 15 min and filtered to a clean and pre weighed test tube. The resin was next washed with THF/5% CH₃OH (2 x 2 mL) and the combined filtrates were evaporated. The resulting gum was dissolved in H₂O (1 mL). The solution was then acidified with 1N HCl to pH 2.0. The precipitate was centrifuged, washed with H₂O (2 x 5 mL) and dried *in vacuum* to furnish the compound of Example 32: ESMS (m/z) 638 (M-H)⁻.

### Example 33: N-[3-[(N'-Carboxymethyl)ureido]benzoyl]-4-[(2,6-dichlorobenzoyl)amino]-L-phenylalanine

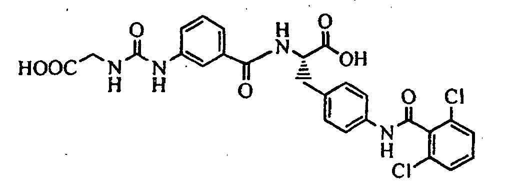

The above titled compound was prepared by the Method I (Y^{A}-X^{A}: 3-[N'-(carboxymethyl)ureido]benzoyl). R. **Compound B-1** was pretreated with CH₂Cl₂ (2 x 3 mL). The swollen resin was then deprotected with 50% TFA/CH₂Cl₂ (3mL, 30min). The resin was rinsed in the following order: CH₂Cl₂ (2 x 3 mL), CH₃OH (2 x 3 mL), CH₂Cl₂ (2 x 3 mL). The resin was then swollen with DMF (3mL). Ethyl isocyanatoacetate (101 mg) dissolved in DMF (3 mL) and DIEA (340 µL) were added to the swollen resin. This reaction mixture was stirred for 6-8 hr at room temperature. The resin was filtered and washed in the following order: DMF (2 x 3 mL), CH₂Cl₂ (2 x 3 mL), CH₃OH (2 x 3 mL), CH₂Cl₂ (2 x 3 mL), respectively. If a Kaiser test on a small quantity of the resin is positive (blue) then repeat the coupling procedure until a negative result is obtained. The resulting resin was then dried *in vacuum* to constant weight. The cleavage of the resin (saponification) was achieved with LiOH/CH₃OH/THF (1:6:20). The 'resin bound material was placed in the polypropylene column and pretreated with THF (3 mL). Then to the swollen resin THF (3.9 mL), CH₃OH (1.0 mL) and 2N LiOH (0.39 mL) were added respectively. The mixture was stirred for 15 min and filtered to a clean and pre weighed test tube. The resin was next 'washed with THF/5% CH₃OH (2mL) and the combined filtrates were evaporated. The resulting gum was dissolved in H₂O (1 mL). The solution was then acidified with 1N HCl to pH 2.0. The precipitate was centrifuged, washed with H₂O (2 x 5 mL) and dried *in vacuum* to furnish the compound of Example 33: ESMS (m/z) 571, (M-H)

### Examples 34-87:

The compounds of the following Examples were prepared in a similar manner as described above.

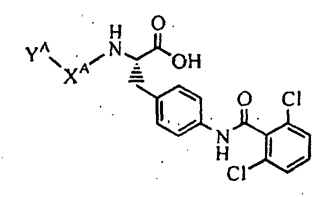

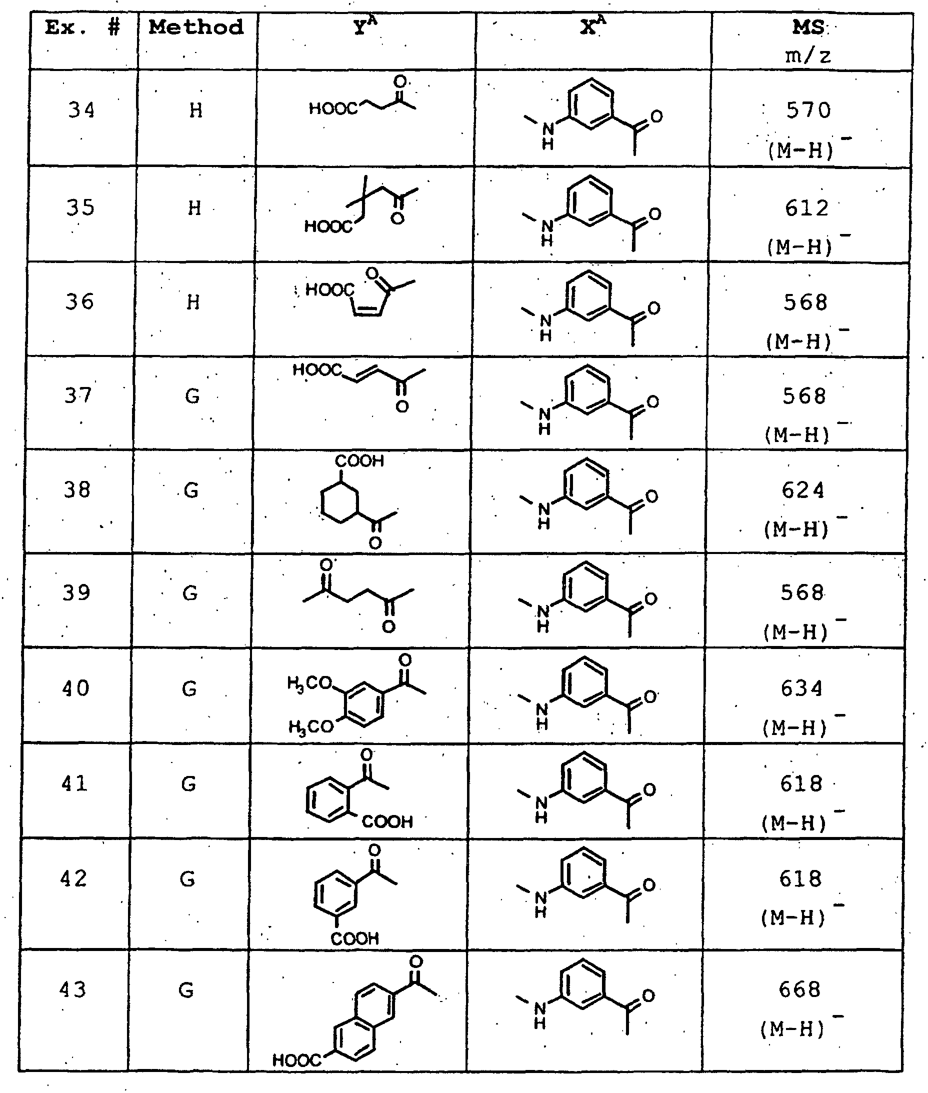

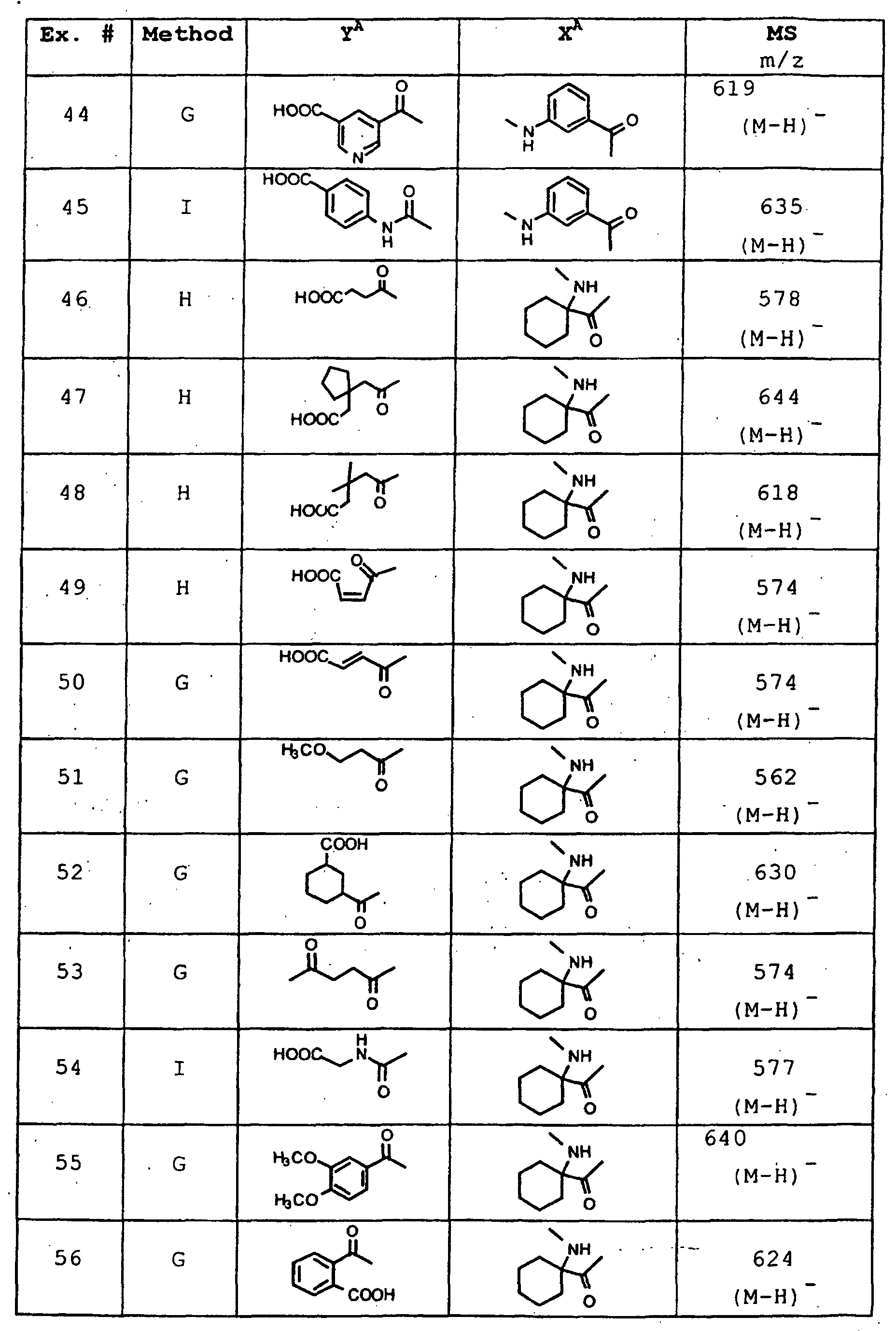

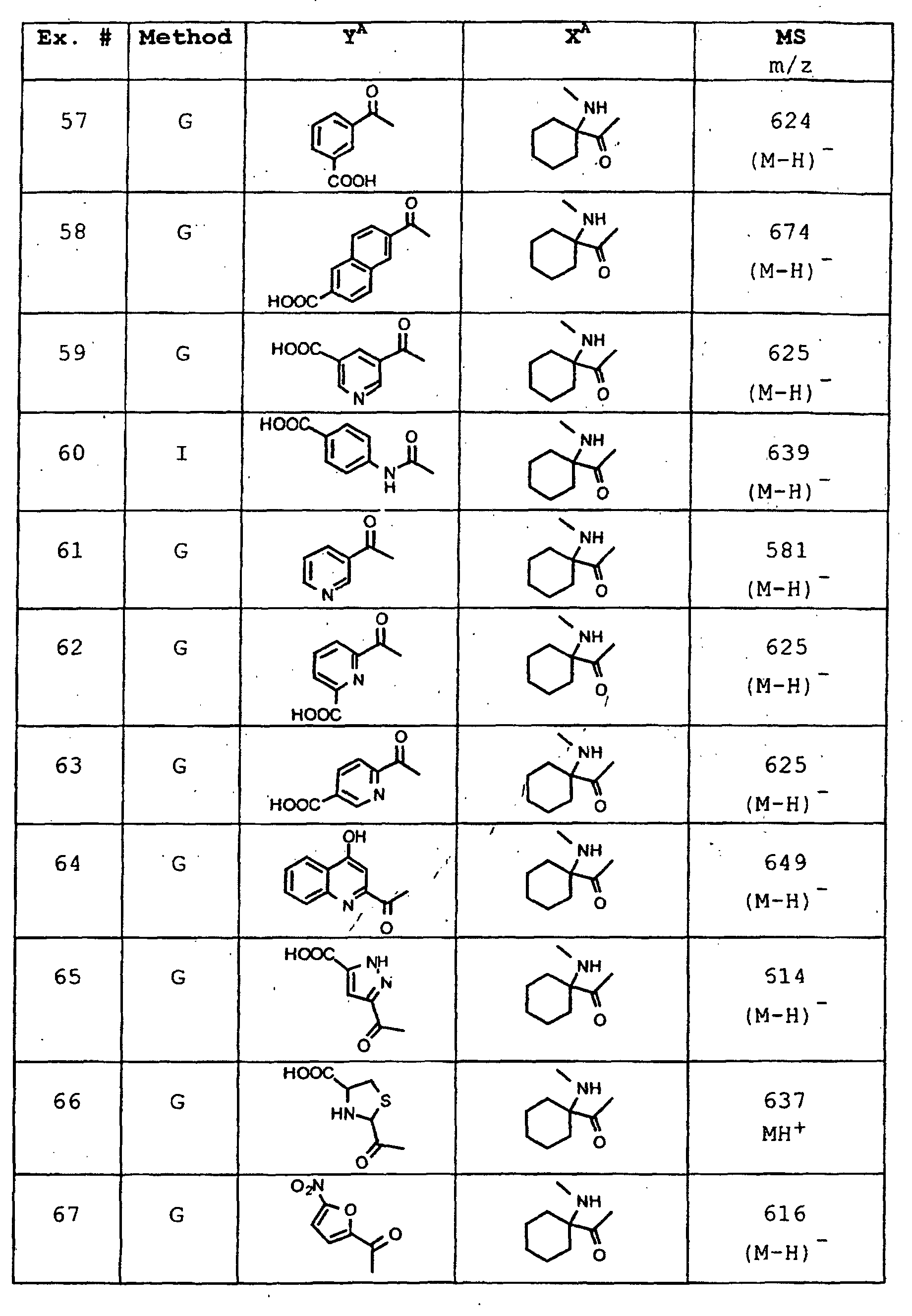

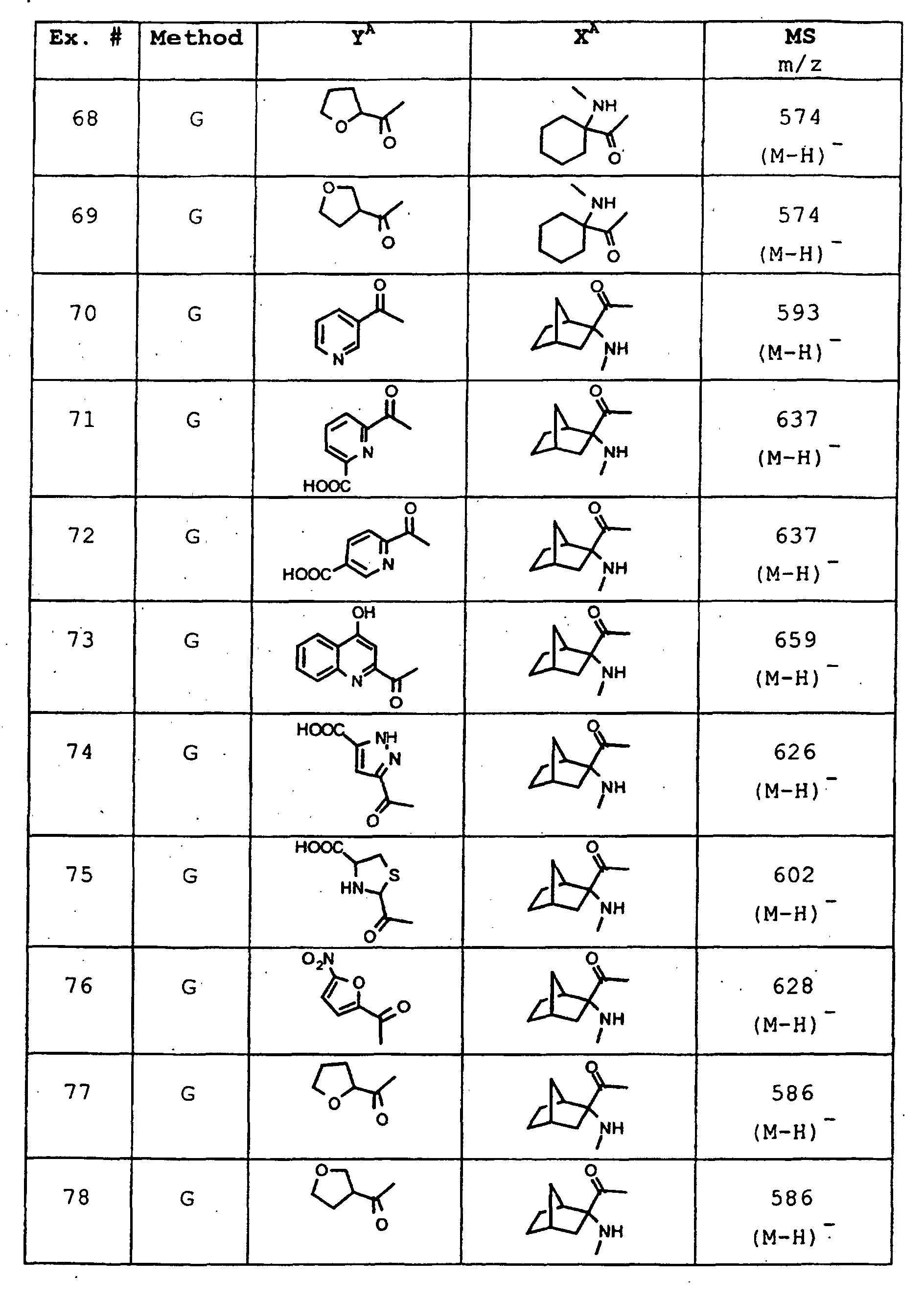

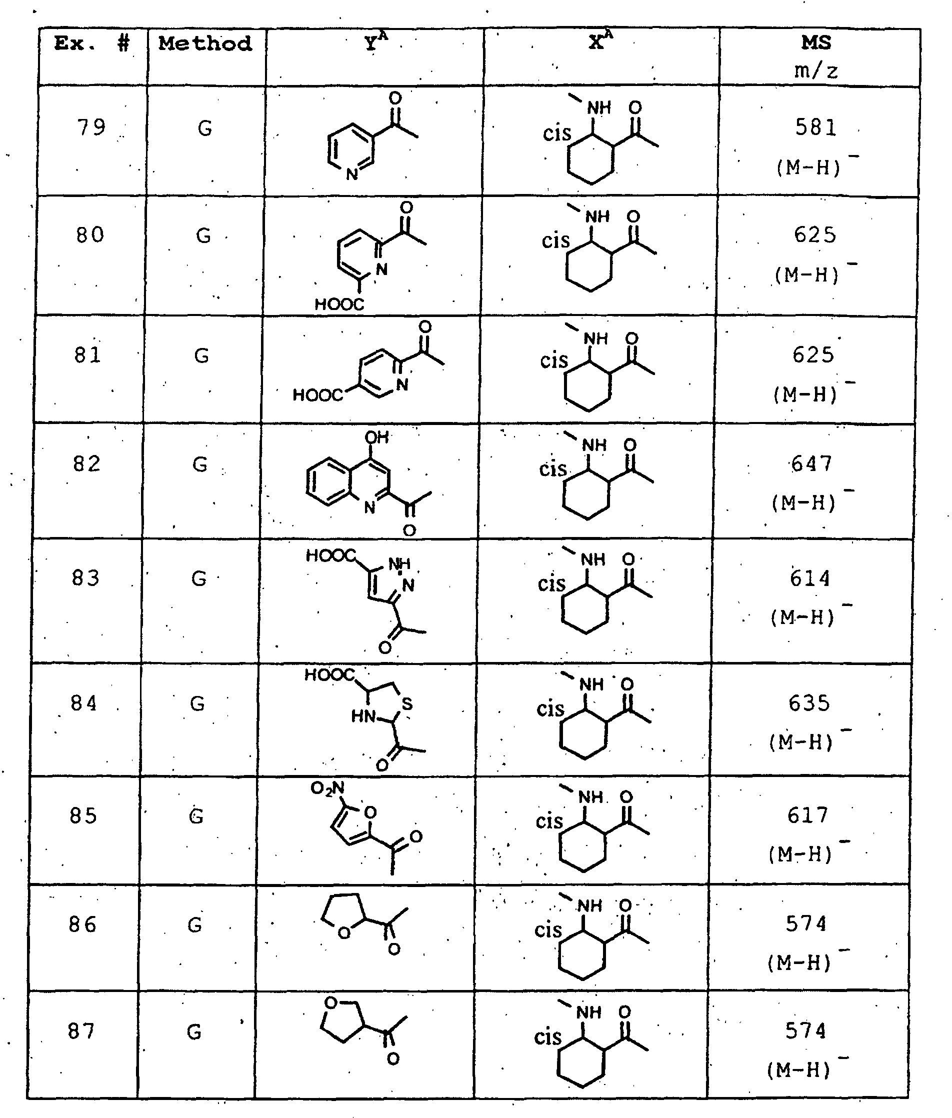

### Reference Compound Examples:

### Reference Compound A:

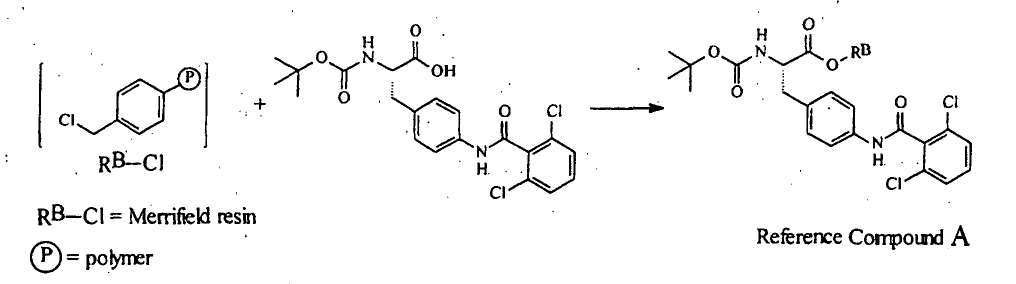

Attachment of N-tert-butoxycarbonyl-[4-[(2,6-dichlorobenzoyl)amino]-L-phenylalanine to Merrifield resin was done using Horiki's method (Horiki et al., *Chem. Lett*. **1978** (2) 165-168). In a 250 mL round bottom flask fitted with a drying tube, Merrifield resin (Biorad, 10.0 g, 13.5 mmol/g) and anhydrous potassium fluoride (1.57g) were added to a solution of N-tert-butoxycarbonyl-[4-[(2,6-dichlorobenzoyl)amino]-L-phenylalanine (**Compound 10,** 6.13 g) in dry DMF (100 mL). The reaction mixture was stirred at 80 °C in an oil bath for 24 hr. The cooled resin was then filtered and washed thoroughly with DMF (2 x 250 mL), 50% aqueous DMF (3 x 250 mL), methanol (3 x 250 mL), dichloromethane (3 x 250 mL), and finally methanol (3 x 250 mL). The resin was then dried under reduced pressure to constant weight to give **R. Compound A.** Incorporation of N-tert-butoxycarbonyl-[4-[(2,6-dichlorobenzoyl)amino]-L-phenylalanine onto the resin was estimated to be 1.3 mmol/g from the increase in resin mass.

### Reference Compounds B-1, B-2, B-3 and B-4:

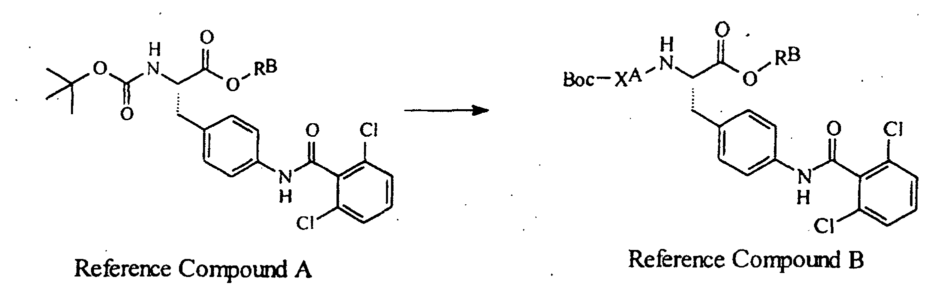

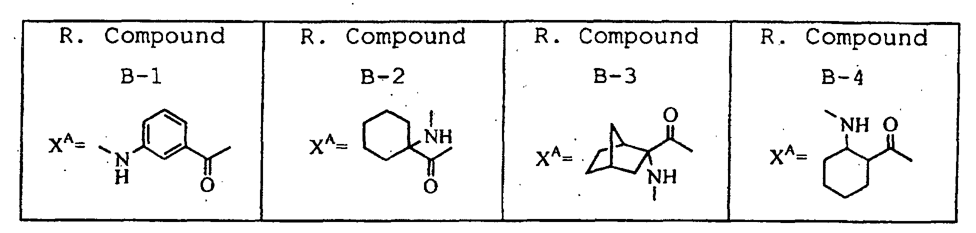

**R. Compound A** (200 mg) was placed in a 8.0 mL, polypropylene filter column fitted with a 2-way polypropylene stopcock. The resin was pretreated with CH₂Cl₂ (2 x 3 mL). The swollen resin was then deprotected with 50% TFA/CH₂Cl₂ (3-4 mL, 30min) with shaking. The resin was rinsed in the following order: CH₂Cl₂ (2 x 3 mL), CH₃OH (2 x 3 mL), CH₂Cl₂ (2 x 3 mL). The resin was swollen with DMF (2 x 3 mL). 3-(tert-butoxycarbonylamino)benzoic acid (180 mg) in DMF (1.0 mL) was activated with 0.5 M HBTU/HOBT in DMF (1.6 mL) and DIEA (0.34 mL). The preactivated 3-(tert-butoxycarbonylamino)benzoic acid was then added to the swollen resin and the mixture was stirred for 2 hr at room temperature. The resin was then washed in the following order: DMF (2 x 3 mL), CH₂Cl₂ (2 x 3 mL), CH₃OH (2 x 3 mL), CH₂Cl₂ (2 x 3 mL) and dried to give **R. Compound B-1.** If a Kaiser test on a small quantity of the resin is positive (blue) then repeat the coupling procedure until a negative result is obtained.

**Reference Compounds B-2, B-3 and B-4** were prepared in a similar manner as **R. Compound B-1.**

### Reference Compound C: 3-(tert-Butoxycarbonylamino)benzoic acid

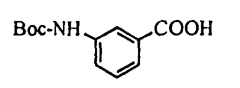

3-Aminobenzoic acid (10.0 g) was dissolved in 1N NaOH (160 mL) and cooled to 0 °C. To this solution was added drop wise, with stirring, t-butyl dicarbonate (17.5 g) in dioxane (100 mL). After complete addition of t-butyl dicarbonate the reaction mixture was warmed to room temperature and stirred vigorously for 14 hours. The dioxane was evaporated and the aqueous phase was extracted with Et₂O (2 X 100 mL). The aqueous layer was then acidified to pH = 2.0 with 1N HCl. The precipitate was filtered and washed with H₂O, and dried under high vacuum to provide 3-(tert-butoxycarbonylamino)benzoic acid (9.25 g): ¹H NMR (400 MHz, DMSO-d₆), δ 9.56 (1H), 8.16 (1H), 7.63 (1H), 7.55 (1H), 7.37 (1H), 1.49 (9H); ESMS (m/z) 238 MH⁺.

### Examples 88-137

The synthesis of the compounds of Examples 88-137 are as follows:

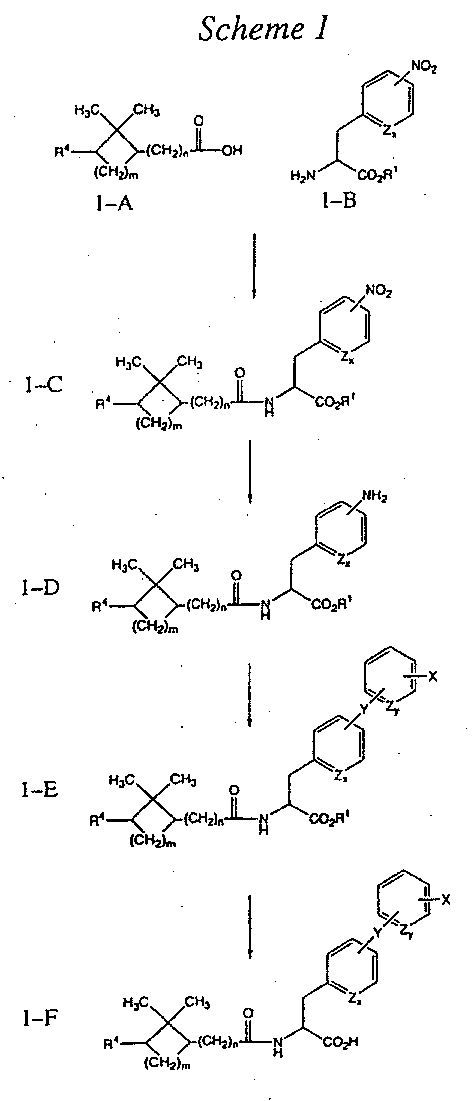

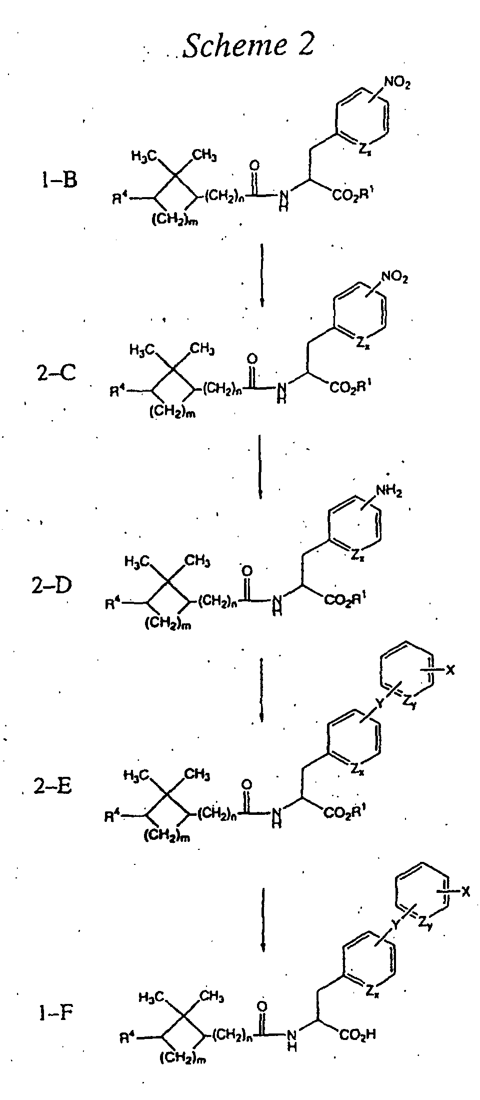

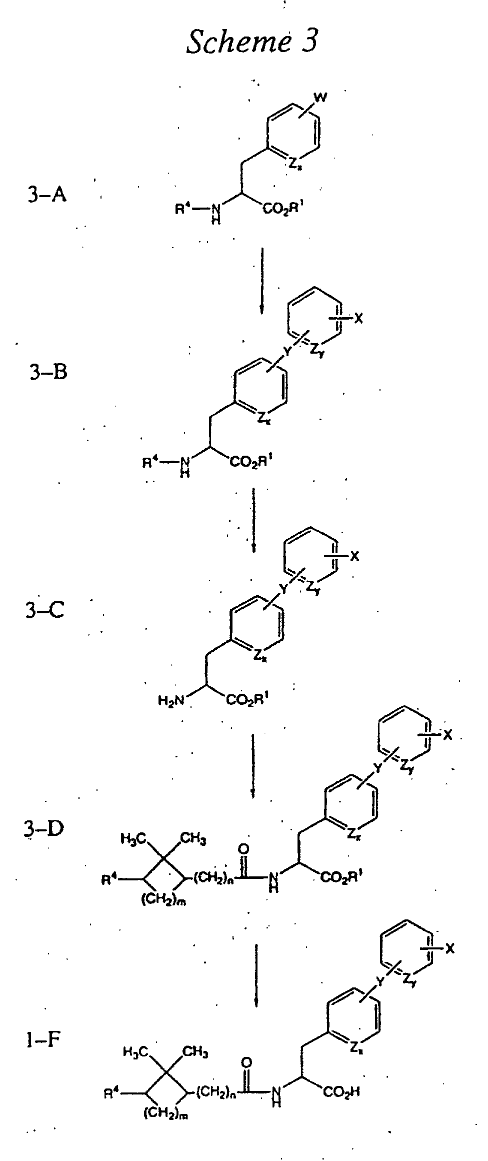

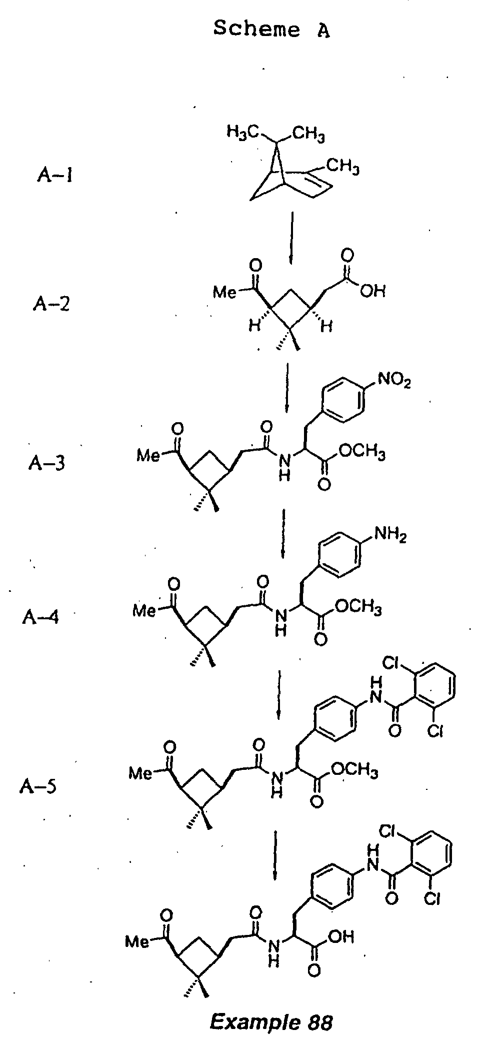

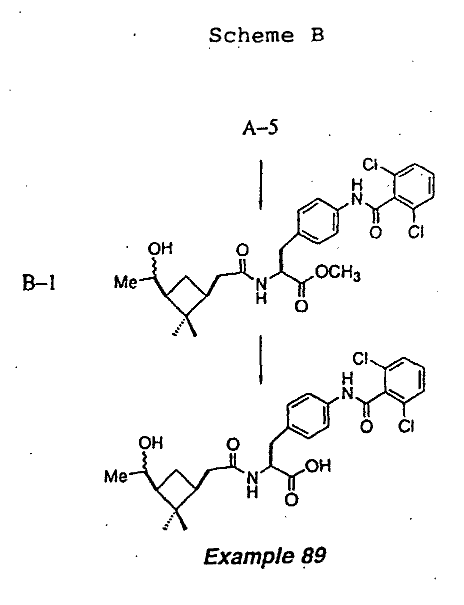

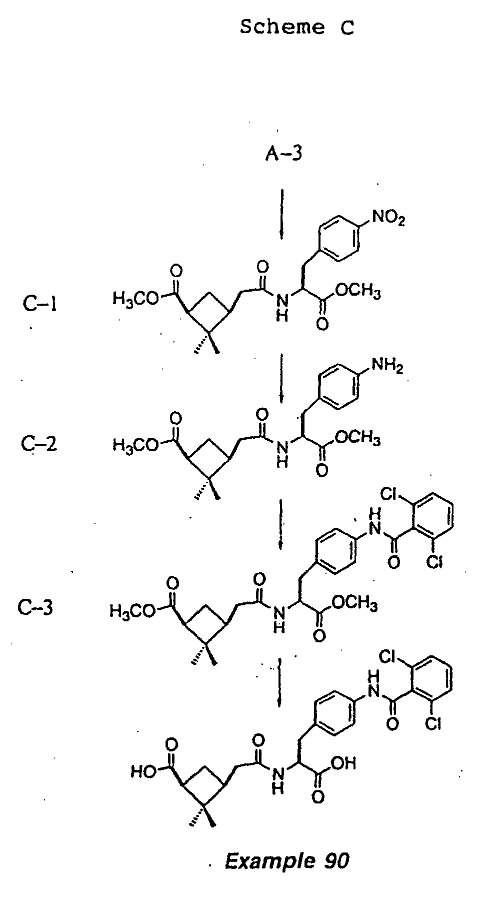

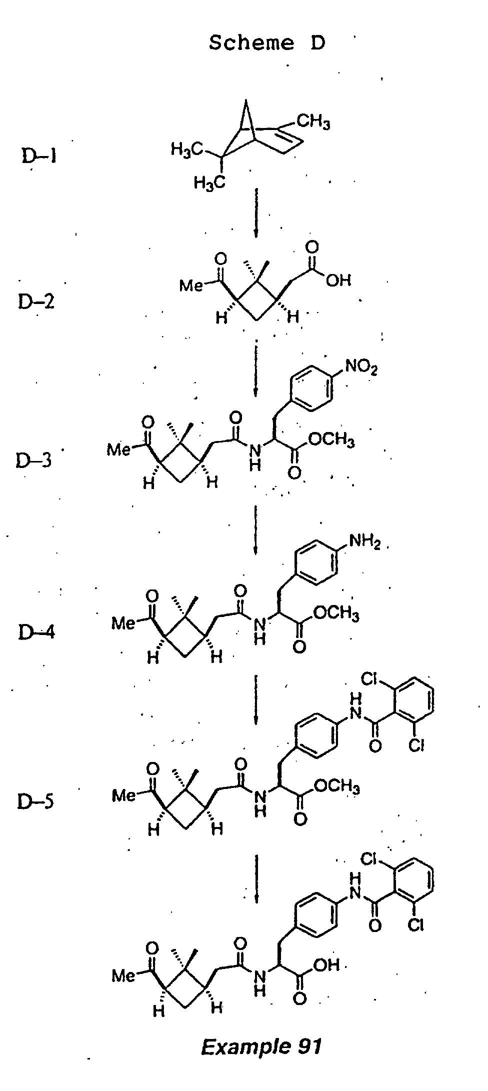

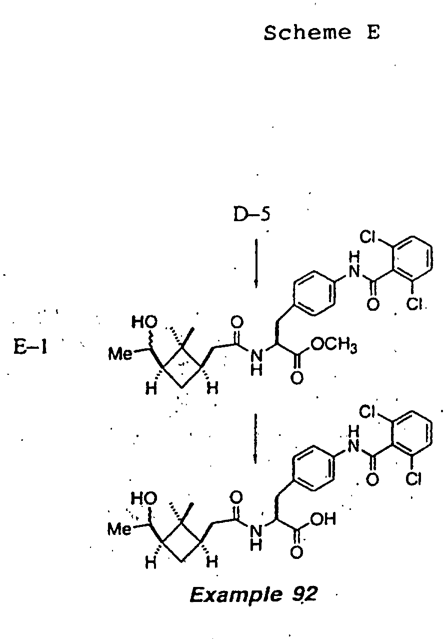

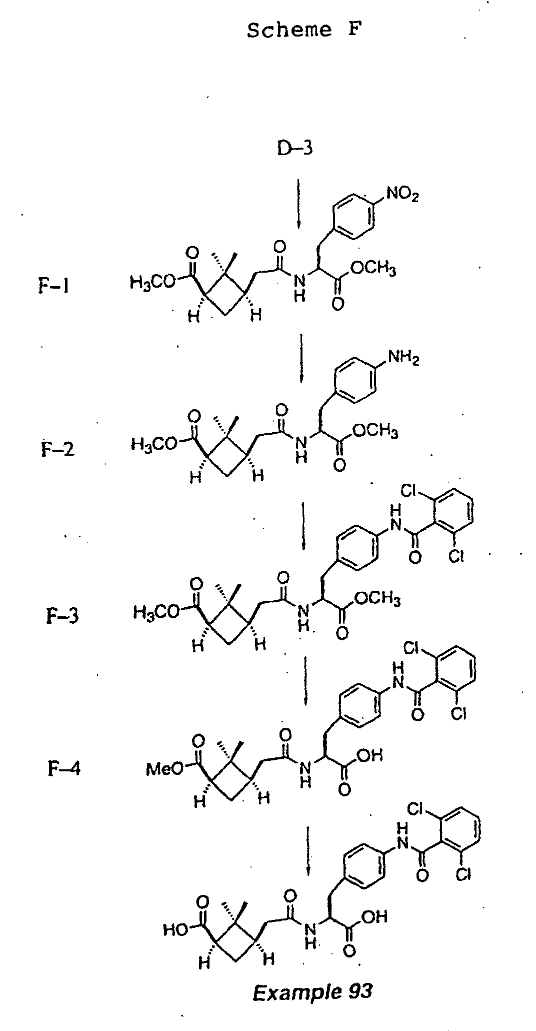

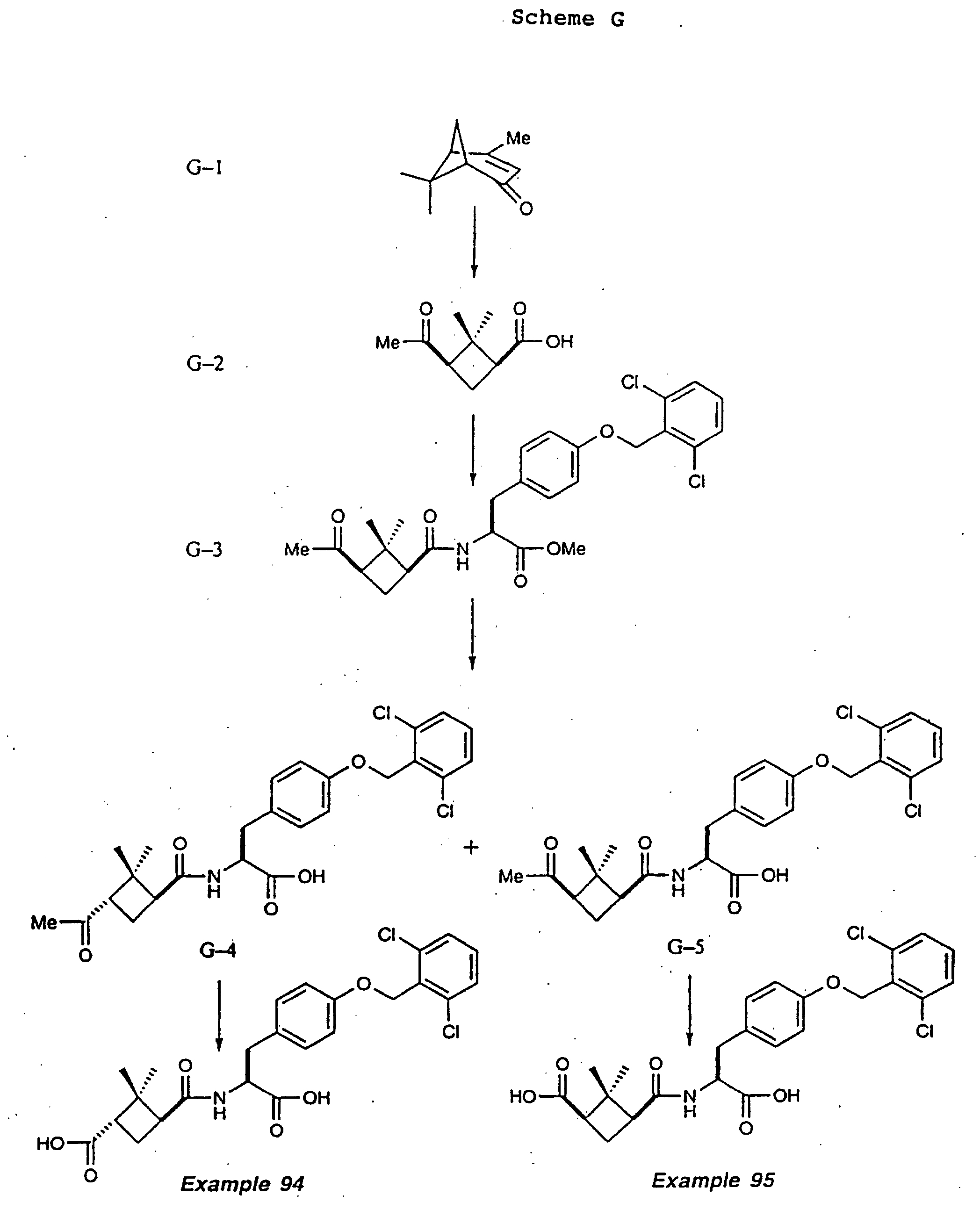

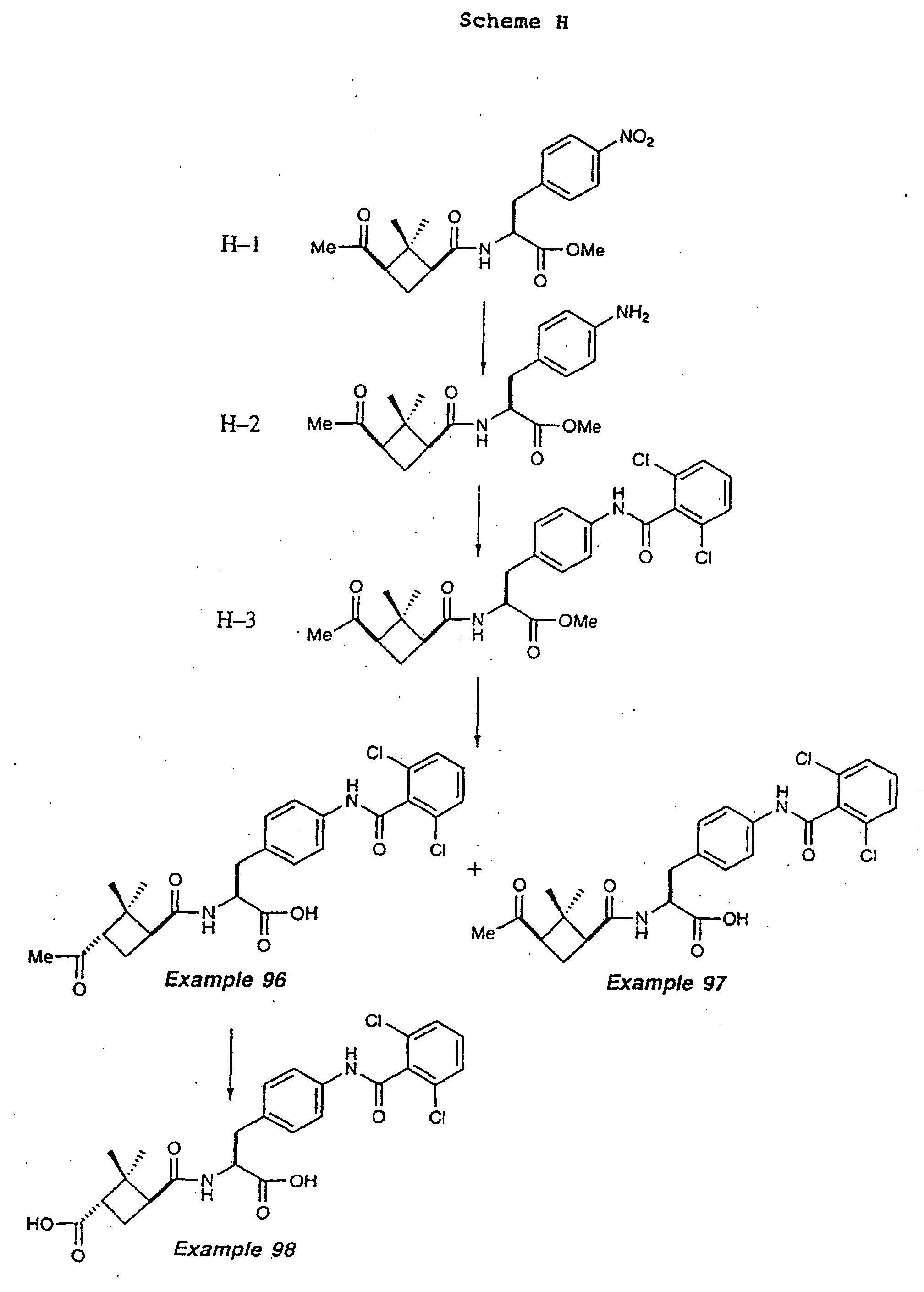

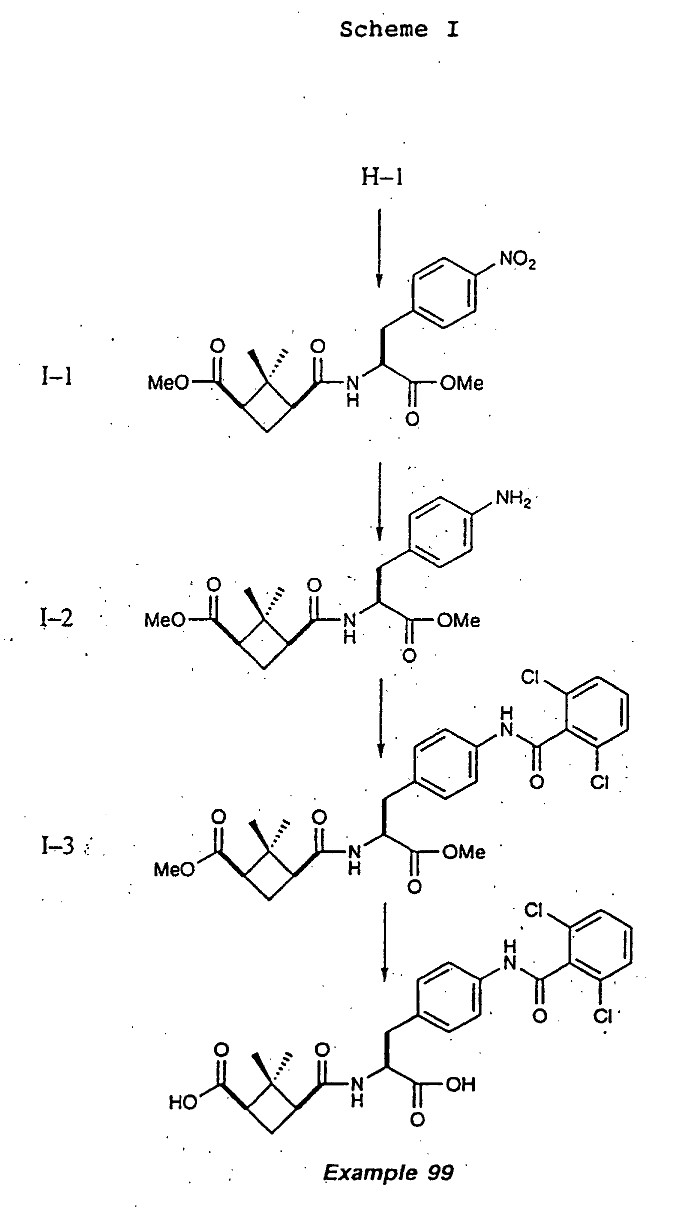

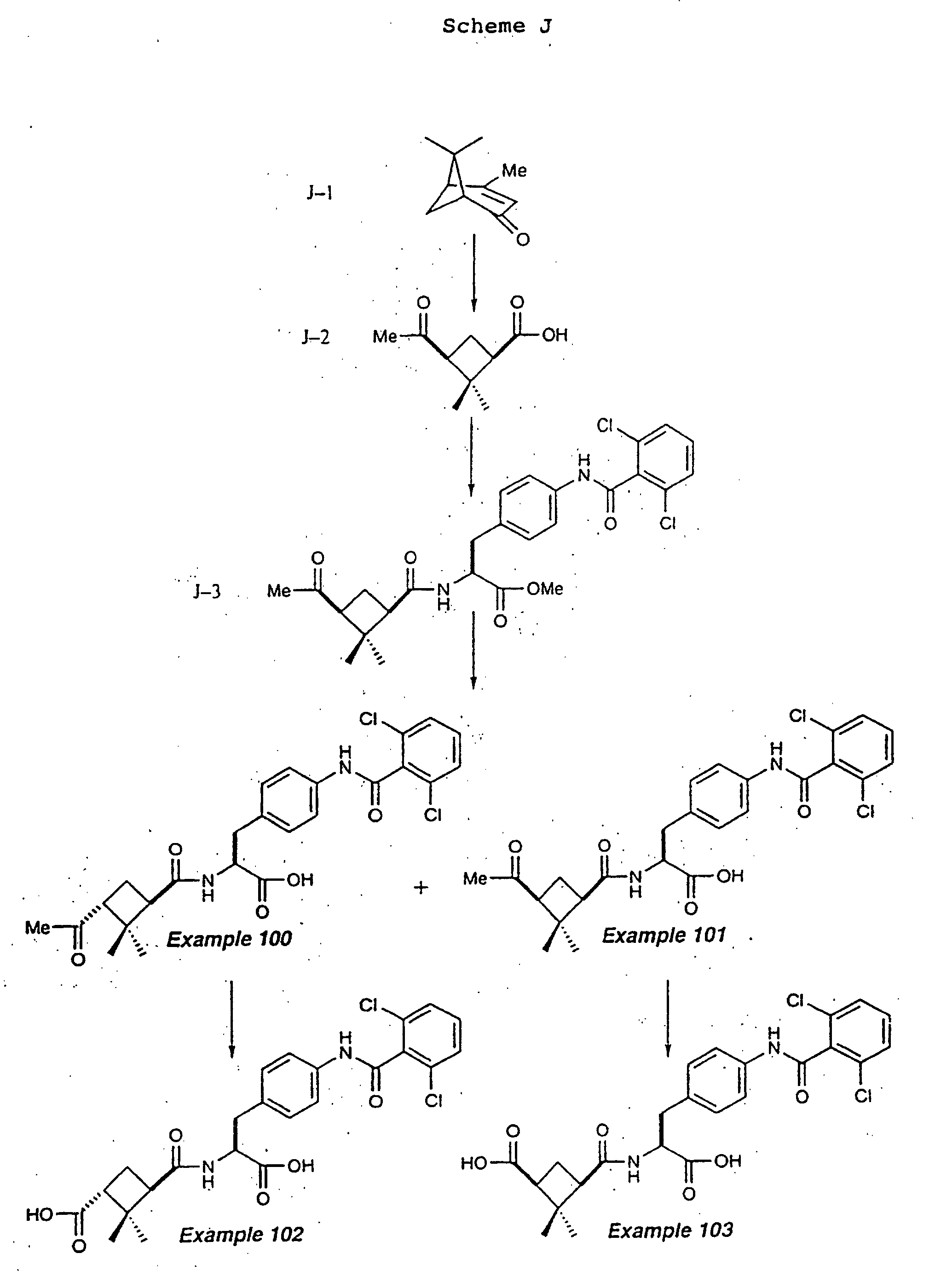

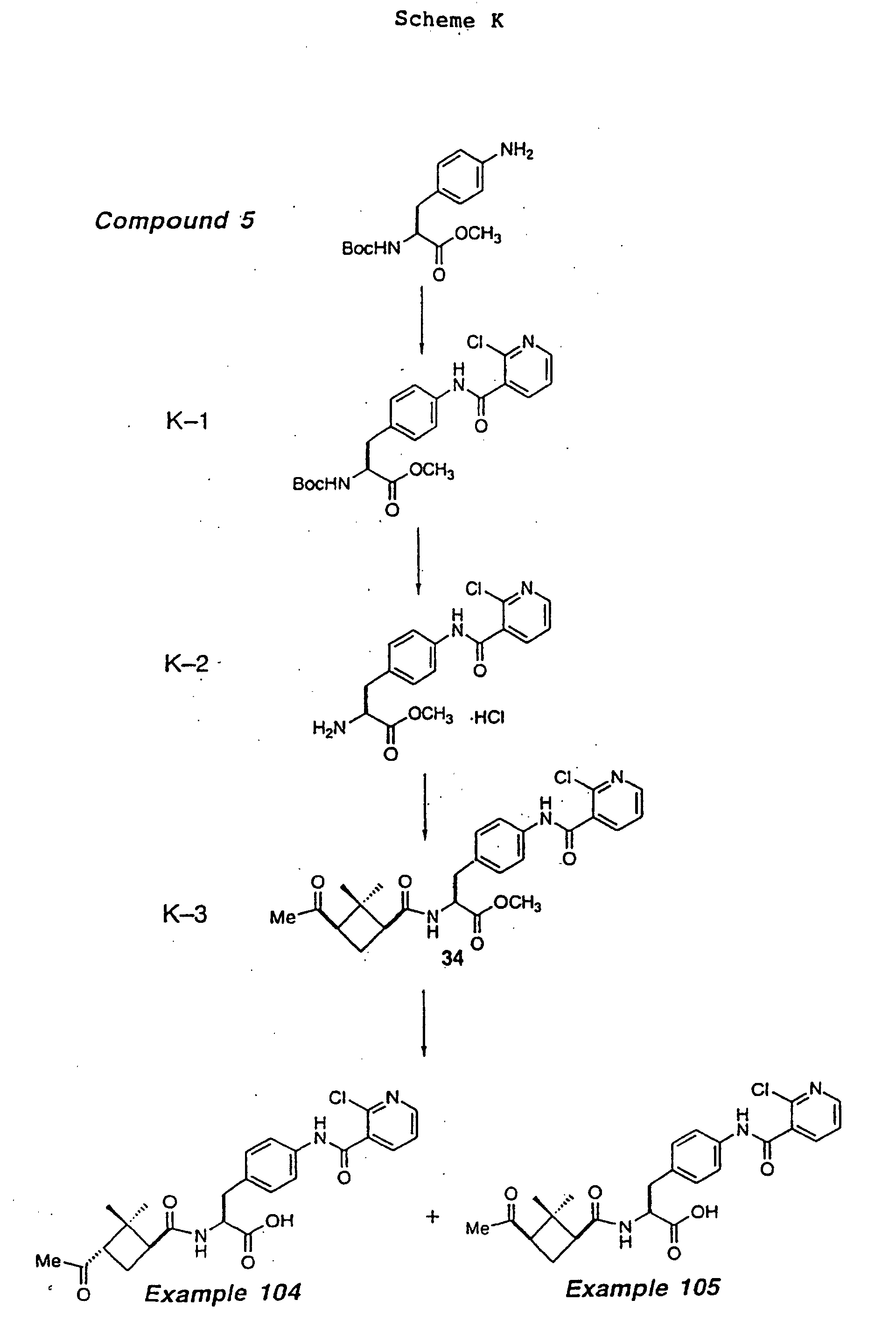

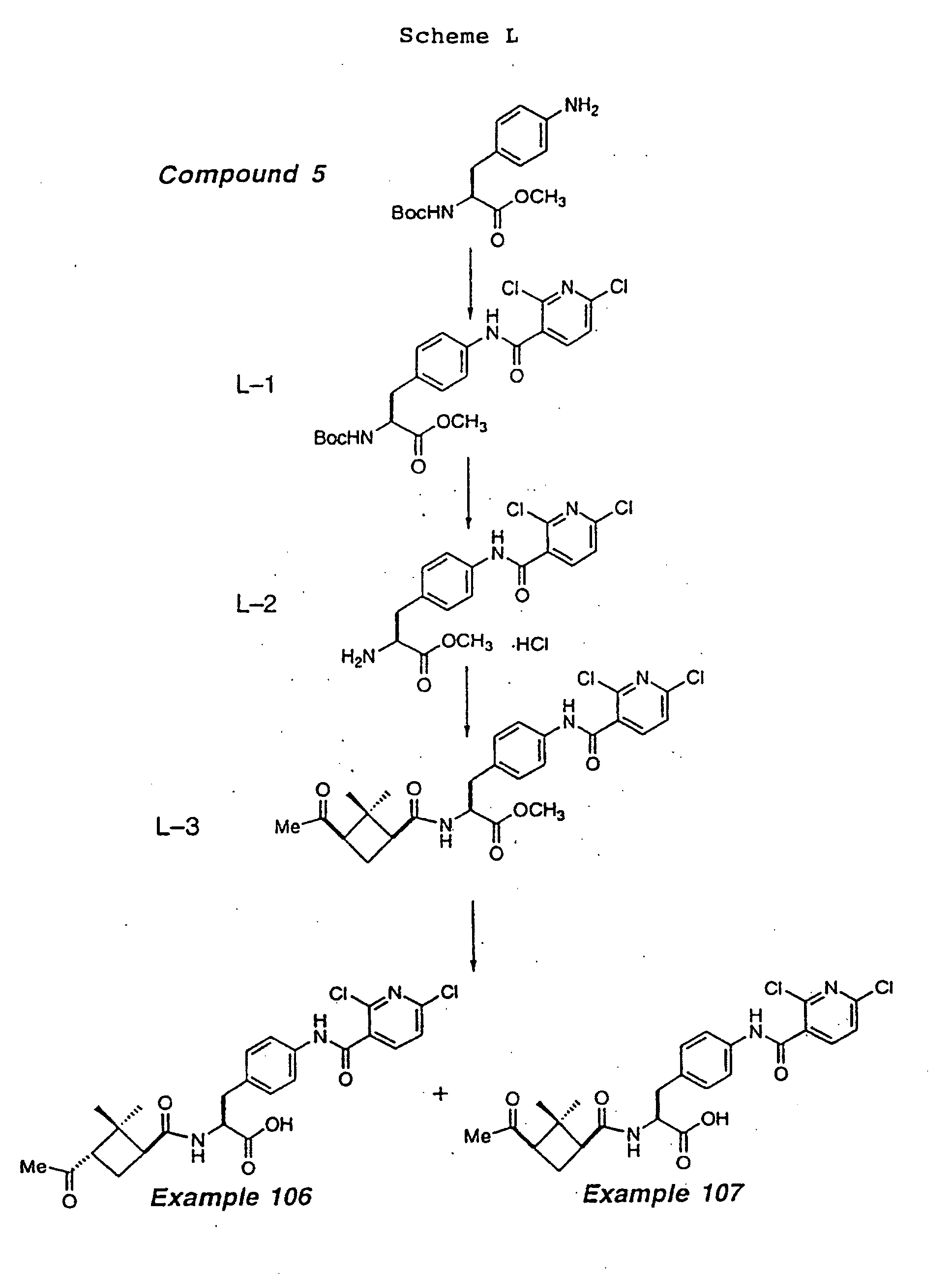

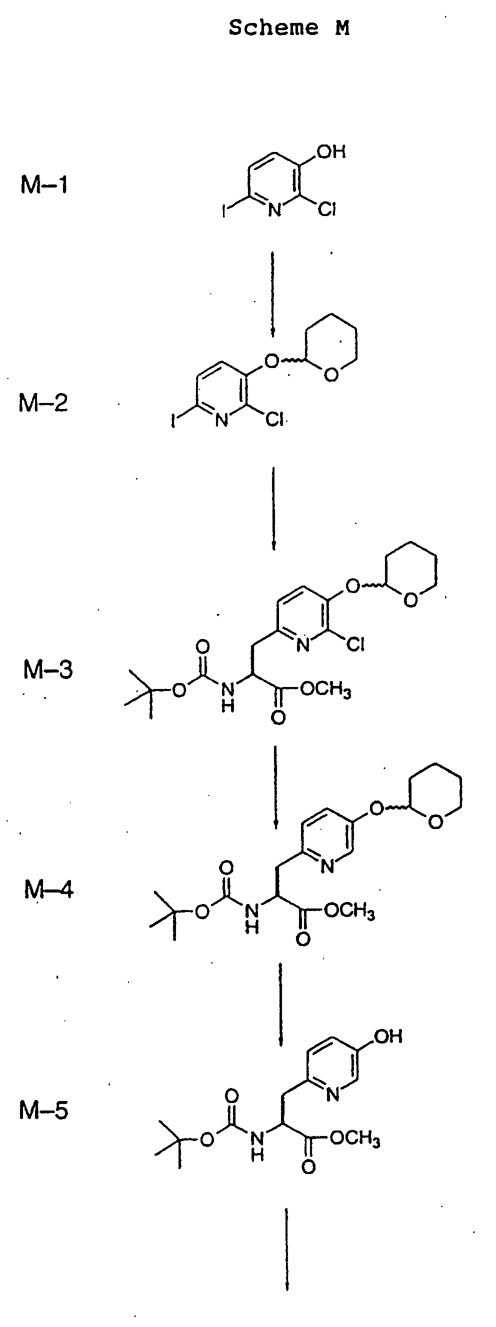

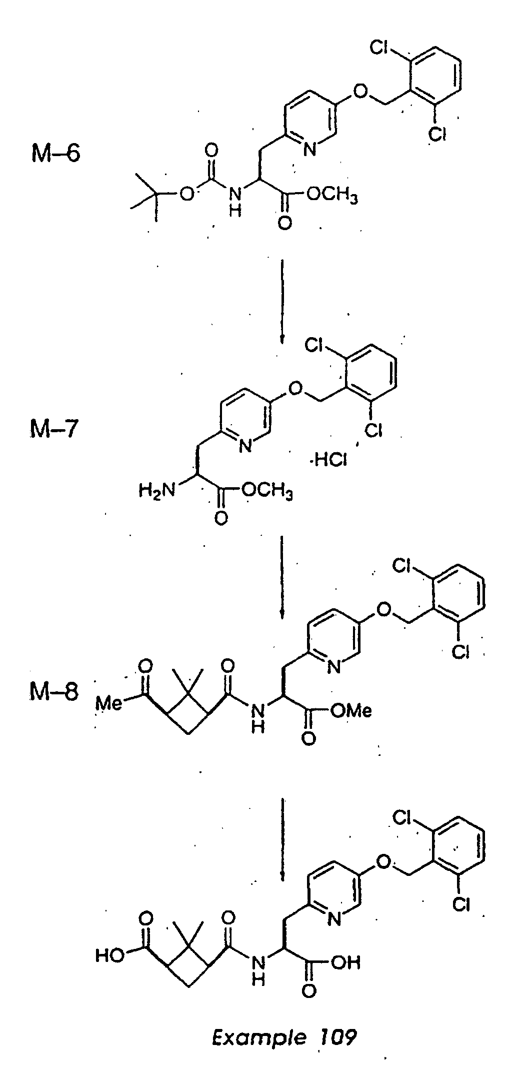

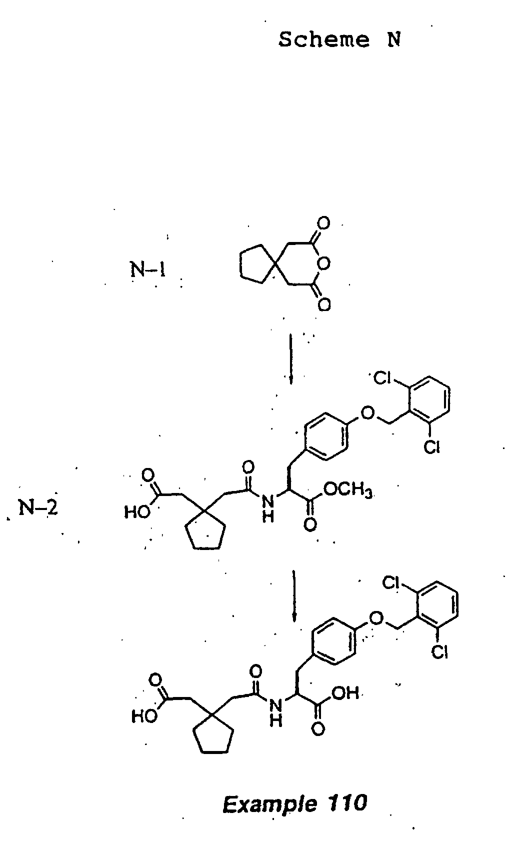

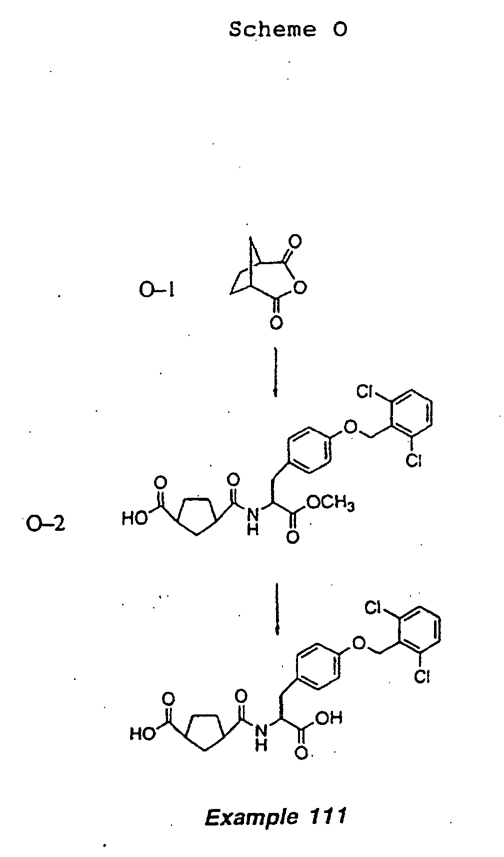

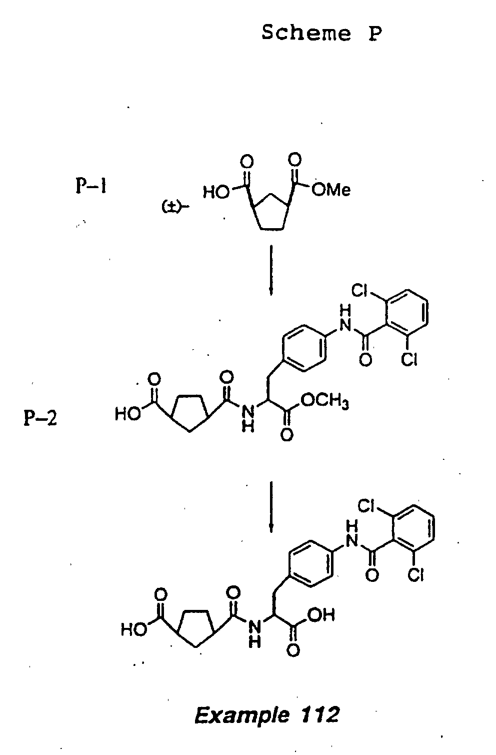

### Scheme 1

One method for the preparation of this general structure **1-F,** having an *N*-substituted cycloalkanoyl segment, is given in **Scheme 1.** Condensation of an R⁴-substituted cycloalkanoic acid **1-A** with aminoester **1-B** gives amidoester **1-C.** The nitro substituent of amidoester **1-C** is reduced by standard methods to give the amine-substituted amidoester **1-D.** Acylation of **1-D** provides **1-E** (Y=NHCO). The functionality of R⁴ may be changed (such as by oxidation, reduction, substitution, epimerization, and /or degradation) by standard synthetic methods. Hydrolysis of **1-E** gives the amidoacid **1-F.**

### Example 88 (TR-9712): Teaching Scheme A

**Teaching Scheme A** exemplifies the practice of **Scheme 1** by teaching the synthesis of **Example 88** where (with reference to structure **1-F**) R⁴ is 3-acetyl, m and n equal 1, Zₓ and Z_{y} are CH, Y is -NHC(O)-, X is 2,6-dichloro, the stereochemistry of the cycloalkanoyl segment is (1*S*-*cis*), and the stereochemistry of the amidoacid is L.

**(1*S*-*cis*)-3-Acetyl-2,2-dimethylcyclobutaneacetic acid [64396-97-0] (C**_{**10**}**H**_{**16**}**O**_{**3**}**, A-2).** This material was prepared as described by Wolk et *al*. (Wolk, J. L.; Goldschmidt, Z.; Dunkelblum, E. *Synthesis* **1986,** 347-348) from (+)-α-pinene **A-1:** mp 67-69 °C; TLC *R*_{*f*} = 0.19 (800:200:1 hexanes/EtOAc/HCO₂H); [α]²⁵_{D} +93 (c 1.0, CHCl₃); ¹³C NMR (CDCl₃, 75 MHz) δ 207.87, 178.94, 54.20, 43.29, 37.71, 34.87, 30.18, 30.15, 22.79, 17.30.

**(1*S*-*cis*)-*N*-[(3-Acetyl-2,2-dimethylcyclobutyl)acetyl]-4-nitro-L-phenylalanine methyl ester (C**_{**20**}**H**_{**26**}**N**_{**2**}**O**_{**6**}**, A-3).** To a mixture of acid **A-2** (1.82 g, 9.88 mmol), EDC (2.00 g, 10.22 mmol), HOBt (1.40 g, 10.33 mmol), DMAP (0.362 g, 2.96 mmol) and amine **Compound 3** (10.22 mmol) in CH₂Cl₂ at 0 °C is added Et₃N (1.67 mL, 11.89 mmol). The reaction mixture is stirred at room temperature for 40 h. It is diluted with CH₂Cl₂. The CH₂Cl₂ mixture is extracted with H₂O, aq 0.5 M HCl, H₂O, aq satd NaHCO₃ and H₂O, and then is dried and concentrated to a yellow oil. The oil is purified by silica chromatography to give **A-3:** TLC *R*_{*f*} = 0.40 (3:1 EtOAC/hexanes); ¹H NMR (CDCl₃, 300 MHz) δ 8.14 (m, 2H), 7.28 (m, 2H), 6.02 (s, 1H), 4.90 (m, 1H), 3.73 (s, 3H), 3.27 (m, 1H), 3.13 (m, 1H), 2.88 (m, 1H), 2.39-1.72 (m, 5H), 2.02 (s, 3H), 1.28 (s, 3H), 0.82 (s, 3H) ; ¹³C NMR (CDCl₃, 75 MHz) δ 207.53, 171.57, 171.53, 147.19, 143.88, 130.14, 123.72, 54.13, 52.76, 52.64, 43.25, 38.27, 37.94, 37.23, 30.26, 30.21, 22.93, 17.50.

**(1*S*-*cis*)-*N*-[(3-Acetyl-2,2-dimethylcyclobutyl)acetyl]-4-amino-L-phenylalanine methyl ester (C**_{**20**}**H**_{**28**}**N**_{**2**}**O**_{**4**}**, A-4).** A mixture of **A-3** (0.80 g, 2.05 mmol) and 10% Pd/C (0.036 g) in MeOH is hydrogenated (38 psi H₂, 2 h). The mixture is filtered and concentrated to give **A-4:** TLC *R*_{*f*} = 0.33 (3:1 EtOAC/hexanes); ¹H NMR (CDCl₃, 300 MHz) δ 6.86 (m, 2H), 6.61 (m, 2H), 5.77 (m, 1H), 4.82-4.75 (m, 1H), 3.72 (s, 3H)., 3.62 (s, 1H), 2.99 (m, 2H), 2.86 (m, 1H), 2.39-2.00 (m, 2H), 2.03 (s, 3H), 1.99-1.82 (m, 2H), 1.59 (m, 2H), 1.29 (s, 3H), 0.84 (s, 3H); MS (FAB) *m*/*z* 361 (M+H)⁺, 203, 177, 132, 106.

**(1*S-cis*)*-N-*[(3-Acetyl-2,2-dimethylcyclobutyl**]**acetyl]-4-[(2**,**6-dichlorobenzoyl) amino] -L-phenylalanine methyl ester (C**_{**27**}**H**_{**30**}**Cl**_{**2**}**N**_{**2**}**O**_{**5**}**, A-5)**. To a solution of 2,6-dichlorobenzoyl chloride (0.28 mL, 1.98 mmol) in THF at room temperature under Ar is added dropwise over 15 min a solution of 17 (0.70 g, 1.94 mmol) in THF. The reaction mixture is stirred overnight at rt. It is diluted with EtOAc. The reaction mixture is extracted with aq 1 M HCl, aq 1 M NaOH and brine. The combined aqueous extracts are back-extracted with EtOAc. The combined EtOAc extracts are dried and concentrated to give A-5 as a beige-colored solid: TLC *R*_{*f*} = 0.14 (1:1 EtOAc/hexanes); ¹H NMR (CDCl₃, 300 MHz) δ 9.57 (s, 1H), 7.57 (m, 2H) , 7.28-7.17 (m, 3H), 6.99 (m, 2H), 6.37 (m, 1H), 4.77 (m, 1H), 3.63 (s, 3H), 3.05-2.91 (m, 2H), 2.76 (m, 1H), 2.28-1.72 (m, 5H), 2.02 (s, 3H), 1.18 (s, 3H), 0.75 (s, 3H); ¹³C NMR (CDCl₃, 75 MHz) δ 207.63, 172.05, 171.67, 162.60, 137.18, 136.75, 132.38, 132.18, 130.40, 129.63, 127.87, 120.38, 54.12, 53.06, 52.20, 43.36, 38.34, 37.10, 36.94, 30.12, 30.05, 23.05, 17.34; MS (FAB) m/z 533 (M+H)⁺.

**(1*S*-*cis*)-*N*-[(3-Acetyl-2,2-dimethylcyclobutyl)acetyl]-4-[(2,6-dichlorobenzoyl)amino]-L-phenylalanine (C**_{**26**}**H**_{**28**}**Cl**_{**2**}**N**_{**2**}**O**_{**5**}**, Example 88).** To a solution of ester A-5 (0.483 g, 0.90 mmol) in 5:1 THF/MeOH (23 mL) at room temperature under Ar is added a solution of LiOH · H₂O (0.088 g, 2.08 mmol) in H₂O (4.6 mL). The reaction mixture is stirred for 2 h. It is acidified with aq 1 M HCl, diluted with H₂O, and extracted with EtOAc. The combined EtOAc extracts are washed with brine. They are dried and concentrated to an off-white solid. The solid is dissolved in MeCN (15 mL), and the solution is diluted with H₂O (15 mL). This solution is frozen and lyophilized to give **Example 88** as a cotton-like solid: mp 129-133 °C; TLC *R*_{*f*} = 0.21 (500:500:5 EtOAc/hexanes/HCO₂H); ¹H NMR (CDCl₃, 300 MHz) δ 8.97 (s, 1H), 7.58 (m, 2H), 7.33-7.18 (m, 4H), 7.11 (m, 2H), 6.11 (m, 1H), 4.74 (m, 1H), 3.21-3.04 (m, 2H), 2.93-2.76 (m, 1H), 2.48-1.74 (m, 5H), 1.97 (s, 3H), 1.24 (s, 3H), 0.79 (s,' 3H); MS (FAB) m/z 519 (M+H)⁺; Anal. C 59.41, H 5.57, Cl 13.20, N 5.31 (calcd corrected for 1.02% H₂O: C 60.12, H 5.43, Cl 13.65, N 5.39).

### Example 89: Teaching Scheme B

**Teaching Scheme B** exemplifies the practice of **Scheme 1** by teaching the synthesis of **Example 89** where (with reference to structure **1-F**) R⁴ is 3-(1-hydroxyethyl), m and n equal 1, Zₓ and Z_{y} are CH, Y is -NHC(O)-, X is 2,6-dichlorc, the stereochemistry of the cycloalkanoyl segment is [1*S*-(1α,3α)], and the stereochemistry of the amidoacid is L. **Example 89** exemplifies a change in the functionality of R⁴ where the acetyl substituent (**1-E,** R⁴ is acetyl) is reduced (**1-E,** R⁴ is 1-hydroxyethyl) prior to the preparation of **1-F.**

**[1*S*-(1α,3α)]-4-[(2,6-Dichlorobenzoyl)amino]-*N*-[[3-(1-hydroxyethyl)-2,2-dimethylcyclobutyl]acetyl]-L-phenylalanine methyl ester (C**_{**27**}**H**_{**30**}**Cl**_{**2**}**N**_{**2**}**O**_{**5**}**, B-1).** To a solution of **A-5** (0.503 g, 0.94 mmol) in THF at 0 °C under Ar is added solid NaBH₄ (0.242 g, 6.47 mmol). The reaction mixture is stirred for 4 h at 0 °C. It is stored overnight at -20 °C, and then stirred for 1 h additional at 0 °C. The reaction mixture is poured into a cold mixture of aq 1 M HCl and brine, and extracted with CH₂Cl₂.The combined CH₂Cl₂ extracts are washed with brine, dried, filtered and concentrated to give a white foam. This foam is purified by silica flash chromatography to give **B-1:** TLC *R*_{*f*} = 0.33 (95:5 CH₂Cl₂/MeOH); ¹H NMR (CDCl₃, 300 MHz) δ 9.26 (s, 1H), 7.59 (m, 2H), 7.33-7.21 (m, 1H), 7.25 (m, 2H), 7.03 (m, 2H), 6.07 (m, 1H), 4.85-4.74 (m, 1H), 3.68 (s, 3H), 3.66-3.52 (m, 1H), 3.12-2.94 (m, 2H), 2.24-1.95 (m, 5H), 1.94-1.83 (m, 1H), 1.76-1.57 (m, 1H), 1.06 (s, 3H), 0.97 (m, 3H), 0.95 (s, 3H); ¹³C NMR (CDCl₃, 75 MHz) δ 172.10, 172.04, 162.61, 137.09, 136.64, 132.47, 132.18, 130.51, 129.75, 127.96, 120.39, 68.94, 52.98, 52.30, 50.26, 38.32, 37.29, 37.19, 30.88, 26.66, 21.26, 16.84; MS (FAB) *m*/*z* 535 (M + H)⁺.

**[1*S*-(1α,3α)]-4-[(2,6-Dichlorobenzoyl)amino]-*N*-[[3-(1-hydroxyethyl)-2,2-dimethylcyclobutyl]acetyl]-L-phenylalanine (C**_{**26**}**H**_{**30**}**Cl**_{**2**}**N**_{**2**}**O**_{**5**}**, Example 89).** Example **89** is prepared from **B-1** as described for Example **88:** mp 145-149 °C; TLC *R*_{*f*} = 0.49 (750:250:5 EtOAc/hexanes/HCO₂H); ¹H NMR (CDCl₃, 300 MHz) δ 9.21 (s, 1H), 7.60 (m, 2H), 7.27 (m, 3H), 7.09 (m, 2H), 6.06 (m, 1H), 4.72 (m, 1H), 3.61 (m, 1H), 3.09 (m, 2H), 2.24-1.96 (m, 2H), 1.99 (s, 3H), 1.87 (m, 1H), 1.68 (m, 1H), 1.20 (m, 1H), 1.05 (s, 3H), 0.97 (m, 3H), 0.94 (s, 3H); MS (FAB) *m*/*z* 523, 521 (M+H)⁺; Anal. C 59.03, H 5.96, Cl 13.20, N 5.27 (calcd for 1.0% H₂O: C 59.28, H 5.85, Cl 13.46, N 5.32).

### Scheme 2

Another method for the synthesis of general structure **1-F** is given in **Scheme 2,** whereby the change of the R⁴ may be accomplished at the point of structure **1-B**. The R⁴ functionality of **1-B** is changed (such as by oxidation, reduction, substitution, epimerization, and/or degradation) to give **2-C**. The nitro substituent of structure **2-C** is reduced to the amine **2-D,** that may be substituted to give **2-E,** that is hydrolyzed to structure **1-F**.

### Example 90: Teaching Scheme C

**Teaching Scheme C** exemplifies the practice of **Scheme 2** by teaching the synthesis of **Example 90** where (with reference to structure **1-F**) R⁴ is 3-carboxy, m and n equal 1, Zₓ and Z_{y} are CH, Y is -NHC(O)-, X is 2,6-dichloro, the stereochemistry of the cycloalkanoyl segment is (1*S-cis*), and the stereochemistry of the amidoacid is L.

**(1*S*-*cis*)-*N*-[[[3-(1-Methoxy-1-oxomethyl)]-2,2-dimethylcyclobutyl]-acetyl]-4-nitro-L-phenylalanine methyl ester (C**_{**20**}**H**_{**26**}**N**_{**2**}**O**_{**7**}**, C-1)**. The haloform degradation of the methyl ketone is carried out as described by Fernández *et al*. (Fernandez, F.; López, C.; Hergueta, A. R. *Tetrahedron* **1995**, *51*, 10317-10322): To a solution of 1 M aq NaOH (18.4 mL) at 0 °C under Ar is added dropwise Br₂ (0.22 mL, 4.2 mmol). This mixture is stirred for 30 min beyond the time that all of the Br₂ has dissolved. At this time a solution of **A-3** (0.405 g, 1.04 mmol) in dioxane (2 mL) is added slowly. The reaction mixture is stirred for 1 h at 0 °C and at room temperature for 2 h. It is cooled to 0 °C and quenched with aq 1 M NaHSO₃. The solution is extracted with Et₂O, and the Et₂O extracts are discarded. The alkaline aqueous solution is acidified with conc aq HCl, and extracted with EtOAc. The combined EtOAc extracts are dried, filtered and concentrated to an off-white solid. To this solid, dissolved in 3:1 EtOAc/THF, is added excess ethereal CH₂N₂. The yellow solution is stirred for 30 min at 0 °C. The excess CH₂N₂ is removed by an N₂ stream. The resulting solution is concentrated to give a pale-yellow colored oil that is purified by silica flash to give **C-1:** TLC R_{f} = 0.36 (1:1 EtOAc/hexanes); ¹H NMR (CDCl₃, 300 MHz) δ 8.15 (m, 2H) , 7.28 (m, 2H), 5.94 (m, 1H) , 4.91 (m, 1H) , 3.74 (s, 3H), 3.65 (s, 3H), 3.28 (m, 1H), 3.15 (m, 1H), 2.73 (m, 1H), 2.37-1.84 (m, 5H), 1.19 (s, 3H), 0.89 (s, 3H); ¹³C NMR (CDCl₃, 75 MHz) δ 173.26, 171.52, 147.22, 143.82, 130.15, 123.74, 60.21, 52.77, 52.66, 51.28, 46.10, 42.62, 38.57, 37.94, 37.49, 29.96, 24.50, 17.87; MS (EI) m/z 406 (M⁺).

**(1*S*-*cis*)-*N*-[[3-(1-Methoxy-1-oxomethyl)-2,2-dimethylcyclobutyl]-acetyl]-4-amino-L-phenylalanine methyl ester (C**_{**20**}**H**_{**28**}**N**_{**2**}**O**_{**5**}**, C-2).** Hydrogenation of **C-1** as described for **A-4** provides **C-2:** TLC *R*_{*f*} = 0.17 (1:1 EtOAc/hexanes); ¹H NMR (CDCl₃, 300 MHz) δ 6.87 (m, 2H), 6.65 (m, 2H), 5.85 (m, 1H), 4.79 (m, 1H), 3.71 (s, 3H), 3.65 (s, 3H), 3.47 (s, 1H), 3.07-2.89 (m, 4H), 2.72 (m, 1H), 2.38-1.83 (m, 4H), 1.18 (s, 3H), 0.88 (s, 3H); ¹³C NMR (CDCl₃, 75 MHz) δ 173.39, 172.29, 171.40, 144.70, 130.12, 125.99, 115.74, 53.15, 52.28, 51.23, 46.15, 42.73, 38.62, 37.51, 37.06, 29.88, 24.61, 17.89; MS (EI) *m*/*z* 376 (M⁺).

**(1*S*-*cis*)-*N*-[[3-(1-Methoxy-1-oxomethyl)-2,2-dimethylcyclobutyl)-acetyl]-4-[(2,6-dichlorobenzoyl)amino]-L-phenylalanine methyl ester (C**_{**27**}**H**_{**30**}**Cl**_{**2**}**N**_{**2**}**O**_{**6**}**, C-3).** Amine **C-2** is acylated, as described for **A-5,** to provide **C-3** as a beige-colored solid: TLC *R*_{*f*} = 0.32 (1:1 EtOAc/hexanes) ; ¹H NMR (CDCl₃, 300 MHz) δ 9.92 (s, 1H), 7.59 (m, 2H), 7.27 (m, 2H), 7.35-7.11 (m, 1H) , 7.02 (m, 2H) , 6.70 (m, 2H) , 4.70 (m, 1H), 3.64 (s, 3H), 3.55 (s, 3H), 3.06-2.89 (m; 2H), 2.62 (m, 1H), 2.13-2.04 (m, 3H), 1.98-1.77 (m, 2H), 1.09 (s, 3H), 0.82 (s, 3H); ¹³C NMR (CDCl₃, 75 MHz) δ 172.82, 171.70, 171.34, 162.23, 136.87, 136.45, 131.97, 131.87, 130.01, 129.23, 127.49, 119.97, 52.75, 51.78, 50.69, 45.66, 42.35, 38.31, 36.71, 29.44, 24.11, 17.33; MS (FAB) *m*/*z* 549 (M+H)⁺.

**(1*S*-*cis*)-*N*-[(3-Carboxy-2,2-dimethylcyclobutyl)acetyl]-4-[(2,6-dichlorobenzoyl)amino]-L-phenylalanine (C**_{**25**}**H**_{**26**}**Cl**_{**2**}**N**_{**2**}**O**_{**6**}**, Example 90).** Ester **C-3** is saponified (as described for **Example 88**) to give **Example 90** as an off-white solid: mp 143-146 °C; TLC *R*_{*f*} = 0.32 (750:250:5 EtOAc/hexanes/HCO₂H); [α]²⁵_{D} +4 (c 0.8, MeOH); ¹H NMR (CDCl₃, 300 MHz) δ 8.96 (s, 1H), 7.57 (m, 2H), 7.32-7.21 (m, 3H), 7.12 (m, 2H), 6.16 (m, 2H), 4.84-4.72 (m, 1H), 3.14-3.06 (m, 2H), 2.72-2.61 (m, 1H), 2.42-1.78 (m, 5H), 1.16 (s, 3H), 0.93 (s, 3H); MS (FAB) *m*/*z* 521 (M+H)⁺; Anal. C 56.55, H 5.09, Cl 13.27, N 5.03 (calcd for 1.49% H₂O: C 57.59, H 5.03, Cl 13.60, N 5.37).

### Example 91: Teaching Scheme D

**Teaching Scheme D** exemplifies the practice of **Scheme 1** by teaching the synthesis of **Example 91** where (with reference to structure **1-F**) R⁴ is 3-acetyl, m and n equal 1, Zₓ and Z_{y} are CH, Y is -NHC(O)-, X is 2,6-dichloro, the stereochemistry of the cycloalkanoyl segment is (1*R*-*cis*), and the stereochemistry of the amidoacid is L.

**(1*R*-*cis*)-3-Acetyl-2,2-dimethylcyclobutane acetic acid [52305-34-7] (C**_{**10**}**H**_{**16**}**O**_{**3**}**, D-2).** This material was prepared as described by Wolk et *al*. (Wolk, J. L.; Goldschmidt, Z.; Dunkelblum, E. *Synthesis* **1986,** 347-348) from (-)-α-pinene **D-1.**

**(1*R*-*cis*)-*N*-[(3-Acetyl-2,2-dimethylcyclobutyl)acetyl]-4-nitro-L-phenylalanine methyl ester (C**_{**20**}**H**_{**26**}**N**_{**2**}**O**_{**6**}**, D-3).** This compound is prepared from **D-2** and **Compound 3** by the procedure taught for **A-3:** TLC *R*_{*f*} = 0.32 (1:1 EtOAc/hexanes); ¹H NMR (CDCl₃, 300 MHz) δ 8.15 (m, 2H), 7.29 (m, 2H), 5.95 (m, 1H), 4.90 (m, 1H), 3.74 (s, 3H), 3.28 (m, 1H), 3.15 (m, 1H), 2.86 (m, 1H), 2.39-1.83 (m, 5H), 2.06 (s, 3H), 1.27 (s, 3H), 0.80 (s, 3H); ¹³C NMR (CDCl₃, 75 MHz) δ 207.50, 171.56, 175.48, 147.21, 143.89, 130.19, 123.73, 54.16, 52.73, 52.66, 43.23, 38.18, 37.97, 37.18, 30.25, 30.20, 22.98, 17.43.

**(1*R-cis*)-*N*-[(3-Acetyl-2,2-dimethylcyclobutyl)acetyl]-4-amino-L-phenylalanine methyl ester (C**_{**20**}**H**_{**28**}**N**_{**2**}**O**_{**4**}**, D-4).** This compound is prepared by the procedure taught for **A-4:** TLC *R*_{*f*} = 0.11 (1:1 EtOAc/hexanes); ¹H NMR (CDCl₃, 300 MHz) δ 6.86 (m, 2H), 6.62 (m, 2H), 5.80 (m, 1H), 4.78 (m, 1H), 3.71 (s, 3H), 3.95-3.17 (m, 2H), 3.05-2.91 (m, 2H), 2.85 (m, 1H), 2.27 (m, 1H), 2.24-1.82 (m, 4H), 2.07 (s, 3H), 1.28 (s, 3H) , 0.82 (s, 3H); MS (FAB) *m*/*z* 361 (M+H)⁺.

**(1*R-cis*)-*N*-[(3-Acetyl-2,2-dimethylcyclobutyl)acetyl]-4-[(2,6-dichlorobenzoyl)amino]-L-phenylalanine methyl ester (C**_{**27**}**H**_{**30**}**Cl**_{**2**}**N**_{**2**}**O**_{**5**}**, D-5).** This compound is prepared by the procedure taught for **D-5:** TLC *R*_{*f*} = 0.16 (1:1 EtOAc/hexanes); ¹H NMR (CDCl₃, 300 MHz) δ 9.32 (s, 1H), 7.59 (d, 2H, *J* = 8.5), 7.32-7.20 (m, 1H), 7.26 (d, 2H, *J* = 8.4), 7.03 (d, 2H, *J* = 8.4), 6.24 (d, 1H, *J* = 7.9), 4.77 (m, 1H), 3.67 (s, 3H), 3.10-2.93 (m, 2H), 2.79 (dd, 1H, *J* = 10.1, 2.4), 2.28-1.74 (m, 5H), 1.98 (s, 3H), 1.21 (s, 3H), 0.75 (s, 3H); MS (FAB) *m*/*z* 533 (M+H)⁺.

**(1*R-cis*)-*N*-[(3-Acetyl-2,2-dimethylcyclobutyl)acetyl]-4-[(2,6-dichlorobenzoyl)amino]-L-phenylalanine (C**_{**26**}**H**_{**28**}**Cl**_{**2**}**N**_{**2**}**O**_{**5**}**, Example 91).** This compound is prepared by the procedure taught for **Example 88:** mp 127-130 °C; TLC *R*_{*f*} = 0.21 (500:500:5 EtOAc/hexanes/HCO₂H); ¹H NMR (CDCl₃, 300 MHz) δ 8.84 (s, 1H), 7.58 (m, 2H), 7.33-7.22 (m, 1H), 7.27 (m, 2H), 7.12 (m, 2H), 6.10 (m, 1H) , 4.76 (m, 1H), 3.19-3.02 (m, 2H), 2.81 (m, 1H), 2.30-1.78 (m, 5H), 1.98 (s, 3H), 1.22 (s, 3H), 0.77 (s, 3H); MS (FAB) *m*/*z* 519 (M+H)⁺; Anal. C 59.65, H 5.45, Cl 13.78, N 5.23 (calcd for 0.75% H₂O: C 59.67, H 5.48, Cl 13.78, N 5.35).

### Example 92: Teaching Scheme E

**Teaching Scheme E** exemplifies the practice of **Scheme 1** by teaching the synthesis of **Example 92** where (with reference to structure **1-F**) R⁴ is 3-(1-hydroxyethyl), m and n equal 1, Zₓ and Z_{y} are CH, Y is -NHC(O)-, X is 2,6-dichloro, the stereochemistry of the cycloalkanoyl segment is [1*R*-(1α,3α)], and the stereochemistry of the amidoacid is L. **Example 92** exemplifies a change in the functionality of R⁴ where the acetyl substituent (1-E, R⁴ is acetyl) is reduced (**1-E,** R⁴ is 1-hydroxyethyl) prior to the preparation of **1-F.**

**[1*R*-(1α,3α)]-4-[(2,6-Dichlorobenzoyl)amino)-*N*-[[3-(1-hydroxyethyl)-2,2-dimethylcyclobutyl]acetyl]-L-phenylalanine methyl ester (C**_{**27**}**H**_{**32**}**Cl**_{**2**}**N**_{**2**}**O**_{**5**}**, E-1).** This compound is prepared from compound **D-5** by the procedure taught for **B-1:** TLC *R*_{*f*} = 0.34 (97:3 CH₂Cl₂/MeOH); ¹H NMR (CDCl₃, 300 MHz) δ 7.57 (m, 2H), 7.52 (s, 1H), 7.39-7.23 (m, 4H), 7.10 (m, 2H), 5.83 (m, 1H), 4.89-4.82 (m, 1H), 3.78-3.63 (m, 1H), 3.75 (s, 3H), 3.18-3.06 (m, 2H), 2.24-1.86 (m, 4H), 1.78-1.68 (m, 1H), 1.20-1.05 (m, 1H), 1.13 (s, 3H), 1.02 (m, 3H), 0.97 (s, 3H); MS (FAB) *m*/*z* 535 (M+H)⁺.

**[1*R*-(1α,3α)]-4-[(2,6-Dichlorobenzoyl)amino]-*N*-[[3-(1-hydroxyethyl)-2,2-dimethylcyclobutyl]acetyl]-L-phenylalanine (C**_{**26**}**H**_{**30**}**Cl**_{**2**}**N**_{**2**}**O**_{**5**}**, Example** **92).** This compound is prepared from compound **E-1** by the procedure taught for **Example 88:** TLC *R*_{*f*} = 0.50 (750:250:5 EtOAc/hexanes/HCO₂H); ¹H NMR (CDCl₃, 300 MHz) δ 8.87 (s, 1H), 7.57 (m, 2H), 7.34-7.22 (m, 4H), 7.13 (m, 2H), 6..02 (m, 1H), 4.76 (m, 1H), 3.63 (m, 1H), 3.15-3.07 (m, 2H), 2.25-1.83 (m, 4H), 1.77-1.54 (m, 1H), 1.17-1.03 (m, 1H), 1.07 (s, 3H), 0.98 (m, 3H), 0.94 (s, 3H); MS (FAB) *m*/*z* 521 (M+H)⁺; Anal. C 59.16, H 5.95, Cl 13.25, N 5.36 (calcd for 1.48% H₂O: C 59.00, H 5.88, Cl 13.40, N 5.29).

### Example 93: Teaching Scheme F

**Teaching Scheme F** exemplifies the practice of **Scheme 2** by teaching the synthesis of **Example 93** where (with reference to structure **1-F**) R⁴ is 3-carboxy, m and n equal 1, Zₓ and Z_{y} are CH, Y is -NHC(O)-, X is 2,6-dichloro, the stereochemistry of the cycloalkanoyl segment is (1*R*-*cis*), and the stereochemistry of the amidoacid is L.

**(1*R-cis*)-*N*-[[[3-(1-Methoxy-1-oxomethyl)]-2,2-dimethylcyclobutyl]-acetyl]-4-nitro-L-phenylalanine methyl ester (C**_{**20**}**H**_{**26**}**N**_{**2**}**O**_{**7**}**, F-1).** This compound is prepared from compound **D-3** by the procedure taught for **C-1:** TLC *R*_{*f*} = 0.36 (1:1 EtOAc/hexanes); ¹H NMR (CDCl₃, 300 MHz) δ 8.15 (m, 2H), 7.28 (m, 2H), 5.91 (m, 1H), 4.91 (m, 1H), 3.74 (s, **3H**), 3.65 (s, 3H), 3.29 (m, 1H), 3.16 (m, 1H), 2.73 (m, 1H), 2.37-2.03 (m, 4H), 1.92 (m, 1H), 1.18 (s, 3H), 0.83 (s, 3H); MS (FAB) m/z 407 (M+H)⁺.

**(1*R-cis*)-*N*-[[3-(1-Methoxy-1-oxomethyl)-2,2-dimethylcyclobutyl]-acetyl]-4-amino-L-phenylalanine methyl ester (C**_{**20**}**H**_{**28**}**N**_{**2**}**O**_{**5**}**, F-2).** This compound is prepared from compound **F-1** by the procedure taught for **A-4**: TLC *R*_{*f*} = 0.31 (3:1 EtOAc/hexanes); ¹H NMR (CDCl₃, 300 MHz) δ 6.87 (m, 2H), 6.64 (m, 2H), 5.81 (m, 1H), 4.79 (m, 1H), 3.78-3.28 (m, 2H), 3.71 (s, 3H), 3.65 (s, 3H), 2.99 (t, 2H, *J* = 5.6), 2.71 (dd, 1H, *J* = 10.3, 2.3), 2.33-2.02 (m, 4H), 1.91 (dd, 1H, *J* = 11.2, 10.3) , 1.18 (s, 3H), 0.88 (s, 3H); MS (FAB) *m*/*z* 377 (M+H)⁺, 345, 316, 195, 177, 135, 106, 95.

**(1*R*-*cis*)-*N*-[[3-(1-Methoxy-1-oxomethyl)-2,2-dimethylcyclobutyl)acetyl]-4-[(2,6-dichlorobenzoyl)amino]-L-phenylalanine methyl ester (C**_{**27**}**H**_{**30**}**Cl**_{**2**}**N**_{**2**}**O**_{**6**}**, F-3)**. This compound is prepared from compound **F-2** by the procedure taught for **A-5:** TLC *R*_{*f*} = 0.34 (1:1 EtOAc/hexanes); ¹H NMR (CDCl₃, 300 MHz) δ 7.57 (m, 2H), 7.29-7.17 (m, 3H), 7.01 (m, 2H),6.36 (s, 1H), 4.71 (m, 1H), 3.63 (s, 3H), 3.54 (s, 3H), 2.93 (m, 2H) , 2.61 (m, 1H), 2.24-1.78 (m, 5H), 1.16 (s, 3H), 0.77 (s, 3H).

**(1*R*-*cis*)-*N*-[[3-(1-Methoxy-1-oxomethyl)-2**,**2-dimethylcyclobutyl)acetyl]-4-[(2,6-dichlorobenzoyl)amino]-L-phenylalanine (C**_{**27**}**H**_{**30**}**Cl**_{**2**}**N**_{**2**}**O**_{**4**}**, F-4).** To a solution of diester **F-3** (0.603 g, 1.10 mmol) in 4:1 THF/MeOH (28 mL) at room temperature under Ar is added a solution of LiOH·H₂O (0.120 g, 2.86 mmol) in H₂O (5. 6 mL), followed by 30% aq H₂O₂ (1.2 mL). The reaction is stirred for 20 h. It is concentrated *in vacuo*, and the residue is taken up in H₂O (45 mL). The aqueous solution is acidified with aq 1 M HCl (12 mL), and extracted with EtOAc. The combined EtOAc extracts are washed with brine, dried, and concentrated to give **F-4** as an off-white foam: TLC *R*_{*f*} = 0.46 (750:250:5 EtOAc/hexanes/HCO₂H); ¹H NMR (CDCl₃, 300 MHz) δ 8.90 (s, 1H), 7.58 (m, 2H), 7.34-7.21 (m, 4H), 7.12 (m, 2H), 6.09 (m, 1H), 4.75 (m, 1H), 3.60 (s, 3H), 3.22-2.98 (m, 2H), 2.67 (m, 1H), 2.37-1.78 (m, 6H), 1.15 (s, 3H), 0.83 (s, 3H).

**(1*R*-*cis*)-*N*-[(3-Carboxy-2,2-dimethylcyclobutyl)acetyl]-4-[(2,6-dichlorobenzoyl)amino]-L-phenylalanine (C**_{**20**}**H**_{**26**}**Cl**_{**2**}**N**_{**2**}**O**_{**6**}**, Example 93).** To a solution of ester **F-4** (0.569 g, 1.10 mmol) in 4:1 THF/MeOH (28.3 mL) at room temperature under Ar is added a solution of LiOH·H₂O (0.120 g, 2.86 mmol) followed by 30% aq H₂O₂ (1.2 mL). The reaction is stirred for 90 h, at which time additional portions of LiOH·H₂O (0.120 g, 2.86 mmol) and 30% aq H₂O₂ (1.2 mL) are added. The reaction mixture is stirred for 18 h. It is concentrated *in vacuo*, and the residue is taken up in ice water (90 g). The solution is acidified with aq 1 M HCl and is extracted with EtOAc. The combined EtOAc extracts are washed with brine, dried, and concentrated to give a cream-colored solid. The solid is dissolved in MeCN (15 mL), and the solution is diluted with H₂O (15 mL). The solution is frozen and lyophilized to give **Example 93:** mp 138-141 °C; TLC *R*_{*f*} = 0.29 (750:250:5 EtOAc/hexanes/HCO₂H); ¹H NMR (CDCl₃, 300 MHz) δ 9.24 (s, 1H), 7.56 (m, 2H), 7.31-7.19 (m, 4H), 7.09, (m, 2H), 6.13 (m, 1H), 4.73 (m, 1H), 3.17-2.96 (m, 2H), 2.64 (m, 1H), 3.17-2.96 (m, 2H), 2.64 (m, 1H), 2.34-2.03 (m, 5H), 1.83 (m, 1H), 1.13 (s, 3H), 0.90 (s, 1H); MS (FAB) *m*/*z* 523, 521 (M+H)⁺; Anal. C 56.38, H 5.14, Cl 13.20, N 5.07 (calcd for 1.27% H₂O: C 58.86, H 5.11, Cl 13.43, N 5.30).

### Scheme 3

Another method for the preparation of general structure **1-F** is given in **Scheme 3** In this general scheme a heteroatom-substituted (W equals OH or NH₂) arylamidoester (or heteroarylamidoester) **3-A,** by standard synthetic methods is alkylated (W equals OH) or acylated (W equals NH₂) to give **3-B.** The *N*-terminus of **3-B** is deprotected to **3-C,** that is acylated with **1-A** to provide **3-D.** The functionality of R⁴ may be changed (such as by oxidation, reduction, substitution, epimerization, and /or degradation) by standard synthetic methods. Hydrolysis of **3-D** gives the amidoacid **1-F.**

### Examples 94 & 95: Teaching Scheme G

**Teaching Scheme G** exemplifies the practice of **Scheme 3** by teaching the synthesis of **Examples 94** and **95** where (with reference to structure **1-F**) for **Example 94** R⁴ is 3-carboxy, m equals 1, n equals 0, Zₓ and Z_{y} are CH, Y is -OCH₂-, X is 2,6-dichloro, the stereochemistry of the cycloalkanoyl segment is (1*S*-*trans*), and the stereochemistry of the amidoacid is L; and for **Example 95** R⁴ is 3-carboxy, m equals 1, n equals 0, Zₓ and Z_{y} are CH, Y is -OCH₂-, X is 2,6-dichloro, the stereochemistry of the cycloalkanoyl segment is (1*S*-*cis*), and the stereochemistry of the amidoacid is L. **Examples 94** and **95** exemplify changes in the functionality R⁴.

**(1*S*-*cis*)-3-Acetyl-2,2-dimethylcyclobutanecarboxylic acid (C**_{**9**}**H**_{**14**}**O**_{**3**}**, G-2)**. Compound **G-2** is prepared from commercial (-)-verbenone **G-1** as taught by Carlson *et al*. *(*Carlsen, P. H. J.; Katsuki, T.; Martin, V. S.; Sharpless, K. B. *J. Org. Chem*. **1981**, *46*, 3936-3938; **Carlsen**, P. H. J.; Odden, W. *Acta Chem. Scand*. **1984**, *38B*, 501-504) and Webster *et al*. (Webster, F. X.; Rivas-Enterrios, J.; Silverstein, R. M. *J. Org. Chem*. **1987**, *52*, 689-691). Compound **G-2**, obtained by these procedures, is purified by silica chromatography and crystallized from 4:1 hexanes/EtOAc to give **G-2** in excellent enantiomeric purity: mp 133-134 °C; TLC (750:250:2.5 EtOAc/hexanes/HCO₂H) *R*_{*f*} = 0.25; [α]_{D}²⁵ -73.7 (*c* 1.0, CHCl₃); ¹³C NMR (75 MHz, CDCl₃)δ 206.92, 177.94, 53.00, 44.99, 44.94, 30.29, 29.99, 18.81, 18.02; MS (FAB) *m*/*z* 171 [M + H]⁺; Anal. C 63.68, H 8.32 (calcd C 63.51, H 8.29).

**(1*S*-*cis*)-*N*-[(3-Acetyl-2,2-dimethylcyclobutyl)carbonyl]-4-[(2,6-dichlolophenyl)methoxy]-L-phenylalanine methyl ester (C**_{**26**}**H**_{**29**}**Cl**_{**2**}**NO**_{**5**}**, G-3):** Compound **G-3** is obtained from compound **G-2** and **Compound 7** as taught by the preparation of compound **A-3:** TLC (3:1 EtOAc/hexanes) *R*_{*f*} = 0.50; ¹H NMR (300 MHz, CDCl₃)δ 7.37 (m, 2H), 7.23 (m, 1H), 7.05 (m, 2H), 6.96 (m, 2H), 5.79 (m, 1H), 5.25 (s, 2H), 4.87 (m, 1H), 3.73 (s, 3H), 3.09 (m, 2H), 2.85 (m, 1H), 2.64 (m, 1H), 2.52 (m, 1H), 2.05 (s, 3H), 1.89 (m, 1H), 1.42 (s, 3H), 0.82 (s, 3H); MS (FAB) m/z 506 [M + H]⁺.

**(1*S*-*trans*)-*N*-[(3-Acetyl-2,2-dimethylcyclobutyl)carbonyl]-4-[(2,6-dichlorophenyl)methoxy]-L-phenylalanine (C**_{**25**}**H**_{**27**}**Cl**_{**2**}**NO**_{**5**}**, G-4)** and **(1*S-cis*)-*N*-[(3-Acetyl-2,2-dimethylcyclobutyl)carbonyl]-4-[(2,6-dichlorophenyl)methoxy]-L-phenylalanine (C**_{**25**}**H**_{**27**}**Cl**_{**2**}**NO**_{**5**}**, G-5)** : To a solution of **G-3** (1.23 mmol) in 4:1 THF/MeOH (32 mL), under Ar at room temperature, is added a solution of LiOH·H₂O (0.121 g, 2.87 mmol) in H₂O (6.3 mL). The reaction mixture is stirred at room temperature for 120 min. It is diluted with ice-cold H₂O (55 mL) and acidified with aq HCl to a pH of 2-3. The resulting mixture is extracted with EtOAc. The combined EtOAc portions are washed with brine, and are dried and concentrated to give a beige-colored foam. This material is purified by silica chromatography to separate **G-4** and **G-5**.

**G-4:** mp 84-86 °C; TLC (750:250:5 EtOAc/hexanes/HCO₂H) *R*_{*f*} = 0.43; ¹³C NMR (75 MHz, 10:1 CDCl₃/CD₃SOCD₃) δ C: 207.00, 173.01, 170.52, 162..38, 136.48, 136.26, 132.60, 132.23, 44.66; CH: 130.40, 129.86, 127.80, 120.21, 52.78, 52.62, 46.18; CH₂: 37.05, 18.76; CH₃: 30.44, 29.81, 17.48; MS (FAB) *m*/*z* 492 [M + H]⁺; Anal. C 60.79, H 5.56, Cl 13.94, N 2.81 (calcd for 0.57% H₂O: C 60.64, H 5.56, Cl 14.32, N 2.83).

**G-5:** mp 86-88 °C; TLC (750:250:5 EtOAc/hexanes/HCO₂H) *R*_{*f*} = 0.30; ¹³C NMR (75 MHz, 10:1 CDCl₃/CD₃SOCD₃) C: 208.12, 172.83, 171.84, 162.19, 136.64, 136.41, 132.22, 131.95, 42.42; CH: 131.87, 129.50, 128.60, 119.89, 53.13, 52.59, 46.45; CH₂: 36.69, 18.25; CH₃: 30.21, 24.93, 24.68; MS (FAB) *m*/*z* 492 [M + H]⁺; Anal. C 60.15, H 5.76, Cl 14.40, N 2.85 (calcd for 0.84% H₂O: C 60.47, H 5.58, Cl 14.28, N 2.82).

**(1*S*-*trans*)-*N*-[(3-Carboxy-2,2-dimethylcyclobutyl)carbonyl]-4- [(2,6-dichlorophenyl)methoxy]-L-phenylalanine (C**_{**24**}**H**_{**25**}**Cl**_{**2**}**NO**_{**6**}**, Example 94) :** Example 94 is prepared by haloform degradation of G-4 as taught for compound **C-1:** mp 94-96 °C; TLC (500:500:2.5 EtOAc/hexanes/HCO₂H) *R*_{*f*} = 0.33; [α]_{D}²⁵ +14 (c 0.96, MeOH) ; ¹H NMR (300 MHz, CDCl₃) δ 7.34 (m, 2H), 7.26-7.19 (m, 2H), 7.09 (m, 2H), 6.93 (m, 2H), 5.81 (m, 1H), 5.22 (s, 2H), 4.93 (m, 1H), 3.13 (m, 2H), 2.93 (m, 1H), 2.71 (m, 1H), 2.32 (m, 2H), 1.19 (s, 3H), 1.14 (s, 3H); MS (EI) *m*/*z* 493 (M⁺); Anal. C 54.85, H 5.20, Cl 13.39, N 2.65 (calcd for 2.65% H₂O: C 55.76, H 5.26, Cl 13.96, N 2.76).

**(1*S-cis*)-*N*-[(3-Carboxy-2,2-dimethylcyclobutyl)carbonyl]-4-[(2,6-dichlorophenyl)methoxy]-L-phenylalanine (C**_{**24**}**H**_{**25**}**Cl**_{**2**}**NO**_{**6**}**, Example 95): Example 95** is prepared by haloform degradation of **G-5** as taught for compound **C-1:** mp 116-118 °C; TLC (120:90:1 EtOAc/hexanes/HCO₂H) *R*_{*f*} = 0.27; ¹H NMR (300 MHz, CDCl₃) δ 7.31 (m, 2H), 7.22 (m, 3H), 7.09 (m, 2H), 6.89 (m, 2H), 6.00 (s, 1H), 5.18 (s, 2H), 4.71 (m, 1H), 3.07 (m, 2H), 2.58. (m, 3H), 2.08 (m, 3H) , 1.28 (s, 3H), 0.93 (s, 3H); ¹³C NMR (75 MHz, CDCl₃)δ 179.00, 178.09, 175.79, 162.48, 141.63, 136.84, 135.21, 135.18, 133.93, 133.18, 119.56, 69.88, 57.65, 51.40, 50.11, 48.62, 41.62, 35.11, 24.88, 22.71.

### Examples 96, 97 and 98: Teaching Scheme H

**Teaching Scheme H** exemplifies the practice of **Scheme 1** by teaching the synthesis of **Examples 96, 97** and **98** where (with reference to structure **1-F**) for **Example 96** R⁴ is 3-acetyl, m equals 1, n equals 0, Zₓ and Z_{y} are CH, Y is -NHC(O)-, X is 2,6-dichloro, the stereochemistry of the cycloalkanoyl segment is (1*S-trans*), and the stereochemistry of the amidoacid is L; for **Example 97** R⁴ is 3-acetyl, m equals 1, n equals 0, Zₓ and Z_{y} are CH, Y is -NHC(O)-, X is 2,6-dichloro, the stereochemistry of the cycloalkanoyl segment is (1*S*-*cis*), and the stereochemistry of the amidoacid is L; and for **Example 98** R⁴ is 3-carboxy, m equals 1, n equals 0, Zₓ and Z_{y} are CH, Y is -NHC(O)-, X is 2,6-dichloro, the stereochemistry of the cycloalkanoyl segment is (1*S-trans*), and the stereochemistry of the amidoacid is L.

**(1*S-cis*)-*N*-[(3-Acetyl-2,2-dimethylcyclobutyl)carbonyl]-4-nitro-L-phenylalanine methyl ester (C**_{**19**}**H**_{**24**}**N**_{**2**}**O**_{**6**}**, H-1) :** Compound **G-2** and **Compound 3** are coupled, as taught for compound **A-3,** to give compound **H-1:** ¹H NMR (300 MHz, CDCl₃) δ 8.16 (m, 2H) , 7.31 (m, 2H), 5.94 (m, 1H), 4.94 (m, 1H), 3.74 (s, 3H), 3.28 (m, 1H), 3.16 (m, 1H), 2.88 (m, 1H), 2.65 (m, 1H), 2.51 (m, 1H), 2.05 (s, 3H), 1.91 (m, 1H), 1.42 (s, 3H), 0.79 (s, 3H) ; ¹³C NMR (75 MHz, CDCl₃) δ 206.94, 171.56, 170.91, 147.20, 143.82, 130.13, 123.74, 52.88, 52.64, 52.49, 46.47, 44.70, 38.20, 30.74, 30.11, 18.85, 17.71.

**(1*S-cis*)*-N-*[(3-Acetyl-2,2-dimethylcyclobutyl)carbonyl]-4-amino-L-phenylalanine methyl ester (C**_{**19**}**H**_{**26**}**N**_{**2**}**O**_{**4**}**, H-2).** Compound **H-2** is prepared from compound **H-1** as taught for the preparation of compound **A-4:** TLC (3:1 EtOAc/hexanes) *R*_{*f*} = 0.28; ¹H NMR (300 MHz, CDCl₃) δ 6.89 (m, 2H) , 6.64 (m, 2H), 5.75 (m, 1H), 4.81 (m, 1H), 3.71 (s, 3H), 3.50 (m, 2H), 2.99 (m, 2H), 2.83 (m, 1H), 2.62 (m, 1H), 2.48 (m, 1H), 2.05 (s; 3H), 1.88 (m, 1H), 1.41 (s, 3H), 0.85 (s, 3H); ¹³C NMR (75 MHz, CDCl₃) δ 207.04, 172.26, 170.65, 145.12, 130.05, 125.64, 115.55, 52.98, 52.79, 52.23, 46.40, 44.87, 37.31, 30.65, 30.03, 19.05, 17.57; MS (FAB) *m*/*z* 347 [M + H]⁺.

**(1*S-cis*)-*N*-[(3-Acetyl-2,2-dimethylcyclobutyl)carbonyl]-4-[(2,6-dichlorobenzoyl)amino]-L-phenylalanine methyl ester (C**_{**26**}**H**_{**28**}**Cl**_{**2**}**N**_{**2**}**O**_{**5**}**, H-3):** Compound **H-3** is prepared from compound **H-2** as taught for the preparation of compound **A-5:** TLC (3:1 EtOAc/hexanes) *R*_{*f*} = 0.36; ¹H NMR (300 MHz, CDCl₃) δ 7.58 (m, 2H), 7.50 (s, 1H), 7.32 (m, 3H), 7.13 (m, 2H), 5.81 (m, 1H), 4.88 (m, 1H), 3.74 (s, 3H), 3.11 (m, 2H), 2.85 (m, 1H), 2.63 (m, 1H), 2.52 (m, 1H), 2.03 (s, 3H), 1.89 (m, 1H), 1.42 (s, 3H), 0.83 (s, 3H); MS (FAB) m/z 519 [M + H]⁺.

**(1*S-trans*)-*N*-[(3-Acetyl-2,2-dimethylcyclobutyl)carbonyl]-4-[(2,6-dichlorobenzoyl)amino]-L-phenylalanine (C**_{**25**}**H**_{**26**}**Cl**_{**2**}**N**_{**2**}**O**_{**5**}**, Example 96)** and **(1*S-cis*)-*N*-[(3-Acetyl-2,2-dimethylcyclobutyl)carbonyl]-4- [(2,6-dichlorobenzoyl)amino)-L-phenylalanine (C**_{**25**}**H**_{**26**}**Cl**_{**2**}**N**_{**2**}**O**_{**5**}**, Example 97).** The mixture of Examples **96** and **97,** prepared as taught for Examples **G-4** and **G-5**, are separated and purified.by silica chromatography.

**Example 96:** mp 146-148 °C; TLC (750:250:5 EtOAc/hexanes/HCO₂H) *R*_{*f*} = 0.28; [α]_{D}²⁵ +43 (*c* 0.86, MeOH); ¹³C NMR (75 MHz, CDCl₃)δ 208.12, 172.83, 171.84, 162.20, 136.64, 136.41, 132.22, 131.96, 129.98, 129.50, 127.48, 120.92, 119.89, 53.13, 52.59, 46.45, 42.42, 36.87, 30.21, 24.93, 24.68, 18.25; Anal. C 58.14, H 5.53, Cl, 13.36, N 5.22 (calcd for 1.59% H₂O: C 58.47, H 5.28, Cl 13.81, N 5.46).

**Example 97:** mp 158-160 °C; TLC (750:250:5 EtOAc/hexanes/HCO₂H) *R*_{*f*} = 0.16; [α]_{D}²⁵ -8 (c 0.92, MeOH); ¹³C NMR (75 MHz, CDCl₃) δ 207.00, 173.01, 170.52, 162.38, 136.48, 136.26, 132.60, 132.24, 130.40, 129.86, 127.80, 120.21, 115.22, 52.78, 52.62, 46.18, 44.66, 37.05, 30.44, 29.81, 18.76, 17.48; MS (FAB) *m*/*z* 505 [M + H]; Anal. C 58.14, H 5.53, Cl 13.36, N 5.22 (calcd for 1.39% H₂O: C 58.53, H 5.27, Cl 13.82, N 5.46).

**(1*S-trans*)*-N-*[(3-Carboxy-2,2-dimethylcyclobutyl)carbonyl]-4-[(2,6-dichlorobenzoyl)amino]-L-phenylalanine (C**_{**24**}**H**_{**24**}**Cl**_{**2**}**N**_{**2**}**O**_{**6**}**, Example 98). Example 98** is prepared by haloform degradation of **Example 96** as taught for compound **C-1:** mp 159-161 °C; TLC (750:250:5 EtOAc/hexanes/HCO₂H) *R*_{*f*} *=* 0.31; [α]_{D}²⁵ +21 (c 0.79, MeOH); ¹³C NMR (75 MHz, CDCl₃)δ 180.42, 177.94, 176.91, 167.31, 141.70, 141.47, 137.22, 137.11, 135.16, 134.68, 132.90, 132.63, 125.02, 57.62, 51.93, 50.82, 46.81, 41.82, 30.32, 29.59, 24.49; MS (FAB) *m*/*z* 507 [M + H]⁺; Anal. C 54.59, H 4.81, Cl 13.21, N 5.30 (calcd for 1.66% H₂O: C 55.91, H 4.87, Cl 13.75, N 5.43).

### Example 99: Teaching Scheme I

**Teaching Scheme I** exemplifies the practice of **Scheme 2** by teaching the synthesis of **Example 99** where (with reference to structure **1-F**) R⁴ is 3-carboxy, m equals 1, n equals 0, Zₓ and Z_{y} are CH, Y is -NHC (O)-, X is 2,6-dichloro, the stereochemistry of the cycloalkanoyl segment is (1*S-cis*), and the stereochemistry of the amidoacid is L.

**(1*S-cis*)-*N*-[[3-(1-Methoxy-1-oxomethyl)-2,2-dimethylcyclobutyl]carbonyl]-4-nitro-L-phenylalanine methyl ester (C**_{**19**}**H**_{**24**}**N**_{**2**}**O**_{**7**}**, I-1):** Compound **H-1** is subjected to haloform degradation, as taught for compound **C-1.** To a solution of the resulting diacid in EtOAc at 0 °C is added an ethereal CH₂N₂ solution. The resulting yellow solution is stirred at 0 °C for 15 min, after which the yellow color is dissipated by the passage of 'an N₂ stream. The solution is concentrated to provide, as a beige-colored foam, compound **I-1:** TLC (3:1 EtOAc/hexanes) *R*_{*f*} = 0.36; ¹H NMR (300 MHz, CDCl₃) δ 8.11 (m, 2H), 7.29 (m, 2H), 6.00 (m, 11H), 4.91 (m, 1H), 3.70 (s, 3H), 3.63 (s, 3H), 3.28 (m, 1H), 3.16 (m, 1H), 2.74 (m, 1H), 2.62 (m, 1H), 2.48 (m, 1H), 2.00 (m, 1H), 1.28 (s, 3H), 0.82 (s, 3H); MS (FAB) *m*/*z* 393 [M + H]⁺.

**(1*S-cis*)-4-Amino-*N*-[[3-(1-methoxy-1-oxomethyl)-2,2-dimethylcyclobutyl]-carbonyl]-L-phenylalanine methyl ester (C**_{**19**}**H**_{**26**}**N**_{**2**}**O**_{**5**}**, I-2):** Compound **I-2** is prepared from compound **I-1** as taught for the preparation of compound **A-4:** TLC (3:1 EtOAc/hexanes) *R*_{*f*} = 0.17; ¹H NMR (300 MHz, CDCl₃)δ 6.91 (m, 2H), 6.68 (m, 2H), 5.72 (m, 1H), 4.82 (m, 1H), 3.71 (s, 3H), 3.67 (s, 3H), 3.48 (s, 2H), 3.01 (m, 2H), 2.75 (m, 1H), 2.62 (m, 1H), 2.51 (m, 1H), 2.02 (m, 1H), 1.31 (s, 3H), 0.91 (s, 3H) ; ¹³C NMR (75 MHz, CDCl₃) δ 172.64, 172.24, 170.66, 144.26, 130.10, 126.28, 115.95, 52.84, 52.25, 51.36, 46.70, 45.22,'44.32, 37.29, 30.35, 20.18, 17.95; MS (FAB) *m*/*z* 363 [M + H]⁺.

**(1*S-cis*)-4-[(Dichlorobenzoyl)amino]-*N*-[[3-(1-methoxy-1-oxomethyl)-2,2-dimethylcyclobutyl]carbonyl]-L-phenylalanine methyl ester (C**_{**26**}**H**_{**28**}**Cl**_{**2**}**N**_{**2**}**O**_{**6**}**, I-3):** Compound **I-3** is prepared from compound **I-2** as taught for the preparation of compound **A-5:** TLC (3:1 EtOAc/hexanes) *R*_{*f*} = 0.34; ¹H NMR (300 MHz, CDCl₃) δ 8.17 (s, 1H), 7.57 (m, 2H), 7.30 (m, 2H), 7.07 (m, 2H), 5.84 (m, 1H), 4.83 (m, 1H), 3.70 (s, 3H), 3.62 (s, 3H), 3.07 (m, 2H), 2.72 (m, 1H), 2.53 (m, 2H), 1.28 (s, 3H), 0.85 (s, 3H).

**(1*S*-*cis*)-*N*-[(3-Carboxy-2,2-dimethylcyclobutyl)carbonyl]-4-[(2,6-dichlorobenzoyl)amino]-L-phenylalanine(C**_{**24**}**H**_{**24**}**Cl**_{**2**}**N**_{**2**}**O**_{**6**}**, Example 99) : Example 99** is prepared by haloform degradation of **I-3** as taught for compound **C-1:** mp 154-156 °C; TLC (750:250:5 EtOAc/hexanes/HCO₂H) *R*_{*f*} = 0.26; [α]_{D}²⁵ +15 (*c* 0.85, MeOH); ¹³C NMR (75 MHz, CDCl₃)δ 174.28, 173.17, 171.09, 162.56, 136.90, 136.71, 132.65, 132.37, 130.40, 129.91 (2C), 127.88, 120.32, 61.29, 52.88, 46.67, 45.39, 43.84, 37.17, 30.41, 20.05, 18.05; MS (FAB) *m*/*z* 507 [M + H]⁺; Anal. C 55.91, H 5.22, Cl 13.01, N 5.18 (calcd for 1.76% H₂O: C 55.82, H 4.88, Cl 13.73, N 5.42).

### Examples 100-103: Teaching Scheme J

**Teaching Scheme J** exemplifies the practice of **Scheme 3** by teaching the synthesis of **Examples 100, 101, 102,** and **103** where (with reference to structure **1-F)** for **Example 100** R⁴ is 3-acetyl, m equals 1, n equals 0, Zₓ and Z_{y} are CH, Y is -NHC(O)-, X is 2,6-dichloro, the stereochemistry of the cycloalkanoyl segment is (1*R-trans*), and the stereochemistry of the amidoacid is L; for **Example 101** R⁴ is 3-acetyl, m equals 1, n equals 0, Zₓ and Z_{y} are CH, Y is -NHC(O)-, X is 2, 6-dichloro, the stereochemistry of the cycloalkanoyl segment is (1*R*-*cis*), and the stereochemistry of the amidoacid is L; for **Example 102** R⁴ is 3-carboxy, m equals 1, n equals 0, Zₓ and Z_{y} are CH, Y is -NHC(O)-, X is 2,6-dichloro, the stereochemistry of the cycloalkanoyl segment is (1*R*-*trans*), and the stereochemistry of the amidcacid is L; and for **Example 103** R⁴ is 3-carboxy, m equals 1, n equals 0, Zₓ and Z_{y} are CH, Y is -NHC(O)-, X is 2,6-dichloro, the stereochemistry of the cycloalkanoyl segment is (1*R*-*cis*), and the stereochemistry of the amidoacid is L.

**(1*R*)-(+)-4,6,6-Trimethylbicyclo[3.1.1]hept-3-en-2-one [18309-32-5] (C**_{**10**}**H**_{**14**}**O, J-1)** : (1*R*)-Verbenone was prepared from. (1*R*)-(+)-α-pinene (97% ee) as described by Sivik *et al*. ( Sivik, M. R.; Stanton, K. J.; Paquette, L. A. *Org. Synth.* **1993**, *72*, 57-61) : TLC (10:1 hexanes/EtOAc) *R*_{*f*} = 0.23; [α]²⁵_{D} +243 (*c* 10.2, EtOH).

**(1*R*-*cis*)-3-Acetyl-2,2-dimethylcyclobutanecarboxylic acid [22571-78-4] (C**_{**9**}**H**_{**14**}**O**_{**3**}**, J-2)**: Compound **J-2** is prepared from **J-1** by the procedures cited in the preparation of compound **G-2**: mp 134-135 °C; [α]_{D}²⁵ +73 (c 1.01, CHCl₃); MS (FAB) m/z 171 [M + H]⁺; Anal. C 63.59, H 8.26 (calcd C 63.51, H 8.29).

**(1*R*-*cis*)-*N*-[(3-Acetyl-2,2-dimethylcyclobutyl)carbonyl]-4-[(2,6-dichlorobenzoyl)amino]-L-phenylalanine methyl ester (C**_{**26**}**H**_{**28**}**Cl**_{**2**}**N**_{**2**}**O**_{**5**}**, J-3)**: Compound **J-3** is obtained from compound **J-2** and **Compound 6** as taught by the preparation of compound **A-3:** TLC (3:1 EtOAc/hexanes) *R*_{*f*} = 0.50; ¹H NMR (300 MHz, CDCl₃)δ 9.37 (s, 1H), 7.59 (m, 2H), 7.26 (m, 3H), 7.05 (m, 2H), 6.08 (m; 1H), 4.79 (m, 1H), 3.67 (s, 3H), 3. 06 (m, 1H), 2.96 (m, 1H), 2.78 (m, 1H), 2.51 (m, 2H), 1.98 (s, 3H), 1.79 (m, 1H), 1.32 (s, 3H), 0.71 (s, 3H); MS (EI) *m*/*z* 516 (M⁺).

**(1*R*-*trans*)-N- [(3-Acetyl-2,2-dimethylcyclobutyl]carbonyl]-4- [(2,6-dichlorobenzoyl)amino]-L-phenylalanine (C**_{**25**}**H**_{**26**}**Cl**_{**2**}**N**_{**2**}**O**_{**5**}**, Example 100)** and **(1*R*-*cis*)-*N*-[(3-Acetyl-2,2-dimethyleyclobutyl)carbonyl]-4-[(2,6-dichlorobenzoyl)-amino]-L-phenylalanine (C**_{**25**}**H**_{**26**}**Cl**_{**2**}**N**_{**2**}**O**_{**5**}**, Example 101) :** These compounds are prepared and separated as taught for the preparation of compounds **G-4** and **G-5.**

**Example 100:** mp 199-196 °C; TLC (750:250:5 EtOAc/hexanes/HCO₂H) *R*_{*f*} = 0.49; [α]_{D}²⁵ -18 (*c* 0.92, MeOH); ¹H NMR (300 MHz, CDCl₃) δ 9.63 (s, 1H), 7.51 (m, 2H), 7.20 (m, 3H) , 7.06 (m, 2H), 6.25 (m, 1H), 4.71 (m, 1H), 3.00 (m, 3H), 2.42 (m, 2H), 2.20 (m, 1H), 2.01 (m, 1H), 1.91 (s, 3H), 0.98 (s, 3H)., 0.93 (s, 3H); MS (EI) m/z 504 (M⁺); Anal. C 58.80, H 5.14, Cl 13.05, N 5.54 (calcd for 1.46% H₂O: C 58.56, H 5.28, Cl 13.80, N 5.45).

**Example 101:** mp 208-209 °C; TLC (600:400:5 EtOAc/hexanes/HCO₂H) *R*_{*f*} *=* 0.35; [α]_{D}²⁵ +59 (*c* 0.96, MeOH); ¹H NMR (300 MHz, CDCl₃)δ 8.89 (s, 1H), 7.57 (m, 2H), 7.28 (m, 2H), 7.12 (m, 2H), 6.01 (m, 1H), 4.79 (m, 1H), 3.09 (m, 2H), 2.79 (m, 1H), 2.55 (m, 2H), 1.97 (s, 3H), 1.81 (m, 2H) 1.34 (s, 3H), 0.76 (s, 3H); MS (FAB) m/z 505 [M + H]⁺; Anal. C 59.17, H 5.56, Cl 12.79, N 6.55 (calcd for 0.50% H₂O: C 59.28, H 5.50, Cl 12.96, N 6.40).

**(1*R*-*trans*)-*N*-[(3-Carboxy-2,2-dimethylcyclobutyl)carbonyl]-4-[(2,6-dichlorobenzoyl)amino]-L-phenylalanine (C**_{**24**}**H**_{**24**}**Cl**_{**2**}**N**_{**2**}**O**_{**6**}**, Example 102) : Example 100** is subjected to haloform degradation, as taught for compound **C-1,** to give **Example 102:** TLC (750:250:5 EtOAc/hexanes/HCO₂H) *R*_{*f*} = 0.29; [α]_{D}²⁵ +2 (*c* 0. 91, MeOH); ¹H NMR (300 MHz, CDCl₃)δ 10.05 (s, 1H), 7.65 (m, 2H) , 7.40-7.29 (m, 3H) , 7.19 (m, 2H), 6.53 (m, 1H), 4.81 (m, 1H), 4.10 (m, 1H), 3.10 (m, 3H), 2.85 (m, 1H), 2.73 (m, 1H), 2.25 (m, 2H), 1.16 (s, 3H), 1.02 (s, 3H); MS (FAB) *m*/*z* 507 [M + H]⁺; Anal. C 55.74, H 4.91, Cl 13.30, N 5.28 (calcd for 0.94% H₂O: C 56.28, H 4.83, Cl 13.84, N 5.45).

**(1*R*-*cis*)-*N*-[(3-Carboxy-2,2-dimethylcyclobutyl)carbonyl]-4-[(2,6-dichlorobenzoyl)amino]-L-phenylalanine (C**_{**24**}**H**_{**24**}**Cl**_{**2**}**N**_{**2**}**O**_{**6**}**, Example 103). Example 101** is subjected to haloform degradation, as taught for compound **C-1,** to give **Example 103**: TLC (600:400:5 EtOAc/hexanes/HCO₂H) *R*_{*f*} = 0.24; [α]_{D}²⁵ +34 (*c* 0.90, MeOH); ¹H NMR (300 MHz, CDCl₃)δ 9.53 (s, 1H), 7.54 (m, 2H), 7.28-7.17 (m, 3H), 7.07 (m, 2H), 6.13 (m, 1H), 4.72 (m. 1H), 4.02 (m, 1H), 3.02 (m, 2H), 2.66-2.35 (m, 4H), 1.89 (m, 1H), 1.20 (s, 3H), 0.83 (s, 3H); MS (FAB) *m*/*z* 507 [M + H]⁺; Anal. C 52.81, H 4.59, Cl 12.41, N 4.85 (calcd for 2.1% H₂O: C 55.62, H 4.91, Cl 13.67, N 5.40).

### Examples 104 and 105: Teaching Scheme K

**Teaching Scheme K** exemplifies the practice of **Scheme 3** by teaching the synthesis of **Examples 104** and **105** where (with reference to structure **1-F**) for **Example 104** R⁴ is 3-acetyl, m equals 1, n equals 0, Zₓ is CH, Z_{y} is 3-NH, Y is -NHC(O)-, X is 2-chloro, the stereochemistry of the cycloalkanoyl segment is (1*S-trans*), and the stereochemistry of the amidoacid is L; and for **Example 105** R⁴ is 3-acetyl, m equals 1, n equals 0, Zₓ is CH, Z_{y} is 3-NH, Y is -NHC(O)-, X is 2-chloro, the stereochemistry of the cycloalkanoyl segment is (1*S*-*cis*), and the stereochemistry of the amidoacid is L.

***N*-[(1,1-Dimethylethoxy)carbonyl]-4-[[(2-chloro-3-pyridinyl) carbonyl]-amino]-L-phenylalanine methyl ester (C**_{**22**}**H**_{**24**}**ClN**_{**3**}**O**_{**5**}**, K-1).** To a mixture of 2-chloro-3-pyridinecarboxylic acid (1.36 g, 8.48 mmol), EDC (1.73 g, 8.85 mmol), HOBT (1.204 g, 8.87 mmol), amine **Compound 5** (2.60 g, 8.85 mmol) and DMAP (0.323 g, 2.6 mmol) in CH₂Cl₂ (55 mL) under N₂ at 0 °C is added Et₃N (1.44 mL, 10.22 mmol). The reaction mixture is stirred for 44 h at rt. Additional portions of acid 2-chloro-3-pyridinecarboxylic acid (1.36 g, 8.48 mmol), EDC (1.73 g, 8.85 mmol), HOBT (1.204 g, 8.87 mmol) and DMAP (0.323 g, 2.6 mmol) and Et3N (1.44 mL, 10.22 mmol) are added. The reaction is stirred an additional 15 h. The reaction mixture is diluted with CH₂Cl₂, and washed with H₂O, 5% aq NaHCO₃, and H₂O. The organics are dried and concentrated to give an amber oil, that solidifies upon standing. The solid is purified by silica chromatography to give **K-1:** TLC *R*_{*f*} = 0.21 (1:1 EtOAc/hexane); ¹H NMR (CDCl₃, 300 MHz) δ 8.51 (s, 1H), 8.18 (m, 2H), 7.58 (m, 2H), 7.41 (m, 1H), 7.14 (m, 2H), 5.07-4.96 (m, 2H), 4.64-4.56 (m, 1H), 3.73 (s, 3H), 3.16-2.97 (m, 2H), 1.42 (s, 9H); MS (FAB) *m*/*z* 434 [M+H]⁺, 436.

**4-[[(2-Chloro-3-pyridinyl)carbonyl]amino]-L-phenylalanine methyl ester hydrogen chloride salt (C**_{**16**}**H**_{**16**}**ClN**_{**3**}**O**_{**3**}**·HCl, K-2). A** solution of **K-1** (3.37 g, 7.77 mmol) in 4 M HCl in dioxane is stirred at room temperature for 20 h. The reaction mixture is concentrated in *vacuo*. The solid is taken up in H₂O and extracted with Et₂O. The Et₂O is discarded. The aqueous solution is frozen and lyophilized to give **K-2** as a solid: ¹H NMR (CDCl₃, 300 MHz) δ 10.74 (s, 1H), 8.64 (s, 3H), 8.52 (m, 1H), 8.04 (m, 1H), 7.65 (m, 2H), 7.55 (m, 1H), 7.21 (m, 2H), 4.23 (m, 1H), 3.23-3.05 (m, 2H); MS (EI) *m*/*z* 333 [M]⁺.

**(1*S-cis*)-*N*-[(3-Acetyl-2,2-dimethylcyclobutyl)carbonyl]-4-[[(2-chloro-3-pyridinyl)carbonyl]amino]-L-phenylalanine methyl ester (C**_{**25**}**H**_{**28**}**ClN**_{**3**}**O**_{**5**}**, K-3).** Compound **K-3** is prepared from acid **G-2** and **K-2** as taught for **A-3:** TLC *R*_{*f*} = 0.35 (EtOAc); ¹H NMR (CDCl₃, 300 MHz) δ 8.51 (m, 1H), 8.20 (m,. 2H) , 7.59 (m, 2H) , 7.41 (m, 1H), 7.14 (m, 2H), 5.83 (m, 1H), 4.88 (m, 1H), 3.73 (s, 3H), 3.11 (m, 2H), 2.64 (m, 1H), 2.51 (m, 1H), 2.05 (s, 3H), 1.89 (m, 1H), 1.42 (s, 3H), 0.84 (s, 3H); ¹³C NMR (CDCl₃, 75 MHz) δ 207.12, 172.01, 170.76, 162.55, 151.39, 146.96, 140.04, 136.26, 132.83, 131.36, 130.00, 122.99, 120.46, 52.94, 52.72, 52.46, 46.40, 44.84, 37.61, 30.70, 30.10, 18.93, 17.68; MS (FAB) *m*/*z* 486 [M + H]⁺.

**(1*S-trans*)-*N*-[(3-Acetyl-2,2-dimethylcyclobutyl)carbonyl]-4-[[(2-chloro-3-pyridinyl)carbonyl]amino]-L-phenylalanine (C**_{**24**}**H**_{**26**}**ClN**_{**3**}**O**_{**5**}**, Example 104)** and **(1*S-cis*)-*N*-[(3-Acetyl-2,2-dimethylcyclobutyl)carbonyl]-4-[[(2-chloro-3-pyridinyl)carbonyl]amino]-L-phenylalanine (C**_{**24**}**H**_{**26**}**ClN**_{**3**}**O**_{**5**}**, Example 105). Examples 104** and **105** are prepared and separated as taught for Compounds **G-4** and **G-5.**

**Example 104:** mp 115-117 °C; TLC *R*_{*f*} *=* 0.41 (1000:2.5 EtOAc/HCO₂H); [α]²⁵_{D} = +42 (c = 1.0, MeOH); ¹H NMR (CDCl₃, 300 MHz) δ 9.47 (s, 1H), 8.41 (m, 1H), 7.90 (m, 1H), 7.55 (m, 2H), 7.28 (m, 1H), 7.09 (m, 2H), 6.00 (m, 1H), 4.77 (m, 1H), 3.07 (m, 2H), 2.46 (m, 1H), 2.28 (m, 1H), 2.07 (m, 1H), 1.98 (s; 3H), 1.14 (s, 3H), 1.01 (s, 3H); ¹³C NMR (CDCl₃, 75 MHz) δ 208.67, 173.25, 172.22, 163.52, 150.49, 147.51, 138.58, 136.90, 132.99, 132.79, 130.01, 122.55, 120.22, 53.51, 52.93, 46.95, 42.84, 37.12, 30.71, 25.34, 25.14, 18.67; MS (ESI-) *m*/*z* 470.1 [M - H]⁻; Anal. C 59.11, H 5.91, Cl 6.68, N 8.32 (calcd for 2.81% H₂O and melt solvate: C 59.36, H 5.72, Cl 7.29, N 8.65).

**Example 105:** mp 129-130 °C; TLC *R*_{*f*} = 0.31 (1000:2.5 EtOAc/HCO₂H); [α]²⁵_{D} = -7 (c = 1.0, MeOH); ¹H NMR (CDCl₃, 300 MHz) δ 9.56 (s, 1H), 8.39 (m, 1H), 7.88 (m, 1H), 7.54 (m, 2H), 7.28 (m, 1H), 7.08 (m, 2H), 4.71 (m, 1H), 3.04 (m, 2H), 2.76 (m, 1H), 2.49 (m, 2H) , 1. 95 (s, 3H), 1.77 (m, 1H), 1.33 (s, 3H), 0.77 (s, 3H); ¹³C NMR (CDCl₃, 75 MHz) δ 207.18, 173.21, 170.67, 163.56, 150.43, 147.53, 138.50, 136.95, 133.06, 132.74, 129.96, 122.53, 120.23, 52.92, 52.80, 46.29, 44.81, 37.21, 30.59, 30.02, 18.55, 17.65; MS (ESI-) m/z 470.0 [M - H]⁻; Anal. C 57.17, H 5.67, Cl 7.17, N 8.55 (calcd for 2.61% H₂O and melt solvate: C 59.19, H 5.70, Cl 7.28, N 8.62).

### Examples 106 and 107: Teaching Scheme L

**Teaching Scheme L** exemplifies the practice of **Scheme 3** by teaching the synthesis of **Examples 106** and **107** where (with reference to structure **1-F**) for **Example 106** R⁴ is 3-acetyl, m equals 1, n equals 0, Zₓ is CH, Z_{y} is 3-NH, Y is -NHC(O)-, X is 2,6-dichloro, the stereochemistry of the cycloalkanoyl segment is (1*S*-*trans*), and the stereochemistry of the amidoacid is L; and for **Example 105** R⁴ is 3-acetyl, m equals 1, n equals 0, Zₓ is CH, Z_{y} is 3-NH, Y is -NHC(O)-, X is 2,6-dichloro, the stereochemistry of the cycloalkanoyl segment is (1*S-cis*), and the stereochemistry of the amidoacid is L.

***N*-[(1,1-Dimethylethoxy)carbonyl]-4-[[(2,6-dichloro-3-pyridinyl)carbonyl]amino]-L-phenylalanine methyl ester (C**_{**21**}**H**_{**23**}**Cl**_{**2**}**N**_{**3**}**O**_{**5**}**, L-1).** A mixture of 2,6-dichloro-3-pyridinecarboxylic acid and **Compound 5** are coupled by the procedure taught for **A-5:** TLC: *R*_{*f*} = 0.42 (1:1 EtOAc/hexanes); ¹H NMR (CDCl₃, 300 MHz) δ 8.14 (m, 1H), 7.56 (m, 2H), 7.42 (m, 1H), 7.14 (m, 2H), 4.99 (m, 1H), 4.58 (m, 1H), 3.72 (s, 3H), 3.17-2.96 (m, 1H), 1.41 (s, 9H); MS (FAB) *m*/*z* 470, 468 [M+H]⁺.

**4-[[(2,6-Dichloro-3-pyridinyl)carbonyl]amino]-L-phenylalanine methyl ester hydrogen chloride salt (C**_{**16**}**H**_{**15**}**Cl**_{**2**}**N**_{**3**}**O**_{**3**}**·HCl, L-2). A** solution of **L-1** is transformed to **L-2** by the procedure taught for **K-2:** ¹H NMR (CDCl₃, 300 MHz) δ 10.84 (s, 1H), 8.60 (s, 3H), 8.14 (m, 1H), 7.72 (m), 7.63 (m, 2H), 7.22 (m, 2H), 4.29-4.17 (m, 1H), 3.69 (s, 3H), 3.19-3.0.6 (m, 2H); MS (ESI+) *m*/*z* 370, 368 [M+H]⁺.

**(1*S*-*cis*)-*N*-[(3-Acetyl-2,2-dimethylcyclobutyl)carbonyl]-4-[[(2,6-dichloro-3-pyridinyl)carbonyl]amino]-L-phenylalanine methyl ester (C**_{**25**}**H**_{**27**}**Cl**_{**2**}**N**_{**3**}**O**_{**5**}**, L-3).** Compound **L-3** is prepared from acid **G-2** and **L-2** as taught for **A-3:** TLC *R*_{*f*} = 0.34 (3:1 EtOAc/hexanes); ¹H NMR (CDCl₃, 300 MHz) δ 8.26 (s, 1H), 8.16 (m, 1H), 7.57 (m, 2H), 7.42 (m, 1H), 7.13 (m, 2H), 5.83 (m, 1H), 4.87 (m, 1H), 3.73 (s, 3H), 3.11 (m, 2H), 2.86 (m, 1H), 2.63 (m, 1H), 2.53 (m, 1H), 2.05 (s, 3H), 1.64 (m, 1H), 1.42 (s, 3H), 0.82 (s, 3H).

**(1*S*-*trans*)-*N*-[(3-Acetyl-2,2-dimethylcyclobutyl)carbonyl]-4-[[(2,6-dichloro-3-pyridinyl) carbonyl] amino]-L-phenylalanine (C**_{**24**}**H**_{**25**}**Cl**_{**2**}**N**_{**3**}**O**_{**5**}**, Example 106)** and **(1*S*-*cis*)-*N*-[(3-Acetyl-2,2-dimethylcyclobutyl)carbonyl]-4-[[(2,6-dichloro-3-pyridinyl)carbonyl]amino]-L-phenylalanine (C**_{**24**}**H**_{**25**}**Cl**_{**2**}**N**_{**3**}**O**_{**5**}**, Example 107).**
**Examples 106** and **107** are prepared and separated as taught for Compounds **G-4** and **G-5.**

**Example 106:** mp 139-141 °C; TLC *R*_{*f*} = 0.28 (750:250:5 EtOAc/hexanes/HCO₂H); [α]²⁵_{D} = +39 (c = 0.7, MeOH); ¹H NMR (CD₃SOCD₃, 300 MHz) δ 12.55 (s, 1H), 10.63 (s, 1H), 8.14 (m, 1H), 8.01 (m, 1H), 7.71 (m, 1H), 7.55 (m, 2H), 7.19 (m, 2H), 4.45 (m, 1H), 3.30 (s, 1H), 3.00 (m, 2H), 2.82 (m, 1H), 2.08 (m, 1H), 1.97 (s, 3H), 1.84 (m, 1H), 1.07 (s, 3H), 1.00 (s, 3H); MS (FAB) m/z 506 [M + H]⁺; Anal. C 55.88, H 5.26, Cl 13.04, N 7.92 (calcd for 2.26% H₂O: C 55.64, H 5.12, Cl 13.69, N 8.11).

**Example 107:** mp 189-191 °C; TLC *R*_{*f*} = 0.21 (750:250:5 EtOAc/hexanes/HCO₂H); [α]²⁵_{D} = -3 (c = 0.8, MeOH); ¹H NMR (CDCl₃, 300 MHz) δ 12.56 (s, 1H), 10.63 (s, 1H), 8.14 (m, 1H), 7.78 (m, 1H), 7.70 (m, 1H), 7.54 (m, 2H), 7.20 (m, 2H), 4.42 (m, 1H), 3.01 (m, 1H), 2.87 (m, 2H), 2.61 (m, 1H), 2.36 (m, 1H), 1.95 (s, 3H), 1.54 (m, 1H), 1.34 (s, 3H), 0.64 (s, 3H); MS (FAB) *m*/*z* 506 [M + H]⁺; Anal. C 56.29, H 5.57, Cl 12.77, N 7.93 (calcd for 2.26% H₂O: C 55.64, H 5.21, Cl 13.69, N 8.11).

### Example 108

From **Example 107** is prepared **Example 108,** wherein R⁴ is 3-carboxy, m equals 1, n equals 0, Zₓ is CH, Z_{y} is 3-NH, Y is -NHC(O)-, X is 2,6-dichloro, the stereochemistry of the cycloalkanoyl segment is (1*S*-*cis*), and the stereochemistry of the amidoacid is L.

**(1*S-cis*)*-N-* [(3-Carboxy-2,2-dimethylcyclobutyl)carbonyl] -4- [[(2,6-dichloro-3-pyridinyl)carbonyl]amino]-L-phenylalanine (C**_{**23**}**H**_{**23**}**Cl**_{**2**}**N**_{**3**}**O**_{**6**}**, Example 108). Example 108** is made from **Example 107** as taught for Compound **C-1:** mp 154-157 °C; TLC *R*_{*f*} = 0.19 (750:250:5 EtOAc/hexanes/HCO₂H); [α]²⁵_{D} = +15 (*c* = 0.9, MeOH); ¹H NMR (CD₃SOCD₃, 300 MHz) δ 12.27 (s, 1H), 10.56 (s, 1H), 8.14 (m, 1H), 7.81 (m, 1H), 7.71 (m, 1H), 7.55 (m, 2H), 7.21 (m, 2H), 4.43 (m, 1H), 3.01 (m, 1H), 2.84 (m, 1H), 2.68 (m, 1H), 2.34 (m, 1H), 1.68 (m, 1H), 1.23 (s, 3H), 0.76 (s, 3H); MS (FAB) *m*/*z* 508 [M + H)⁺; Anal. C 52.49, H 4.97, Cl 12.49, N 7.62 (calcd for 2.29% H₂O: C 53.10, H 4.71, Cl 13.63, N 8.08).

### Example 109: Teaching Scheme M

**Teaching Scheme M** exemplifies the practice of **Scheme 3** by teaching the synthesis of **Example 109** where (with reference to structure **1-F)** R⁴ is 3-carboxy, m equals 1, n equals 0, Zₓ is 2-NH, Z_{y} is CH, Y is -OCH₂-, X is 2,6-dichloro, the stereochemistry of the cycloalkanoyl segment is (1*S-cis*), and the stereochemistry of the amidoacid is L.

**(±)-2-Chloro-3-[(2-tetrahydropyranyl)oxy]-6-iodopyridine (C**_{**10**}**H**_{**11**}**ClINO**_{**2**}**, M-2):** To a solution of 2-chloro-6-iodo-3-pyridinol (Wishka, D. G.; Graber, D. R.; Seest, E. P.; Dolak, L. A.; Han, F.; Watt, W.; Morris, J. *J. Org. Chem*. **1998,** *63*, 7851-7859) **M-1** (1.00 g, 3.91 mmol) and dihydropyran (1.0 mL, 10.6 mmol) in CH₂Cl₂ (10 mL) under Ar at room temperature is added pyridinium chloride (0.050 g). The reaction mixture is stirred for 72 h. It is diluted with CH₂Cl₂ and is washed with satd aq NaHCO₃ and brine. The CH₂Cl₂ solution is dried and concentrated to an oil, that is purified by silica chromatography: TLC (19:1 hexanes/EtOAc) *R*_{*f*} = 0.24; ¹H NMR (CDCl₃, 300 MHz) δ 7.55 (m, 1H), 7.17 (m, 1H), 5.50 (m, 1H), 3.77 (m, 1H), 3.61 (m, 1H), 2.07-1.57 (m, 6H); MS (+ESI) *m*/*z* 361.9 [M + Na]⁺, 339.9 [M + H]⁺.

**(*S*)-2-Chloro-α-[[(1,1-dimethylethoxy)carbonyl]amino]-3-[(2-tetrahydropyranyl)oxy]-6-pyridinepropanoic acid methyl ester (C**_{**19**}**H**_{**27**}**ClN**_{**2**}**O**_{**6**}**, M-3) :** To an amberized flask containing activated Zn dust (0.349 g, 5.51 mmol) under Ar is added THF (2 mL) and 1,2-dibromoethane (0.018 mL, 0.21 mmol). The suspension is brought to reflux for several minutes, cooled to approximately 30 °C, and TMSCl (0.17 mL of a 1 M solution in THF) is added. The reaction mixture is stirred at 40±5 °C for 30 min. A degassed solution of Boc-L-iodoalanine methyl ester [93267-04-0] (1.814 g, 5.51 mmol) in 11:7 dimethylacetamide/THF (9.0 mL) is added to the suspension. The reaction mixture is stirred for 5 h at 45±5 °C. It is then cooled in an ice bath, and solid PdCl₂ (PPh₃)₂ (0.192 g) is added, followed by a degassed solution of the iodide **M-2** (0.936 g, 2.76 mmol) in 1:1 THF/dimethylacetamide (9.4 mL). This reaction mixture is stirred for 11 h at 45±5 °C. It is cooled to 0 °C, quenched with satd aq NH₄Cl, and extracted with EtOAc. The combined EtOAc portions are washed with satd aq NH₄Cl and brine, dried and concentrated to a green-yellow colored foam that is purified by silica chromatography: TLC (7:3 hexanes/EtOAc) *R*_{*f*} = 0.21; ¹H NMR (CDCl₃, 300 MHz) δ 7.39 (m, 1H), 7.00 (m, 1H), 5.46 (m, 1H), 4.61 (m, 1H), 4.13 (m, 1H), 3.80 (s, 3H), 3.62 (m, 1H), 3.20 (m, 1H), 2.13-1.53 (m, 6H), 1.42 (s, 9H); MS (+ESI) *m*/*z* 474.0 [M + H]⁺.

**(*S*)-α-[[(1,1-Dimethylethoxy)carbonyl]amino]-3-[(2-tetrahydropyranyl)oxy]-6-pyridinegropanoic acid methyl ester (C**_{**19**}**H**_{**28**}**N**_{**2**}**O**_{**6**}**, M-4):** A suspension of pre-reduced Pd/CaCO₃ (3.5 g) and **M-3** (1.15 g, 2.77 mmol) in EtOH (40 mL) is hydrogenated (30 psi H₂) for 19 h at rt. The mixture is filtered, and the filtrate is evaporated to give a yellow-colored foam, that is purified by silica chromatography: TLC (1:1 hexanes/EtOAc) *R*_{*f*} = 0.27; ¹H NMR (CDCl₃, 300 MHz) δ 8.30 (m, 1H), 7.29 (m, 1H), 7.03 (m, 1H), 5.81 (m, 1H), 5.39 (s, 1H), 4.65 (m, 1H), 3.86 (m, 1H), 3.73 (s, 3H), 3.62 (m, 1H), 3.21 (m, 2H), 1.96-1.53 (m, 6H), 1.42 (s, 9H); MS (+ESI) *m*/*z* 381.1 [M + H]⁺.

**(*S*)-α-[[(1,1-Dimethylethoxy)carbonyl]amino]-5-hydroxy-2-pyridinepropanoic acid methyl ester (C**_{**14**}**H**_{**20**}**N**_{**2**}**O**_{**5**}**, M-5):** A solution of **M-4** (0.346 g, 0.91 mmol) and pyridinium *p*-toluenesulfonate (0.031 g, 0.12 mmol) in EtOH (8 mL) is stirred at 55±5 °C for 20 h. The reaction mixture is cooled to room temperature, and concentrated *in vacuo*. The residue is taken up in EtOAc. This solution is washed with brine, dried, and concentrated to a pale yellow-colored oil that is purified by silica chromatography: TLC (1:1 hexanes/EtOAc) *R*_{*f*} = 0.18; ¹H NMR (CDCl₃, 300 MHz) δ 8.13 (s, 1H), 7.13 (m, 1H), 7.03 (m, 1H), 5.71 (m, 1H), 4.65 (m, 1H), 3.70 (s, 3H), 3.20 (m, 2H), 1.39 (s, 9H); MS (+ESI) *m*/*z* 297.1 [M + H]⁺.

**(*S*)-5-[(2,6-Dichlorophenyl)methoxy]-α-[[(1,1-dimethylethoxy)carbonyl]amino]-2-pyridinepropanoic acid methyl ester (C**_{**21**}**H**_{**24**}**Cl**_{**2**}**N**_{**2**}**O**_{**5**}**, M-6):** To a solution of **M-5** (0.126 g, 0.43 mmol), 2,6-dichlorobenzylalcohol (0.075 g, 0.43 mmol) and PPh₃ (0.113 g, 0.43 mmol) in dry THF (4 mL) at 0 °C under Ar is added DEAD (0.068 mL). The reaction mixture is permitted to warm to room temperature, and is stirred for 18 h. It is concentrated, and the residue is purified by silica chromatography: TLC (7:3 hexanes/EtOAc) *R*_{*f*} = 0.34; ¹H NMR (CDCl₃, 300 MHz) δ 8.31 (m, 1H), 7.37 (m, 2H), 7.25 (m, 2H), 7.08 (m, 1H), 5.81 (m, 1H), 5.29 (s, 2H), 4.65 (m, 1H), 3.70 (s, 3H), 3.24 (m, 2H), 1.63 (m, 1 H), 1.43 (s, 9H); ¹³C NMR (CDCl₃, 75 MHz) δ 172.47, 155.50, 153.82, 149.71, 137.33, 137.00, 131.51, 130.72, 128.56, 123.99, 122.78, 79.74, 65.64, 53.25, 52.27, 38.43, 28.33 (3C); MS (+ESI) *m*/*z* 454.9 [M + H]⁺.

**(*S*)-α-Amino-5-[(2,6-dichlorophenyl)methoxy]-2-pyridinepropanoic acid methyl ester dihydrogen chloride salt (C**_{**16**}**H**_{**16**}**Cl**_{**2**}**N**_{**2**}**O**_{**3**}**·2HCl, M-7) :** A solution of carbamate **M-6** (0.546 g, 1.20 mmol) in 4 M HCl in dioxane (12 mL) is stirred at room temperature under Ar for 16 h. The reaction mixture is concentrated *in vacuo.* The residue is dissolved in H₂O, and this solution is extracted with Et₂O. The aqueous solution is frozen and lyophilized to give **M-7:** ¹H NMR (CD₃SOCD₃, 300 MHz) δ 8.75 (s, 3H), 8.47 (m, 1H), 7.81 (m, 1H), 7.57 (m, 3H), 7.48 (m, 1H), 5.35 (s, 2H) , 4.49 (m, 1H), 3.67 (s, 3H), 3.42 (m, 2H); ¹³C NMR (CD₃SOCD₃, 75 MHz) δ 169.42, 154.95, 146.54, 136.57, 134.35, 132.50, 131.30, 129.36, 126.72, 126.52, 66..40, 53.32, 51.79, 34.81.

**[1*S*-[1α(*R**),3α]]-α-[[(3-Acetyl-2,2-dimethylcyclobutyl)carbonyl]amino]-3-[(2,6-dichlorophenyl)methoxy]-6-pyridinepropanoic acid methyl ester (C**_{**25**}**H**_{**28**}**Cl**_{**2**}**N**_{**2**}**O**_{**5**}**, M-8):** To a mixture of acid G-2 (0.936 g, 5.50 mmol), HOAt (0.749 g, 5.50 mmol), EDC (1.055 g, 5.50 mmol), and **M-7** (2.14 g, 5.00 mmol) in 4:1 CH₂Cl₂/DMF (20 mL) at 0 °C is added N-methylmorpholine (NMM) (1.38 mL, 12.5 mmol). The solution is kept at 0 °C for 40 h, and then is concentrated. The residue is taken up in CH₂Cl₂, and this solution is washed with water, satd aq NaHCO₃, and H₂O. The combined aqueous portions are back-extracted with CH₂Cl₂. The combined CH₂Cl₂ portions are dried and concentrated to give a green-brown colored foam, that is purified by silica chromatography: TLC *R*_{*f*} = 0.32 (3:1 EtOAc/hexanes); ¹H NMR (CDCl₃, 300 MHz) δ 8.32 (m, 1H), 7.38 (m, 2H), 7.30-7.25 (m, 3H), 7.08 (m, 1H), 5.31 (s, 2H), 4.87 (m, 1H), 3.67 (s, 3H), 3.28 (m, 1H), 3.20 (m, 1H), 2.85 (m, 1H), 2.05 (s, 3H), 1.91 (m, 1H), 1.46 (s, 3H), 0.86 (s, 3H); MS (+ESI) m/z 509.2 507.2 [M + H]⁺.

**[1*S*-[1α(*R**),3α]]-α-[[(3-Carboxy-2,2-dimethylcyclobutyl) carbonyl]amino]-3-[(2,6-dichlorophenyl)methoxy]-6-pyridinepropanoic acid (C**_{**23**}**H**_{**24**}**Cl**_{**2**}**N**_{**2**}**O**_{**6**}**, Example 109):** To a solution of 1 M aq NaOH (21.2 mL) at 0 °C is added portionwise Br₂ (0.254 mL, 4.93 mmol). The mixture is stirred for 30 min beyond the point that all of the Br₂ is dissolved. At that time a solution of **M-7** (0.60 mL, 1.18 mmol) in 1:1 dioxane/THF (16.0 mL) is added. The reaction mixture is stirred for 1 h at 0 °C and for 2.5 h at rt. It is cooled to 0 °C and quenched with cold 1 M aq NaHSO₃ (50 mL). The aqueous mixture is extracted with Et₂O. The aqueous mixture is acidified with concentrated aq HCl, and extracted with EtOAc. The combined EtOAc portions are dried and concentrated to give a colorless paste, that is purified by silica chromatography. The solid that is obtained upon concentration of the column fractions is dissolved in 1:1 MeCN/H₂O (40 mL). This solution is lyophilized to give **Example 109** as an amorphous solid: mp 121-124 °C; TLC R_{f} = 0.11 (750:250:5 EtOAc/hexanes/HCO₂H); ¹H NMR (CD₃SOCD₃, 300 MHz) δ 12.27 (s, 2H), 8.27 (m, 1H), 7.82 (m, 1H), 7.56 (m, 2H), 7.48-7.42 (m, 2H), 7.23 (m, 1H), 5.25 (s, 2H), 4.62 (m, 1H), 3.30 (s, 2H), 3.14-2.97 (m, 2H), 2.67-2.55 (m, 2H), 2.29 (m, 1H), 1.71 (m, 1H), 1.21 (s, 3H), 0..72 (s, 3H); ¹³C NMR (CD₃OD, 75 MHz) δ C: 175.93, 174.5, 173.72, 155.6, 151.23, 132.98, 45.90; CH: 138.04, 132.30, 129.79, 125.9, 124.31, 53.6, 47.38, 46.40; CH₂: 66.85, 39.02, 20.96; CH₃: 30.54, 18.39; MS (+ESI, MeOH) *m*/*z* 497.2, 495.2 [M + H]⁺; Anal. C 55.77, H 4.88, N 5.39 (calcd for 1.50% H₂O: C 54.93, H 4.98, N 5.57).

### Example 110: Teaching Scheme N

**7,9-Dioxaspiro[4.5]decan-8-one (C**_{**9**}**H**_{**12**}**O**_{**3**}**, N-1).** Anhydride **N-1** is prepared from 1,1-cyclopentanediacetic acid [16713-66-9] by standard methods (Balo, M. B.; Fernandez, F.; Lens, E.; Lopez, C. *Nucleosides Nucleotides* **1996,** *15*, 1335-1346).

***N*-[(1-Carboxymethyl)cyclopentaneacetyl)-*O*-([(2,6-dichlorophenyl)methyl]-L-tyrosine methyl ester (C**_{**26**}**H**_{**29**}**Cl**_{**2**}**NO**_{**6**}**, N-2).** To a mixture of anhydride **N-1** (0.84 g, 5.0 mmol), **Compound 8** (1.95 g, 5.0 mmol) and iPr₂EtN (4.4 mL, 25 mmol) in THF (15 mL) is heated at reflux for 17 h. The reaction mixture is cooled, and is concentrated *in vacuo*. The residue is taken up in aqueous 1 M HCl (40 mL), and this mixture is extracted with EtOAc. The combined EtOAc extracts are dried and concentrated, and the residue is purified by silica chromatography. The column fractions are concentrated, and the resulting solid is dissolved in MeCN/H₂O. This solution is lyophilized to give **N-2:** ¹³C NMR (CDCl₃, 75 MHz) δ 175.0, 172.8, 171.7, 157.9, 136.7, 131.8, 130.3, 130.0, 128.3, 127.9, 115.0, 65.0, 53.2, 52.4, 44.2, 44.1, 43.1, 38.2, 36.6, 23.5; MS (EI) *m*/*z* 523, 521; Anal. C 59.62, H 5.82, Cl 13.44, N 2.57 (calcd C 59.78, H 5.60, Cl 13.57, N 2.68).

***N*-[(1-Carboxymethyl)cyclopentaneacetyl]-*O*-([(2,6-dichlorophenyl)methyl]-L-tyrosine (C**_{**25**}**H**_{**27**}**Cl**_{**2**}**NO**_{**6**}**, Example 110).** A mixture of ester **N-2** (1.00 g, 1.90 mmol) and LiOH·H₂O (0.84 g, 20.0 mmol) in H₂O (12 mL) is stirred at 0 °C for 4 h. The reaction mixture is acidified with aqueous 1 M HCl (24 mL) to give a white solid. The solid is recovered by filtration. It is dissolved in 1:1 MeCN/H₂O (20 mL), and this solution is lyophilized to give **Example 110:** mp 108 °C (softens) ; ¹³C NMR (CD₃SOCD₃, 75 MHz) δ 175.2, 174.0, 173.0, 158.0, 132.0, 130.5, 130.3, 130.1, 128.7, 128.5, 115.0, 65.2, 53.6, 53.5, 44.2, 42.0, 25.6, 21.3; MS (EI) m/z 521; Anal. C 60.37, H 5.84, Cl 14.33, N 2.55 (calcd for 0.73% H₂O: C 58.59, H 5.33, Cl 14.62, N 2.73).

### Example 111: Teaching Scheme O

**3-Oxabicyclo[3.2.1]octane-2,4-dione [6054-16-6] (C**_{**7**}**H**_{**8**}**O**_{**3**}**, O-1)**. Anhydride **O-1** is prepared from *cis*-1,3-cyclopentanedicarboxylic acid [876-05-1] by standard methods (Balo, M. B.; Fernandez, F.; Lens, E.; Lopez, C. *Nucleosides Nucleotides* **1996**, *15*, 1335-1346).

***N*-[*cis*-(3-Carboxycyclopentyl)carbonyl]-*O*-[(2,6-dichlorophenyl)methyl]-L-tyrosine methyl ester (C**_{**25**}**H**_{**27**}**Cl**_{**2**}**NO**_{**6**}**, O-2)** . Compound **O-2** is prepared from anhydride **O-1** and **Compound 8** as taught for Compound **N-2:** ¹³C NMR (CDCl₃, 75 MHz) δ 179.0, 178.9, 175.8, 175.7, 171.9, 157.9, 136.8, 131.9, 130.3, 130.1, 128.3, 128.1, 115.0, 114.9, 65.1, 53.1, 53.0, 52.4, 52.3, 45.5, 44.0, 43.9, 36.8, 36.7, 32.5, 32.1, 31.3, 30.8, 29.7, 29.6; Anal. C 59.27, H 5.22, Cl 14.26, N 2.78 (calcd for 0.39% H₂O: C 58.31, H 5.10, Cl 14.34, N 2.83).

***N*-[*cis*-(3-Carboxycyclopentyl)carbonyl]-*O*-[(2,6-dichlorophenyl)methyl]-L-tyrosine (C**_{**23**}**H**_{**23**}**Cl**_{**2**}**NO**_{**6**}**, Example 111)**. **Example 111** is prepared from ester **O-2** as taught for **Example 110:** ¹H NMR (CDCl₃, 300 MHz) δ 7.40-7.32 (m, 2H), 7.30-7.22 (m, 1H), 7.13 (m, 2H), 6.93 (m, 2H) , 6.75-6.60 (m, 1H), 5.23 (s, 2H), 4.85-4.70 (m, 1H), 3.25-3.00 (m, 2H), 2.90-2.60 (m, 2H), 2.22-1.80 (m, 6H); Anal. C 56.56, H 4.76, Cl 14.66, N 2.79 (calcd for 0.90% H₂O: C 57.51, H 4.83, Cl 14.76, N 2.92).

### Example 112: Teaching Scheme P

**(±)-*cis*-1,3-Cyclopentanedicarboxylic acid monomethyl ester [96382-85-3] (C**_{**8**}**H**_{**12**}**O**_{**4**}**, P-1).** Monoester **P-1** is prepared from *cis*-1,3-cyclopentane-dicarboxylic acid by standard methods (Chenevert, R.; Martin, R. *Tetrahedron: Asymmetry* **1992,** *3*, 199-200).

**4-[(2**,**6-Dichlorobenzoyl)amino]-*N*-[*cis*-[3-(1-methoxy-1-oxomethyl)cyclopentyl] carbonyl]- L-phenylalanine methyl ester (C**_{**25**}**H**_{**26**}**Cl**_{**2**}**N**_{**2**}**O**_{**6**}**, P-2).** To a mixture of **P-1** (0.5 g, 2.9 mmol), HOBt (0.45 g, 3.3. mmol) DMAP (0.04 g, 0.32 mmol), NEt₃ (1.45 mL, 10.4 mmol) in CH₂Cl₂ (25 mL) at 0 °C is added **Compound 7** (1.15 g, 2.8 mmol). The reaction mixture is stirred at room temperature for 48 h. The CH₂Cl₂ is removed from the reaction mixture in vacuo, and the residue is taken up in aqueous 1 M HCl (50 mL). The aqueous mixture is extracted with CH₂Cl₂. The combined CH₂Cl₂ extracts are 'washed with said aq NaHCO₃, dried, and concentrated. The residue is purified by silica chromatography to provide, as a diastereomeric mixture, **P-2:** MS (EI) *m*/*z* 520, 351, 350, 349, 280, 278, 175, 173, 155, 95, 67; Anal. C 57.55, H 5.03, Cl 13.62, N 5.40 (calcd for 0.11% H₂O: C 57.59, H 5.03, Cl 13.60, N 5.37).

**N-[cis-(3-Carboxycyclopentyl)carbonyl]-4-[(2,6-dichlorobenzoyl)amino]-L-phenylalanine (C**_{**23**}**H**_{**22**}**Cl**_{**2**}**N**_{**2**}**O**_{**6**}**, Example 112).** Example **112** is prepared, as a diastereomeric mixture, from **P-2** as taught for Example **110:** MS (FAB) *m*/*z* 496, 495, 494, 493, 492, 476, 475, 337, 335, 175, 173; Anal. C 55.40, H 4.55, Cl 14.08, N 5.62 (calcd for 1.87% H₂O: C 56.00, H 4.50, Cl 14.37, N 5.68).

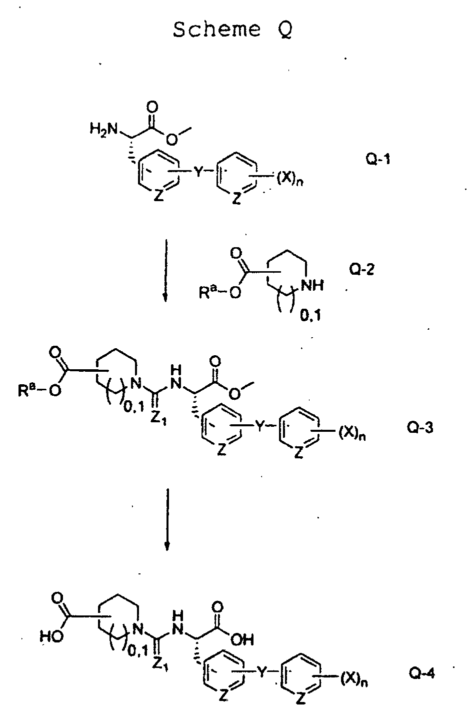

Scheme Q describes a general method for preparing ureas and thioureas of general structure Q-4. This method is not preferred for preparing ureas containing a carboxyl substituent in the 2 position of the piperidine or pyrrolidine ring of structure Q-2. An amino acid of general structure Q-1 is reacted with carbonyldiimidazole or thiocarbonyldiimidazole followed by the cyclic amino acid of general structure Q-2 to afford the diester intermediate Q-3. Mild base hydrolysis afford the diacid of general structure Q-4.

### Example 113

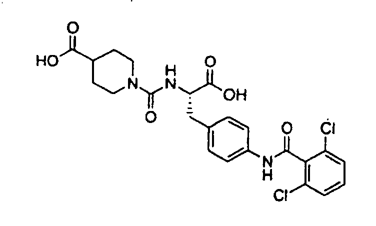

**1-[[[(1S)-1-carboxy-2-[4-[(2,6-dichlorobenzoyl)amino]phenyl]ethyl]amino]carbonyl]-4-piperidinecarboxylic acid.** To a cooled (0-5°C ) solution of **Compound 11** (410 mg, 0.74 mmol) in THF (23 mL) and MeOH (7.5 mL) was added an aqueous (5 mL) solution of lithium hydroxide monohydrate (94 mg, 2.24 mmol) via a syringe pump over 1 h. After an additional 1 h at 0-5°C, the ice bath was removed and the solution stirred 2 h at ambient temperature. The reaction mixture was 'diluted with ethyl acetate and 0.1 N HCl and the organic layer separated, washed with water, dried (Na₂SO₄), filtered and concentrated *in vacuo.* Lyophilization of the residue from glacial acetic acid afforded the title compound (360 mg) as a white amorphous powder: IR (mull) 3123, 3034, 1717, 1665, 1606, 1562, 1535, 1517, 1432, 1414, 1326, 1272, 1213, 1195, 799 cm⁻¹; ¹H NMR (300 MHz, CD₃OD) δ 7.57 (2 H), 7.43 (3 H), 7.24 (2 H), 4.48 (1 H), 3.85 (2 H), 3.19 (1 H), 3.95 (3 H), 2.47 (1 H), 1.83 (2 H), 1.55 (2 H); ¹³C NMR (75 MHz, CD₃OD) δ 178.3, 176.2, 165.2, 159.4, 138.1, 137.8, 133.4, 132.4, 131.0, 129.4, 121.5, 56.8, 94.6, 44.5, 42.0, 38.1, 29.2; MS (FAB) *m*/*z* (rel. intensity) 508 (MH⁺, 99), 511 (19), 510 (64), 509 (36), 508 (99), 391 (34), 356 (16), 173 (20), 149 (29), 128 (17), 57 (16); HRMS (FAB) calcd for C₂₃H₂₃Cl₂N₃O₆+H₁ 508.1042, found 508.1033. Anal. Calcd for C₂₃H₂₃Cl₂P₃O₆ · 0.25 H₂O: C, 53. 87; H, 4.62; N, 8.19. Found: C, 53.85; H, 4.87; N, 8.04.

### Example 114

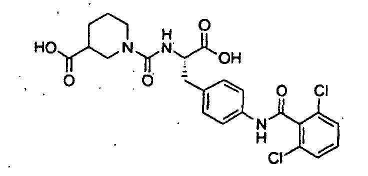

**1-[[[(1S)-1-carboxy-2-[4-[(2,6-dichlorobenzoyl)amino]phenyl]ethyl]amino]carbonyl]-3-piperidinecarboxylic acid Example 114** was prepared as described in Scheme Q from (*R,S*)-ethyl nipecotate. Physical properties as follows: IR (mull) 3062, 3035, 1718, 1665, 1628, 1606, 1562, 1536, 1517, 1432, 1414, 1326, 1268, 1216, 1195 cm⁻¹; ¹H NMR (300 MHz, CD₃OD) δ 7.58 (2 H), 7.43 (3 H), 7.25 (2 H), 4.52 (1 H), 4.01 (1 H), 3.73 (1 H), 3.29 (1 H), 3.95 (3 H), 2.38 (1 H), 1.99 (1 H), 1.63 (2 H), 1.37 (1 H); ¹³C NMR (75 MHz, CD₃OD) δ 176.4, 169.4, 165.2, 159.4, 138.1, 137.7, 135.8, 133.4, 132.4, 131.0, 129.4, 121.5, 60.1, 56.5, 47.4, 45.7, 42.4, 38.2, 28.6, 25.5; MS (FAB) m/z (rel. intensity) 508 (MH⁺, 99), 512 (12), 511 (18), 510 (66), 509 (31), 508 (99), 335 (10), 173 (11), 130 (11), 128 (20), 84 (11). Anal. Calcd for C₂₃H₂₃Cl₂N₃O₆ • 0.5 H₂O: C, 53.39; H, 4.67; N, 8.12. Found: C, 53.56; H, 4.60; N, 7.95.

### Example 115

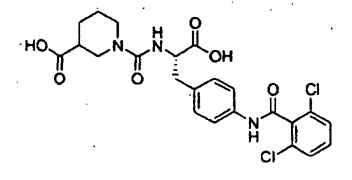

**1-[[((1S)-1-carboxy-2-[4-[(2,6-dichlorobenzoyl)amino]phenyl]ethyl)amino]carbonyl]-3-piperidinecarboxylic acid. Example 115** was prepared as described in Scheme Q from (*R,S*)-ethyl nipecotate. The intermediate diester products of general structure Q-3 were separated by chiral HPLC. The individual diastereomers were saponified as described for **Example 113** to provide single stereoisomers of undetermined absolute stereochemistry. Physical properties of one stereoisomer as follows: ¹H NMR (300 MHz, CD₃OD) δ 7.57 (2 H), 7.45 (3 H), 7.25 (2 H), 4.52 (1 H), 4.02 (1 H), 3.74 (1 H), 3.23 (1 H), 2.93 (3 H), 2.34 (1 H), 2.01 (1 H), 1.65 (2 H), 1.44 (1 H); ¹³C NMR (75 MHz, CD₃OD) δ 177.0, 176.1, 165.2, 159.4, 138.1, 137.7, 135.8, 133.4, 132.4, 131.0, 129.4, 121.5, 62.4, 56.7, 47.4, 45.6, 42.4, 38.2, 28.6, 25.5; MS (FAB) *m*/*z* (rel. intensity) 508 (MH⁺, 99), 510 (69), 509 (32), 508 (99), 173 (18), 130 (21), 128 (29), 84 (19), 57 (16), 55 (18), 43 (17); HRMS (FAB) calcd for C₂₃H₂₃Cl₂N₃O₆+H₁ 508.1042, found 508.1033.

### Example 116

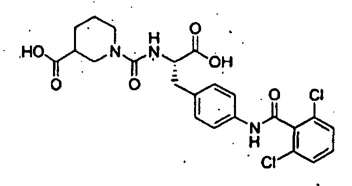

**1-[[[(1S)-1-carboxy-2-[4-[(2,6-dichlorobenzoyl)amino]phenyl]ethyl]amino]carbonyl]-3-piperidinecarboxylic acid. Example 116** is the other individual stereoisomer related to **Example 115.** Physical properties as follows: ¹H NMR (300 MHz, CD₃OD) δ 7.58 (2 H), 7.43 (3 H), 7.25 (2 H) , 4.53 (1 H), 4.05 (1 H) , 3.71 (1 H), 3.23 (1 H), 2.95 (3 H) , 3.37 (1 H), 2.01 (2 H), 1.63 (2 H); ¹³C NMR (75 MHz, CD₃OD) δ 177.1, 176.1, 165.2, 159.4, 138.1, 137.7, 135.8, 133.4, 132.4, 131.0, 129.4, 121.5, 56.7, 47.2, 45.7, 42.4, 38.1, 32.9, 28.6, 25.5, 23.8; MS (FAB) *m*/*z* (rel. intensity) 508 (MH⁺, 99), 511 (19), 510 (69), 509 (34), 508 (99), 335 (15), 1.73 (21), 130 (21), 128 (32), 123 (18), 84 (20); HRMS (FAB) calcd for C₂₃H₂₃Cl₂N₃O₆+H₁ 508.1042, found 508.1038.

### Example 117

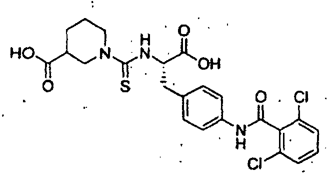

**1-[[[(1S)-1-carboxy-2-[4-[(2,6-dichlorobenzoyl)amino] phenyl]ethyl]amino] thiocarbonyl]-3-piperidinecarboxylic acid. Example 117** was prepared as described in Scheme Q from (*R*,*S*)-ethyl nipecotate and thiocarbonyldiimidazole as reagents. Physical properties as follows: IR (mull) 3109, 3030, 1713, 1661, 1606, 1561, 1542, 1516, 1431, 1415, 1330, 1274, 1234, 1224, 1195 cm⁻¹; ¹H NMR (300 MHz, CD₃OD) δ 7.57 (2 H), 7.42 (3 H), 7.24 (2 H), 5.34 (1 H), 4.60 (1 H), 4.24 (1 H), 3.23 (4 H), 2.48 (1 H), 2.06 (1 H), 1.64 (3 H) ; ¹³C NMR (75 MHz, CD₃OD) δ 183.1, 183.0, 176.9, 176.8, 175.7, 175.6, 165.2, 138.1, 137.7, 135.4, 133.4, 132.4, 131.0, 129.4, 121.6, 121.5, 61.1, 51.5, 51.3, 42.3, 42.2, 38.0, 28.7, 28.5, 25.5, 25.3, 20.9; MS (FAB) *m*/*z* (rel. intensity) 524 (MH⁺, 99), 602 (40), 600 (51), 526 (68), 525 (42), 524 (99), 188 (28), 173 (33), 172 (67), 130 (29), 128 (35) ; HRMS (FAB) calcd for C₂₃H₂₃Cl₂N₃O₅S +H₁ 524.0814, found 524.0816. Anal. Calcd for C₂₃H₂₃Cl₂N₃O₅S · 0.25 H₂O: C, 52.23; H, 4.48; N, 7.94. Found: C,

### Compound Preparations cont.

### Compound 3

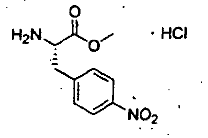

**4-Nitro-L-phenylalanine methyl ester hydrochloride (Compound 3).** To a cold (0-5°C) solution of anhydrous methanolic HCl was added L-4-nitrophenylalanine (Advanced ChemTech, 100 g) portionwise over 15 min. The mechanically stirred mixture was heated to.gentle reflux for 48 h. The mixture was allowed to cool and filtered through a sintered glass filter funnel, washing the collected solids with hot MeOH until only insoluble residues remained. The filtrate was concentrated *in vacuo* to afford the methyl ester as waxy off white solid which was used without further purification.

### Compound 4

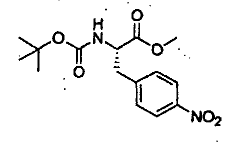

***N-tert*-Butoxycarbonyl-4-nitro-L-phenylalanine methyl ester (Compound 4).**
To a suspension of methyl ester described above (87 g, 0.33 mole) in CH₂Cl₂ (1500 mL) at ambient temperature was added di-*t*-butyldicarbonate (109 g, 0.50 mole) followed by the dropwise addition of Et₃N (51 mL, 0.37 mole). After 15 min additional Et₃N (40 mL, 0.29 mol) was added to maintain a slightly basic mixture (*ca*. pH 8). The reaction mixture was stirred 18 h and additional CH₂Cl₂ (1400 mL) and Et₃N (15 mL, 0.11 mol) were added. After an additional 2 h the reaction mixture was quenched by the slow addition of MeOH (100 mL), stirred for 1 h and then partitioned between CH₂Cl₂ and cold 10% aqueous KHSO₄. The organic layer was washed with saturated NaHCO₃ and brine, dried (Na₂SO₄), filtered and concentrated *in vacuo*. Purification of the residue by flash chromatography using hexane and a gradient of a 1:1 mixture of ETOAc/CH₂Cl₂ (25-33%) afforded the title compound (69 g) as a white solid. Physical properties as follows: ¹H NMR (300 MHz; CDCl₃) δ 8.16 (2H), 7.31 (2H), 5.04 (1H), 4.63 (1H), 3.73 (3H), 3.18 (2H), 1.41 (9H); MS (ES+) for C₁₅H₂₀N₂O₆ *m*/*z* 325.2 (M+H)⁺.

### Compound 5

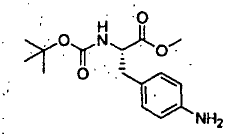

***N*-*tert*-Butoxycarbonyl-4-amino-L-phenylalanine methyl ester (Compound 5).** Palladium on carbon (10% w/w, 1.25 g) was added to a Parr hydrogenation flask under a N₂ atmosphere and carefully wetted with 100 mL of MeOH/THF (1:1). A solution of the Boc-methyl ester described above (25 g, 77 mmol) in 400 mL of MeOH/THF (1:1) was added and the mixture shaken on a hydrogenation apparatus under a hydrogen atmosphere (20 psi) for 1 h at ambient temperature. The reaction mixture was filtered through a pad of Celite and the solids washed several times with MeOH. The combined filtrates were concentrated *in vacuo* to afford the title compound which was used without further purification. Physical properties as follows: ¹H NMR (300 MHz, CDCl₃) δ 6.89 (2H), 6.61 (2H), 4.96 (1H), 4.50 (1H), 3. 69 (3H), 2.95 (2H), 1.41 (9H); MS (ES+) for C₁₅H₂₂N₂O₄ *m*/*z* 295.2 (M+H)⁺.

### Compound 6

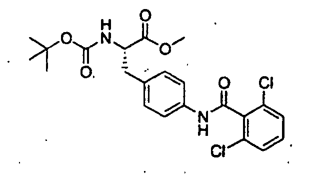

***N*-*tert*-Butoxycarbonyl-4-[(2,6-dichlorobenzoyl)amino]-L-phenylalanine methyl ester (Compound 6).** To a cooled (0-5°C) solution of 2,6-dichlorobenzoyl chloride (11.1 mL, 77.5 mmol) in THF (125 mL) was added a solution of the compound 5 (22.7 g, 77.1 mmol) and Et₃N (16 mL, 115 mmol) in THF (125 mL). The reaction mixture was allowed to warm to temperature and stir an additional 18 h. The mixture was diluted with EtOAc (2 L) and washed with 1N aqueous HCl, H₂O, 1N aqueous NaOH and brine. The organic extract was dried (Na₂SO₄), filtered, and concentrated *in vacuo* to give the crude product as a pale yellow solid. This material was recrystallized from acetone/hexanes to afford the amide as a crystalline solid. Physical properties as follows: mp 192.2-193.1°C; IR (mull) 3305, 1747, 1736, 1690, 1665, 1609, 1548, 1512, 1433, 1414, 1325, 1277, 1219, 1171, 781 cm⁻¹; ¹H NMR (300 MHz; CDCl₃) δ 7.57 (2H), 7.34 (4H), 7.14 (2H), 4.98 (1H), 4.60 (1H), 3.74 (3H), 3.11 (2H), 1.42 (9H); MS (ES+) for C₂₂H₂₄Cl₂N₂O₅ *m*/*z* 467.0 (M+H)⁺.

### Compound 7

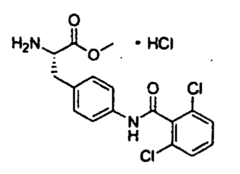

**4-[(2,6-Dichlorobenzoyl)amino]-L-phenylalanine methyl ester hydrochloride (Compound 7).** To 650 mL of anhydrous 4M HCl in dioxane at ambient temperature was added compound 6 (30.6 g, 65.5 mmol) portionwise and the resulting mixture stirred until all the solids had dissolved (ca. 1 h). Volatiles were removed *in vacuo* to give a light yellow solid which was partitioned between water (500 mL) and ether (1 L). The water layer was separated and concentrated *in vacuo* to approximately 200 mL. The aqueous solution was then frozen and lyophilized to afford the title compound as a light yellow solid. Physical properties as follows: [α]²⁵_{D} = +5 (c 1, MeOH); IR (mull) 3244, 3186, 3112, 1747, 1660, 1604, 1562, 1539, 1516, 1431, 1416, 1327, 1273, 1243, 799 cm⁻¹; ¹H NMR (300 MHz; CD₃OD) δ 7.69 (2H), 7.45 (3H), 7.29 (2H), 4.34 (1H), 3.83 (3H), 3.21 (2H); ¹³C NMR (300 MHz; CD₃OD) δ 169.0, 163.9, 137.8, 136.08, 131.8, 131.0, 130.3, 129.7, 127.9, 120.6, 53.8, 52.3, 35.4; MS (ES+) for C₁₇H₁₆Cl₂N₂O₃ *m*/*z* 367.1 (M+H)⁺.

### Compound 8

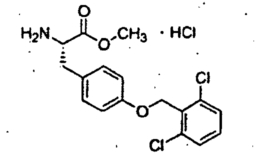

***O*-2,6-Dichlorobenzyl-L-tyrosine methyl ester hydrochloride (Compound 8).** To a cooled (0-5°C) solution of anhydrous methanolic HCl (200 mL) was added 25 g of *N*-α-*tert*-Butoxycarbonyl-*O*-2,6-dichlorobenzyl-L-tyrosine (Sigma) portionwise over 15 min. After 30 minutes at 0-5°C, the mixture was heated to 50°C for 2 h. The solution was cooled to room temperature and the volatiles removed *in vacuo*. The solid was suspended in ethyl ether and collected by filtration to afford the title compound which was used without further purification. Physical properties as follows: [α]²⁵_{D} = +16 (c 1.00, ethanol); ¹H NMR (300 MHz, CD₃OD) δ 7.44 (2 H), 7.35 (1 H), 7.21 (2 H), 7.02 (2 H), 5.28 (2 H), 4.29 (1 H), 3.81 (3 H), 3.18 (2 H); MS (ESI+) for C₁₇H₁₇Cl₂NO₃ m/z 354.1 (M+H)⁺; Anal. Calcd for C₁₇H₁₇Cl₂NO₃•HCl: C, 52.26; H, 4.64; N, 3.59. Found: C, 52.17; H, 4.74; N, 3.61.

### Compound 9

**4-Amino-*N*-[(1,1-dimethylethoxy)carbonyl]-L-phenylalanine (C**_{**14**}**H**_{**20**}**N**_{**2**}**O**_{**4**}**, Compound 9).** To a mixture of *N*-[(1,1-dimethylethoxy)carbonyl]-4-nitro-L-phenylalanine (12.0 g, 38.7 mmol) and 10% Pd/C (0.8 g) in MeOH (0.15 L) is hydrogenated (35 psi H₂) at room temperature for 2 h. The catalyst is removed by filtration, and the MeOH is removed in.vacuo to give, after vacuum drying, **Compound 9:** ¹H NMR (CDCl₃, 300 MHz) d 6.95 (m, 2H), 6.63 (m, 2H), 5.1 (s, 1H), 4.4 (s, 1H), 3.0 (m, 2H), 1.4 (s, 9H).

### Compound 10

**4-[2,6-(Dichlorobenzoyl)amino]-*N*-[(1,1-dimethylethoxy)carbonyl]-L-phenylalanine (C**_{**21**}**H**_{**22**}**Cl**_{**2**}**N**_{**2**}**O**_{**5**}**, Compound 10).** To a mixture of **Compound 9** (12.0 g, 42.7 mmol), NaOH (3.4 g, 85.5 mmol) in H₂O (80 mL) is added a solution of 2,6-dichlorobenzoylchloride (10.7 g, 51.3 mmol) in dioxane (24 mL). The mixture is stirred for 24 h at rt. It is concentrated partially to remove the dioxane, and is then acidified to precipitate the product. The precipitate is collected by filtration, washed with cold H₂O, and dried in a vacuum oven at room temperature overnight. The solid is triturated with THF, and the THF is discarded. The solid is dissolved in MeCN (2 L), and the solution is concentrated to a volume of approximately 300 mL. Water (150 mL) is added. The solution is frozen and lyophilized to give **Compound 10:** MS (FAB) *m*/*z*453, 399, 398, 397, 353, 307, 280, 278, 175, 173, 57; Anal. C 55.48; H 4.74, Cl 15.37, N 6.25 (calcd corrected for 2.68% H₂O: C 55.64, H 4.89, Cl 15.64, N 6.18).

### Compound 11

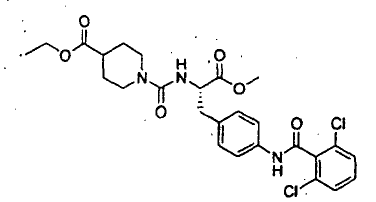

To a cooled (0-5 °C) solution of **Compound** 7 (0.75 g, 1.86 mmol) in THF (20 mL) was added NEt₃ (520 µL, 3.72 mmol) and CDI (0.30 g, 1.86 mmol). After 60 min, ethyl isonipecotate was added and the mixture stirred for an additional hour at 0-5 °C. The ice bath was removed and the solution stirred overnight at room temperature and diluted with ethyl acetate and 0.25 N aqueous HCl. The organic layer was separated and washed with 0.25 N aqueous HCl, brine, dried (MgSO₄) filtered and concentrated *in vacuo.* Crystallization of the residue from aqueous ethanol afforded the above compound as a colorless solid: ¹H NMR (300 MHz, CDCl₃) δ 7.60 (2 H), 7.32 (3 H), 7.10 (2 H), 4.70 (1 H), 4.13 (2 H) , 3.78 (2 H), 3.74 (3 H), 3.12 (2 H), 2.89 (2 H), 2.48 (1 H), 1.88 (2 H), 1.63 (2 H), 1.23 (3 H); ¹³C NMR (75 MHz, CDCl₃) δ 179.8, 173.4, 163.2, 156.8, 136.8, 136.3, 132.9, 132.4, 130.8, 129.9, 128.1, 120.5, 60.8, 56.8, 54.6, 52.4, 43.4, 41.0, 37.7, 27.8, 14.2; MS (ESI+) for C₂₆H₂₉Cl₂N₃O₆ *m*/*z* 550.0 (M+H)⁺; MS (ESI-) for C₂₆H₂₉Cl₂N₃O₆ *m*/*z* 548.0 (M-H)⁻; Anal. Calcd for C₂₆H₂₉Cl₂N₃O₆: C, 56.73; H, 5.31; N, 7.63. Found: C, 56.42; H, 5.43; N, 7.58.

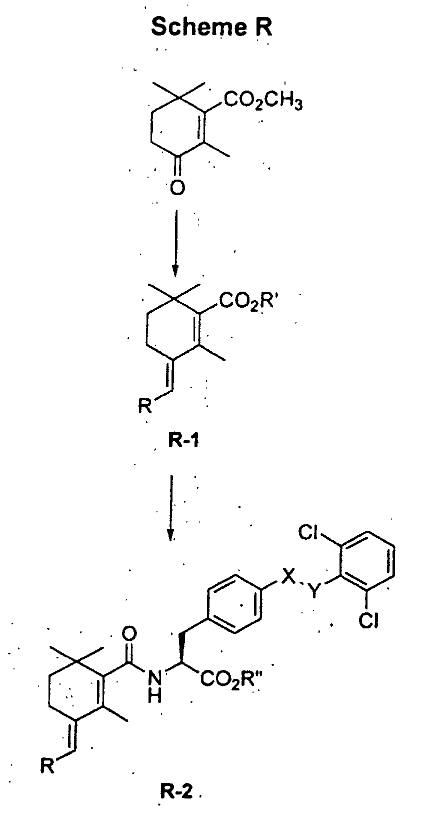

### Preparation R-1-1

### Scheme R, R-1, wherein R = CO₂tBu, R' = CH₃ 3-Carbomethoxy-2,4,4-trimethyl-2-cyclohexen-1-ylideneacetic acid, 1,1-dimethylethyl ester, (E) (C₁₇H₂₆O₄)

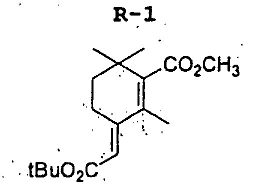

To a stirring solution of potassium t-butoxide in THF (44mL) at 0°C was added a solution of t-butyl diethylphosphonoacetate (12.6g, 49.8mmol) in THF(15mL) dropwise. The solution was allowed to warm to RT and stirred for 30 minutes before being recooled to 0°C. A solution of the known ketone 3-carbomethoxy-2,4,4-trimethyl-2-cyclohexen-1-one (Boulin, B.; Miguel, B. Arreguy-San; D.B. *Tetrahedron* **1998**, *54*, 2753) (5.75g, 29.3mmol) in THF (10mL) was added dropwise. The solution was allowed to slowly warm to RT and was stirred for 16h. The solution was evaporated *in vacuo*, and the residue was partitioned between H₂O (250mL) and EtOAc (250L). The aqueous phase was extracted with EtOAc (250mL). The combined organic phases were washed with H₂O (500mL), brine (500mL), filtered, and evaporated *in vacuo.* The resulting orange oil was chromatographed on silica gel (450g, 230-400 mesh, 70mm OD column, packed and eluted with EtOAc/heptane, 5:95 (2L), then EtOAc/heptane, 1:9, collecting 270mL fractions) using the flash technique. Fractions 5-13 were combined and evaporated *in vacuo* to afford the desired diester **R-1** (R = CO₂tBu, R' = CH₃) 6.41g of clear colorless oil, (74%). ^{**1**}**H-NMR** (300 MHz., CDCl₃): δ = 5.8I (1), 3.80 (3), 3.00-3.07 (2), 1.79 (3), 1.56-1.69 (2), 1.50 (9), 1.13 (6). **IR** (mull) 1723, 1702, 1610, 1602, 1395, 1300, 1286,1249, 1210, 1 180, 1147, 113(), 1()63, 982, and 864cm ⁻¹. **MS** (FAB) m/z (rel. intensity) 295 (MH+, 20), 295 (20), 263 ( 1()), 24() (15), 239 (99), 237 (8), 221 (39), 207 (40), 133 (9), 91 (8), 57 (29). Anal. Calcd for C17H26O4: C, 69.36; H, 8.90. Found: C, 69.02; H, 8.80.

### Preparation R-1-2

### Scheme R, R-1, wherein R = CO₂tBu, R' = H 3-Carboxy-2,4,4-trimethyl-2-cyclohexen-1-ylideneacetic acid, 1,1-dimethylethyl ester, (E) (C₁₆H₂₄O₄) R-1

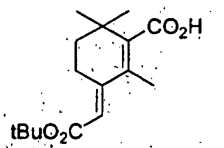

To a stirring solution of the **R-1** (R = CO₂tBu, R' = CH₃) (6.08g, 20.7mmol) in DMF (75mL) was added KOH (1.27g, 22.7mmol) and thiophenol (2.3mL, 22.7mmol), and the mixture was heated at 100°C for 16h. The mixture was allowed to cool to RT, and evaporated *in vacuo*. The resulting residue was partitioned between Et₂O (500mL) and 5% aqueous NaOH (500mL), and the organic phase was discarded. Aqueous 6N HCl was added to the aqueous phase until the pH was ca. 2-3; and the mixture was extracted with Et₂O (2x500mL). The combined extracts were washed with brine (500mL), dried (Na₂SO₄), filtered, and evaporated *in vacuo* to afford the carboxylic acid **R-1** (R = CO₂tBu, R' = H) as a creamcolored solid, 5.55g (96%) **.MP** 83-87°C; ^{**1**}**H-NMR** (300 MHz,..DMSO-d₆) : δ = 12.91 (1), 5.70-5.77 (2), 2.88-2.97 (2), 1.75 (3), 1.40-1.55 (10), 1.09 (6); **IR** (mull) 3488, 3389, 2584,1683, 1604, 1303, 1283, 1262, 1230, 1218, 1150, 1132, 987, 865, and 854cm⁻¹. **MS** (EI ) m/z (rel. intensity): 280 (M+, 1), 224 (71), 207 (54), 206 (95), 179 (99), 178 (62), 161 (48), 133(52), 119 (49), 111 (50), 57 (97). **HRMS** (EI) calcd for C₁₆H₂₄O₄ 280.1764. Found 280.1668.

### Preparation R-2-1

### Scheme R, R-2, wherein R = CO₂tBu, R" = CH₃, X = NH, Y = C=O N-[[(3E)-3-[[(1,1-Dimethylethoxy)carbonyl]methylene]-2,6,6-trimethyl-1-cyclohexen-1-yl]carbonyl]-4-[(2,6-dichlorobenzoyl)amino]-L-phenylalanine methyl ester (C₃₃H₃₈Cl₂N₂O₆) R-2

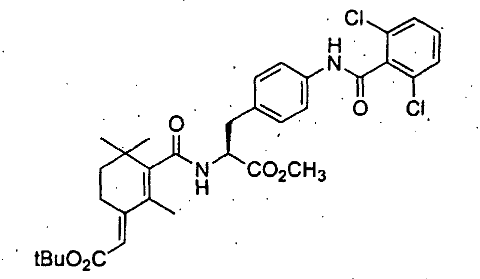

To a stirring solution of the carboxylic acid **R-1** (R = CO₂tBu, R' = H) (2.54g, 9.06mmol) in DMF (75mL) was added 4-[(2,6-dichlorobenzoyl)amino]-L-phenylalanine methyl ester. hydrochloride (11.0g, 27.2mmol), HATU (6.89g, 18.1mmol), and diisopropylethylamine (7.9mL, 45.3mmol). The solution was allowed to stir at RT for 16h and then was evaporated *in vacuo*. The residue was partitioned. between 1M aqueous HCI (500mL) and EtOAc (500mL), and the aqueous phase was extracted with EtOAc (500mL). The combined extracts 'were washed with brine (500mL), dried (Na,SO₄), filtered, and evaporated *in vacuo*. The resulting yellow oil was chromatographed on silica gel (450g, 230-400 mesh, 70mm OD column, packed and eluted with EtOAc/CH₂Cl₂, (1:9), collecting 270mL fractions) using the flash technique. Fractions 8- 16 were combined and evaporated *in vacuo.* to afford the desired product **R-2** (R = CO2tBu, R" = CH₃, X = NH, Y = C=O) as a white solid, 2.22g (39%). **MP** 158-160°C; ^{**1**}**H-NMR** (300 MHz, CDCl₃) δ = 7.56-7.64 (2), 7.30-7.41 (3), 7.17-7.24 (2), 5.74-5.83 (2), 5.03-5.13 (1), 3.79 (3), 3.07-3.26 (2), 2.95-3.05 (2), 1.73 (3), 1.45-1.59 (12), 1.12 (3), 1.05 (3); **IR** (mull) 3259, 175(), 1707, 169(), 1671, 1639, 1610, 1548, 1515, 1432, 1332, 1279, 1216, 1151, and 1135cm⁻¹; **MS** (FAB) m/z; (rel. intensity): 629 (MH+, 3), 557 (32), 555 (46), 349 (18), 263 (31), 208 (18), 207 (99), 175 (19), 173 (30), 161 (18), 57 (26); **HRMS** (FAB) calcd for C₃₃H₃₈Cl₂N₂O₆+Hl 629.2185, found 629.2176.

### Preparation R-2-2

### Scheme R, R-2, wherein R = CO₂H, R" = CH₃, X = NH, Y = C=O N-[[(3E)-3-Carboxymethylene-2,6,6-trimethyl-1-cyclohexen-1-yl]carbonyl]-4-[(2,6-dichlorobenzoyl)amino]-L-phenylalanine methyl ester (C₂₉H₃₀Cl₂N₂O₆) R-2

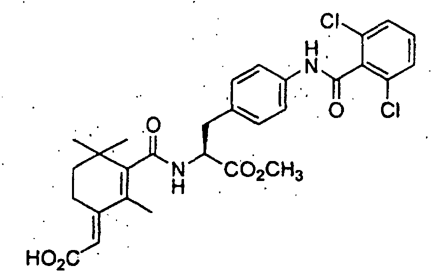

The diester **R-2-1** (**R-2** (R = CO2tBu, R" = CH₃, X = NH, Y = C=O) (3.95g, 6.27mmol) was dissolved in trifluoroacetic acid (70mL), and the solution stirred at RT for 12h. Evaporation in *vacuo* gave a viscous, light brown oil which, upon trituration with Et₂O/heptane (1 :1, 100mL), afforded the target carboxylic acid **R-2-2** (**R-2** R = CO₂H, R" = CH₃, X = NH, Y = C=O) as a powdery white solid which was collected by suction filtration and dried (3.45g, 96%). **MP** 163-168°C; ^{**1**}**H-NMR** (300 MHz, DMSO-d₆): δ = 10.65 (1), 8.39 (1), 7.43-7.65 (5), 7.26 (2), 5.72 (1), 9.59-4.72 (1), 3.66 (3), 3.05-3.18 (1), 2.75-3.02 (3), 0.72-1.83 (12); **IR** (mull) 3258, 1746, 1664, 1639, 1607, 1562, 1544, 1515, 1432, 1414, 1328, 1277, 1216, 1194, and 799cm⁻¹; **MS** (FAB) m/z (rel. intensity): 573 (MH+, 21), 573 (21), 557 (41), 555 (59), 351 (20), 3,49 (31), 207 (99),. 177 (65), 175 (26), 173 (42), 105 (25); **HRMS** (FAB) calcd. for C₂₉H₃₀Cl₂N₂O₆+H1 573.1559, found 573.1544.

### Example 118

### Scheme R, R-2, wherein R = CO₂H, R" = H, X = NH, Y = C=O N-[[(3E)-3-Carboxymethylene-2,6,6-trimethyl-1-cyclohexen-1-yl]carbonyl]-4-[(2,6-dichlorobenzoyl)amino]-L-phenylalanine (C₂₈H₂₈Cl₂N₂O₆) R-2

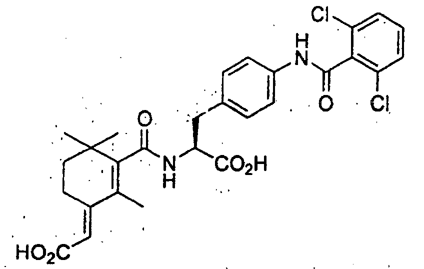

To a stirring solution **R-2-2** (**R-2** R = CO₂H, R" = CH₃, X = NH, Y = C=O) (4.25g, 7.41mmol) in CH₃OH (50mL) was added a solution of LiOH·H₂O (0.62g, 14.8mmol) in H₂O (15mL) and 30% H₂O₂ (25 drops). The solution stirred at RT for 16h and was then evaporated *in vacuo*. The residue was dissolved in H₂O (50mL) and acidified to pH *ca*. 1-2 with 1N aqueous HCl. The resulting gelatinous solid was collected by suction filtration and dried in a vacuum oven at 70°C for 16h. The resulting solid was crushed, washed thoroughly with H₂O, and dried in a vacuum oven at 70°C for 16h to afford the target diacid **R** (R = CO₂H, R" = H, X = NH, Y = C=O) as an off-white, crystalline solid, 3.98g ('96%). **MP:** 175-185°C; ^{**1**}**H-NMR** (300 MHz, DMSO-d6): δ = 11.94-12.80 (1), 10.64 (I), 8.23 (1), 7.44-7.66 (5), 7.24 (2), 5.71 (1), 4.53-4.66 (1), 3.06-3.19 (1), 2.76-3.01 (3), 0.61-1.87 (12); **IR** (mull) 3257, 3195, 3128, 3067, 1712, 1663, 1606, 1563, 1540, 1517, 1432, 1415, 1329, 1275, and 1194cm⁻¹; **MS** (FAB) m/z (rel. intensity): 559 (MH+, 61), 559 (61), 543 (74), 541 (98), 517 (42), 515 (55), 207 (99), 175 (46), 173 (69), 161 (54), 135 (65); **HRMS** (FAB) calcd for C₂₈H₂₈Cl₂N₂O₆+H1 559.1403, found 559.1387.

### Preparation R-2-3

### Scheme R, R-2, wherein R = CO2tBu, R" = CH₃, X = O, Y = CH₂ N-[[(3E)-3-[[(1,1-Dimethylethoxy)carbonyl]methylene]-2,6,6-trimethyl-1-cyclohexen-1-yl]carbonyl]-4-O-(2,6-dichlorophenylmethyl)-L-tyrosine methyl ester (C₃₃H₃₉Cl₂NO₆) R-2

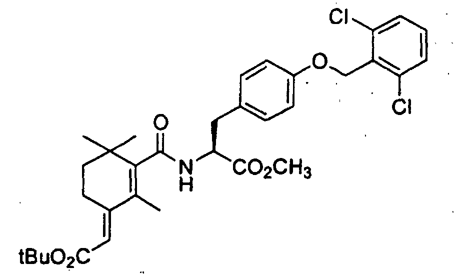

To a stirring solution of the carboxylic acid **R-1** (R = CO₂tBu, R' = H) (1.10g, 3.92mmol) in DMF (30mL) was added 4-*O*-(2,6-dichlorophenylmethyl)-L-tyrosine methyl ester hydrochloride (2.30g, 5.89mmol), HATU (1.79g, 4.71 mmol), and diisopropylethylamine (2.7mL, 15.7mmol). The solution stirred at RT for 16h and then was evaporated *in vacuo.* The residue was partitioned between 1M aqueous HCl (150mL) and CH₂Cl₂ (150mL). The organic phase was washed with saturated aqueous NaHCO₃ (150L), brine (150mL), dried (Na₂SO₄), filtered, and evaporated *in vacuo*. The resulting amber oil was chromatographed on silica gel (300g, 230-400 mesh, 70mm OD column, packed and eluted with CH₂Cl₂ (2L), then EtOAc/CH₂Cl₂ (5:95), collecting 270mL fractions) using the flash technique. Fractions 16-20 were combined and evaporated *in vacuo* to afford the desired product **R-2-3** (R = CO2tBu, R" = CH₃, X = O, Y = CH₂) as a light yellow solid, 0.87g (36%). ^{**1**}**H-NMR** (300 MHz, CDCl₃): δ = 7.33-7.38 (2), 7.21-7.28 (1), 7.10 (2), 6.95 (2), 5.69-5.73 (2), 5.24 (2), 5.01 (1), 3.76 (3), 3.18 (1), 2.94-3.07 (3), 1.66-1.69 (4), 1.46-1.55 (10), 1.09 (3), 1.00 (3); **IR** (mull) 1746, 1705, 1636, 1611, 1586, 1512, 1439, 1301, 1277, 1241, 1196, 1179, 1152, 1134, and 1018cm⁻¹; **HRMS** (EI) : calcd for C₃₃H₃₉Cl₂NO₆ 615.2155, Found: 615.2156. **Anal.** Calcd for C₃₃H₃₉Cl₂NO₆: C, 64,28; H, 6.38; N, 2.27; Cl, 11.50. Found: C, 63.91; H, 6.38; N, 2.52; Cl, 11.33.

### Preparation R-2-4

### Scheme R, R-2, wherein R = CO₂H, R" = CH₃, X = O, Y₁ = CH₂ N-[[(3E)-3-Carboxymethylene-2,6,6-trimethyl-1-cyclohexen-1-yl]carbonyl]-4-O-(2,6-dichlorophenylmethyl)-L-tyrosine methyl ester (C₂₉H₃₁Cl₂NO₆) R-2

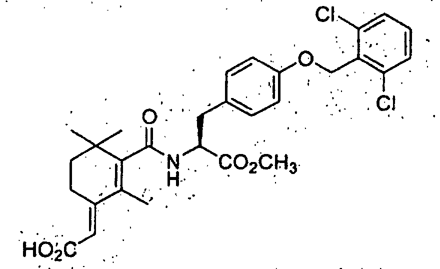

The diester (0.80g, 1.30mmol) was dissolved in trifluoroacetic acid (15mL), and the solution stirred at RT for 16h. Evaporation *in vacuo* gave a viscous, amber oil which, upon trituration with Et₂O/hexanes (1: 1, 100mL), afforded the target carboxylic acid **R-2** (R = CO₂H, R" = CH₃, X = O, Y = CH₂) as a powdery white solid which was collected by suction filtration and dried, 0.63g (86%). **MP:** 140-143°C; ^{**1**}**H-NMR** (300 MHz, CDCl₃): δ = 7.37 (2), 7.26 (2), 7.11 (2), 6.96(2), 5.82-5.89(2), 5.24 (2), 5.05(1), 3.78(3), 2.90-3.25 (4), 1.70 (3), 1.54 (2), 1.09 (3), 1.00 (3).

### Example 119

### Scheme R, R-2, wherein R = CO₂H, R" = H, X = O, Y = CH₂ N-[[(3E)-3-Carboxymethylene-2,6,6-trimethyl-1-cyclohexen-1-yl]carbonyl]-4-O-(2,6-dichlorophenylmethyl)-L-tyrosine (C₂₈H₂₉Cl₂NO₆) R-2

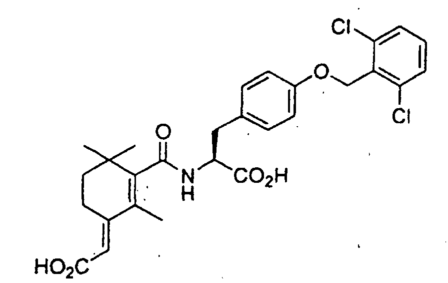

To a stirring solution of the methyl ester **R-2** (R = CO₂H, R" = H, X = O, Y = CH₂) (0.59g, 1.05mmol) in CH₃OH (10mL) was added ,a solution of LiOH·H₂O (0.09g, 2.11mmol) in H₂O (5mL) and 30% H₂O₂ (5 drops). The solution stirred at RT for 16h and was then evaporated *in vacuo*. The residue was dissolved in H₂O (50mL) and acidified to pH ca. 1-2 with 1N aqueous HCl. The resulting gelatinous solid was collected by suction filtration and dried in a vacuum oven at 70°C for 16h. The resulting solid was crushed, washed thoroughly with H₂O, and dried in a vacuum oven at 70°C for 16h to afford the target diacid **R-2** (R = CO₂H, R" = H, X = O, Y = CH₂) as an off-white, crystalline solid, 0.47g (82%). **MP:** 115-138°C; ^{**1**}**H-NMR** (300 MHz, DMSO-d₆) : d = 11.67-12.88 (1), 8.15 (1), 7.54 (2), 7.40-7.48 (1), 7.20 (2), 6.93 (2), 5.68 (1), 5.16 (2), 4.47-4.58 (1), 3.04-3.13 (1), 2.73-2.95 (4), 1.33-1.65 (5), 0.68-0.98 (6): **IR** (mull): 3058, 1693, 1611, 1601, 1565, 1539, 1512, 1439, 1299, 1278, 1244, 1196, 1179, 777, and 769cm⁻¹: **HRMS** (EI) calcd for C₂₈H₂₉Cl₂NO₆ 545.1372, found 545.1377; **% Water** (KF) : 2.53.

### Preparation R-1-4

### Scheme R, R-1, wherein R = CN, R' = CH₃ 3-Carbomethoxy-2,4,4-trimethyl-2-cyclohexen-1-ylindeneacetonitrile, (E) (C₁₃H₁₇NO₂)

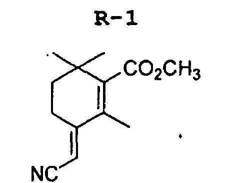

Sodium hydride (0.73g of a 60% oil dispersion, 18.3mmol) was washed with hexane (3 x 5 mL) and suspended in THF (10mL) and cooled to 0°C. To this suspension was added a solution of t-butyl diethylphosphonoacetate (4.60g, 26.0mmol) in THF (10mL) dropwise. After stirring at 0°C for 1h, the ice water bath was removed and a solution of the ketone, 3-carbomethoxy-2,4,4-trimethyl-2-cyclohexen-1-one (Boulin, B.; Miguel, B. Arreguy-San; D.B. *Tetrahedron* **1998,** *54*, 2753) , (3.00g, 15.3mmol) in THF(10mL) was added dropwise. The reaction mixture stirred at RT for 16h before being evaporated in *vacuo.* The residue was partitioned between H₂O (100mL) and EtOAc (100ml), and the aqueous phase was extracted with EtOAc (50mL). The combined organic phases were washed with H₂O (100mL), brine (100mL), filtered, and evaporated *in vacuo.* The resulting light brown oil was chromatographed on silica gel (400g, 230-400 mesh, 70mm OD column, packed and eluted with Et₂O/hexanes, 15:85, collecting 270mL fractions) using the flash technique. Fractions 11-18 were combined and evaporated *in vacuo* to afford the vinyl nitrile **R-1** (R = CN, R' = CH₃) as a mixture of geometric isomers (E:Z = 3.5:1, as determined by ¹H-NMR); 3.20g (95%) of clear, colorless oil. ^{**1**}**H-NMR** (300 MHz, CDCl₃): δ = 5.30 (0.78), 5.19 (0.22), 3.79 (3), 2.74-2.80 (1.56), 2.44-2.51 (0.44), 2.14 (0.66), 1.75 (2.34), 1.60-1.67 (2), 1.14 (4.68), 1.13 (1.32); IR (neat) 2966, 2954, 2871, 2212, 1726, 1584, 1450, 1433, 1330, 1291, 1247, 1222, 1067, 1027, and 802cm⁻¹; **MS** (EI ) m/z (rel. intensity): 219 (M+, 19), 219 (19), 188 (12), 172 (16), 161 (13), 160 (99), 159 (13), 144 (12), 133 (8), 118 (11), 91 (9); **Anal.** Calcd for C₁₃H₁₇NO₂: C, 71.21; H, 7.81; N, 6.39. Found: C, 71.04; H, 7.74; N, 6.37.

### Preparation R-1-5

### Scheme R, R-1, wherein R = CN, R' = H 3-Carboxy-2,4,4-trimethyl-2-cyclohexen-1-ylideneacetonitrile, (E) (C₁₅H₁₅NO₂)

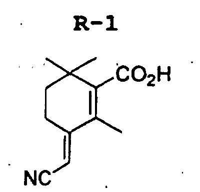

To a stirring solution of the nitrile **R-1** (R = CN, R' = CH₃) (2.05g, 9.35mmol) in DMF (35mL) was added KOH (0.58g, 10.3mmol) and thiophenol (1.1mL, 10.3mmol), and the mixture stirred at 100°C for 16h. After cooling to RT, the reaction mixture was evaporated *in vacuo.* The residue was partitioned between 5% aqueous NaOH (100mL) and Et₂O (100mL), and the organic phase was discarded. Then, 6N aqueous HCl was added dropwise to the aqueous phase until the pH reached ca. 1, and it was then extracted with Et₂O (2x100mL). The combined extracts were washed with H₂O (100mL), brine (100mL), dried (Na₂SO₄), filtered, and evaporated *in vacuo* to afford the carboxylic acid **R-1** (R = CN, R' = H) as a mixture of geometric isomers (E:Z = 4.7: 1, as determined by ¹H NMR); 1.81g (94%) as a white solid. **MP:** 88-92°C; ^{**1**}**H-NMR** (300 MHz, CDCl₃): δ = 8.02-9.24 (1), 5.36 (0.82), 5.24 (0.18), 2.77-2.84 (1.64), 2.48-2.54 (0.36), 2.27 (0.54), 1.88 (2.46), 1.64-1.71 (2), 1.22 (4.92), 1.21 (1.08); **MS** (ESI-) for C₁₅H₁₅NO₂ *m*/*z* 204.2 (M-H).

### Preparation R-2-5

### Scheme R, R-2, wherein R = CN, R" = CH₃, X = NH, Y = C=O N-[[(3E)-3-Cyanomethylene-2,6,6-trimethyl-1-cyclohexen-1-yl]carbonyl]-4-[(2,6-dichlorobenzoyl)amino]-L-phenylalanine methyl ester (C₂₉H₂₉Cl₂N₃O₄) R-2

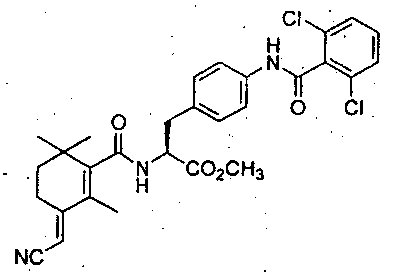

To a stirring solution of the carboxylic acid **R-1** (R = CN, R' = H) (1.80g, 8.77mmol) in DMF (35mL) was added 4-[(2,6-dichlorobenzoyl)amino]-L-phenylalanine methyl ester. hydrochloride (7.08g, 17.5mmol), HATU (4.00g, 1 0.5mmol), and diisopropylethylamine (6.1mL, 35.1 mmol). The solution stirred at RT for 62h and then was evaporated *in vacuo.* The residue was partitioned between 1N aqueous HCI (250mL) and CH₂Cl₂ (250mL), and the aqueous phase was extracted with CH₂Cl₂ (250mL). The combined extracts were washed with saturated aqueous NaHCO₃ (500mL), brine (500mL), dried (Na₂SO₄), filtered, and evaporated *in vacuo*. The resulting orange oil was chromatographed on silica gel (400g, 230-400 mesh, 70mm OD column, packed and eluted with EtOAc/CH₂Cl₂ (1:9), collecting 260mL fractions) using the flash technique. Fractions 12-25 were combined and evaporated in *vacuo* to afford the desired product **R-2** (R = CN, R" = CH₃, X = NH, Y = C=O) as a mixture of geometric isomers (E:Z = 4.2:1, as determined by ¹H-NMR), 4.93g (100%) of light yellow solid. **MP:** 120°C; ^{**1**}**H-NMR** (300 MHz, DMSO-d₆): δ = 10.65 (1), 8.48 (1), 7.43 7.62 (5), 7.23 (2), 5.55 (0.81), 5.52 (0.19), 4.58-4.69 (1), 3.64 (3), 3.33 (3), 3.10 (1), 2.81-2.92 (1), 2.62 (1.62), 2.37-2.45 (0.38), 1.42-1.69 (2), 0.68-1.05 (6); **IR** (mull) 3292, 1745, 1665, 1642, 1605, 1580, 1562, 1536, 1517, 1431, 1414, 1325, 1270, 1217, and 799cm⁻¹; **HRMS** (EI) calcd for C₂₉H₂₉Cl₂N₃O₄ 553.1535, found 553.1551.

### Example 120

### Scheme R; R-2, wherein R = CN, R" = H, X = NH, Y = C=O N-[[(3E)-3-Cyanomethylene-2,6,6-trimethyl-1-cyclohexen-1-yl]carbonyl]-4-[(2,6-dichlorobenzoyl)amino]-L-phenylalanine (C₂₈H₂₇Cl₂N₃O₄) R-2

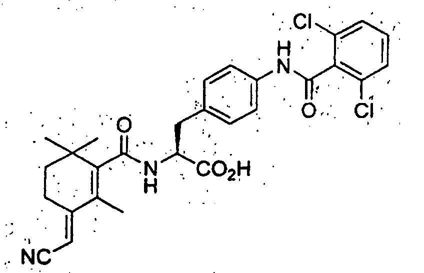

To a stirring solution of the methyl ester **R-2** (R = CN, R" = CH₃, X = NH, Y = C=O) (1.80g, 3.25mmol) in CH₃OH (40mL) was added a solution of LiOH^{·}H₂O (0.27g, 6.50mmol) in H₂O (10mL) and 30% H₂O₂ (20 drops). The solution was stirred at RT for 16h and was then evaporated *in vacuo*. The residue was dissolved in H₂O (50mL) and acidified to pH *ca*. 1-2 with 1N aqueous HCl. The resulting gelatinous solid was collected by suction filtration and dried in a vacuum oven at 70°C for 16h. The resulting solid was crushed, washed thoroughly with H₂O, and dried in a vacuum oven at 70°C for 16h to afford the target carboxylic acid **R-2** (R = CN, R" = H, X = NH, Y = C=O) as an off-white, crystalline solid, 1.43g (81%). **MP:** 172-180°C; ^{**1**}**H-NMR** (300 MHz, DMSO-d₆): δ = 12.05-13.42 (1), 10.63 (1), 8.30 (1), 7.42-7.61 (5), 7.23 (2), 5.52 (1), 4.50-4.62 (1), 2.34-3.55 (7), 1.41-1.68 (2), 0.71-1.06 (6); **IR** (mull) 3291, 1734, 1664, 1645, 1605, 1581, 1562, 1537, 1517, 1432, 1414, 1327, 1272, 1195, and 799cm⁻¹; **MS** (FAB) *m*/*z* (rel. intensify): 540 (MH+, 80), 543 (20), 542 (57), 541 (32), 540 (8()), 335 (17), 189 (18), 188 (99), 175 (19), 173 (30), 161 (41); **HRMS** (FAB) calcd for C₂₈H₂₇Cl₂N₃O₄+H1, 540.1457, found 540.1436; **% Water** (KF): 3.07.

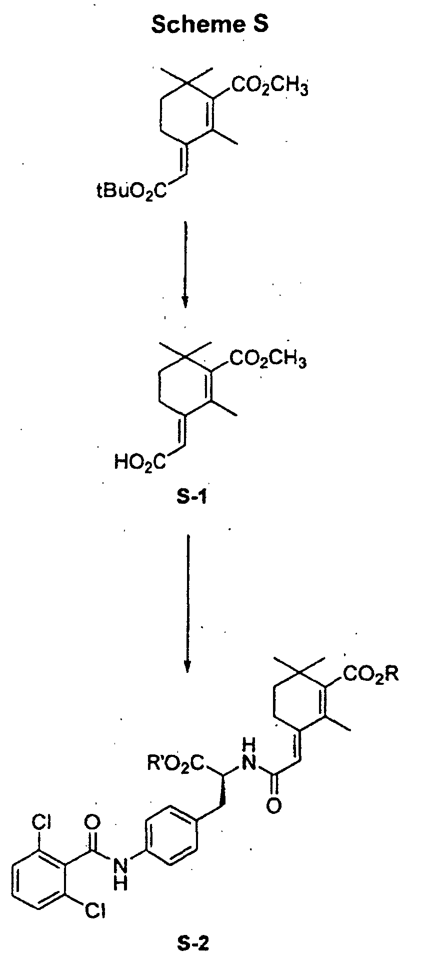

### Preparation S-1

### Scheme S, S-1 (3E)-3-Carbomethoxy-2,4,4-trimethyl-2-cyclohexen-1-ylideneacetic acid (C₁₃H₁₈O₄) S-1

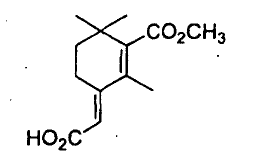

A solution of 3-carbomethoxy-2,4,4-trimethyl-2-cyclohexen-1-ylideneacetic acid, 1,1-dimethylethyl ester, prepared as described in Scheme R (**Preparation R-1-1,** Scheme R, **R-1,** wherein R = CO₂tBu, R' = CH₃) (1.82g, 6.18mmol) was stirred in TFA (10mL) at RT for 16h and was then evaporated *in vacuo.* The resulting black oil was chromatographed on silica gel (150g, 230-400 mesh, 35mmOD column, packed and eluted with MeOH/CH₂Cl₂, 2.5:97.5, collecting 30mL fractions) using the flash technique. Fractions 24-56 were combined and evaporated in vacuo to afford 0.92g (62%) of the carboxylic acid **S-1** as an off-white solid, as an 83:17 mixture of E/Z geometric isomers. **MP** 115-120°C (sublimes); TLC: R_{f} = 0.31 (MeOH/CH₂Cl₂, 7.5:92.5); ^{**1**}**H-NMR** (300 MHz, CDCl₃) : δ = 5.92 (0.83), 5.73 (0.17), 3.82 (2.49), 3.80 (0.51), 3.06 (1.63), 2.43 (0.37), 1.89 (0.54), 1.81 (2.46), 1.61 (2), 1.14 (1.02), 1.13 (4.98); **IR** (mull) 1718, 1685, 1664, 1600, 1433, 1416, 1326, 1303, 1288, 1258, 1227, 1210, 1060, 1027, and 866cm⁻¹; **MS** (EI ) *m*/*z* (rel. intensity) 238 (M+, 30), 238 (30), 207 (31), 206 (33), 179 (99), 178 (38), 161 (51), 133 (63), 119 (33), 91 (41), 77 (24); **Anal.** Calcd for C₁₃H₁₈O₄: C, 65.53; H, 7.61. Found: C, 65.32; H, 7.79.

### Preparation S-2-1

### Scheme S, S-2, wherein R = R' = CH₃ N-[(1E)-3-Carbomethoxy-2,4,4-trimethyl-2-cyclohexen-1-ylideneacetyl]-4-[(2,6-dichlorobenzoyl)amino]-L-phenylalanine methyl ester (C₃₀H₃₂Cl₂N₂O₆) S-2

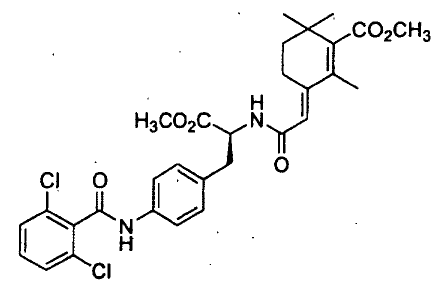

To a stirring solution of the carboxylic acid **S-1** (0.88g, 3.69mmol) in CH₂Cl₂ (15mL) was 4-[(2, 6-dichlorobenzoyl)amino]-L-phenylalanine methyl ester hydrochloride (1.49g, 3.69mmol), EDC (0.71g, 3.69mmol), DMAP (0.14g, 1.10mmol), HOBT (0.50g, 3.69mmol) and triethylamine (3mL). The solution was allowed to stir at RT for 16h and then was evaporated *in vacuo*. The residue was partitioned between 1N aqueous HCl (150mL) and CH₂Cl₂ (150mL). the organic phase was washed with saturated aqueous NaHCO₃ (150mL), brine (150mL), dried (Na₂SO₄), filtered, and evaporated *in vacuo*. The resulting yellow solid was chromatographed on silica gel (400g, 230-400 mesh, 70mm OD column, packed and eluted with MeOH/CH₂Cl₂, 5:95, collecting 150mL fractions) using the flash technique. Fractions 4-7 were combined and evaporated *in vacuo* to afford the desired product **S-2** (R = R' = CH₃) as a yellow solid, 1.31g (60%). An analytical sample was obtained by recrystallization from CH₂Cl₂/CH₃OH/Et₂O. **MP:** 193°C; **TLC:** Rf = 0.22 (MeOH/CH₂Cl₂, 5:95); ^{**1**}**H-NMR** (300 MHz, DMSO-d₆): δ = 10.66 (1), 8.39 (1), 7.52-7.61 (4), 7.44-7.50 (1), 7.19 (2), 6.00 (1), 4.51 (1), 3.70 (3), 3.62 (3), 3.01 (1), 2.78-2.91 (3 ), 1.68 (3), 1.42 (2), 1.01 (6); **IR** (mull) 1749, 1723, 1669, 1643, 1612, 1562, 1553, 1534, 1514, 1433, 1335, 1281, 1243, 1218, 782cm⁻¹; **MS** (FAB) m/z (rel. intensity): 587 (MH+, 13), 589 (8), 587 (13), 557 (8), 555 (12), 222 (15), 221 (99), 175 (14), 173 (23), 161 (21), 133 (14); **HRMS** (FAB) calcd for C₃₀H₃₂Cl₂N₂O₆+H, 587.1715, found 587.1705; **Anal.** Calcd for C₃₀H₃₂Cl₂N₂O₆: C, 61.33; H, 5.49; N, 4.77. Found: C, 60.94; H, 5.42; N, 4.80.

### Example 121 Scheme S, S-2, wherein R = R' = H N-[(1E)-3-Carboxy-2,4,4-trimethyl-2-cyclohexen-1-ylideneacetyl]-4-[(2,6-dichlorobenzoyl)amino]-L-phenylalanine (C₂₈H₂₈Cl₂N₂O₆) S-2

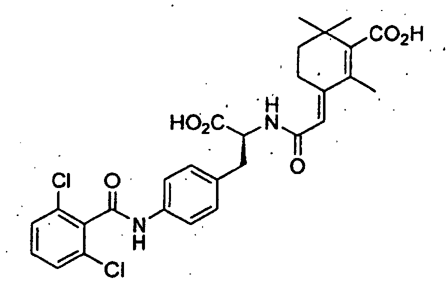

To a stirring solution of the diester **S-2** (R = R' = CH₃) (1.13g, 1.92mmol) in DMF (15mL) was added KOH (0.24g, 4.23mmol) and thiophenol (0.44mL, 4.23mmol), and the mixture was heated at 100°C for 16h. Evaporation *in vacuo* afforded a brown residue which was dissolved in H₂O (50mL). To this solution was added 1N aqueous HCl until the pH of the mixture was *ca*. 3, and it was extracted with MeOH/Et₂O (5:95, 50mL). The extract was washed with brine (50mL), dried (Na₂SO₄), filtered, and evaporated *in vacuo.* The resulting orange solid was recrystallized from EtOAc/cyclohexane to give the diacid **S-2** (R = R' = H) as a light yellow solid. Residual volatiles were removed by heating (100°C) in a vacuum oven for 6h to give 0.90g (84%) of **S-2** (R = R' = H). **MP:** 159°C (decomposition); ^{**1**}**H-NMR** (300 MHz, DMSO-d₆): δ = 12.70 (1), 10.65 (1), 8.23 (1), 7.44-7.60 (6), 7.20, (2), 5.97 .(1), 4.46 (1), 3.03 ( 1), 2.77-2.88 (3), 1.73 (3), 1.41 (2), 1.05 (6); **IR** (mull) 3265, 3123, 3055, 3035, 1720, 1645, 1605, 1562, 1537, 1516, 1432, 1414, 1326, 1217, and 1196cm⁻¹; **MS** (FAB) *m*/*z* (rel. intensity): 559 (MH+, 27), 559 (27), 543 (30), 541 (41), 335 (18), 221 (51), 207 (99), 175 (31), 173 (48), 161 (30), 133 (19); **HRMS** (FAB) calcd for C₂₈H₂₈Cl₂N₂O₆+H1 559.1403, found 559.1398; **% Water** (KF): 1.24.

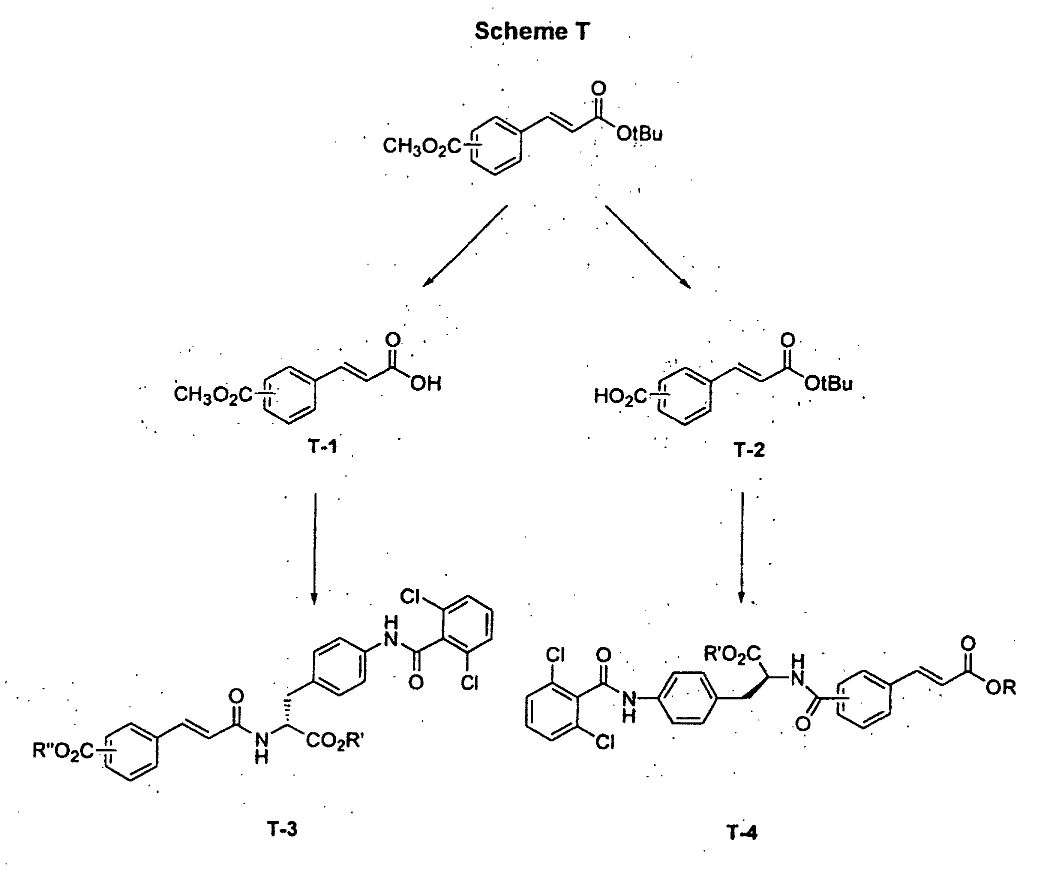

### Preparation T-1-1

### Scheme T, T-1, wherein 3-CO₂CH₃ (E)-3-[3-Hydroxy-3-oxo-1-propenyl]benzoic acid methyl ester (C₁₁H₁₀O₄)

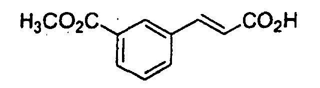

A solution of the known diester 3-[3-(1,1-dimethylethoxy)-3-oxo-1-propenyl]benzoic acid methyl ester (Mjalli, A.M.M.; Cao, X.; Moran, E.J. PCT Int. Appl., 89pp. CODEN: PIXXD2. WO 9708934 A2 970313. CAN 126:277769) (1.00g, 3.81mmol) in TFA (10mL) was stirred at RT for 16 hours. Evaporation *in vacuo* afforded 0.83g (100%) of the **T-1** (3-CO₂CH₃) as a white solid. **MP:** 186-187°C; ^{**1**}**H-NMR**(300MHz, DMSO-d₆): δ = 8.16(1), 7.94(2), 7.52-7.67(2), 6.59(1), 3.85(3); **IR** (mull) 1730, 1678, 1634, 1438, 1346, 1329, 1297, 1227, 1201, 1079, 985, 871, 757, 721, and 670cm⁻¹; **MS** (EI ) m/z (rel. intensity) 206 (M⁺, 54), 206 (54), 175 (99), 174 (14), 147 (24), 130 (14), 129 (19), 102 (16), 91 (33), 65 (16), 51 (19); **HRMS** (EI) calcd for C₁₁H₁₀O₄ 206.0579, found 206.0586.

### Preparation T-1-2

### Scheme T, T-1, wherein 4-CO₂CH₃ (E)-4-[3-Hydroxy-3-oxo-1-propenyl]benzoic acid methyl ester (C₁₁H₁₀O₄)

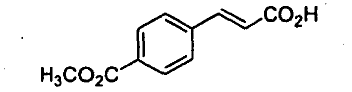

A solution of the known diester 4-[3-(1,1-dimethylethoxy)-3-oxo-1-propenyl]benzoic acid methyl ester (Mjalli, A.M.M.; Cao, X.; Moran, E.J. PCT Int. Appl., 89pp. CODEN: PIXXD2. WO 9708934 A2 970313. CAN 126:277769) (1.00g, 3.81mmol) in TFA (10mL) was stirred at RT for 2 hours. Evaporation *in vacuo* afforded 0.79g (100%) of the title compound **T-1** (4-CO₂CH₃) as a white solid. ^{**1**}**H-NMR**(300MHz, DMSO-d₆): δ = 7.94(2), 7.80(2), 7.62(1), 6.62(1), 3.84(3); **IR** (mull): 1715, 1685, 1634, 1569, 1438, 1426, 1329, 1309, 1284, 1227, 1185, 1111, 992, 775, and 72 cm⁻¹; **MS** (EI ): *m*/*z* (rel. intensity) 206 (M⁺, 44), 206 (44), 191 (19), 176 (12), 175 (99), 147 (28), 102 (12), 101 (9), 91 (23), 65 (11), 51 (10); **HRMS** (EI) calcd for C₁₁H₁₀O₄ 206.0579, found 206.0583.

### Preparation T-3-1

### Scheme T, T-3, wherein 3-substituted, R = CH₃, R" = CH₃ N-[(E)-3-(3-Carbomethoxyphenyl)-1-oxo-2-propenyl]-4-[(2,6-dichlorobenzoyl)amino]-L-phenylalanine methyl ester T-3 (C₂₈H₂₄Cl₂N₂O₆)

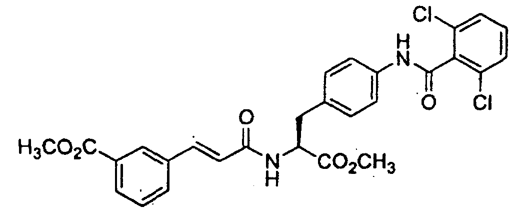

To a stirring suspension of the acid **T-1** (3-CO₂CH₃) (0.73g, 3.54mmol) in CH₂CL₂(30mL) was added 4-[(2,6-dichlorobenzoyl)amino]-L-phenylalanine methyl ester. hydrochloride (1.43g, 3.54mmol), EDC(0.69g, 3.54mmol), DMAP (0.13g, 1.06mmol), and HOBT(0.48g, 3.54mmol). To this mixture was added Et₃N dropwise until all solids went into solution. After stirring for 16h at RT, the resulting precipitate was collected by suction filtration, washed with CH₂CT₂ and Et₂O, and dried to afford 1.26g(64%) of **T-2-1** (3-CO₂CH₃) as an off-white solid. **MP:** 216-218°C; ^{**1**}**H-NMR**(300MHz, DMSO-d₆) : δ = 10.35(1), 8.58(1), 8.13(1), 7.93(1), 7.81(1), 7.43-7.60(6), 7.21(2), 6.82(1), 4.61(1), 3.86(3), 3.64(3), 2.88-3.10(3); **IR** (mull): 1746, 1741, 1728, 1670, 1655, 1624, 1611, 1562, 1552, 1513, 1444, 1433, 1334, 1282, and 1207 cm⁻¹; **MS** (FAB) *m*/*z* (rel. intensity): 555 (MH⁺, 68), 558 (16), 557 (48), 556 (26), 555 (68), 349 (21), 189 (58), 175 (20), 174 (99), 173 (30), 103 (15); **HRMS** (FAB) calcd for C₂₈H₂₄CL₂N₂O₆+H₁ 555.1089, found 555.1083.

### Example 122

### Scheme T, T-3, wherein 3-substituted, R = H, R" = H N-[(E)-3-(3-Carboxyphenyl)-1-oxo-2-propenyl]-4-[(2,6-dichlorobenzoyl)amino]-L-phenylalanine methyl ester T-3 (C₂₆H₂₀Cl₂N₂O₆)

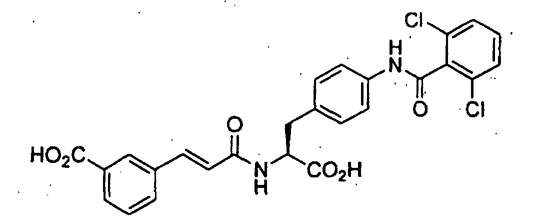

To a stirring solution of the diester **T-3** (3-substituted, R = CH₃, R" = CH₃) (0.83g, 1.50mmol) in MeOH/DMSO (1:1, 20mL) was added a solution of LiOH•H₂O (0.12g, 3.00mmol) in H₂O (5mL). After stirring for 16h at RT, the reaction mixture was evaporated *in vacuo.* The residue was dissolved in H₂O (25mL) and 6M aq. HCl was added until the pH of the mixture was ca. 1. The resulting gelatinous solid was collected by suction filtration and dried in the vacuum oven at 70°C for 16h. The resulting solid was washed with H₂O and dried in the vacuum oven at 70°C for 16h to afford 0.33g (42%) of **T-3** (3-substituted, R = H, R" = H) as a light yellow solid. **MP:** 165°C; ^{**1**}**H-MMR**(300MHz, DMSO-d₆): δ = 10.37(1), 8.37(1), 8.12(1), 7.90(1), 7.75(1), 7.40-7.58(6), 7.21(2), 6.84(1), 4.5491), 2.80-3.15(3); **IR** (mull) 3280, 3062, 1709, 1661, 1606, 1584, 1562, 1539, 1516, 1432, 1414, 1326, 1267, 1196, and 799 cm⁻¹; **MS** (FAB) *m*/*z* (rel. intensity) 527 (MH⁺, 51), 530 (11), 529 (32), 528 (17), 527 (51), 225 (12), 175 (25), 174 (99), 173 (9), 161 (16), 12 3 (12); **HRMS** (FAB) calcd for C₂₆H₂₀CL₂N₂O₆+H₁ 527.0776, found 527.0770.

### Preparation T-3-2

### Scheme T, T-3, wherein 4-substituted, R = CH₃, R" = CH₃ N-[(E)-3-(4-Carbomethoxyphenyl)-1-oxo-2-propenyl]-4-[(2,6-dichlorobenzoyl)amino]-L-phenylalanine methyl ester T-3 (C₂₈H₂₄Cl₂N₂O₆)

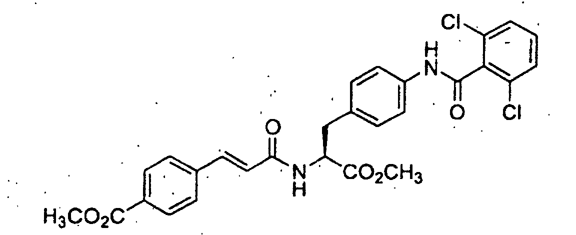

To a stirring suspension of the acid **T-1** (4-CO₂CH₃) (0.74g, 3.59mmol) in CH₂CL₂(30mL) was added the 4-[(2,6-dichlorobenzoyl)amino]-L-phenylalanine methyl ester. hydrochloride (1.45g, 3.59mmol), EDC (0.69g, 3.59mmol), DMAP (0.13g, 1.08mmol), and HOBT (0.48g, 3.59mmol). To this mixture was added N,N-diisopropyl ethylamine (3mL). After stirring for 16h at RT, the reaction mixture was evaporated *in vacuo*. The residue was partitioned between CH₂Cl₂(50mL) and 1N aq. HCl (50mL), and an insoluble white solid formed. This was collected by suction filtration, washed with H₂O, washed with Et₂O, and dried to afford 1.28g(64%) of the title compound **T-3** (4- CO₂CH₃, R = CH₃, R" = CH₃). **MP:** >250°C; ^{**1**}**H-NMR**(300MHz, DMSO-d₆): δ = 8.62(1), 7.96(2), 7.68(2), 7.42-7.60(6), 7.21(2), 6.81(1), 4.61(1), 3.84(3), 3.64(3), 2.89-3.10(2); **IR** (mull) 3284, 3071, 1745, 1721, 1671, 1654, 1622, 1611, 1554, 1513, 1433, 1416, 1337, 1283, and 121 cm⁻¹; **MS** (FAB) : *m*/*z* (rel. intensity) 555 (MH⁺, 99), 558 (21), 557 (70), 556 (35), 555 (99), 349 (22), 189 (73), 175 (19), 174 (21), 173 (27), 130 (38); **HRMS** (FAB) calcd for C₂₈H₂₄CL₂N₂O₆+H₁ 555.1089, found

### Example 123

### Scheme T, T-3, wherein 4-substituted, R = H, R" = H N-[(E)-3-(4-Carboxyphenyl)-1-oxo-2-propenyl]-4-[(2,6-dichlorobenzoyl)amino]-L-phenylalanine methyl ester T-3 (C₂₆H₂₀Cl₂N₂O₆)

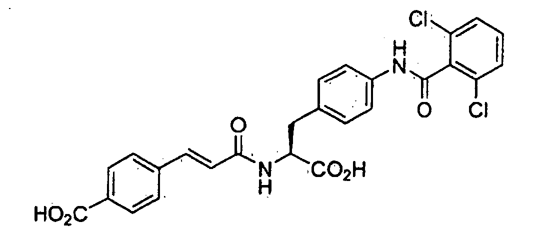

To a stirring solution of the diester (4- CO₂CH₃, R = CH₃, R" = CH₃) (0.75g, 1.35mmol) in MeOH/DMSO (3:1, 20mL) was added a solution, of LiOH·H₂O (0.23g, 5.40mmol) in H₂O(5mL). After stirring for 16h at RT, the reaction mixture was evaporated *in vacuo*. The residue was dissolved in H₂O (30mL) and 6N aq. HCl was added until the pH of the mixture was *ca*. 1. The resulting precipitate was collected by suction filtration, washed with H₂O, and dried in a vacuum oven at 70°C for 16 hours to afford 0.69g (97%) of the title compound **T-3** (4-CO₂H) as a white solid. **MP:** 177-181°C; ^{**1**}**H-NMR**(300MHz, DMSO-d₆): δ = 8.46(1), 17.93(2), 7.64(2), 7.40-7.58(6), 7.21(2), 6.82(1), 4.54(1),2.86-3.13(2); **IR** (mull) 3278, 1708, 1661, 1608, 1562, 1538, 1517, 1432, 1415, 1325, 1270, 1230, 1196, 1179, and 778cm⁻¹; **MS** (FAB): *m*/*z* (rel. intensity) 527 (MH⁺, 99), 531 (11), 530 (20), 529 (73), 528 (33), 527 (99) , 335 (11), 315 (13), 175 (64), 173 (15), 161 (45); **HRMS** (FAB) calcd for C₂₆H₂₀CL₂N₂O₆+H₁ 527.0776, found 527.0770.

### Preparation T-2-1

### Scheme T, T-2, wherein 3-CO₂H (E)-3-[3-(1,1-dimethylethoxy)-3-oxo-1-propenyl]benzoic acid T-2 (C₁₄H₁₆O₄)

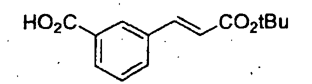

To a stirring solution of the known diester 3-[3-(1,1-dimethylethoxy)-3-oxo-1-propenyl]benzoic acid methyl ester (Mjalli, A.M.M.; Cao, X.; Moran, E.J. PCT Int. Appl., 89pp. CODEN: PIXXD2. WO 9708934 A2 970313. CAN 126:277769) (1.00g, 3.81mmol) in CH₃OH (10mL) was added a solution of LiOH•H₂O (0.32g, 7.62mmol) in H₂O (5mL). After stirring at RT for 16 hours, the reaction mixture was evaporated *in vacuo*. The residue was dissolved in H₂O (25mL), and 6N aq. HCl was added until the pH of the mixture was *ca*. 4-5. The resulting white precipitate was extracted with EtOAc/THF (1:1, 2X25mL). The combined extracts were washed with sat'd. aq. NaCl (50mL), dried (Na₂SO₄), filtered, and evaporated *in vacuo.* The resulting white solid was dissolved in MeOH and chromatographed (flash) on silica gel (300g, 230-400 mesh, 70mm OD, column, packed and eluted with MeOH/CH₂Cl₂ (1:9), collecting 200 mL fractions). Fractions 4-18 were combined and evaporated *in vacuo* to give 0.42g(44%) of **T-2** (3-CO₂H) as a white solid. **MP:** 132-136°C; ^{**1**}**H-NMR**(300MHz, DMSO-d₆): δ = 8.14(1), 7.86-7.96(2), 7.59(1), 7.98(1), 6.54(1), 1.46(9); **IR** (mull) 1703, 1693, 1641, 1605, 1585, 1415, 1326, 1291, 1270, 1260, 1212, 1157, 975, 755, and 664cm⁻¹; **MS** (EI ) *m*/*z* (rel. intensity): 248 (M⁺, 8), 193 (44), 192 (83), 191 (19), 175 (80), 174 (43), 157 (19), 130 (22), 57 (99), 56 (78), 55 (24); **HRMS** (EI) calcd for C₁₄H₁₆O₄ 248.1048, found 248.1055.

### Preparation T-2-2

### Scheme T, T-2, wherein 4-CO₂H (E)-4-[3-(1,1-dimethylethoxy)-3-oxo-1-propenyl]benzoic acid T-2 (C₁₄H₁₆O₄)

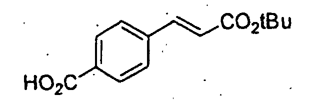

To a stirring solution of the known diester 4-[3-(1,1-dimethylethoxy)-3-oxo-1-propenyl]benzoic acid methyl ester (Mjalli, A.M.M.; Cao, X.; Moran, E.J. PCT Int. Appl., 89pp. CODEN: PIXXD2. WO 9708934 A2 970313. CAN 126:277769) (1.00g, 3..81mmol) in MeOH(20mL) was added a solution of LiOH•H₂O (0.40g, 9.50mmol) in H₂O(5mL). After stirring for 16h at RT, the reaction mixture was evaporated *in vacuo* and 6N aq. HCl was added until the pH of the mixture was *ca*. 1. The resulting mixture was extracted with Et₂O (2X25mL). The combined organic phases were washed with saturated aq. NaCl, dried (Na₂SO₄), filtered, and evaporated *in vacuo* to afford 0.41g(43%) of the title compound **T-2,** (4-CO₂H) as a white solid. MP: sublimes; ^{**1**}**H-NMR**(300MHz, DMSO-d₆): δ = 7.92(2), 7.78(2), 7.58(1), 6.60(1), 1.47(9); **IR** (mull) 1705, 1679, 1608, 1426, 1412, 1322, 1296, 1256, 1180, 1156, 1129, 1113, 975, 966, and 781cm⁻¹; **MS** (EI): *m*/*z* (rel. intensity) 248 (M⁺, 11), 248 (11), 193 (49), 192 (99), 191 (39), 175 (62), 147 (52), 103 (18), 77 (15), 57 (73), 56 (19); **Anal.** Calcd for C₁₄H₁₆O₄: C, 67.73; H, 6.50.
Found: C, 67.49; H, 6.76; N, 0.22.

### Preparation T-4-1

### Scheme T, T-4, wherein 3-substituted, R = tBu, R' = CH₃ N-[[3-[(E)-3-(1,1-dimethylethoxy)-3-oxo-1-propenyl]phenyl]carbonyl]-4- [(2,6-dichlorobenzoyl)amino]-L-phenylalanine methyl ester T-4 (C₃₁H₃₀Cl₂N₂O₆)

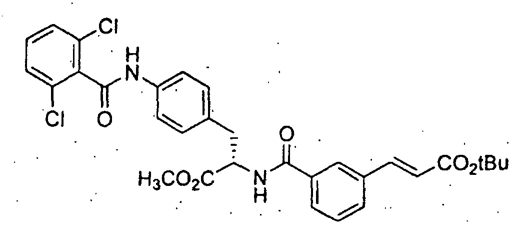

To a stirring suspension of the acid **T-2** (3-CO₂H) (0.36g, 1.45mmol) in CH₂CL₂ (25mL) was added 4-[(2,6-dichlorobenzoyl)amino]-L-phenylalanine methyl ester. hydrochloride (0.59g, 1.45mmol), EDC (0.28g, 1.45mmol), DMAP( 0.05g, 0.44mmol), and HOBT(0.20g, 1.45mmol). To this mixture was added Et₃N dropwise until all solids went into solution. After stirring for 16h at RT, reaction mixture was partitioned between CH₂Cl₂ (50mL) and 1N aq. HCl(50mL). The organic phase was washed with sat'd aq. NaHCO₃ (50mL), sat'd aq. NaCl( 50mL), dried (Na₂SO₄), filtered, and evaporated *in vacuo*. The resulting white solid was chromatographed (flash) on silica gel (400g, 230-400 mesh, 70mm OD column, packed and eluted with EtOAc/CH₂Cl₂ (1:9), collecting 275 mL fractions). Fractions 18-27 were combined and evaporated *in vacuo* to give 0.72g(83%) of **T-4** (3-substituted, R = tBu, R' = CH₃) as a white solid. **MP:** 189-193°C; **IR** (mull) 3347, 1746, 1740, 1708, 1660, 1641, 1608, 1549, 1531, 1512, 1431, 1330, 1280, 1219, and 789cm⁻¹; ; ^{**1**}**H-NMR**(300MHz, DMSO-d₆): δ = 10.66(1), 8.90(1), 8.15(1), 7.83(2), 7.43-7.60(6), 7.26(2), 6.61(1), 4.69(1), 3.64(3), 3.01-3.20(2), 1.47(9); **MS** (FAB) *m*/*z* (rel. intensity) 597 (MH⁺, 19), 597 (19), 543 (21), 541 (31), 525 (21), 523 (31), 351 (22), 349 (32), 175 (99), 173 (47), 57 (38); **HRMS** (FAB) calcd for C₃₁H₃₀Cl₂N₂O₆+H₁ 597.1559, found 597.1541.

### Preparation T-4-2

### Scheme T, T-4, wherein 3-substituted, R = H, R' = CH₃ N-[[3-[(E)-3-Hydroxy-3-oxo-1-propenyl]phenyl]carbonyl]-4-[(2,6-dichlorobenzoyl)amino]-L-phenylalanine methyl ester T-4 (C₂₇H₂₂Cl₂N₂O₆)

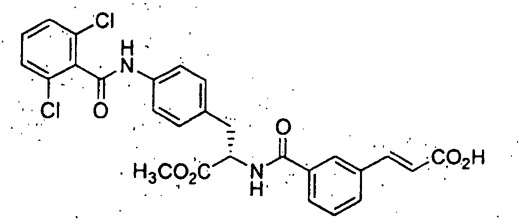

A solution of the diester **T-4** (3-substituted, R = tBu, R' = CH₃) (0.50g, 0.84mmol) in TFA(10mL) stirred at RT for 16h. Evaporation *in vacuo* gave a sticky solid which, upon trituration with Et₂O afforded 0.42g (92%) of the title compound **T-4** (3-substituted, R = H, R' = CH₃) as a fine white solid which was collected by suction filtration and dried. **MP:** 170-171°C; ^{**1**}**H-NMR**(300MHz, DMSO-d₆): δ = 10.70(1), 8.93(1), 8.13(1), 7.83(2), 7.43-7.64(6). 7.27(2), 6.61(1), 4.67(1), 3.64(3), 3.01-3.20(2); **IR** (mull) 1748, 1695, 1665, 1644, 1610, 1579, 1562, 1550, 1540, 1514, 1432, 1415, 1331, 1279, and 1217cm⁻¹. **MS** (FAB) *m*/*z* (rel. intensity) 541 (MH⁺, 93), 544 (20), 543 (65), 542 (35), 541 (93), 525 (20), 523 (31), 351 (21), 349 (41), 175 (99), 173 (32); **HRMS** (FAB) calcd for C₂₇H₂₂Cl₂N₂O₆+H₁ 541.0933, found 541.0938.

### Example 124

### Scheme T, T-4, wherein 3-substituted, R = H, R' = H N-[[3-[(E)-3-Hydroxy-3-oxo-1-propenyl]phenyl]carbonyl]-4-[(2,6-dichlorobenzoyl)amino]-L-phenylalanine T-4 (C₂₆H₂₀Cl₂N₂O₆)

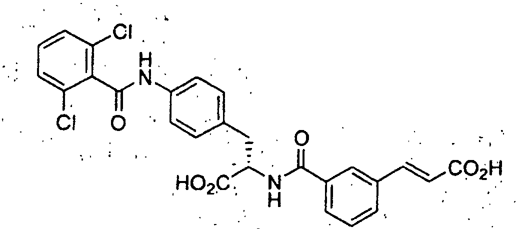

To a stirring solution of the diester **T-4** (3-substituted, R = H, R' = CH₃) (0.38g, 0.72mmol) in MeOH (10mL) was added a solution of LiOH·H₂O (0.06g, 1.44mmol) in H₂O(3mL). After stirring for 16h at RT, the reaction mixture was filtered and 6N aq. HCl was added until the pH of the mixture was *ca*. 1. The resulting precipitate was collected by suction filtration, washed with H₂O and dried in the vacuum oven at 70°C for 16h to afford 0.25g (66%) of the title compound **T-4** (3-substituted, R = H, R' = H) as a white, powdery solid. **MP:** 156-161°C; ^{**1**}**H-NMR** (300MHz, DMSO-d₆): δ = 12.70(2), 10.70(1), 8.81 (1), 8.14(1), 7.82(2), 7.46-7.64(6), 7.29(2), 6.62(1), 4.63(1), 2.97-3.20(2); IR (mull) 3261, 3065, 3035, 1695, 1641, 1605, 1579, 1562, 1538, 1517, 1432, 1414, 1326, 1271, and 1196cm⁻¹; **MS** (FAB) *m*/*z* (rel. intensity): 527 (MH⁺, 99), 530 (23), 529 (70), 528 (40), 527 (99), 511 (42), 509 (59), 337 (22), 335 (32), 175 (90), 173 (51); **HRMS** (FAB) calcd for C₂₆H₂₀Cl₂N₂O₆+H₁ 527.0776, found 527.0786.

### Preparation T-4-3

### Scheme T, T-4, wherein 4-substituted, R = tBu, R' = CH₃ N-[[4-[(E)-3-(1,1-dimethylethoxy)-3-oxo-1-propenyl]phenyl]carbonyl]-4-[(2,6-dichlorobenzoyl)amino]-L-phenylalanine methyl ester T-4 (C₃₁H₃₀Cl₂N₂O₆)

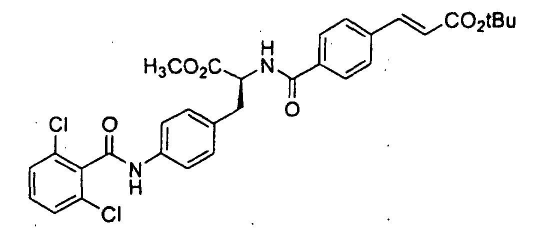

To a stirring suspension of the acid T-2, (4-CO₂H) (0.52g, 2.09mmol) in CH₂Cl₂ (30mL) was added the 4-[(2,6-dichlorobenzoyl)amino]-L-phenylalanine methyl ester. hydrochloride (0.85g, 2.09mmol), EDC (0.40g, 2.09mmol), DMAP (0.08g, 0.63mmol), and HOBT (0.28g, 2.09mmol). To this mixture was added Et₃N dropwise until all solids went into solution. After stirring for 16h at RT, the resulting precipitate was collected by suction filtration, washed with H₂O, washed with CH₂Cl₂, and dried to give 0.86g (69%) of the title compound **T-4** (4-substituted, R = tBu, R' = CH₃) as a white solid. **MP:** >225°C; ^{**1**}**H-NMR**(300MHz, DMSO-d₆): δ = 10.66(1), 8.92(1), 7.76-7.84(4), 7.43-7.59(6), 7.27(2), 6.60(1), 4.62(1), 3.64(3), 3.01-3.18(2), 1.47(9); **IR** (mull) 1742, 1733, 1708, 1666, 1640, 1610, 1547, 1513, 1431, 1415, 1332, 1320, 1305, 1166, and 1155cm⁻¹; **MS** (FAB): *m*/*z* (rel. intensity) 597 (MH⁺, 1), 598 (12), 543 (14), 541 (19); 351 (9), 349 (13), 176 (11), 175 (99), 173 (31), 131 (9), 57 (31); **HRMS** (FAB): calcd for C₃₁H₃₀Cl₂N₂O₆+H₁ 597.1559, found 597.1547.

### Preparation T-4-4

### Scheme T, T-4, wherein 4-substituted, R = H, R' = CH₃ N-[[4-[(E)-3-Hydroxy-3-oxo-1-propenyl]phenyl]carbonyl]-4-[(2,6-dichlorobenzoyl)amino]-L-phenylalanine methyl ester T-4 (C₂₇H₂₂Cl₂N₂O₆)

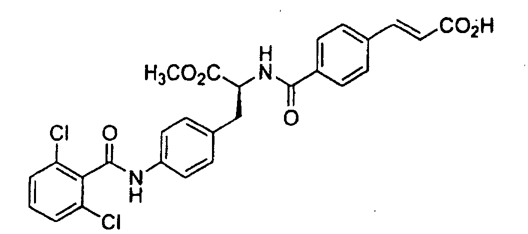

A solution of the diester **T-4** (4-substituted, R = tBu, R' = CH₃) (0.84g, 1.40mmol) in TFA (10mL) was stirred at RT for 16 hours. Evaporation *in vacuo* afforded 0.76g (100%) of the title compound **T-4** (4-substituted, R = H, R' = CH₃) as an off-white solid. **MP:** 80°C(dec.); ^{**1**}**H-NMR**(300MHz, DMSO-d₆): δ = 10.70(1), 8.92(1), 7.75-7.82(4), 7.43-7.62(6), 7.26(2), 6.61(1), 4.63(1), 3.64(3), 2.99-3.20(2); **IR** (mull) 1731, 1693, 1639, 1608, 1562, 1540, 1516, 1504, 1433, 1415, 1326, 1277, 1215, 1195, and 1172cm⁻¹; **MS** (FAB) : *m*/*z* (rel. intensity) 541 (MH⁺, 99), 545 (14), 544 (23), 543 (72), 542 (37), 541 (99), 351 (13), 349 (27), 176 (12), 175 (99), 173 (26); **HRMS** (FAB): calcd for C₂₇H₂₂Cl₂N₂O₆+H₁ 541.0933, found 541.0927.

### Example 125

### Scheme T, T-4, wherein 4-substituted, R = H, R' = H N-[[4-[(E)-3-Hydroxy-3-oxo-1-propenyl]phenyl]carbonyl]-4-[(2,6-dichlorobenzoyl)amino] -L-phenylalanine T-4 (C₂₆H₂₀Cl₂N₂O₆)

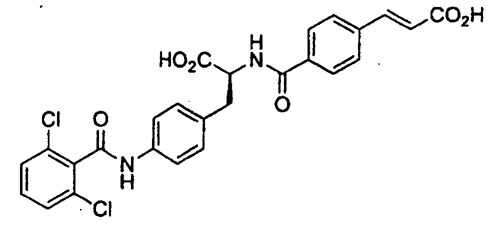

To a stirring solution of the ester **T-4** (4-substituted, R = H, R' = CH₃) (0.76g, 1.40mmol) in MeOH (10mL) was added a solution of LiOH·H₂O (0.17g, 4.20mmol) in H₂O( 5mL). After stirring for 16h at RT, the reaction mixture was evaporated in *vacuo.* The residue was dissolved in H₂O (30mL) and 6N aq. HCl was added until the pH of the mixture was *ca*.1. The resulting mixture was extracted with EtOAc/THF (1:1, 50mL). The extract was dried (Na₂SO₄), filtered, and evaporated *in vacuo* to afford 0.43g (58%) of the title compound **T-4** (4-substituted, R = H, R' = H) as a white solid. **MP:** 161-165°C; ^{**1**}**H-NMR**(300MHz, DMSO-d₆): δ = 12.70(1), 10.60 (1), 7.75-7.84(4), 7.39-7.62(6), 7.28(2), 6.61(1), 4.58(1), 2.95-3.20(2); **IR** (mull): 3057, 1715, 1693, 1658, 1639, 1608, 1562, 1533, 1505, 1445, 1431, 1415, 1328, 1278; and 1195cm⁻¹; **MS** (FAB): *m*/*z* (rel. intensity) 527 (MH⁺, 88), 530 (19), 529 (63) , 528 (33), 527 (88), 220 (21), 219 (20), 175 (99), 173 (26), 163 (18), 57 (44); **HRMS** (FAB): calcd for C₂₆H₂₀Cl₂N₂O₆+H₁ 527.0776, found 527.0765.

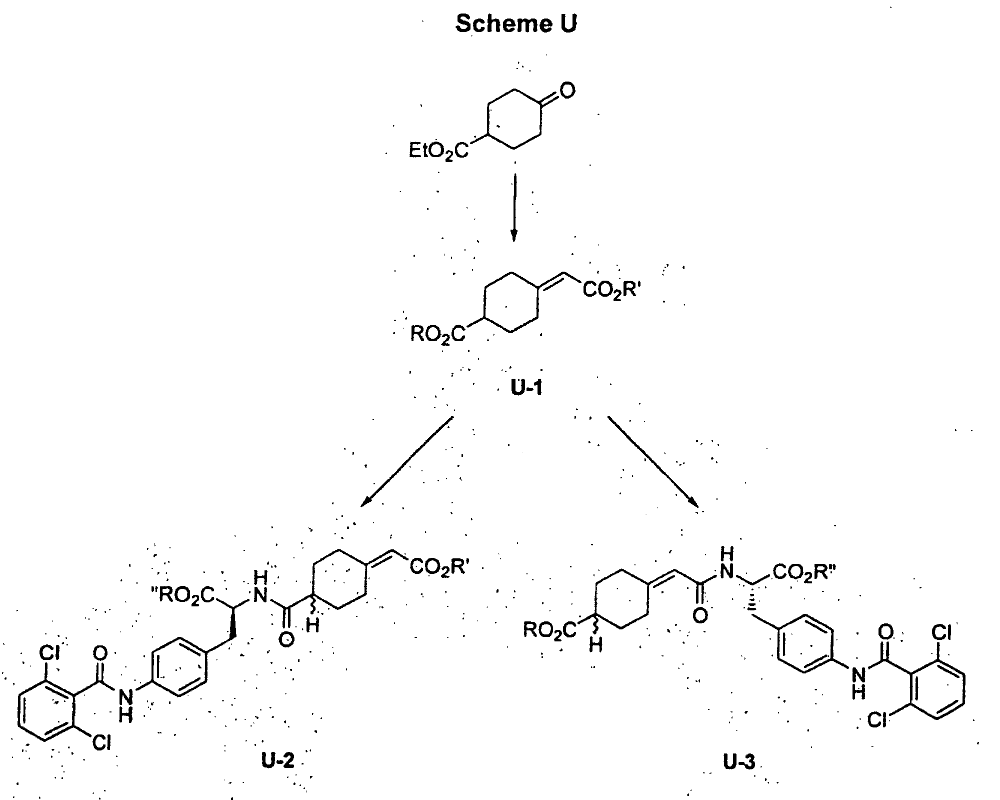

### Preparation U-1-1

### Scheme U, U-1, wherein R = CH₂CH₃, R' = tBu 4-Carboethoxy-1-cyclohexylideneacetic acid 1,1-dimethylethyl ester (C₁₅H₂₄O₄)

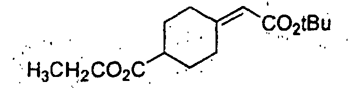

Sodium hydride (0.56g of a 60% mineral oil dispersion, 14.1mmol) was washed with hexanes (3 x 10mL), suspended in THF (10mL), and the suspension was cooled to 0°C. To this suspension was added a solution of tert-butyl diethyl phosphonoacetate (5.0g, 19.8mmol) in THF (5mL) dropwise. The resulting solution was allowed to stir for 1h at 0°C, and then a solution of ethyl-4-oxocyclohexane carboxylate (Aldrich Chemical Co., Milwaukee, WI, Catalog #32062-5) (2.0g, 11.8mmol) in THF (5mL) was added dropwise. The solution was allowed to slowly warm to RT and stirred for 16h. The reaction mixture was evaporated *in vacuo*. The residue was partitioned between EtOAc (100mL) and H₂O (100mL), and the organic phase was washed with brine (100mL), dried (Na₂SO₄), filtered, and evaporated *in vacuo*. The resulting yellow oil was chromatographed on silica gel (350g, 230-400 mesh, 70mmOD column, packed and eluted with EtOAc/hexanes, 1:9, collecting 230mL fractions) using the flash technique. Fractions 8-1S5were combined and evaporated *in vacuo* to afford 2.04g (64%) of the vinylogous ester **U-1** (R = CH₂CH₃, R' = tBu) as a colorless oil. ^{**1**}**H-NMR** (300 MHz, CDCl₃) : δ = 5.56 (1), 4.12 (2), 3.58 (1), 2.52 (1), 2.31 (1), 1.98-2.22 (4), 1.57-1.76 (2), 1.46 (9), 1.24 (3); **IR** (neat.): 2979, 2939, 1732, 1710, 1650, 1384, 1368, 1314, 1307, 1264, 1249, 1206, 1162, 1140, and 1041cm⁻¹; **MS** (EI ) *m*/*z* (rel. intensity): 195 (30) , 194 (99), 1.67 (11), 139 (13), 138 (18), 121 (55), 120 (55), 93 (24), 60 (8), 57 (37); **Anal.** Calcd for C₁₅H₂₄O₄ : C, 67.14; H, 9.01. Found: C, 67.23; H, 9.00.

### Preparation U-1-2

### Scheme U, U-1, wherein R = H, R' = tBu 4-Carboxy-1-cyclohexylideneacetic acid 1,1-dimethylethyl ester (C₁₃H₂₀O₄)

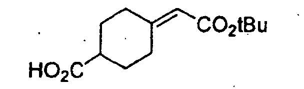

To a stirring solution of the diester **U-1** (R = CH₂CH₃, R' = tBu) (1.00g, 3.73mmol) in MeOH (20mL) was added a solution of LiOH^{^{.}}H₂O (0.78g, 18.6mmol) in H₂O (5mL). The reaction mixture at RT for 16h and was then evaporated *in vacuo*. The residue was partitioned between H₂O (100mL) and CHCl₃ (100mL), and the aqueous phase was neutralized with 1N aq. HCl. The organic phase was washed with H₂O (100mL), brine (100mL), dried (Na₂SO₄), filtered, and evaporated *in vacuo* to afford 0.60g (67%) of the carboxylic acid **U-1,** (R = H, R' = tBu) as a viscous, colorless oil, which crystallized upon standing to afford a colorless solid. **MP:** 63-64°C; ^{**1**}**H-NMR** (300 MHz, CDCl₃): δ = 5.57 (1), 3.58 (1), 2.58 (1), 2.33 (1), 2.02-2.23 (4), 1.61-1.78 (2), 1.47 (9); **IR** (mull) 3002, 1706, 1652, 1434, 1317, 1267, 1256, 1206, 1197, 1166, 1142, 1088, 1011, 874, and 639cm⁻¹; **MS** (EI)*m*/*z* (rel. intensity): 184 (16), 167 (92), 166 (99), 138 (21), 121 (30), 94 (10), 93 (19), 57 (65), 56 (8), 55 (8); **MS** (FAB) *m*/*z* (rel. intensity): 241 (MH+, 46), 481 (1) ), 395(7), 241 (46), 186 (9), 185 (99), 167 (62), 139 (14), 57 (67), 41 (13), 29 (13); **HRMS** (FAB) calcd for C₁₃H₂₀O₄+H, 241.1440, found 241.1434; **Anal.** Calcd for C₁₃H₂₀O₄: C, 64.98; H, 8.39. Found: C, 65.16; H, 8.35.

### Preparation U-2-1

### Scheme U, U-2, wherein R' = tBu, R" = CH₃ N-[[4-[[(1,1-Dimethylethoxy)carbonyl]methylene]cyclohexyl]carbonyl]-4-[(2,6-dichlorobenzoyl)amino]-L-phenylalanine methyl ester (C₃₀H₃₄Cl₂N₂O₆) U-2

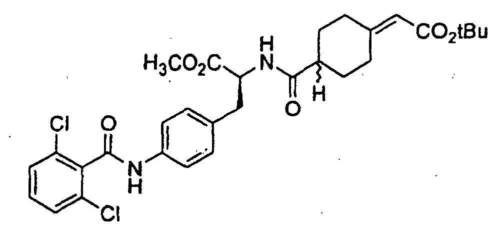

To a stirring solution of the carboxylic acid **U-1** (R = H, R' = tBu) (0.54g, 2.25mmol) in CH₂Cl₂ (30mL) was added 4-[(2,6-dichlorobenzoyl)amino]-L-phenylalanine methyl ester. hydrochloride (0.91g, 2.25mmol), EDC (0.43g, 2.25mmol), DMAP (0.08g, 0.67mmol), HOBT (0.30g, 2.25mmol), and diisopropyl ethylamine (3 mL). Finally, DMF (2mL) was added to facilitate dissolution. The solution stirred at RT for 16h and then was evaporated *in vacuo*. The residue was partitioned between 1N aq. aqueous HCl (150mL) and CH₂Cl₂ (150mL). The organic phase was washed with saturated NaHCO₃ (150mL), brine (150mL), dried (Na₂SO₄), filtered, and evaporated *in vacuo.* The resulting pale yellow solid was chromatographed on silica gel (300g, 230-400 mesh, 70mm OD column, packed and eluted with EtOAc/CH₂Cl₂, (1:4), collecting 230mL fractions) using the flash technique. Fractions 7-12 were combined and evaporated *in vacuo* to afford the desired product **U-2** (R' = tBu, R" = CH₃) as a white solid, 0.87g (66%). **MP:** 199-200°C; ^{**1**}**H-NMR** (300 MHz, DMSO-d₆) : δ = 10.64 (1), 8.19 (1), 7.44-7.59 (5), 7.17 (2), 5.50 (1), 4.43 (1), 3.60 (3), 3.65-3.58 (1), 3.00 (1), 2.86 (1), 1.64-2.46 (8), 1.39 (9); **IR** (mull) 1749, 1703, 1667, 1649, 1609, 1561, 1548, 1513, 1432, 1415, 1332, 1250, 1197, 1167, and 1140cm⁻¹; **MS** (FAB) *m*/*z* (rel. intensity): 589 (MH+, 25), 533 (31), 517 (68), 516 (36), 515 (99), 351 (37), 349 (55), 175 (42), 173 (64), 121 (45), 57 (50). HRMS (FAB) calcd for C₃₀H₃₄Cl₂N₂O₆+H, 589.1872, found 589.1 846.

### Preparation U-2-2

### Scheme U, U-2, wherein R' = H, R" = CH₃ N-[[4-(Carboxymethylene)cyclohexyl]-carbonyl]-4-[(2,6-dichlorobenzoyl)amino]-L-phenylalanine methyl ester (C₂₆H₂₆Cl₂N₂O₆) U-2

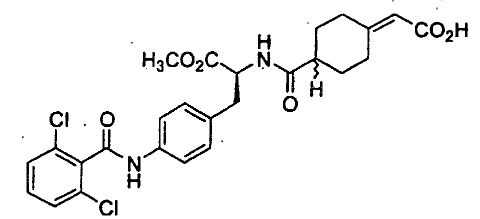

A solution of the diester **U-2** (R' = tBu, R" = CH₃) (0.82g, 1.39mmol) in TFA (10mL) was stirred at RT for 16h. The reaction mixture was evaporated *in vacuo* and the residue was dissolved in toluene and evaporated *in vacuo* (2x10mL). The residue was triturated with Et₂O, and the resulting solid was collected by suction filtration and dried to afford 0.66g (89%) of the carboxylic acid **U-2** (R' = H, R" = CH₃) as powdery, white solid. **MP:** 140°C (dec.); ^{**1**}**H-NMR** (300 MHz, DMSO-d₆): δ = 11.93 (1), 10.64 (1), 8.19 (1), 7.52-7.60(4), 7.44-7.51(1), 7.17(1), 5.54(1), 4.43 (1), 3.52-3.63 (4), 3.24-3.39 (1), 2.99 (1), 2.79 2.91 (1), 1.64-2.45 (6), 1.21-1.50 (2); **IR** (mull) 3069, 1748, 1652, 1609, 1561, 1545, 1514, 1432, 1415, 1331, 1274, 1250, 1215, 1195, and 1184 cm⁻¹; **MS** (FAB) *m*/*z* (rel. intensity): 533 (MH+, 62), 535 (40), 533 (62), 517 (52), 515 (74), 351 (48), 349 (69), 175 (63), 173(99), 121 (55), 93 (35); **HRMS** (FAB) calcd for C₂₆H₂₆Cl₂N₂O₆+H1 533.1246, found 533.1237.

### Example 126

### Scheme U, U-2, wherein R' = H, R" = H N-[[4-(Carboxymethylene)cyclohexyl]carbonyl]-4-[(2,6-dichlorobenzoyl)amino]-L-phenylalanine (C₂₅H₂₄Cl₂N₂O₆) U-2

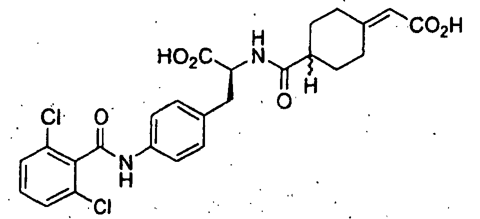

To a stirring solution of the methyl ester **U-2** (R' = H, R" = CH₃) (0.60g, 1.12mmol) in CH₃OH (10mL) was added a solution of LiOH·H₂O (0.24g, 5.6mmol) in H₂O (5mL). The solution stirred at RT for 16h and was then evaporated *in vacuo*. The residue was dissolved in H₂O (20mL) and acidified to pH *ca*. 1-2 with 6N aqueous HCl. The resulting white solid was collected by suction filtration, washed with H₂O, and dried in a vacuum oven at 70°C for 16h. The resulting solid was crushed, washed thoroughly with H₂O, and dried in a vacuum oven at 100°C for 16h to afford the target diacid **U-2** (R' = H, R" = H) as a white, crystalline solid, 0.45g (78%). **MP:** 145-148°C; ^{**1**}**H-NMR** (300 MHz, DMSO-d₆): δ = 12.27 (1), 10.63 (1), 8.04 (1), 7.52-7.58 (4), 7.47 (1), 7.18 (2), 5.53 (1), 4.37 (1), 3.57 (1), 3.30 (1), 3.01 (1), 2.77-2.88 (1), 1.63-2.46 (6), 1.21-1.51 (2); **IR** (mull) 3270, 3124, 3070, 1655, 1607, 1562, 1539, 1517, 1432, 1414, 1327, 1269, 1250, 1195, and 799cm⁻¹; **MS** (FAB) *m*/*z* (rel. intensity): 519 (MH+, 83), 521 (53), 519 (83), 503 (62), 501 (89), 335 (52), 175 (65), 173 (99), 139 (76), 121 (58), 91 (47); **HRMS** (FAB) calcd for C₂₅H₂₄Cl₂N₂O₆+H1 519.1089, found 519.1082.

### Preparation U-1-3

### Scheme U, U-1, wherein R = CH₂CH₃, R' = H 4-Carboethoxy-1-cyclohexylideneacetic acid (C₁₁H₁₆O₄)

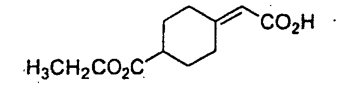

The diester **U-1** (R = CH₂CH₃, R' = tBu) (0.86g, 3.20mmol) was dissolved in trifluoroacetic acid (5mL), and the solution stirred at RT for 4h. Evaporation *in vacuo* afforded 0.68g (100%) of the carboxylic acid **U-1** (R = CH₂CH₃, R' =, H) as a viscous oil which was used without further purification. ^{**1**}**H-NMR** (300 MHz, CDCl₃): δ = 10.96 (1), 5.68 (1), 4.14 (2) , 3.58 (1), 2.57 (1), 2.40 (1), 2.16-2.30 (2), 2.01-2.13 (2), 1.61-1.81 (2), 1.26 (3); **IR** (neat) 2985, 2944, 2872, 1790, 1730, 1692, 1645, 1449, 1421, 1305, 1271, 1252, 1213, 1175, and 1042cm⁻¹; **MS** (EI) *m*/*z* (rel. intensity): 194 (99), 139 (31), 138 (40), 121 (62), 120 (93), 94 (24), 93 (64), 91 (22), 73 (23), 55 (21); **MS** (FAB): m/z (rel. intensity) 213 (MH+, 5), 213 (5), 196 (12), 195 (99), 194 (6), 139 (9), 121 (12), 93 (11), 39 (4), 29 (8), 27 (6); **HRMS** (FAB) calcd for C₁₁H₁₆O₄+H1, 213.1127, found 213.1130.

### Preparation U-3-1 Scheme U, U-3, wherein R = CH₂CH₃, R" = CH₃ N-[4-Carboethoxy-1-cyclohexylideneacetyl]-4-[(2,6-dichlorobenzoyl)amino]-L-phenylalanine methyl ester (C₂₈H₃₀Cl₂N₂O₆) U-3

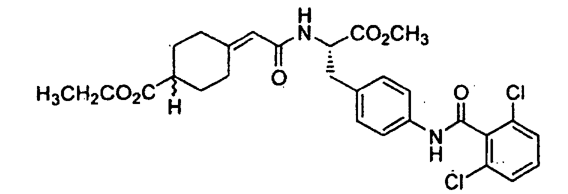

To a stirring solution of the carboxylic acid **U-1** (R = CH₂CH₃, R' = H) (0.70g, 3.20mmol) in CH₂Cl₂ (30mL) was added 4-[(2,6-dichlorobenzoyl)amino]-L-phenylalanine methyl ester. hydrochloride (1.29g, 3.20mmol), EDC (0.61g, 3.20mmol), DMAP (0.12g, 0.96mmol), HOBT (0.43g, 3.20mmol) and diisopropylethylamine (4 mL). Finally, DMF (2mL) was added in order to assist dissolution. The solution stirred at RT for 16h and then was evaporated *in vacuo*. The residue was partitioned between 1N aqueous HCl (150mL) and CH₂Cl₂ (150mL). The organic phase was washed with saturated NaHCO₃ (150mL), brine (150mL), dried (Na₂SO₄), filtered, and 'evaporated *in vacuo* to give a white solid. Trituration with Et₂O gave the desired product **U-3** (R = CH₂CH₃, R" = CH₃) 'as a powdery, white solid, 1.08g (60%). **MP:** 240°C; ^{**1**}**H-NMR** (300 MHz, DMSO-d₆): δ = 10.65 (1), 8.22 (1), 7.52-7.60(4), 7.47(1), 7.18(2), 5.64(1), 4.52 (1), 3.98-4.08 (2), 3.61 (3), 3.46 (1), 2.94-3.04 (1), 2.78-2.90 (1), 2.47-2.58 (1), 1.78-2.19 (5 ), 1.30-1.55 (2), 1.12-1.18 (3); **IR** (mull): 3316, 1746, 1730, 1668, 1656, 1634, 1611, 1562, 1551, 1513, 1434, 1415, 1334, 1281; and 1210cm⁻¹; **MS** (FAB) *m*/*z* (rel. intensity) 561 (MH+, 69), 563 (47), 562 (28), 561 (69), 195 (99), 175 (31), 173 (47), 121 (93), 105 (25), 93 (24), 91 (33); **HRMS** (FAB) calcd for C₂₈H₃₀Cl₂N₂O₆+H1 561.1559, found 561.1556.

### Example 127

### Scheme U, U-3, wherein R = H, R" = H N-[4-Carboxy-1-cyclohexylideneacetyl]-4-[(2,6-dichlorobenzoyl)amino]-L-phenylalanine (C₂₅H₂₄Cl₂N₂O₆) U-3

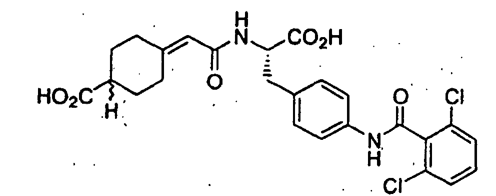

To a stirring suspension of the diester **U-3** (R = CH₂CH₃, R" = CH₃) (1.00g, 1.78mmol) in MeOH (15mL) was added a solution of LiOH-H₂O (0.37g, 8.9mmol) in H₂O (5mL). H₂O (10mL) and THF (5mL) were added in an attempt to achieve a homogenous solution. The cloudy mixture Stirred at RT for 16h and was then evaporated *in vacuo.* The residue was dissolved in H₂O and 6N aqueous HCI was added dropwise until the pH was *ca. 1*. The resulting precipitate was collected by suction filtration, washed with H₂O, and dried in a vacuum oven at 80°C for 16h to afford the diacid U-3 (R = H, R" = H) as a white solid, 0.75g (81%). **MP:** 148-155°C^{**1**}**H-NMR** (300 MHz, DMSO-d₆) : δ = 12.27 (1), 10.64 (1), .8.06 (1), 7.52-7.59 (4), 7.47 (1), 7.19 (2), 5.64 (1), 4.40 (1), 3.41-3.54 (1), 3.30 (1), 2.96-3.06 (1), 2.75-2.87 (1), 2.35-2.45 (1), 1.78-2.19 (5), 1.30-1.54 (2); **IR** (mull): 3274, 3064, 1717, 1662, 1607, 1562, 1538, 1516, 1432, 1414, 1327, 1270, 1254, 1211, and 1195cm⁻¹; **MS** (FAB): *m*/*z* (rel. intensity) 519 (MH+, 99), 522 (20), 521 (67), 520 (38), 519 (99), 518 (16), 335 (23), 175 (21), 173 (30), 167 (73), 121 (36); **HRMS** (FAB): calcd for C₂₅H₂₄Cl₂N₂O₆+H1, 519.1089, 'found 519.1104.

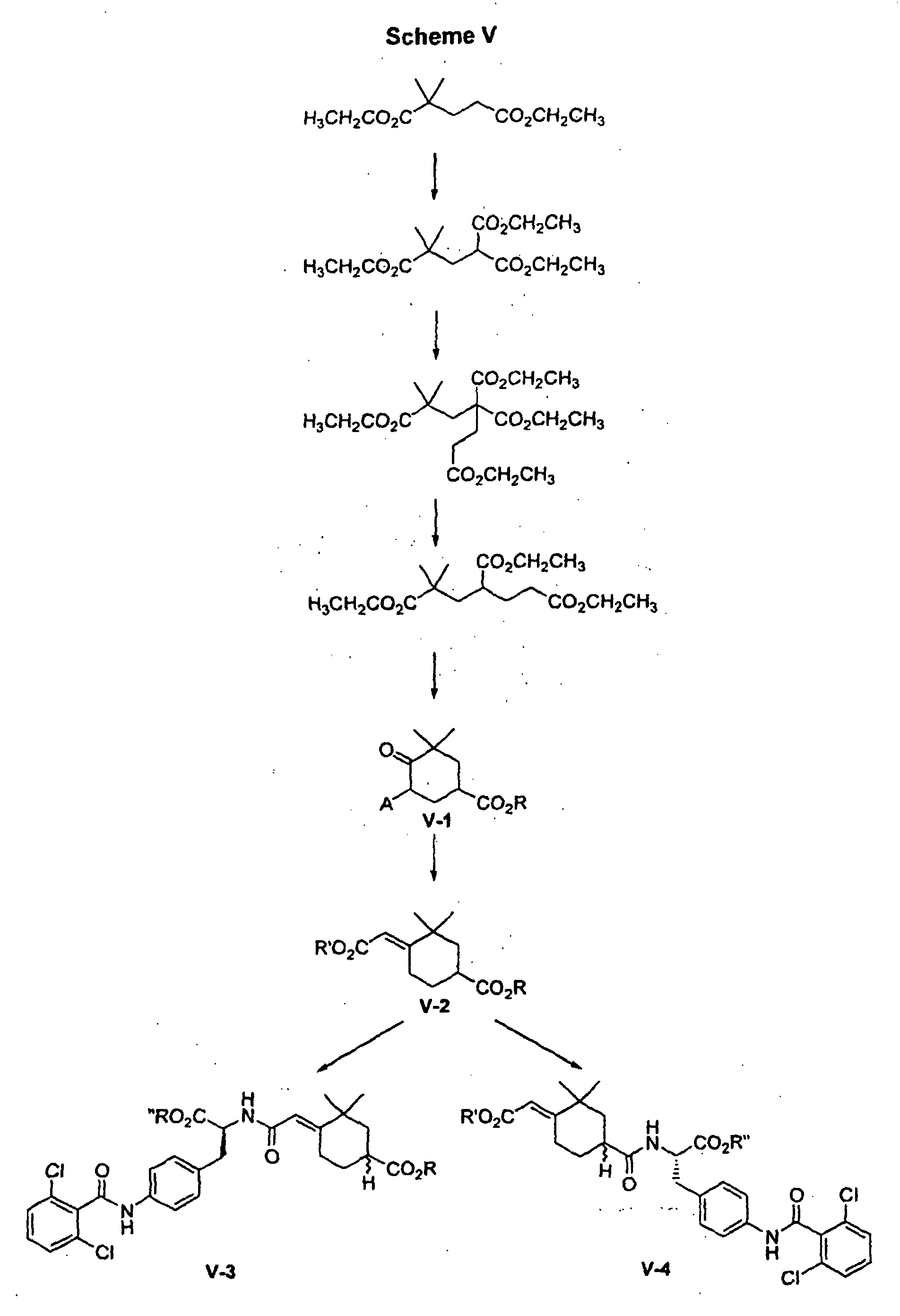

### Preparation of

### 2-Carboethoxy-4,4-dimethylglutaric acid diethyl ester (C₁₄H₂₄O₆)

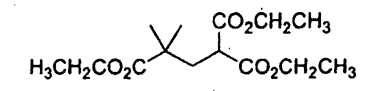

A flame dried 3-neck flask was charged with absolute ethanol (90mL), and sodium (2.53g, 110mmol) was added portionwise. After the sodium completely dissolved; diethyl oxalate (16.3mL) and the starting diester (21.6g, 100mmol) (Inesi, A.; Zeuli, E. *J. Electroanal. Chem. Interfacial Electrochem*. 1983, 149, 167) were added. The resulting suspension stirred at RT for 16h, and then the ethanol was removed via distillation under reduced pressure. The residue was dissolved in H₂O (500mL), and 6N aqueous HCl was added until the pH of the mixture was *ca.* 1-2. The mixture was extracted with EtOAc (2 x 500mL), and the combined extracts were washed with sat'd aqueous NaHCO₃ (500mL), brine (500mL), dried (Na₂SO₄), filtered, and evaporated *in vacuo.* This crude product was purified by distillation under reduced pressure to afford the triester as a clear, colorless oil, 18,1 g (63%). **BP** 100-110°C (0.1atm); ^{**1**}**H-NMR** (300 MHz, CDCl₃) : δ = 4.02-4.22 (6), 2.21-2.27 (2), 1.82-1.93 (1), 1.19-1.30 (9), 1.17 (6) ; **IR** (neat) : 2983, 1753, 1733, 1477, 1448, 1390, 1369, 1302, 1251, 1232, 1178, 1148 (s), 1114, 1097, and 1030cm⁻¹; **MS** (EI ) : *m*/*z* (rel. intensity) 288 (M+, 3), 243 (49), 215 (77), 197 (21), 173 (83), 169 (20), 160 (25), 142 (19), 141 (99), 123 (49), 95 (25).

### Preparation of

### 4,4-Bis-carboethoxy-2,2-dimethylheptandedioic acid diethyl ester (C₁₉H₃₂O₈)

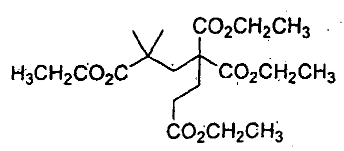

To sodium (0.13g, 5.80mmol) covered with Et₂O (25mL) was added a solution of EtOH (2.4mL) in Et₂O (45mL) dropwise. When the sodium was consumed, the triester **V** (8.39g, 29.1mmol) was added followed by ethyl acrylate (3.5mL, 32.0mmol). The solution was refluxed for 10h, then allowed to cool to RT. The reaction mixture was partitioned between 2% aqueous HOAc (500mL) and Et₂O (500mL). The organic phase was washed with H₂O (500mL), brine (500mL), dried (Na₂SO₄), filtered, and evaporated *in vacuo*. The resulting clear, colorless oil was distilled under reduced pressure (0.1atm) to afford 8.61 g (76%) of the target tetraester **V+1** as a clear, colorless oil. **BP** 147°C; ^{**1**}**H-NMR** (300 MHz, CDCl₃): δ = 4.00-4.24 (8), 2.35 (2), 2.06-2.31 (4), 1.17-1.30 (12), 1.16 (6); **IR** (neat) : 2982, 2939, 1735, 1479, 1448, 1391, 1368, 1298, 1266, 1250, 1180, 1143, 1113, 1097, and 1025cm⁻¹; **Anal.** Calcd for C₁₉H₃₂O₈:C, 58.75; H, 8.30. Found: C, 58.87; H, 8.15.

### Preparation of 4-Carboethoxy-2,2-dimethylheptandedioic acid diethyl ester

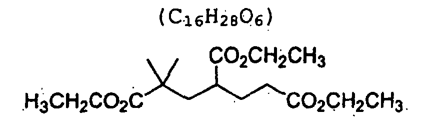

To a solution of the tetraester **V+1** (8.55g, 22.0mmol) in DMSO (75mL) was added NaCl (2.62g, 44.0mmol) and H₂O (4mL). The mixture was refluxed for 16h, then allowed to cool to RT. Following evaporation *in vacuo*, the reaction mixture was partitioned between H₂O (500mL) and EtOAc (500mL), and the aqueous phase was extracted with EtOAc (250mL). The combined organic phases were washed with H₂O (500mL), brine (500mL), dried (Na₂SO₄), filtered, and evaporated *in vacuo* to afford 5.0g (72%) of the desired triester **V+2** as a pale yellow oil which was used without further purification. ^{**1**}**H-NMR** (300 MHz, CDCl₃): δ = 4.00-4.21 (6), 1.62-2.42 (7), 1.08-1.31 (15); **IR** (neat): 2981, 2938, 1734, 1477, 1465, 1449, 1388, 1379, 1304, 1256, 1179, 1159, 1137, 1097, and 028cm⁻¹; **MS** (FAB) *m*/*z* (rel. intensity) 317 (MH+, 59), 318 (10), 317 (59), 272(15),27 (99), 243(19), 197(13), 169(56), 151(16), 95(21), 29(11); **HRMS** (FAB) calcd for C₁₆H₂₈O₆+H1 317.1964, found 317.1969.

### Preparation V-1-1

### Scheme V, V-1, wherein A = CO₂CH₂CH₃, R = CH₂CH₃ 2,4-Bis-carboethoxy-6,6-dimethylcyclohexanone (C₁₄H₂₂O₅) V-1

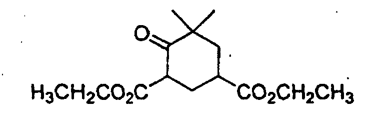

Sodium hydride (0.70g of a 60% oil dispersion, 17.5mmol) was washed with hexanes (3x10mL) and suspended in THF (25mL). To this stirring suspension was added a solution of the triester V+2 (4.95g, 15.6mmol) in THF (25mL) dropwise. The mixture was refluxed for 4h, allowed to cool to RT, and evaporated in *vacuo.* The resulting orange foam was partitioned between EtOAc (250mL) and H₂O (250mL), and the aqueous phase was neutralized with IN aqueous HCl. The phases were separated, and the organic phase was washed with saturated aqueous NaHCO₃ (250mL), brine (250mL), dried (Na₂SO₄), filtered, and evaporated in vacuo. The resulting yellow oil was purified via Kugelrohr distillation to afford the desired cyclohexanone **V-1** (A = CO₂CH₂CH₃, R = CH₂CH₃) diester as a clear, colorless oil, 2.73g (65%). ^{**1**}**H-NMR** (300 MHz, CDCl₃) : δ = 4.05-4.24 (5), 2.62(1), 2.39(1), 1.84(1), 1.66(1), 1.05-1.35(6), 1.17(6); **IR** (neat): 2982, 2937, 1734, 1652, 1612, 1399, 1375, 1363, 1304, 1285, 1271, 1255, 1198, 1147, and 1033cm⁻¹; **MS** (EI ) m/z (rel. intensity) 168 (27), 150 (28), 142 (74), 141 (34), 139 (47), 115 (31), 114 (39), 97 (99), 73 (28), 69 (90); **HRMS** (EI) calcd for C₁₉H₂₂O₅ 270.1467, found 270.1488.

### Preparation V-1-2

### Scheme V, V-1, wherein A = H, R = H 4-Carboxy-2,2-dimethylcyclohexanone (C₉H₁₄O₃) V-1

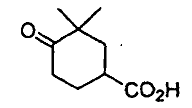

A mixture of the diester **V-1** (A = CO₂CH₂CH₃, R = CH₂CH₃) (1.90g, 7.03mmol) in 6N aqueous HCl (25mL) was refluxed for 12h. After cooling to RT, NaHCO₃ was added to the reaction mixture until the pH was ca.10. The mixture was extracted with Et₂O (50mL), and the extract was discarded. 1N aqueous HCl was added to the aqueous phase until the pH was ca. 4, and it was extracted with Et₂O (50mL). The extract was washed with brine (50mL), dried (MgSO₄), filtered, and evaporated *in vacuo* to afford the desired carboxylic acid **V-1** (A = H, R = H) as a pale yellow oil which solidified upon standing, 0.85g (71%). ^{**1**}**H-NMR** (300 MHz, CDCl₃): δ = 2.94(1), 2.60(1), 2.38(1), 2.34(1), 2.03(1), 1.76-1.93(2), 1.22(3), 1.09(3); **HRMS** (EI): calcd for C₉H₁₄O₃ 170.0943, found 170.0948.

### Preparation V-1-3

### Scheme V, V-1, wherein A = H, R = CH₃ 4-Carboxymethoxy-2,2-dimethylcyclohexanone (C₁₀H₁₆O₃) V-1

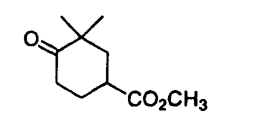

To a stirring solution of the carboxylic acid **V-1** (A = H, R = H) (1.20g, 7.05mmol) in CH₃OH (40mL) was added concentrated H₂SO₄ (2mL), and the mixture refluxed for 16h, then was stirred at RT for 24h. The reaction mixture was evaporated *in vacuo,* and the residue was partitioned between EtOAc (50mL) and sat'd aqueous NaHCO₃ (50mL). The organic phase was washed with brine (50mL), dried (Na₂SO₄), filtered, and evaporated *in vacuo*. The resulting yellow oil was chromatographed on silica gel (150g, 230-400 mesh, 35mm OD column, packed and eluted with EtOAc/hexanes, 15:85, collecting 200mL fractions) using the flash technique. Fractions 9-11 were combined and evaporated *in vacuo* to afford the desired methyl ester **V-1** (A = H, R = CH₃) as a pale yellow oil, 0.65g (50%). ^{**1**}**H-NMR** (300 MHz, CDCl₃): δ = 3.68 (3), 2.83-2.96 (1), 2.49-2.63 (1), 2.17-2.38 (2), 1.94-2.04 (1), 1.71-1.92 (2), 1.17 (3), 1.05 (3); **IR** (neat): 2966, 2956, 1737, 1712, 1463, 1436, 1388, 1317, 1297, 1275, 1250, 1236, 1197, 1166, and 1125cm⁻¹; **HRMS** (E1) calcd for C₁₀H₁₆O₃ 184.1099, found 184.1101; Anal. Calcd for C₁₀H₁₆O₃: C, 65.19; H, 8.75. Found: C, 64.88; H, 8.76.

### Preparation V-2-1

### Scheme V, V-2, wherein R = CH₃, R' = tBu (E)-4-Carbomethoxy-2,2-dimethylcyclohexylideneacetic acid 1,1-dimethylethyl ester (C₁₆H₂₆O₄)

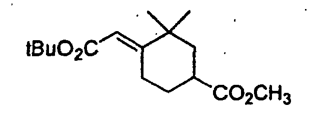

Sodium hydride (0.25g of a 60% mineral oil dispersion, 6.19mmol) was washed with hexanes (3x10mL), suspended in THF (8mL), and cooled to 0°C. To this suspension was added a solution of tert-butyl diethylphosphonoacetate (2.21g, 8.77mmol) in THF (3mL) dropwise. After stirring at 0°C for 1h, a solution of the ketone **V-1** (A = H, R = CH₃) (0.95g, 5.16mmol) in THF (3mL) was added dropwise. The mixture was allowed to slowly warm to RT and stirred for 16h. The reaction mixture was evaporated *in vacuo,* the residue was partitioned between H₂O (50mL) and EtOAc (50mL), and the aqueous phase was extracted with EtOAc (50mL). The combined organic phases were washed with H₂O (100mL), brine (100mL), dried (Na₂SO₄), filtered, and evaporated *in vacuo*. The resulting pale yellow oil was chromatographed on silica gel (300g, 230-400 mesh, 35mm OD column, packed and eluted with EtOAc/hexanes, 1:9, collecting 200mL fractions) using the flash technique. Fractions 5-7 were combined and evaporated *in vacuo* to afford the desired diester **V-2** (R = CH₃, R' = tBu) as a clear, colorless oil, 1.26g (86%). ^{**1**}**H-NMR** (300 MHz, CDCl₃): δ = 5.61 (1), 3.76-3.89 (1), 3.65 (3), 2.66-2.80 (1), 2.01-2.15 (2), 1.72-1.82 (1), 1.49-1.59 (1), 1.47 (9), 1.12 (6); **IR** (neat): 2976, 2952, 1738, 1711, 1637, 1390, 1378, 1368, 1301, 1265, 1213, 1197, 1183, 1150, and 1120cm⁻¹; **MS** (EI): *m*/*z* (rel. intensity) 209 (33), 208 (99), 166 (15), 149 (34), 148 (20), 121 (25), 107 (23), 79 (14), 57 (50), 55 (11); **HRMS** (FAB): calcd for C₁₆H₂₆O₄+H∼ 283.1909, found 283.1316.

### Preparation V-2-2

### Scheme V, V-2, wherein R = CH₃, P' = 'tBu (E)-4-Carbomethoxy-2,2-dimethylcyclohexylideneacetic acid (C₁₂H₁₈O₄)

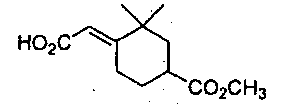

The diester **V-2** (R = CH₃, R' = tBu) (0.50g, 1.77mmol) was dissolved in trifluoroacetic acid (5mL), and the solution stirred at RT for 12h. Evaporation *in vacuo* gave a viscous, pale yellow oil which crystallized upon standing to afford the carboxylic acid **V-2** (R = CH₃, R' = H) as a waxy white solid, 0.40g (100%). **MP** 126-128°C. ^{**1**}**H-NMR** (300 MHz, CDCl₃): δ = 8.32 (1), 5.76 (1), 3.79-3.89 (1), 3.68 (3), 2.71-2.85 (1), 2.05-2.24 (2), 1.78- 1.88 (1), 1.47-1.63 (2), 1.16 (6); **IR** (mull): 3013, 2766, 1735, 1687, 1630, 1436, 1416, 1327, 1299, 1275, 1233, 1193, 1183, 1172, and 879cm⁻¹; MS (EI) : m/z (rel. intensity) 315 (99), 165 (27), 105 (30), 104 (48), 85 (21), 83 (20), 71 (24), 69 (26), 57 (44), 55 (35); **HRMS** (FAB) calcd for C₁₂H₁₈O₄ 226.1205, found 226.1193.

### Preparation V-3-1

### Scheme V, V-3, wherein R = CH₃, R" = CH₃ N-[(1E)-4-Carbomethoxy-2,2-dimethyl-1-cyclohexylideneacetyl]-4-[(2,6-dichlorobenzoyl)amino]-L-phenylalanine methyl ester (C₂₉H₃₂Cl₂N₂O₆) V-3

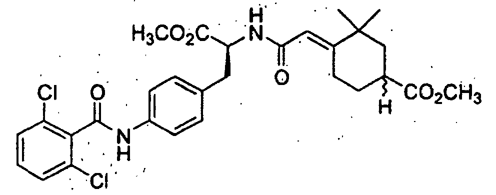

To a stirring solution of the carboxylic acid **V-2** (R = CH₃, R' = H) (0.42g, 1.86mmol) in CH₂Cl₂ (30mL) was added 4-[(2,6-dichlorobenzoyl)amino]-L-phenylalanine methyl ester. hydrochloride (0.75g, 1.86mmol), EDC (0.36g, 1.86mmol), DMAP (0.07g, 0.56mmol), HOBT (0.25g, 1.86mmol). To this suspension was added triethylamine dropwise until 'a solution was attained, and it was allowed to stir at RT for 16h. The solution was washed with 1N aqueous HCl (50mL), and the aqueous phase was extracted with CH₂Cl₂ (50mL). The combined extracts were washed with saturated aqueous NaHCO₃ (50mL), brine (50mL), dried (Na₂SO₄), filtered, and evaporated in *vacuo.* The resulting off-white solid was chromatographed on silica gel (200g, 230-400 mesh, 35mm OD column, packed and eluted with MeOH/CH₂Cl₂ (2.5:97.5), collecting 42mL fractions) using the flash technique. Fractions 58-82 were combined and evaporated *in vacuo* to afford the desired product **V-3** (R = CH₃, R" = CH₃) as a white solid, 0.44g (41%). **MP** 160-163°C; ^{**1**}**H-NMR** (300 MHz, CDCl₃) : δ = 7.57 (2), 7.28-7.39 (3), 7.13 (2) , 5.90 (1), 5.59 (1), 4.87-4.96 (1), 3.75 (3), 3.61-3.71 (4), 3.05-3.23 (2), 2.65-2.78 (1), 1.98-2.17 (2), 1.72-1.82 (1), 1.90-1.58 (2), 1.09-1.14 (6); **IR** (mull): 1741, 1667, 1650, 1627, 1608, 1546, 1513, 1433, 1414, 1327, 1278, 1267, 1207, 1198, and 1167cm⁻¹; **MS** (FAB) :m/z (rel. intensity) 575 (MH+, 86), 577 (59), 576 (35), 575 (86), 351 (20), 349 (30), 209 (99), 175 (23), 173 (39), 149 (26), 107 (49); **HRMS** (FAB): calcd for C₂₉H₃₂Cl₂N₂O₆+H, 575.1715, found 575.1706.

### Example 128

### Scheme V, V-3, wherein R = H, R" = H N-[(1E)-4-Carboxy-2,2-dimethyl-1-cyclohexylideneacetyl]-4-[(2,6-dichlorobenzoyl)amino]-L-phenylalanine (C₂₇H₂₈Cl₂N₂O₆) V-3

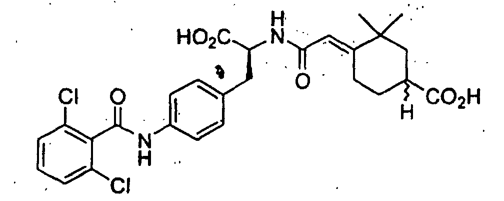

To a stirring solution of the diester **V-3** (R = CH₃, R" = CH₃) (0.31g, 0.54mmol) in MeOH (10mL) was added a solution of LiOH^{·}H₂O (0.05g, 1.08mmol) in H₂O (3mL). The reaction mixture stirred at RT for 16h and was then evaporated *in vacuo*. The residue was dissolved in H₂O (20mL), and 6N aqueous HCI was added dropwise until the pH of the mixture was *ca*. 4. The resulting precipitate was washed with H₂O, and dried in the vacuum oven at 70°C for 12h to afford 0.23g (78%) of the diacid V-3 (R = H, R" = H) as a while, crystalline solid. **MP** 155-162°C; ^{**1**}**H-NMR** (300 MHz, DMSO-d₆): δ = 11.84-12.75 (1), 8.16 (1), 7.52-7.60 (4), 7.43-7.51 (1), 7.20 (2), 5.71 (1), 4.34-4.45 (1), 3.67-3.77 (1), 2.95-3.05 (1), 2.76-2.88 (1), 2.51-2.65 (1), 1.81-2.01 (2), 1.64-1.74 (1), 1.01-1.37 (8); **IR** (mull): 3286, 3193, 3062, 1717, 1658, 1607, 1562, 1539, 1516, 1432, 1414, 1327, 1271, 1222, and 1196cm⁻¹. **MS** (FAB): m/z (rel. intensity) 547 (MH+, 92), 561 (27), 549 (61), 548 (37), 547 (92), 335 (34), 195 (99), 175 (37), 173 (59), 149 (36), 107 (35); **HRMS** (FAB) : calcd for C₂₇H₂₈Cl₂N₂O₆) +H1 547.1403, found 547.1392; **% Water** (KF): 3.12.

### Preparation V-2-3

### Scheme V, V-2, wherein R = H, R' = tBu (E)-4-Carboxy-2,2-dimethylcyclohexylideneacetic acid 1,1-dimethylethyl ester (C₁₅H₂₄O₄)

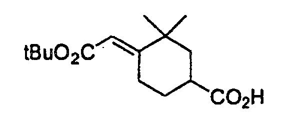

To a stirring solution of the diester **V-2** (R = CH₃, R' = tBu) (0.65g, 2.30mmol) in MeOH (8mL) was added a solution of LiOH^{·}H₂O (0.19g, 4.60mmol) in H₂O (4mL). The solution stirred at RT for 16h and was then evaporated *in vacuo.* The residue was dissolved in H₂O (25mL), and 6N aqueous HCl was added until the pH was *ca.* 4. The mixture was extracted with CHCI₃ (2x25mL), and the combined extracts were washed with brine (50mL), dried (MgSO₄), filtered, and evaporated in vacuo to afford 0.61g (99%) of the carboxylic acid **V-2** (R = H, R' = tBu) as a clear, colorless oil. ^{**1**}**H-NMR** (300 MHz, CDCl₃): δ = 5.62 (1), 3.78-3.91 (1), 2.68-2.84 (1), 2.02-2.18 (2), 1.76-1.86 (1), 1.51-1.62 (1), 1.47 (9), 1.13 (3); **IR** (neat) 3006, 2977, 2937, 2874, 1709, 1637, 1379, 1368, 1303, 1265, 1215, 1151, 1124, 1117, and 758cm⁻¹; **MS** (EI ): *m*/*z* (rel. intensity) 195 (32), 194 (99), 166 (17), 149 (18), 138 (14), 121 (21), 107 (20), 79 (12), 57 (60), 55 (13); **HRMS** (FAB) calcd for C₁₅H₂₄O₄+H1 269.1753, found 269.1755.

### Preparation V-4-1

### Scheme V, V-4, wherein R' = tBu, R" = CH₃ N-[[(4E)-3,3-Dimethyl-4-[[(1,1-dimethylethoxy)carbonyl]methylene]cyclohexyl]carbonyl]-4-[(2,6-dichlorobenzoyl)amino]-L-phenylalanine methyl ester (C₃₂H₃₈Cl₂N₂O₆) V-4

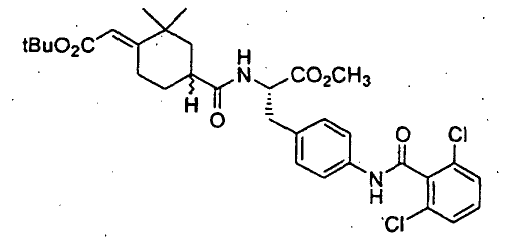

To a stirring solution of the acid V-2 (R = H, R' = tBu) (0.44g, 1. 64mmol) in CH₂CI₂ (30mL) was added 4-[(2,6-dichlorobenzoyl)amino]-L-phenylalanine methyl ester. hydrochloride (0.66g, 1.64mmol), EDC (0.31g, 1.64mmol), DMAP (0.06g, 0.49mmol), HOBT (0.22g, 1.64mmol), and Et₃N (0.9mL, 4.92mmol). The reaction mixture was stirred at RT for 16h, then was evaporated *in vacuo.* The residue was dissolved in 2% MeOH/CH₂Cl₂ (50mL), and this solution was washed with 1N aqueous HCI (50mL), saturated aqueous NaHCO₃ (50mL), brine (50mL), dried (Na₂SO₄), filtered, and evaporated *in vacuo*. The resulting white solid was chromatographed on silica gel (400g, 230-400 mesh, 70mm OD column, packed and eluted with MeOH/CH₂Cl₂, 3:97, collecting 270mL fractions) using the flash technique. Fractions 4-6 were combined and evaporated *in vacuo* to afford the desired product **V-4** (R' = tBu, R" = CH₃) as a white solid, 0.82g, (81%). **MP** 204-208°C; ^{**1**}**H-NMR** (300 MHz, CDCl₃): δ = 7.53-7.64 (3), 7.28-7.41 (3), 7.02-7.13 (2), 5.91-5.98 (1), 5.61 (1), 4.82-4.90 (1), 3.79-3.91 (1), 3.75 (3), 3.03-3.20 (2), 2.47-2.61 (1), 1.71-1.83 (2), 1.38-1.70 (12), 1.11 (6); **IR** (mull): 1749, 1711, 1672, 1651, 1610, 1562, 1548, 1514, 1432, 1415, 1331, 1278, 1215, 1174, and 1152cm⁻¹; **MS** (FAB:) *m*/*z* (rel. intensity) 617 (MH+, 13), 545 (69), 544 (35), 543 (99) , 351 (39) , 349 (57), 175 (50), 173 (50) , 149 (41), 121 (50), 57 (80); **HRMS** (FAB): calcd for C₃₂H₃₈Cl₂N₂O₆+H1 617.2185, found 617.2178.

### Preparation V-4-2

### Scheme V, V-4, wherein R' = H, R" = CH₃ N-[[[(4E)-4-Carboxymethylene-3,3-dimethyl]cyclohexyl]carbonyl]-4-[(2,6-dichlorobenzoyl)amino]-L-phenylalanine methyl ester (C₂₈H₃₀Cl₂N₂O₆) V-4

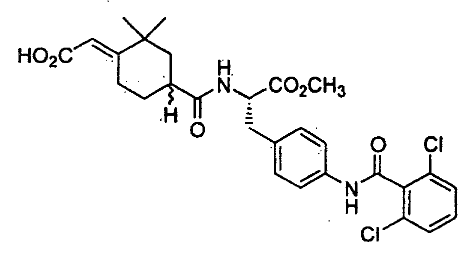

A solution of the diester **V-4** (R' = tBu, R" = CH₃) (0.50g, 0.89mMol) in trifluoroacetic acid (5mL) was stirred at RT for 16h. The reaction mixture was evaporated *in vacuo* to give a pale yellow glass. Trituration with Et₂O gave the desired carboxylic acid **V-4** (R' = H, R" = CH₃) as a white solid which was collected by suction filtration and dried, 0.38g (76%). **MP** 228-230°C; ^{**1**}**H-NMR** (300 MHz, DMSO-d₆): δ = 11.67-12.28 (1), 10.67 (1), 8.16-8.31 (1), 7.40-7.68 (5), 7.17 (2), 5.54 (1), 4.36 4.49 (1), 3.71 (1), 3.60 (3), 2.91-3.07 (1), 2.75 2.91 (1), 2.53-2.71 (1), 0.91-2.13 (11); **IR** (mull): 1750, 1692, 1672, 1648, 1611, 1553, 1540, 1514, 1444, 1432, 1415, 1335, 1278, 1225, and 1213cm⁻¹; **MS** (El ) *m*/*z* (rel. intensity) 560 (M+, 1), 351 (66), 350 (19), 349 (99), 280 (20), 278 (29) , 177 (16), 175 (64), 173 (99), 107 (13), 106 (17); **HRMS** (EI) calcd for C₂₈H₃₀Cl₂N₂O₆ 560.1481, found 560.1477.

### Example 129

### Scheme V, V-4, wherein R' = H, R" = H N-[[[(4E)-4-Carboxymethylene-3,3-dimethyl]cyclohexyl]carbonyl]-4-[(2,6-dichlorobenzoyl)amino]-L-phenylalanine (C₂₇H₂₈Cl₂N₂O₆) V-4

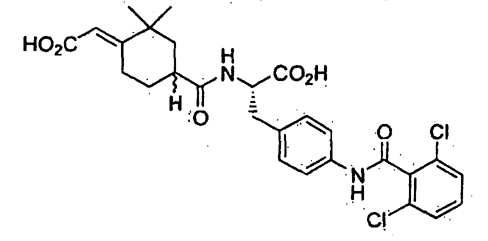

To a stirring solution of the methyl ester **V-4** (R' = H, R" = CH₃) (0.33g, 0.59mmol) in MeOH (7mL) was added a solution of LiOH^{·}H₂O (0.05g, 1.18mmol) in H₂O (3mL). The reaction mixture stirred at RT for 16h and was then evaporated *in vacuo*. The residue was dissolved in H₂O (20mL), and 6N aqueous HCI was added dropwise until the pH of the mixture was ca. 1. The resulting gelatinous solid was collected by suction filtration and dried in a vacuum oven at 70°C for 12h. The resulting white crystalline solid was crushed, thoroughly washed with H₂O, and dried in the vacuum oven at 70°C for 12h to afford 0.12g (37%) of the diacid **V-4** (R' = H, R" = H). **MP** 165-167°C; ^{**1**}**H-NMR** (300 MHz, DMSO-d₆): δ = 11.82-12.65 (2), 10.65 (1), 8.04-8.13 (1), 7.51-7.61 (4), 7.43-7.51 (1), 7.17 (2), 5.54 (1), 4.31-4.42 (1), 3.64-3.76 (1), 2.95-3.06 (1), 2.73-2.87 (1), 2.53-2.69 (1), 0.88-2.06 (11); **IR** (mull) 3281, 3071, 3036, 1.659, 1608, 1562, 1540, 1517, 1432, 1414, 1327, 1271, 1220, 1196, and 1170cm⁻¹; **MS** (FAB) m/z (rel. intensity) 547 (MH+, 82), 549 (54), 548 (32), 547 (82), 531 (67), 529 (99), 337 (30), 335 (45), 175 (44), 173 (70), 123 (41); **HRMS** (FAB) calcd for C₂₇H₂₈Cl₂N₂O₆+H1 547.1403, found 547.1417. **% Water** (KF): 2.51.

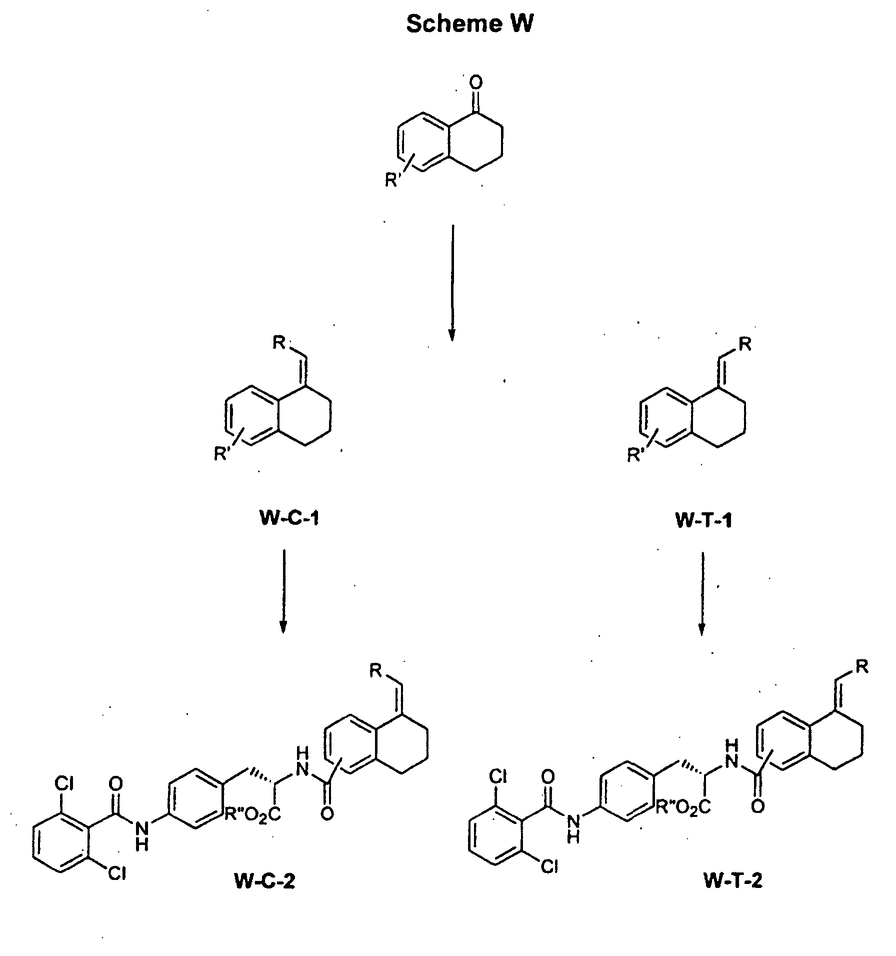

### Preparation W-C-1 and W-T-1

### Scheme X, W-C-1 and W-T-1, wherein R = CO₂tBu, R' = 5-CO₂Me (Z)-5-Carbomethoxy-3,4-dihydro-1(2H)-naphthalenylideneacetic acid 1,1-dimethylethyl ester W-C-1 (C₁₈H₂₂O₄) and (E)-5-Carbomethoxy-3,4-dihydro-1(2H)-naphthalenylideneacetic acid 1,1-dimethylethyl ester W-T-1 (C₁₈H₂₂O₄)

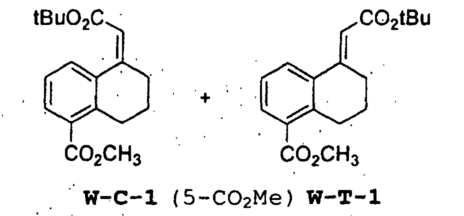

To a dry, 50mL two-neck flask was added diethylphosphono t-butylacetate (Aldrich, 6.4 mL, 27.2mmol). The flask was flushed with Ar/house vac (3X) and then dry THF(10mL) was added. The flask was immersed in an ice/water bath and, five minutes later, NaH/oil (60% NaH, 1.0g., 25.0mmol) was cautiously added in portions. After stirring for 30 minutes, a solution of the known 5-carbomethoxy-2-tetralone (Gerlach, U.; Wollmann, T. *Tetrahedron Lett.* **1992,** *33*, 5499) (4.62g., 22.6mmol) in dry THF (20mL) was added. The mixture stirred for 18 hours, warming slowly as the ice melted, then the reaction was quenched by the addition of ice (20mL) and the mixture was partitioned between water (100mL) and hexanes (200mL). The aqueous layer was extracted with hexanes (3X150mL) and the combined organic phases were washed with brine (1X100mL), dried (MgSO₄), and then evaporated to dryness, giving a pale brown oil (11.0g.).The crude product was purified by chromatography via medium pressure liquid chromatography (MPLC) and eluted with a gradient from 0 to 4% ethyl acetate/hexane. Two fractions were collected and isolated by evaporation *in vacuo* to give colorless oils. The first eluted (MPLC) fraction afforded 2.47g, 36% yield of the trans olefin **W-T-1.** ^{**1**}**H-NMR** (CDCl₃) δ = 7.77(2), 7.22(1), 6.19(1), 3.88(3), 3.11(4), 1.79(2), 1.51(9). **MS** (EI ) m/z (rel. intensity) 302 (M+, 1), 247 (16), 246 (99), 229 (21), 214 (36), 186 (12), 169 (39), 142 (15), 141 (35), 115 (21), 57 (35). **Anal.** Calcd for C₁₈H₂₂O₄^{·}0.3C₃H₆O: C, 71.50; H, 7.33. Found: C, Found: C, 70.89; H, 7.31..Further elution (MPLC) afforded 2.61g (38%) of the cis-olefin **W-C-1** as a colorless oil **.**^{**1**}**H NMR** (CDCl₃) δ = 7.85(1), 7.65(1), 7.18(1), 5.75(1), 3.87(3), 3.17(2), 2.44(2), 1.94(2), 1.42(9). **MS** (EI ) m/z (rel. intensity) 302 (M+, 1), 247 (16), 246 (99), 229 (20), 214 (38), 186 (13), 169 (43), 142 (17), 141 (39), 115 (25), 57 (35). **Anal.** Calcd for C₁₈H₂₂O₄^{·}0.3C₃H₆O: C, 71.50; H, 7.33. Found: C, 71.58; H, 7.19.

### Preparation W-C-1 and W-T-1

### Scheme X, W-C-1 and W-T-1, wherein R = CO₂tBu, R' = 6-CO₂Me (Z)-6-Carbomethoxy-3,4-dihydro-1(2H)-naphthalenylideneacetic acid 1,1-dimethylethyl ester W-C-1 (C₁₈H₂₂O₄) and (E)-6-Carbomethoxy-3,4-dihydro-1(2H)-naphthalenylideneacetic acid 1,1-dimethylethyl ester W-T-1 (C₁₈H₂₂O₄)

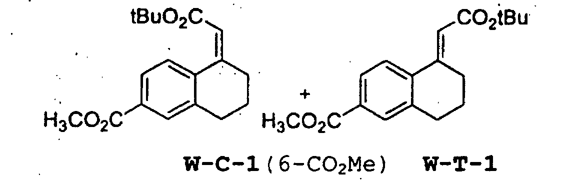

To a dry, 50mL two-neck flask 'was added diethylphosphono t-butylacetate (Aldrich, 7 mL, 29.8mmol). The flask was flushed with Ar/house vac (3X) and then dry THF (10mL) was added . The flask was immersed in an ice/water bath and, five minutes later, NaH/oil (60% NaH, 1.15g., 28.8mmol) was cautiously added in portions. Thirty minutes later, a solution of the known 6-carbomethoxy-2-tetralone (Gerlach, U.; Wollmann, T. *Tetrahedron Lett*. **1992,** *33*, 5499) (4.62g., 22.6mmol) (5.22g., 25.55mmol) in dry THF(20mL) was added. After two 36 hours, the mixture was quenched by addition of ice (20g) and then partitioned between water (100mL) and hexanes (200mL). The aqueous layer was shaken with hexanes (3X150mL) and the combined hexanes layers washed with brine (1X100mL) and then evaporated to dryness, giving a pale brown oil (12.5g.). Crystallization from hexanes (100mL, freezer) gave **W-T-1** (6-CO₂CH₃) as a white solid (2.37g). The mother liquor was transferred to a medium pressure liquid chromatography apparatus and eluted with a gradient from 0 to 4% ethyl acetate/hexane. Two fractions were collected and isolated by evaporation *in vacuo* to give colorless solids. Band 1 (0.559g) was combined with the above hexanes crystals, with which it is spectroscopically identical, to provide a total of 2.93g (38%) of **W-T-1** (6-CO₂CH₃). ^{**1**}**H-NMR** (CDCl₃) : δ = 7.71(2), 7.56(1), 6.22(1), 3.81(3), 3.07(2), 2.71(2), 1.75(2); **IR** (mull) 2133, 1935, 1718, 1700, 1618, 1438, 1296, 1275, 1260,

1236, 1201, 1181, 1152, 1144, and 1105 cm⁻¹; **MS** (EI ) m/z (rel. intensity) 302 (M+, 1), 246 (99), 231 (22), 229 (28), 228 (21), 187 (24), 169 (23), 142 (30), 141 (48), 115 (27), 57 (53); **Anal.** Calcd. for C₁₈H₂₂O₄: C, 71.50; H, 7.33. Found: C, 71.03; H, 7.07.
Fraction 2 gave 2.50g. (32%) of **W-C-1** (6-CO₂CH₃) as a white solid. ^{**1**}**H-NMR** (CDCl₃) : δ = 7.78(2), 7.59(1), 5.79(1), 3.89 (3), 2.87(2), 2.47(2), 1.95(2), 1.44(9); **IR** (liq.) 2951, 2396, 1982, 1927, 1721, 1437, 1368, 1306, 1287, 1266, 1226, 1200, 1147, 1110, and 77.5cm⁻¹; **MS** (EI ) m/z (rel. intensity) 302 (M+, 1), 246 (99), 231 (19), 229 (22), 228 (20), 187 (21), 169 (17), 142 (19), 141 (29) , 115 (16), 57 (29); **Anal.** Calcd for C₁₈H₂₂O₄: C, 71.50; H, 7.33. Found: C, 71.29; H, 7.19.

### Preparation W-C-1

### Scheme X, W-C-1 wherein R = CO₂tBu, R' = 5-CO₂H (Z)-5-Carboxy-3,4-dihydro-1(2H)-naphthalenylideneacetic acid 1,1,-dimethylethyl ester W-C-1 (C₁₇H₂₀O₄)

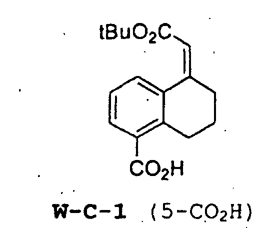

Sodium thiophenoxide (5.05g, 38mmol) was added to'a stirred mixture of the diester **W-C-1** (2.3g, 7.6mmol) in dry THF (50mL).

The mixture was stirred for two days at room temperature and then heated for two days in a 35°C oil bath. The mixture was then evaporated to dryness in vacuo, stirred with H₂O (50mL), and filtered through a sintered glass funnel, and the filter cake is rinsed with water (4X20mL). The filtrate was treated with aqueous HCl (1.2N) to pH6 and then filtered (4X10mL H₂O rinses). The filtered solid was air dried to give a white solid (1.73g) which was transferred to a medium pressure chromatography column and eluted with a gradient from 0% to 5% CH₃OH/CH₂Cl₂. The UV absorbing band was isolated by evaporation of eluant *in vacuo* to give **W-C-1** (R = CO₂tBu, R' = 5-CO₂H ) as a white solid (1.56g, 70% yield). ^{**1**}**H-NMR** (CDCl₂) δ = 7.98 (1), 7.82 (1), 7.28 (1), 6.21 (1), 3.16 (4), 1.82 (2), 1.52 (9); **MS** (ESI-) for C₁₇H₂₀O₄ *m*/*z* 287.2 (M-H).

### Preparation W-T-1

### Scheme X, W-T-1 wherein R = CO₂tBu, R' = 5-CO₂H (E)-5-Carboxy-3,4-dihydro-1(2H)-naphthalenylideneacetic acid 1,1,-dimethylethyl ester W-C-1 (C₁₇H₂₀O₄)

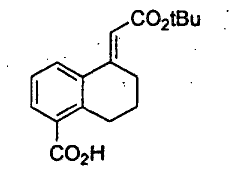

Sodium thiophenoxide (5.05g, 38mmol) was added to a stirred mixture of the diester **W-T-1** (R = CO₂tBu, R' = 5-CO₂CH₃) (2.3g, 7.6mmol) in dry THF (50mL). The mixture was stirred for 36 hours at room temperature and then heated for 48 hours in a 35°C oil bath. The mixture was then evaporated to dryness in *vacuo,* stirred with H₂O (50mL), and filtered through a sintered glass funnel (4X20mL H₂O rinses). The filtrate was treated with aqueous HCl (1.2N) to pH6 and then filtered (4X10mL H₂O rinses). The filtered solid was air dried to give a white solid (1.73g) which was transferred to a medium pressure chromatography column and eluted with a gradient from 0% to 5% CH₃OH/CH₂Cl₂. The UV absorbing band was isolated by evaporation of eluant *in vacuo* to give **W-T-1** (R = CO₂tBu, R' = 5-CO₂H) as a powdery, white solid (1.56g, 70% yield). ^{**1**}**H-NMR** (CDCl₃): δ = 7.98 (1), 7.82 (1), 7.28 (1), 6.21 (1), 3.16 (4), 1.82 (2), 1.52 (9); **MS** (ESI-) for C₁₇H₂₀O₄ *m*/*z* 287.2 (M-H).

### Preparation W-T-1

### Scheme X, W-T-1 wherein R = CO₂tBu, R' = 6-CO₂Na (E)-6-Carboxy-3,4-dihydro-1(2H)-naphthalenylideneacetic acid 1,1,-dimethylethyl ester sodium salt W-C-1 (C₁₇H₁₉O₄Na)

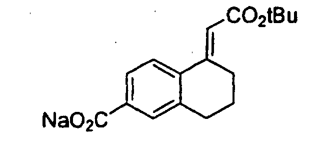

A solution of the diester **W-T-1** (R = CO₂tBu, R' = 6-CO₂Me) (0.94g., 3.1mmol) and sodium thiophenoxide (0.62g., 4.69mmol) in dry DMF (5mL) was stirred under Ar and heated in a 60°C oil bath. After overnight stirring, copious precipitation had occurred. The mixture was removed from the oil bath, cooled to room temperature, mixed with dry DMF (6mL), and filtered (with 4X2mL DMF rinses) through a sintered glass funnel. The filter cake was air-dried to give **W-T-1** (6-CO₂Na) as a white solid (0.72g., 74%). ^{**1**}**H-NMR** (DMSO-d₆): δ = 7.52 (3) , 6.21(1), 3.03(2), 2.73 (2) , 1.72(2), 1.44(9); **MS** (ESI-) for C₁₇H₁₉O₄ m/z

### Preparation W-C-2

### Scheme X, W-C-2 wherein R = CO₂tBu, 5-substituted, R" = CH₃ N-[[(5Z)-7,8-Dihydro-5-[[(1,1-dimethylethoxy) carbonyl]methylene]-1(6H)-naphthyl]carbonyl]-4-[(2,6-dichlorobenzoyl)amino]-L-phenylalanine methyl ester (C₃₄H₃₄Cl₂N₂O₆)

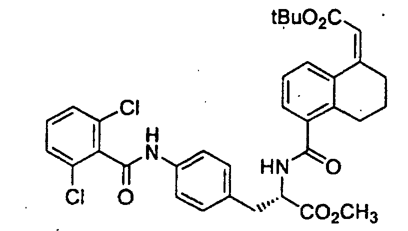

To a mixture of the **W-C-1** (R = CO₂tBu, R' = 5-CO₂H) (1. 18g. , 4.08mmol) in dry DMF (20mL) under N₂ and cooled in an ice water bath was added EDC (0.86g, 4.49mmol), HOBT (0.61g, 4.51mmol), diisopropylethyl amine(3.8mL, 21.82mmol), and 4-dimethylaminopyridine (0.05g., 0.41mmol). After thirty minutes, 4-[(2,6-dichlorobenzoyl)amino]-L-phenylalanine methyl ester. hydrochloride (1.8g., 4.46mmol) and more dry DMF (5mL) were added. After overnight stirring, the mixture was evaporated to dryness *in vacuo,* giving an oil which was dissolved in CHCl₃ (150mL) and washed with water (50mL), aq. HCl (1N, 2X50mL), water (5X50mL, to pH7) and evaporated to dryness, giving **W-C-2** (R = CO₂tBu, 5-substituted, R" = CH₃) as a pale yellow powder (2.68g., 93%). An analytical sample was recrystallized from toluene/chloroform. ^{**1**}**H-NMR** (CDCl₃): δ = 7.59(4), 7.27(7), 6.20(2), 5.08(1), 3.80(3), 3.31(1), 3.13(3), 2.73(2), 1.74(2), 1.51(9); **IR** (mull) 1751, 1700, 1675, 1640, 1612, 1555, 1534, 1515, 1433, 1336, 1278, 1225, 1201, 1153, and 1147cm⁻¹; **MS** (FAB) m/z (rel. intensity) 637 (MH+, 38), 639 (28), 637 (38), 581 (25), 565 (24), 563 (35), 271 (24), 215 (99), 197 (29), 173 (35), 57 (30); **Anal.** Calcd for C₃₄H₃₄Cl₂N₂O₆: C, 64.05; H, 5.38; N, 4.39. Found: C, 63.76; H, 5.41; N, 4.45.

### Example 130

### Scheme X, W-C-2 wherein R = CO₂H, 5-substituted, R" = CH₃ N-[[(5Z)-5-Carboxymethylene-7,8-dihydro-1(6H)-naphthyl]carbonyl]-4-[(2,6-dichlorobenzoyl)amino]-L-phenylalanine methyl ester (C₃₀H₂₆Cl₂N₂O₆)

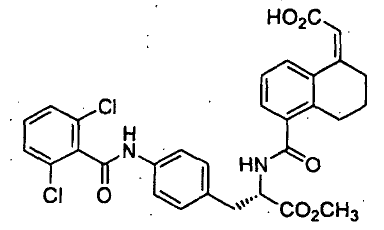

A solution of **W-C-2** (R = CO₂tBu, 5-substituted, R" = CH₃) (0.244g., 0.38mmol) in methylene chloride was cooled in an ice water bath and then trifluoroacetic acid (1.0mL, 13mmol) was added. After 10 minutes, the cooling bath was removed. After four days, the heterogeneous mixture was filtered through a sintered glass funnel and the filter cake washed with methylene chloride (3X2mL) and air dried to give a white solid, **W-C-2,** R = CO₂H, 5-substituted, R" = CH₃) (0.185g., 82%). An analytical sample was prepared by stirring the compound with saturated aq. NaHCO₃ to convert it to its water soluble salt followed by reversed phase chromatography (0% to 30% acetonitrile/water) and acidification. ^{**1**}**H-NMR** (DMSO-d₆) δ = 12.21(1), 10.69(1), 8.74(1), 7.51(6), 7.25(2), 7.12(2), 5.80(1), 4.69(1), 3.68(3), 3.15(1), 2.89(1), 2.41(4), 1.71(2); **IR** (mull) 3261, 1743, 1696, 1659, 1642, 1610, 1529, 1433, 1414, 1320, 1265, 1226, 1216, 1206, and 1195cm⁻¹; **MS** (FAB) m/z (rel. intensity) 581 (MH+, 63), 583 (43), 582 (28), 581 (63), 565 (15), 563 (22), 349 (21), 215 (99), 197 (46), 175 (16), 173 (28); **HRMS** (FAB) calcd. for C₃₀H₂₆Cl₂N₂O₆+H1 581.1246, found 581.1232.

### Example 131

### Scheme X, W-C-2 wherein R = CO₂H, 5-substituted, R" = H N-[[(5Z)-5-Carboxymethylene-7,8-dihydro-1(6H)-naphthyl]carbonyl]-4-[(2,6-dichlorobenzoyl)amino]-L-phenylalanine (C₂₉H₂₄Cl₂N₂O₆)

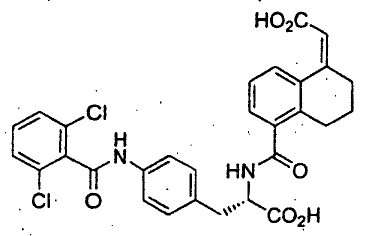

To **W-C-2** (R = CO₂H, 5-substituted, R" = CH₃) (0.9g., 1.5mmol) in methanol (50mL) was stirred and cooled in an ice water bath. To the cooled solution was added an ice cold solution of LiOH•H₂O (0.33g., 7.9mmol) in H₂O (10mL). After overnight stirring, the mixture was evaporated to dryness *in vacuo,* keeping the water bath temperature at or below room temperature to give a white solid. The mixture was then dissolved in water (50mL) and the pH was adjusted to approximately pH8 using 1N HCl. The solution was then filtered and the filtrate transferred to a reversed phase (C-18) medium pressure chromatography column and eluted with a gradient from 0 to 30% acetonitrile/water. Concentration, *in vacuo*, of the fractions containing **W-C-2** (R = CO₂H, 5-substituted, R" = CH₃) gave 0.337g (35%) of **W-C-2** (R = CO₂H, 5-substituted, R" = CH₃) as a white solid. ^{**1**}**H-NMR** (DMSO-d₆) δ = 10.73(1), 8.58(1), 7.52(5), 7.18(5), 5.94(1), 4.63(1), 3.15(1), 2.86(1), 2.48-1.70(6); **IR** (mull) 3257, 3068, 3037, 1720, 1663, 1645, 1608, 1581, 1562, 1540, 1516, 1432, 1414, 1327, and 1196cm⁻¹; **MS** (FAB) m/z (rel. intensity) 567 (MH+, 99), 570 (27), 569 (71) , 568 (57), 567 (99), 566 (26), 216 (26), 215 (89), 175 (22), 173 (30), 169 (20); **HRMS** (FAB) calcd. for C₂₉H₂₄Cl₂N₂O₆+H1 567.1089, found 567.1102; **% Water** (KF): 3.72.; **Anal.** Calcd for C₂₉H₂₄Cl₂N₂O₆^{·}1.21H₂O: C, 59.11; H, 4.52; N, 4.75. Found: C, 58.89; H, 4.38; N, 4.70.

### Example 132

### Scheme X, W-T-2 wherein R = CO₂tBu, 5-substituted, R" = CH₃ N-[[(5E)-7,8-Dihydro-5-[[(1,1-dimethylethoxy)carbonyl]methylene]-1(6H)-naphthyl]carbonyl]-4-[(2,6-dichlorobenzoyl)amino]-L-phenylalanine methyl ester (C₃₄H₃₄Cl₂N₂O₆)

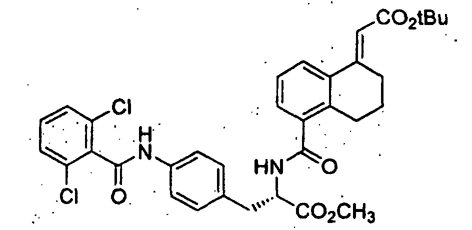

To a mixture of **W-T-1** (R = CO₂tBu, R' = 5-CO₂H) (1.18g., 4.08mmol) in dry DMF (20mL) under N₂ and cooled in an ice water bath was added EDC (0.86g, 4.49mmol), HOBT (0.61g, 4.51mmol), diisopropylethyl amine (3.8mL, 21.82mmol), and 4-dimethylaminopyridine (0.05g., 0.41mmol). After thirty minutes, 4-[(2,6-dichlorobenzoyl)amino]-L-phenylalanine methyl ester hydrochloride (1.8g., 4.46mmol) and more dry DMF .(5mL) were added. After overnight stirring, the mixture was evaporated to dryness *in vacuo,* giving an oil which was dissolved in CHCl₃ (150mL) and washed with water (50mL), aq. HCl (1N, 2X50mL), water (5X50mL, to pH7) and evaporated to dryness, giving **W-T-2** (R = CO₂tBu, 5-substituted, R" = CH₃) as a pale yellow powder (2.68g., 93%). An analytical sample was recrystallized from toluene/chloroform. ^{**1**}**H-NMR** (CDCl₃) δ = 7.59(4), 7.27(7), 6.20(2), 5.08(1), 3.80(3), 3.31(1), 3.13(3), 2.73(2), 1.74(2), 1.51(9) ; **IR** (mull) 1751, 1700, 1675, 1640, 1612, 1555, 1534, 1515, 1433, 1336, 1278, 1225, 1201, 1153, and 1147cm⁻¹; **MS** (FAB) m/z (rel. intensity) 637 (MH+, 38), 639 (28), 637 (38), 581 (25), 565 (24), 563 (35), 271 (24), 215 (99), 197 (29), 173 (35), 57 (30); **Anal.** Calcd. for C₃₄H₃₄Cl₂N₂O₆: C, 64.05; H, 5.38; N, 4.39. Found: C, 63.76; H, 5.41; N, 4.45.

### Example 133

### Scheme X, W-T-2 wherein R = CO₂H, 5-substituted, R" = CH₃ N-[[(5E)-5-Carboxymethylene-7,8-dihydro-1(6H)-naphthyl]carbonyl]-4-[(2,6-dichlorobenzoyl)amino]-L-phenylalanine methyl ester (C₃₀H₂₆Cl₂N₂O₆)

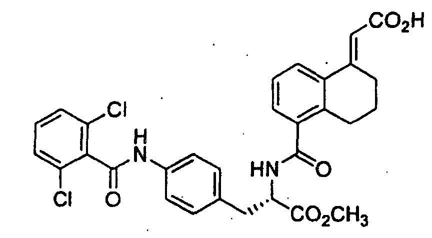

To a solution of **W-T-2** (R = CO₂tBu, 5-substituted, R" = CH₃) (1.4g, 2.2mmol) in methylene chloride (20mL) under N₂ was added trifluoroacetic acid (1mL, 13mmol) . After overnight stirring, an additional portion of trifluoroacetic acid was added (1mL, 13mmol). After stirring for two more days, the mixture was mixed with toluene (30mL) and evaporated to dryness *in vacuo* to give a yellow solid. This solid was mixed with chloroform (25mL) and acetone (2mL), heated (40°C), allowed to cool to room temperature overnight and then filtered through a sintered glass funnel. The filter cake was washed with chloroform (3X3mL) and air dried to give **W-T-2** (R = CO₂H, 5-substituted, R" = CH₃) (1.08g, 84%) as a pale yellow solid. ^{**1**}**H-NMR** (DMSO-d₆) : δ = 12.10(1), 10.69(1), 8.73(1), 7.74(1), 7.57(5), 7.18(4), 6.26(1), 4.71(1), 3.69(3), 2.97(4), 2.36(2), 1.59(2); **IR** (mull) 1753, 1677, 1644, 1636, 1602, 1544, 1515, 1443, 1433, 1277, 1239, 1220, 1210, 1199, and 1189cm⁻¹; **HRMS** (FAB) calcd for C₃₀H₂₆Cl2N₂O₆+H1 581.1246, found 581.1268.

### Example 134

### Scheme X, W-T-2 wherein R = CO₂H, 5-substituted, R" = H N-[[(5E)-5-Carboxymethylene-7,8-dihydro-1(6H)-naphthyl]carbonyl]-4-[(2,6-dichlorobenzoyl)amino]-L-phenylalanine (C₂₉H₂₄Cl₂N₂O₆)

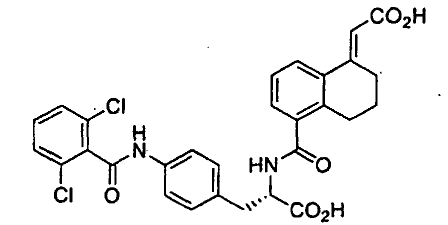

A solution of LiOH·H₂O (0.3g, 7.15mmol) in H₂O(5mL) was added to a solution of **W-T-2** (R = CO₂H, 5-substituted, R" = CH₃) (0.8g, 1.3mmol) in methanol (10mL). After overnight stirring, the mixture was evaporated to dryness *in vacuo*, giving a pale yellow solid which was dissolved in water (20mL) and cooled in an ice/water bath. The pH was adjusted to ca. 3 using 1N aq. HCl. After an hour in the ice bath, the mixture was filtered through a sintered glass funnel to give a pale yellow solid which was washed with water (3X10mL) and air dried affording **W-T-2** (R = CO₂H, 5-substituted, R" = H) (0.79g, 96%). ^{**1**}**H-NMR** (DMSO-d₆) δ = 12.90(1), 10.67(1), 8.57(1), 7.73(1), 7.56(5), 7.21(4), 6.26(1), 4.63(1,), 2.96(4), 2.30(2), 1.57(2); **IR** (mull) 3258, 3193, 3123, 3066, 1660, 1608, 1584, 1562, 1539, 1516, 1432, 1414, 1327, 1273, and 1195cm⁻¹; **MS** (FAB) m/z (rel. intensity) 567 (MH+, 96), 570 (20), 569 (65), 568 (36), 567 (96), 551 (17), 549 (31), 215 (99), 197 (21), 175 (25), 173 (46); **HRMS** (FAB) calcd for C₂₉H₂₄Cl₂N₂O₆+H1 567.1089, found 567.1082; % **Water** (KF): 6.64; **Anal.** Calcd for C₂₉H₂₄Cl₂N₂O₆^{·}2.24H₂O: C, 57.31; H, 4.72; N, 4.61. Found: C, 57.31; H, 4.43; N, 4.64.

### Preparation W-T-2

### Scheme X, W-T-2 wherein R = CO₂tBu, 6-substituted, R" = CH₃ N-[[(5E)-7,8-Dihydro-5-[[(1,1-dimethylethoxy)carbonyl]methylene]-2(6H)-naphthyl]carbonyl]-4-[(2,6-dichlorobenzoyl)amino]-L-phenylalanine methyl ester (C₃₄H₃₄Cl₂N₂O₆)

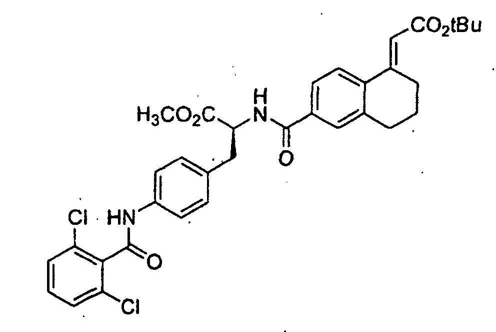

To a heterogeneous mixture of the salt **W-T-1** (6-CO₂Na) (0.65g., 2.1mmol) in dry DMF (20mL) under N₂ and cooled in an ice water bath were added EDC (0.45g., 2.3mmol), HOBT(0.31g., 2.3mmol), diisopropylethyl amine(2mL, 11.5mmol), and 4-dimethylaminopyridine (0.03g., 0.3mmol). After thirty minutes, 4-[(2,6-dichlorobenzoyl)amino]-L-phenylalanine methyl ester. hydrochloride (1.0g., 2.48mmol) and more dry DMF (5mL) were added. After stirring for 60 hours, the mixture was evaporated to dryness in *vacuo,* giving an oil which was dissolved in CHCl₃ (150mL) and washed with water (50mL), aq. HCl (1N, 2X50mL), water (5X50mL, to pH7), and evaporated to dryness, giving **W-T-2** (R = CO₂tBu, 6-substituted, R" = CH₃) as a pale yellow powder (1.2g, 89%). ^{**1**}**H-NMR** (CDCl₃): δ = 7.67(1), 7.56(4), 7.31(4), 7.13(2), 6.62(1), 6.29(1), 5.07(1), 3.79(3), 3.25(2), 3.14(2), 2.81(2), 1.84(2), 1.51(9); **MS** (ESI-) for C₃₄H₃₃Cl₂N₂O₆ *m*/*z* 634.9 (M-H); **IR** (mull) 1748, 1705, 1664, 1641, 1608, 1561, 1546, 1536, 1513, 1441, 1429, 1329, 1203, 1148, and 1141cm⁻¹; **Anal.** Calcd for C₃₄H₃₄Cl₂N₂O₆: C, 64.05; H, 5.38; N, 4.39. Found: C, 63.67; H, 5.39; N, 4.38.

### Preparation W-T-2

### Scheme X, W-T-2 wherein R = CO₂tBu, 6-substituted, R" = Na N-[[(5E)-7,8-Dihydro-5-[[(1,1-dimethylethoxy)carbonyl]methylene]-2(6H)-naphthyl]carbonyl]-4-[(2,6-dichlorobenzoyl)amino]-L-phenylalanine sodium salt (C₃₃H₃₁Cl₂N₂O₆Na)

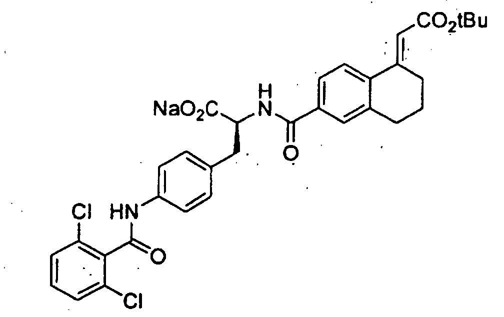

Sodium thiophenoxide was added to a stirred solution of **W-T-2** (R = CO₂tBu, 6-substituted, R" = CH₃) (0.418g, 0.66mmol) in dry THF(20mL) under N₂. After overnight stirring, the mixture was evaporated to dryness *in vacuo* to give a pale yellow solid which was stirred with water (50 mL) for 10 minutes and then filtered. The filtrate was transferred to a reversed phase (C-18) chromatography column and eluted with water(1L) followed by 20% acetonitrile/water. Evaporation of solvent *in vacuo* gave **W-T-2** (R = CO₂tBu, 6-substituted, R" = Na) as a white solid (0.184g, 43%). ^{**1**}**H-NMR** (DMSO-d₆): δ = 10.59(1), 7.76(2), 7.48(7), 7.06(2), 6.29(1), 4.09(1), 3.15(1), 3.03(3), 2.78(2), 1.73(2), 1.45(9); **IR** (mull) 3392, 3297, 1704, 1664, 1605, 1562, 1535, 1517, 1485, 1431, 1411, 1402, 1393, 1321, and 1145cm⁻¹; **MS** (FAB) m/z (rel. intensity) 622 (MH+, 0), 669 (13), 667 (18), 647 (13), 645 (18) , 331 (9), 215 (17), 179 (9), 177 (89), 57 (11), 23 (99); **HRMS** (FAB) calcd for C₃₃H₃₁Cl₂N₂O₆Na+H1 622.1637, found 645.1550.

### Example 135

### Scheme X, W-T-2 wherein R = CO₂Na, 6-substituted, R" = Na N-[[(5E)-5-Carboxymethylene-7,8-dihydro-2(6H)-naphthyl]carbonyl]-4-[(2,6-dichlorobenzoyl)amino]-L-phenylalanine sodium salt (C₂₉H₂₂Cl₂N₂O₆Na₂)

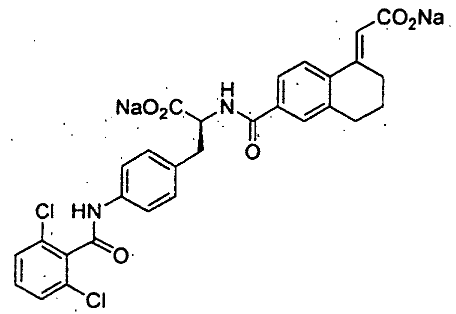

A solution of trifluoroacetic acid (4 mL) and **W-T-2** (R = CO₂tBu, 6-substituted, R" = Na) (0.21g, .32mmol) was stirred overnight under N₂. The reaction mixture was then mixed with toluene (50mL) and evaporated to dryness *in vacuo*, giving an off-white solid. This solid was then dissolved in methanol (10mL) and stirred with saturated aqueous NaHCO₃ (10mL). The mixture was then evaporated to dryness *in vacuo,* dissolved in water, filtered and the filtrate transferred to a reversed phase C-18 chromatography column. Elution with 0 to 10% acetonitrile/H₂O gave **W-T-2** (R = CO₂Na, 6-substituted, R" = Na) as a white solid after evaporation of solvent *in vacuo* (0.14g, 70%).
^{**1**}**H-NMR** (DMSO-d₆): δ = 7.69(1), 7.48(7), 6.49(1), 4.70(1), 3.37(1), 3.16(2), 2.98(2), 2.84(2), 1.85(2); **IR** (mull) 3388, 3261, 3123, 1656, 1606, 1561, 1543, 1517, 1485, 1432, 1412, 1349, 1328, 1195, and 1114cm⁻¹; **HRMS** (FAB) calcd for C₂₉H₂₂Cl₂N₂O₆Na₂+H1 611.0729, found 611.0755; % **Water** (KF): 6.40; **Anal.** Calcd for C₃₃H₃₁Cl₂N₂O₆Na·2.45H₂O: C, 57.47; H, 5.25; N, 4.06. Found: C, 57.31; H, 5.18; N, 3.97.

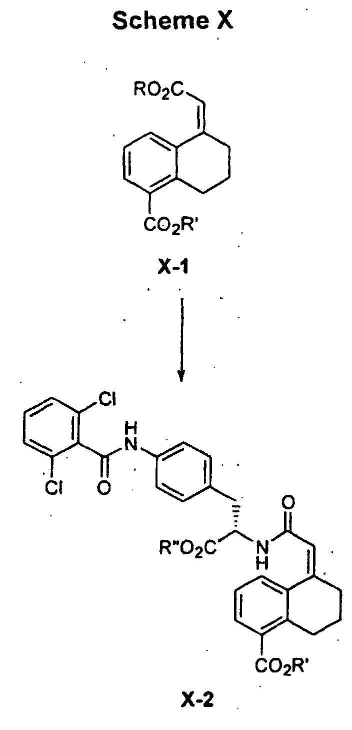

### Preparation X-1

### Scheme Y, X-1 wherein R = H, R' = CH₃ (Z)-5-Carbomethoxy-3,4-dihydro-1(2H)-naphthalenylideneacetic acid X-1 (C₁₄H₁₄O₄)

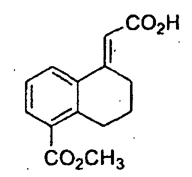

To a solution of the diester **X-1** (R = tBu, R' = CH₃), prepared as described in Scheme X (Preparation **X-C-1** and **X-T-1,** R = CO₂tBu, R' = 5-CO₂CH₃) (0.77g., 2.55mmol) in methylene chloride (40mL) was added trifluoroacetic acid (2mL, 26mmol). After stirring for 16 hours an additional portion of trifluoroacetic acid (0.5mL, 6.5mmol) was added. 'After stirring for an additional 24 hours, the reaction mixture was diluted with toluene (50mL) and evaporated to dryness *in vacuo;* giving a pale. yellow solid. The solid thus obtained was dissolved in ethyl acetate (150mL) and washed with water (1X100mL) followed by saturated aqueous sodium bicarbonate (1X30mL). The bicarbonate wash was brought to pH3 using 1N aq. HCl. The resultant heterogeneous mixture was then placed in the freezer for thirty minutes followed by filtration through a sintered glass funnel. The filtered solid was washed with water (3X30mL) and air dried to give **X-1** (R = H, R' = CH₃) as a white solid (0.308g., 49%yield). ^{**1**}**H-NMR** (DMSO-d₆): δ = 12.21(1), 7.72(1), 7.61(1), 7.23(1), 5.84(1), 3.81(3), 3.02(2), 2.42(2), 1.84(2).

### Preparation X-2

### Scheme Y, X-2 wherein R' = CH₃, R" = CH₃ N-[(1Z)-5-Carbomethoxy-3,4-dihydro-1(2H)-naphthalenylideneacetyl]-4-[(2,6-dichlorobenzoyl)amino]-L-phenylalanine methyl ester (C₃₁H₂₈Cl₂N₂O₆)

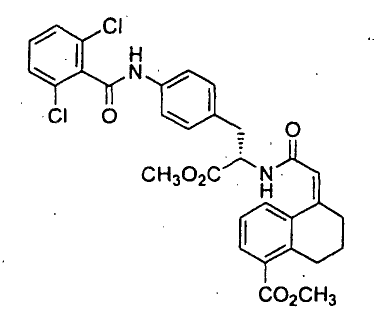

A mixture of the acid **X-1** (Scheme Y, **X-1,** R = CO₂H, R' = CO₂CH₃) (0.3g., 1.22mmol) and methylene chloride (20mL) was stirred in a 100mL round bottom flask immersed in an ice water bath. To this mixture were added EDC (0.26g., 1.36mmol), HOBT (0.19g., 1.41mmol), 4-dimethylaminopyridine (0.05g., 0.41mmol), and diisopropylethyl amine (2mL, 11.48mmol). After thirty minutes, 4-[(2,6-dichlorobenzoyl)amino]-L-phenylalanine methyl ester. hydrochloride (0.56g., 1.39mmol) was added. and the cold bath was removed. The mixture was allowed to stir for 36 hours then an additional portion of EDC (0.28g., 1.46mmol) and diisopropylethyl amine (1mL, 0.57mmol) were added. The mixture was stirred at room temperature for an additional 120 hours and the reaction mixture was then diluted with methylene chloride (40mL), shaken with water (2X20mL), 1N aq. HCl (1X20mL), and water (4X20mL, to pH7). The organic layer was then evaporated to dryness *in vacuo,* giving **X-2** (R' = CH₃, R" = CH₃) as a pale yellow solid (0.639g, 85%). This was ascertained (1H-NMR) to be a 1:7.5 mixture of the respective *E-* and (desired) *Z-* isomers. ^{**1**}**H-NMR** (DMSO-d₆) δ = 10.68(1), 8.50(1)7.35(9), 6.47(0.12), 5.85(0.88), 4.56(1), 3.80(3), 3.63(3), 2.93(6), 2.27(0.88), 1.86(0.88), 1.66(0.24); **IR** (mull) 1742, 1723, 1664, 1642, 1605; 1547, 1529, 1513, 1433, 1413, 1330, 1280, 1246, 1213, and 1197cm⁻¹; **MS** (FAB) m/z (rel. intensity) 595 (MH+, 47), 598 (13), 597 (34), 596 (21), 595 (47), 349 (14), 230 (15), 229 (99), 175 (13), 173 (19), 123 (45); **HRMS** (FAB) calcd for C₃₁H₂₈Cl₂N₂O₆+Hl 595.1403, found 595.1401.

### Example 136

### Scheme Y, X-2 wherein R' = CH₃, R" = H N-[(1Z)-5-Carbomethoxy-3,4-dihydro-1(2H)-naphthalenylideneacetyl]-4-[(2,6-dichlorobenzoyl)amino]-L-phenylalanine (C₃₀H₂₆Cl₂N₂O₆)

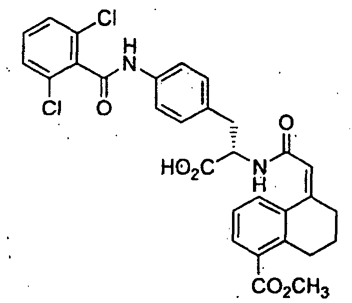

A solution of **X-2** (R' = CH₃, R" = CH₃) (0.275g, 0.46mmol) was stirred with methanol (50mL) in a flask immersed in an ice-water bath. To this cooled solution was added a solution of LiOH·H₂O (0.1g, 2.4mmol) in H₂O (20mL). After stirring for 16 hours, the mixture was brought to pH7 using 1N aq. HCl and then evaporated to dryness *in vacuo*. The resultant solid was then dissolved in water, filtered and the filtrate transferred to a C-18 reversed phase chromatography column and eluted with a gradient from 0 to 28% acetonitrile/ 0.02% aq.. NaHCO₃ . Two UV(250nm) absorbing bands were collected and isolated by evaporation of solvent *in vacuo* until all of the acetonitrile was gone, followed by acidification to pH3 using 1N aq. HCl. The resultant white precipitates were isolated by suction filtration through a sintered glass funnel. The solids were washed with H₂O (3X5mL) and air dried. UV band 1 afforded **X-2** (R' = CH₃, R" = H, 0.16g, 60% yield) as a powdery white solid. ^{**1**}**H NMR** (DMSO-*d*₆) δ = 12.7(1), 10.68(1), 8.36(1), 7.52(6), 7.33(1)7.21(2), 7.00(1), 5.86(1), 4.48(1), 3.79(3), 2.88(4), 2.35(2), 1.82(2); **IR** (mull) 3271, 1739, 1724, 1715, 1696, 1680, 1667, 1650, 1644, 1612, 1609, 1517, 1430, 1255, and 1193cm⁻¹; **HRMS** (FAB) calcd for C₃₀H₂₆Cl₂N₂O₆+H1 581.1246, found 581.1229.

### Example 137

### Scheme Y, X-2 wherein R' = CH₃, R" = H N-[(1Z)-5-Carboxy-3,4-dihydro-1(2H)-naphthalenylideneacetyl]-4- [(2,6-dichlorobenzoyl)amino] -L-phenylalanine (C₂₉H₂₄Cl₂N₂O₆)

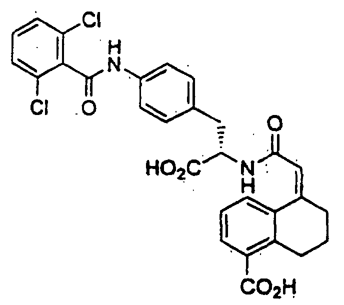

A solution of **X-2** (R' = CH₃, R" = H, 0.208g, 0.35mmol) in methanol (50mL) was stirred in a flask immersed in an ice-water bath. To this cooled solution was added a solution of LiOH·H₂O (0.1g, 5.4mmol) in H₂O (20mL). After 18 days, the pale yellow solution was diluted with water (20mL), evaporated to a volume of 30mL, acidified to pH5 using 1N aq. HCl, and filtered. The colorless filtrate was brought to pH3 using 1N aq. HCl, and the resulting precipitate was isolated by suction filtration and, after air drying, gave **X-2** (R' = H, R" = H) as a white powdery solid (0.092g., 46% yield). ^{**1**}**H-NMR** (DMSO-*d*₆): δ = 12.7(1), 8.35(1), 7.54(6), 7.25(3), 6.97(1), 5.84(1), 4.50(1), 2.91(4), 2.34(2), 1.82(2); **IR** (mull) 3263, 3194, 3121, 3066, 2941, 1727, 1714, 1693, 1666, 1647, 1605, 1518, 1431, 1412, and 1264cm⁻¹; **HRMS** (FAB) calcd for C29H24CL2N2O6+H1 567.1089, found 567.1091.% **Water** (KF): 4.83; **Anal.** Calcd for C₂₉H₂₄Cl₂N₂O₆·1.6H₂O: C, 58.42; H, 4.60; N, 4.70. Found: C, 58.57; H, 4.72; N, 4.87.

### Biological Assays

### Jurkat-Endothelial Cell Adhesion Assay:

The following assay established the activity of the present compounds in inhibiting β₁-mediated cell adhesion in a representative *in vitro* system. This assay measures the adhesive interactions of a T-cell line, Jurkat, known to express the α₄β₁ integrin, to endothelial monolayers in the presence of test compounds. The test compounds were added in increasing concentrations to T-cells and then the T-cell compound mixture was added to IL-1 stimulated endothelial cell monolayers. The plates were incubated, washed and the percentage of attached cells was quantitated. The present assay directly demonstrates the cell adhesion inhibitory activity and adhesion modulatory activity of the compounds.

Human umbilical vein endothelial cells were purchased from Clonetics (San Diego, CA.) at passage number 2. The cells were grown on 0.5% porcine skin gelatin pre-coated flasks (Sigma, St. Louis MO.) in EGM-UV media (Clonetics, San Diego, CA) supplemented with 10% fetal bovine serum. Cells are refed every 2-3 days reaching confluence by day 4 to 6. The cells are monitored for factor VIII antigen and results show that at passage 12, the 'cells are positive for this antigen. The endothelial cells are not used following passage 6.

The T-cell line Jurkat was obtained from American Type Tissue Culture Collection (Rockville, MD) and the cells were cultured in RPMI containing 10 % fetal calf serum. The cells were washed twice in Hank's Balanced Salt Solution (HBSS) and resuspended in Dulbecco's Minimal Eagle's Media (DMEM) containing 2.5 mg/ml Human Serum Albumin (HSA). Jurkat cells (1x10⁶ cells/ml) were stained with 10 ng/ml BCECF-AM (Molecular Probes, Eugene, OR)) in HBSS without phenol red. The cells were loaded with BCECF for 60 minutes in the dark at 37°C, washed 2 times, and resuspended in DMEM-HSA solution.

Confluent endothelial monolayers, grown in 96-well tissue culture plates, were stimulated for 4 hr. at 37 °C with 0.1 ng/ml (∼50 U/ml) recombinant IL-1 (Amgen, Thousand Oaks, CA). Following this incubation, the monolayers were washed twice with HBSS and 0.1 ml of DMEM-HSA solution was added. Jurkat cells (5 x 10⁵ cells) were combined with the appropriate concentration of the test compound and 0.1 ml of the Jurkat cell-compound mixture was added to the endothelial cell monolayers. Generally, 100, 20, 5 and 1.25 µM compound concentrations were tested. These concentrations are adjusted downward for analogs found or thought to be more potent. 'The plates were placed on ice for 5 minutes to allow for Jurkat cell settling and the plates were incubated at 37 °C for 20 minutes. Following this incubation, the monolayers were washed twice with PBS containing 1 mM calcium chloride and 1 mM magnesium chloride' and the plates were read using a Millipore Cytofluor 2300 (Marlboro, MA.). Fluorescence in each well was measured as Arbitrary Fluorescence Units and percent adhesion in the absence of compound was adjusted to 100% and the % adhesion in the presence of compound was calculated. Monolayers were also fixed in 3% paraformaldehyde and evaluated microscopically to verify the adhesion. This procedure is a modification of a previously published method (Cardarelli et al., *J. Biol. Chem.* 269:18668-18673 (1994)).

### Jurkat-CS-1 assay

The CS-1 derived peptide, CLHPGEILDVPST, and the scrambled control peptide, CLHGPIELVSDPT, were synthesized on a Beckman 990 synthesizer using t-Boc methodology. The peptides were immobilized onto microtiter plates using the heterobifunctional crosslinker 3-(2-pyridyldithio)propionic acid N-hydroxysuccinimide ester (SPDP) as reported by Pierschbacher et al., Proc. *Natl. Acad. USA,* 80:1224-1227 (1983). Microtiter plates were coated with 20 µg/ml HSA for 2 hr. at room temperature, washed once with PBS and derivatized with 10 µg/ml SPDP for 1 hr. After washing, 100 µl of a 100 µg/ml cysteine containing peptide solution which had been recently dissolved was added to the wells and allowed to crosslink to the plates overnight at 4 °C. Unbound peptide was removed from plates by washing with PBS. To block non-reacted sites, the plates are coated with 100 µl of a 2.5 mg/ml BSA solution in PBS for 1 hr. at 37 °C. 100 µl of Jurkat cells (2.5 x 10⁶ cells/ml) in DMEM plus BSA (2.5 mg/ml) was mixed with an appropriate concentration of the compound to be tested and the mixture was added to peptide coated dishes and incubated for 1 hr. at 37 °C. Generally 100, 20, 5 and 1.25 µM concentrations of the compound were tested. The concentrations of the compound were adjusted downward for compounds thought or found to be more potent.

Following this incubation the plates were washed once with PBS and the attached cells were fixed with 3% paraformaldehyde in PBS and stained with 0.5% toluidine blue in 3.7% formaldehyde. The cells were stained overnight at room temperature and the optical density at 590 nm of toluidine blue stained cells was determined using a vertical pathway spectrophotometer to quantitate attachment (VMAX Kinetic Microplate Reader, Molecular Devices, Menlo Park, CA). This procedure is a modification of a previously published method (Cardarelli et al, J. Biol. Chem., 269:18668-18673 (1994) and Cardarelli et al, Proc. Natl. Acad. Sci. USA, 83:2647-2651 (1986)).

The preferred compounds are those which have low IC₅₀ values in the Jurkat EC assay or the Jurkat-CS-1 assay described above or which have at least moderate activity in both assays. All of the compounds of the present invention have an activity of less than 50µM in the Jurkat CS-1 assay or less than 500µM in the Jurkat EC assay. Compounds with activity in the Jurkat CS-1 assay preferably have IC₅₀ values of less than 1µM, more preferably less than 0.5pM, most preferably less than or equal to 0.08µM. Compounds with activity in the Jurkat EC assay preferably have IC₅₀ values of less than 10µM, more preferably less than 5µM, most preferably less than or equal to 0.8µM.

In the Jurkat EC Assay, IC₅₀ value ranges (µM) are depicted by A, B, and C and in the Jurkat CS-1 Assay, IC₅₀ value ranges are depicted by D, E, and F. These ranges are as follows:
Jurkat EC: 5 ≤ A < 10, 0.8 < B < 5, and C ≤ 0.8
Jurkat CS-1: 0.5 ≤ D < 1, 0.08 < E < 0.5, and F ≤ 0.08

The following chart illustrates the IC₅₀ values for selected compounds of the present invention in the Jurkat EC Assay and the Jurkat CS-1 Assay. The ranges are as described above.

### IN VITRO BIOLOGICAL DATA

| **Example No.** | **Jurkat EC** | **Jurkat CS-1** |
|---|---|---|
| 4 | B | - |
| 8 | B | - |
| 9 | B | D |
| 11 | A | - |
| 12 | - | D |
| 14 | B | - |
| 15 | B | - |
| 16 | B | - |
| 18 | B | - |
| 19 | B | D |
| 21 | A | - |
| 22 | B | - |
| 24 | A | - |
| 25 | C | D |
| 28 | B | - |
| 33 | - | E |
| 34 | - | D |
| 35 | - | - |
| 36 | B | D |
| 37 | A | D |
| 38 | B | D |
| 39 | - | D |
| 41 | A | - |
| 42 | - | D |
| 44 | A | E |
| 46 | B | E |
| 47 | A | E |
| 48 | B | E |
| 49 | C | E |
| 50 | A | D |
| 51 | B | F |
| 52 | B | F |
| 53 | C | F |
| 54 | - | D |
| 55 | B | E |
| 56 | B | E |
| 57 | C | E |
| 59 | B | E |
| 60 | - | D |
| 61 | C | E |
| 62 | B | E |
| 63 | C | E |
| 64 | B | E |
| 65 | B | E |
| 66 | C | E |
| 67 | C | E |
| 68 | C | E |
| 69 | C | E |
| 70 | B | E |
| 71 | B | E |
| 72 | C | E |
| 73 | B | D |
| 74 | B | E |
| 75 | C | E |
| 76 | B | E |
| 77 | C | E |
| 78 | C | E |
| 79 | B | E |
| 80 | B | E |
| 81 | B | E |
| 82 | C | E |
| 83 | C | F |
| 84 | C | E |
| 85 | C | F |
| 86 | C | F |
| 87 | C | E |
| 88 | A | D |
| 89 | A | D |
| 90 | A | - |
| 91 | B | D |
| 92 | B | - |
| 93 | B | E |
| 94 | C | E |
| 95 | - | D |
| 96 | C | F |
| 97 | C | F |
| 98 | C | E |
| 99 | B | E |
| 100 | B | E |
| 101 | B | E |
| 102 | C | E |
| 103 | C | E |
| 104 | B | D |
| 105 | B | E |
| 106 | B | E |
| 107 | B | E |
| 108 | B | - |
| 109 | B | - |
| 112 | B | E |
| 113 | B | - |
| 114 | C | E |
| 115 | B | E |
| 116 | C | E |
| 117 | C | E |
| 118 | C | F |
| 119 | C | F |
| 120 | C | F |
| 121 | B | - |
| 123 | A | D |
| 124 | B | D |
| 125 | B | F |
| 126 | B | F |
| 127 | B | E |
| 128 | B | E |
| 130 | B | D |
| 131 | C | F |
| 134 | C | E |
| 135 | A | D |
| 137 | A | - |

### Rationale for Developing an α₄β₁ Integrin Antagonist to Treat Inflammatory Diseases

VLA-4, a member of the β1 integrin family of adhesion molecules, is thought to play a critical role in several types of inflammatory disease processes by promoting leukocyte adhesion to vascular cell adhesion molecule (VCAM-1) and the CS-1 domain of fibronectin in extracellular tissue matrix (Elices MJ, Osborn L, Takada'Y, Crouse C, Luhowskyj S, Hemler M, Lobb RR. VCAM-1 on activated endothelium interacts with the leukocyte integrin VLA-4 at a site distinct from the VLA-4-fibronectin binding site. Cell; 60: 577-584, 1990, Humphries MJ, Akiyama SK, Komoriya A, Olden K, Yamada KM. Identification of an alternatively-spliced site in human plasma fibronectin that mediates cell type-specific adhesion. J Cell Biol; 103: 2637-2647, 1986, Wayner EA, Garcia-Pardo A, Humphries MJ, McDonald JA, Carter WG. Identification and characterization of the T lymphocyte adhesion receptor for an alternative cell attachment domain (CS-1) in plasma fibronectin. J Cell Biol; 109: 1321-1330, 1989, Guan J-L, Hynes RO. Lymphoid cells recognize an alternatively-spliced segment of fibronectin via the integrin α₄β₁. Cell; 60: 53-61, 1990) Of the cell types expressing VLA-4, the major emphasis has been on eosinophils, lymphocytes, and monocytes. Validation of the role of VLA-4 has relied predominantly on the use of anti-VLA-4 antibodies which have been shown to suppress delayed-type hypersensitivity responses (Issekutz TB. Dual inhibition of VLA-4 and LFA-1 maximally inhibits cutaneous delayed-type hypersensitivity-induced inflammation. Am J Pathol; 143: 1286-1293, 1993, Scheynius A, Camp RL, Puré E. Reduced contact sensitivity reactions in mice treated with monoclonal antibodies to leukocyte function-associated molecule-1 and intercellular adhesion molecule-1. J Immunol; 150: 655-663, 1993, Ferguson TA, Kupper TS. Antigen-independent processes in antigen-specific immunity. J Immunol; 150: 1172-1182, 1993, Chisholm PL, Williams CA, Lobb RR. Monoclonal antibodies to the integrin α-4 subunit inhibit the murine contact hypersensitivity response. Eur J Immunol; 23: 682-688, 1993, Elices MJ, Tamraz S, Tollefson V, Vollger LW. The integrin VLA-4 mediates leukocyte recruitment to skin inflammatory sites *in vivo*. Clin Exp Rheumatol; 11 (Suppl 8) S77-80), 1993, experimental allergic encephalomyelitis (Yednock TA, Cannon C, Fritz LC, Sanchez-Madrid F, Steinman LM, Karin N. Prevention of experimental autoimmune encephalomyelitis by antibodies against α₄β₁ integrin. Nature; 356: 63-66, 1992, Canella B, Raine CS. The VCAM-1/VLA-4 pathway is involved in chronic lesion expression in multiple sclerosis (MS). J Neuropathol Exp Neurol; 52: 311, 1993), HIV-induced encephalitis (Sasseville VG, Newman W, Brodie SJ, Hesterberg P, Pauley D, Ringler DJ. Monocyte adhesion to endothelium in simian immunodeficiency virus-induced AIDS encephalitis is mediated by vascular cell adhesion molecule-1/α₄β₁ integrin reactions. Am J Pathol; 144: 27-40, 1994), pulmonary inflammation and airway hyperreactivity in asthma (Abraham WM, Sielczak MW, Ahmed A, Cortes A, Lauredo IT, Kim J. Pepinsky, B, et al. α₄-integrins mediate antigen-induced late bronchial responses and prolonged airway hyperresponsiveness in sheep. J Clin Invest; 93: 776-787, 1994, Pretolani M, Ruffié C, Roberto LapaeSilva J, Joseph D, Lobb RR, Vargaftig BB. Antibody to very late activation antigen 4 prevents antigen-induced bronchial hyperreactivity and cellular infiltration in the guinea-pig airways. J Exp Med; 180: 795-805, 1994), experimental models of autoimmune-mediated diabetes (Yang X-D, Karin N, Tisch R, Steinman L, McDevitt HO. Inhibition of insulitis and prevention of diabetes in non-obese diabetic mice by blocking L-selectin and very late antigen 4 adhesion receptors. Proc Natl Acad Sci USA; 90: 10494-10498, 1993, Burkly LC, Jakubowski A, Hattori M. Protection against adoptive transfer of autoimmune diabetes medicated through very late antigen-4 integrin. Diabetes; 43: 529-534, 1994), and experimental colitis (Podolsky DK, Lobb R, King N, Benjamin CD, Pepinsky B, Sehgal P, et al. Attenuation of colitis in the cotton-top Tamarin by anti-α4 integrin monoclonal antibody. J Clin Invest; 92: 372-380, 1993). Since eosinophils represent a major component of the inflammatory cell influx in asthmatic lung tissue we developed a simple acute inflammatory model of VLA-4 integrin-dependent eosinophil infiltration which could be used to identify VLA-4 antagonists; such compounds would be of potential value in the treatment of asthma as well as other 'diseases in which VLA-4 played a role.

## Claims

1. A compound of the formula: 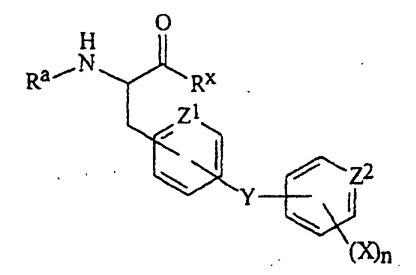 wherein
X is selected from the group consisting of halogen, CF₃, NO₂, OH, C₁₋₃ alkoxy, NH₂ and C₁₋₄ alkyl;
Z¹ is CH or N;
Z² is CH or N;
n is 1, 2 or 3;
Y is -OCH₂- or -NHC(=O)-;
R^{x} is OH or C₁₋₆ alkoxy;
R^{a} is selected from the group consisting of 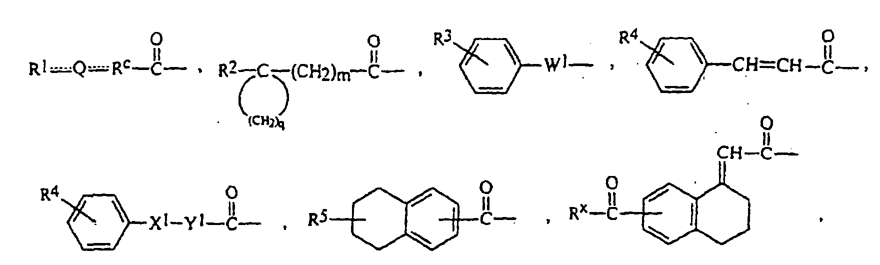 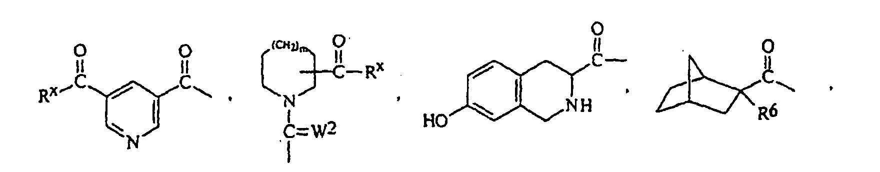 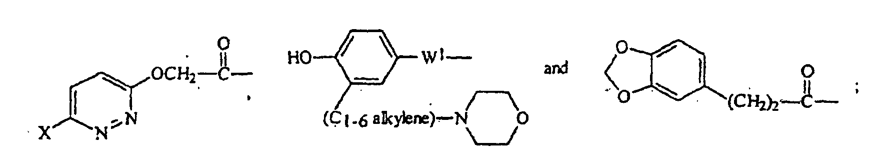 the symbol - - - - - represents a single or a double bond;
W¹ is selected from the group consisting of -CO-, -OCH₂CO-, -OCH(CH₃)CO-, -OC(CH₃)₂CO-, -SCH₂CO-, -SCH(CH₃)CO-, -SC(CH₃)₂CO-, -CH=CHCO-, -OCH(C₆H₅)CO- and -SCH(C₆H₅)CO-;
W² is 0 or S;
q is 5 or 6;
m is 0 or 1;
X¹ is O, S or a bond;
Y¹ is C₁₋₃ alkylene or -CH(C₆H₅);
R^{c} is a bond, -CH₂- or =CH-;
Q is a ring of C₃₋₁₀ cycloalkane or C₃₋₁₀ cycloalkene, which ring is substituted by a group of R¹ and may be further substituted by 1 to 3 methyl groups, with the proviso that Q is other than a ring of cyclopentane which is substituted by three methyl groups;
R¹ is selected from the group consisting of -H, -COR^{x}, -CH(OH)CH₃, -(C₂₋₇ alkenylene)COR^{x}, -NHCO(C₁₋₆ alkylene)COR^{x}, =CHCOR^{x}, -NHCO(C₁₋₆ alkylene)O(C₁₋₆ alkyl), -NHCO(C₂₋₇ alkenylene)COR^{x}, -NHCO(C₁₋₆ alkylene)CO(C₁₋₆ alkyl), -NHCONH(C₁₋₆ alkylene)COR^{x}, -C₂₋₇ alkanoyl, =CHCN, -(C₁₋₆ alkylene)COR^{x}, 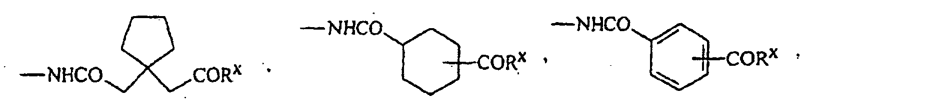 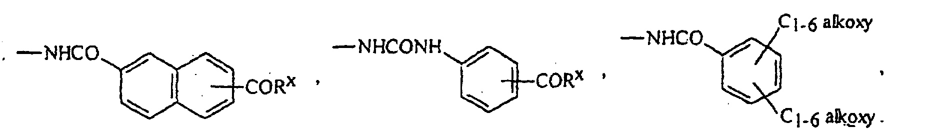 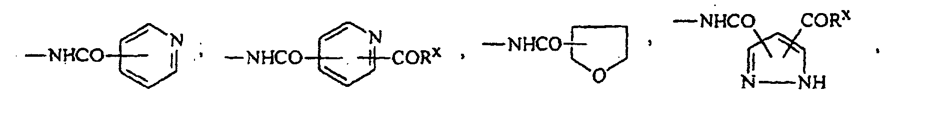 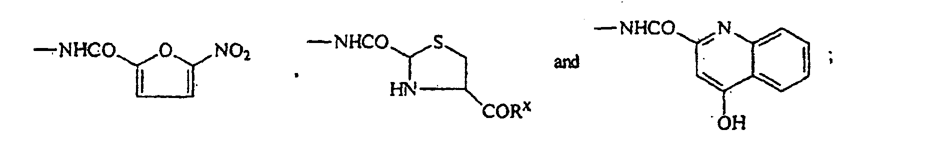 R² is selected from the group consisting of -CH₂COR^{x}, -NHCO(C₁₋₆ alkylene)COR^{x}, -NHCO(C₂₋₇ alkenylene)COR^{x}, -NHCO(C₁₋₆ alkylene)O(C₁₋₆ alkyl), -NHCONH(C₁₋₆ alkylene)COR^{x}, 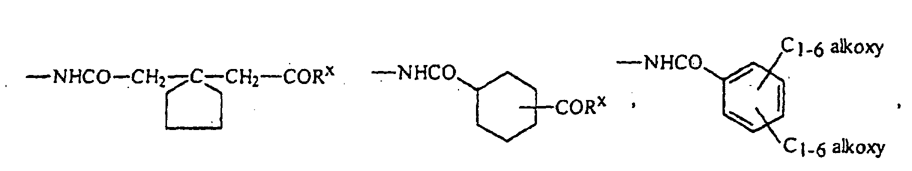 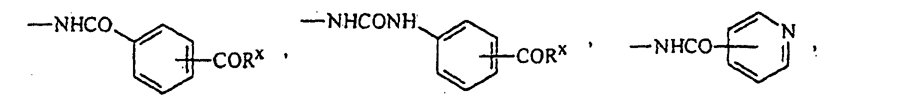 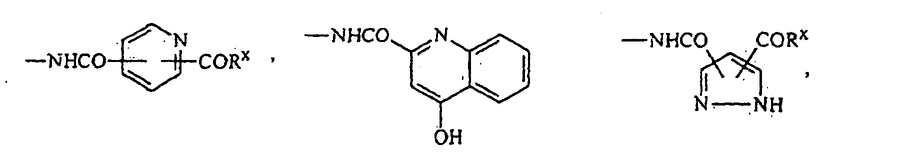 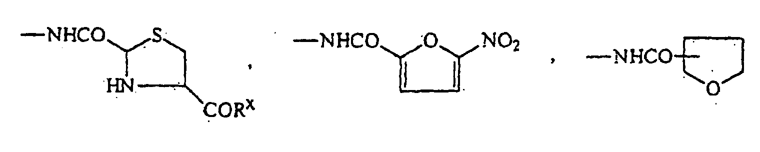 and 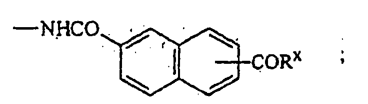 R³ is selected from the group consisting of -OH, -COR^{x}, - (C₂₋₇ alkenylene)COR^{x}, -NHCO(C₁₋₆ alkylene)COR^{x}, -NHCO(C₁₋₆ alkylene)O(C₁₋₆ alkyl), -NHCO(C₂₋₇ alkenylene)COR^{x}, - NHCO(C₁₋₆ alkylene)CO(C₁₋₆ alkyl), -NHCONH(C₁₋₆ alkylene)COR^{x}, - C₂₋₇ alkanoyl, 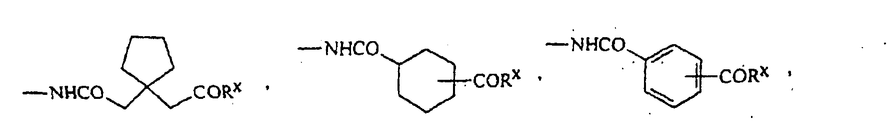 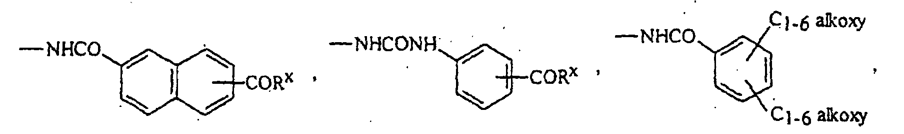 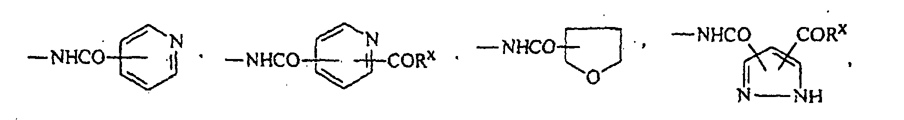 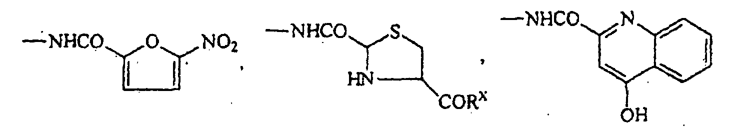 and 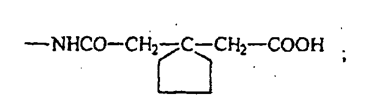 R⁴ is selected from the group consisting of -COOH, -CH=CHCOOH and methylenedioxy;
R⁵ is =CHCOR^{x} or =CHCN; and
R⁶ is selected from the group consisting of 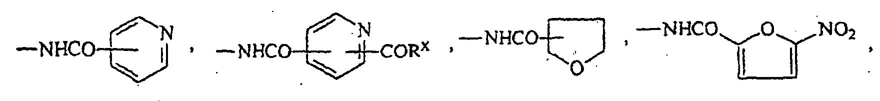 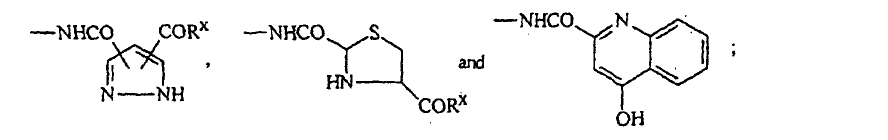 or a pharmaceutically acceptable salt thereof.

2. The compound according to claim 1, having the formula:

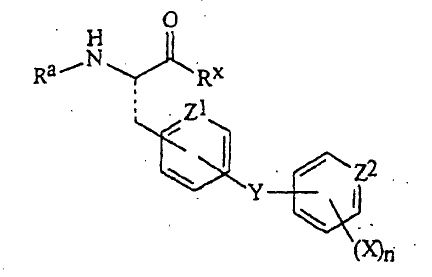

3. The compound according to claim 2, wherein
X is halogen;
Y is -OCH₂- or -NHC(=O)-;
Z¹ is CH or N;
Z² is CH or N;
R^{x} is OH or C₁₋₆ alkoxy;
R^{a} is selected from the group consisting of 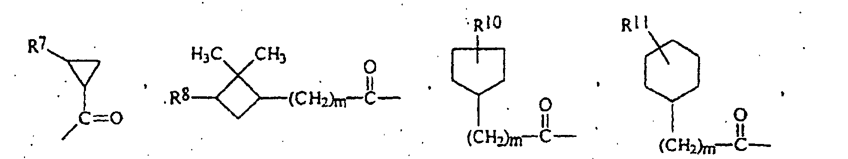 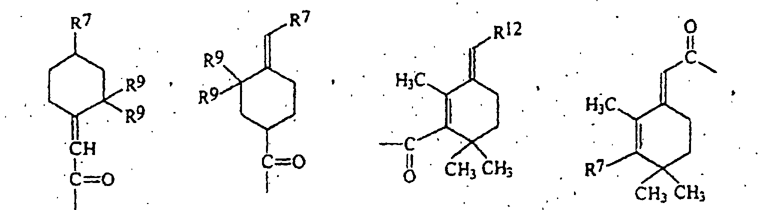 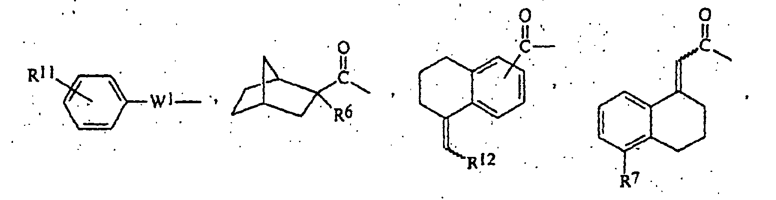 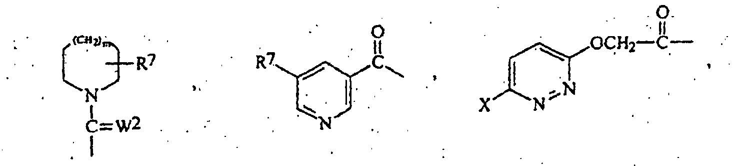 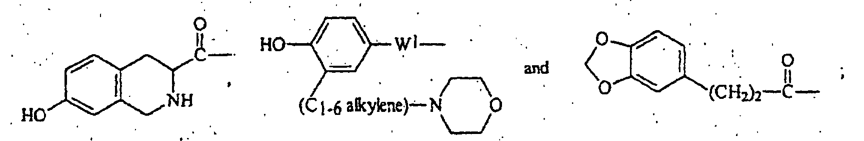 R⁶ is selected from the group consisting of: 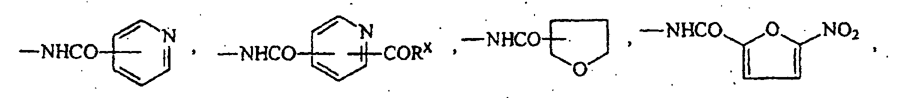 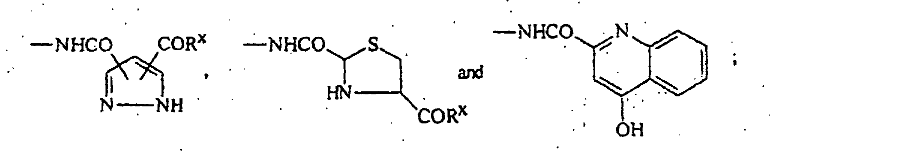 R⁷ is -COR^{x};
R⁸ is selected from the group consisting of hydrogen, -COR^{x}, -COCH₃ and -CH(OH)CH₃;
R⁹ is hydrogen or methyl;
R¹⁰ is -COR^{x} or -(C₁₋₆ alkylene)COR^{x};
R¹¹ is selected from the group consisting of -COR^{x}, -(C₂₋₇ alkenylene)COR^{x}, -NHCO(C₁₋₆ alkylene)COR^{x}, -NHCO(C₁₋₆ alkylene)O(C₁₋₆ alkyl), -NHCO(C₂₋₇ alkenylene)COR^{x}, -NHCO(C₁₋₆ alkylene)CO(C₁₋₆ alkyl), -NHCONH(C₁₋₆ alkylene)COR^{x}, -C₂₋₇ alkanoyl, 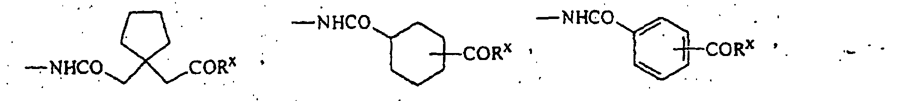 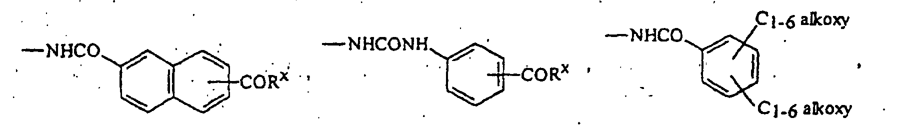 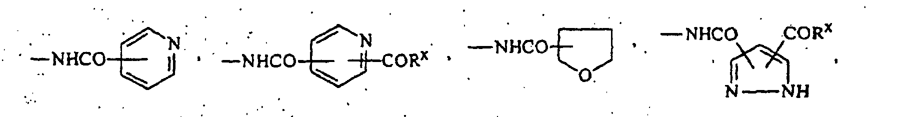 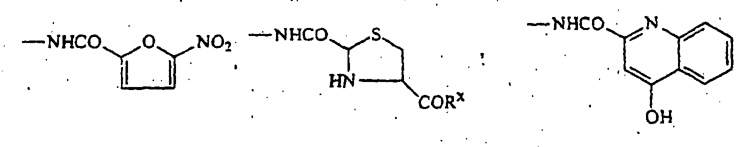 and 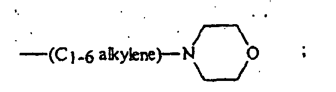 R¹² is -COR^{x} or -CN;
W¹ is selected from the group consisting of -CO-, - OCH₂CO-, -OCH(CH₃)CO-, -OC(CH₃)₂CO-, -SCH₂CO-, -SCH(CH₃)CO-, -SC(CH₃)₂CO-, -CH=CHCO-, -OCH(C₆H₅)CO- and -SCH(C₆H₅)CO-;
W² is O or S;
n is 1 or 2; and
m is 0 or 1;
or a pharmaceutically acceptable salt thereof.

4. The compound according to claim 1, wherein Y is -NHC(=O)-.

5. The compound according to claim 3, wherein the formula is the following:

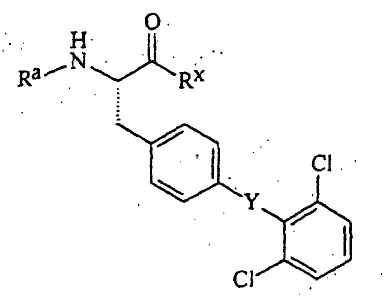

6. The compound according to claim 5, wherein R^{a} is selected from the group consisting of:

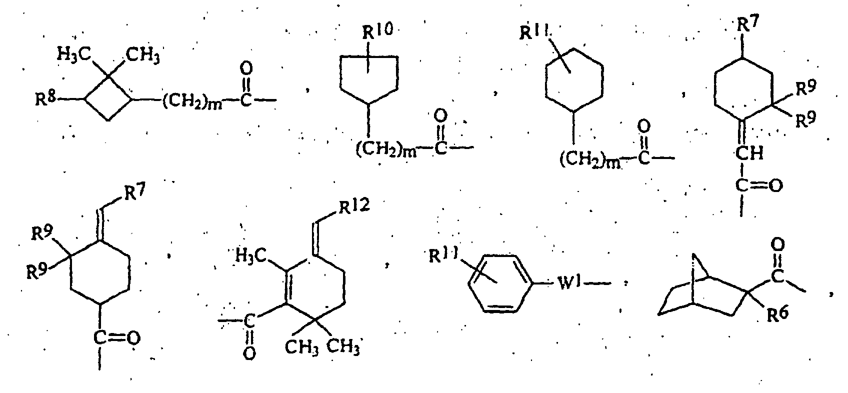

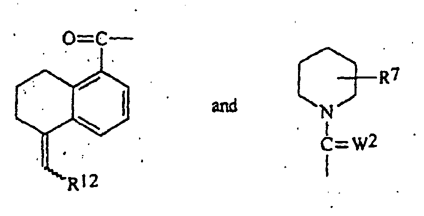

7. The compound according to claim 6, wherein R^{a} is selected from the group consisting of: 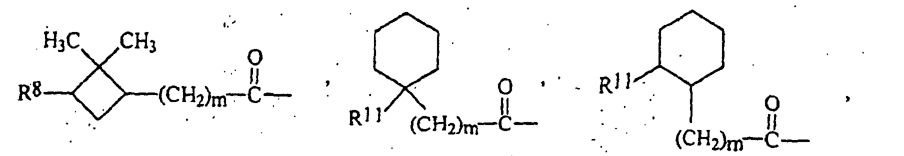 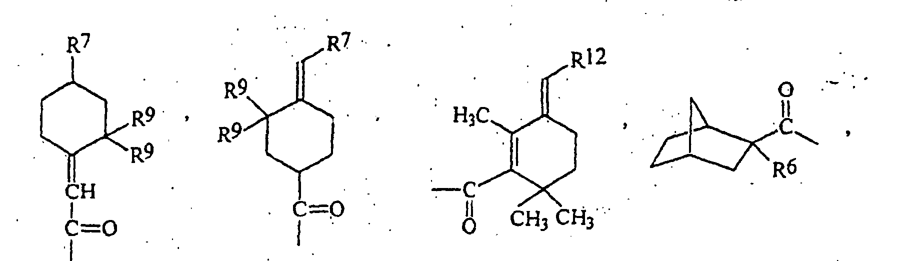 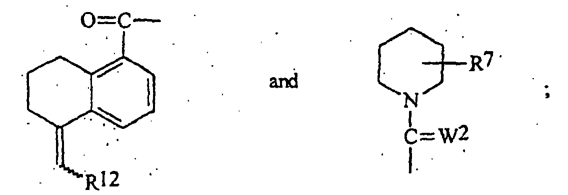 R⁶ is selected from the group consisting of: 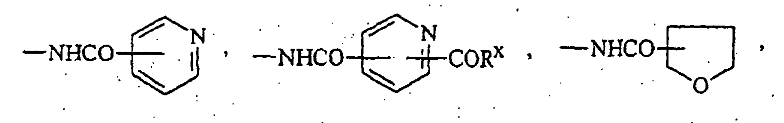 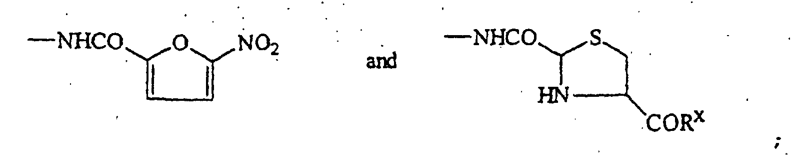 and
R¹¹ is selected from the group consisting of -NHCO(C₂₋₇ alkenylene)COR^{x}, -NHCO(C₁₋₆ alkylene)CO(C₁₋₆ alkyl),

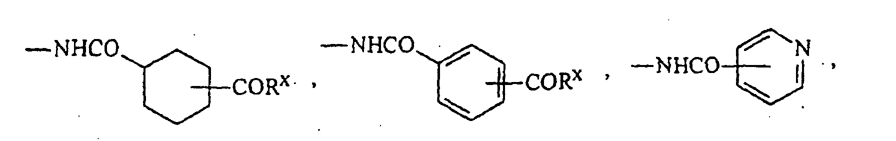

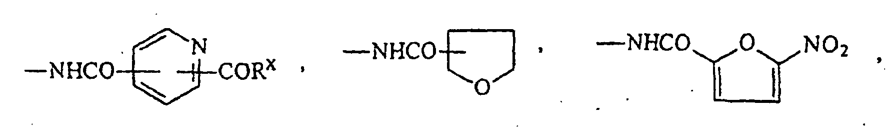

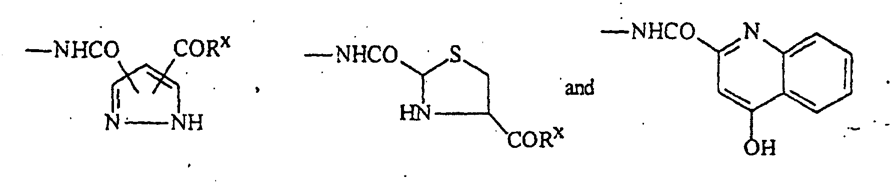

8. The compound according to claim 7, wherein R^{a} is 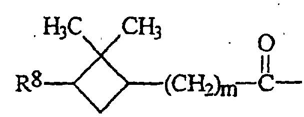 and R⁸ is COR^{x} or COCH₃.

9. The compound according to claim 7, wherein R^{a} is 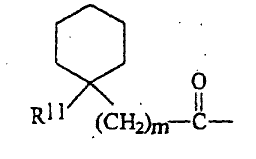 and R¹¹ is selected from the group consisting of -NHCO(C₁₋₆ alkylene)CO(C₁₋₆ alkyl), 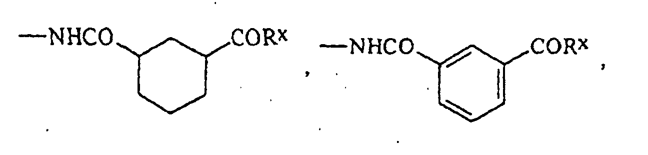 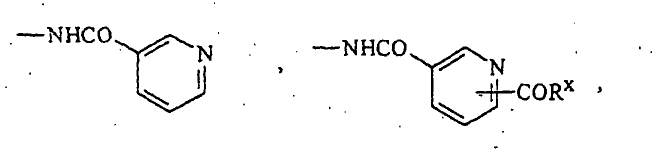 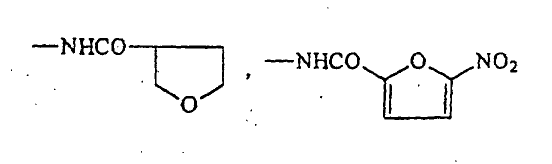 and

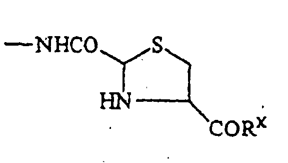

10. The compound according to claim 7, wherein R^{a} is 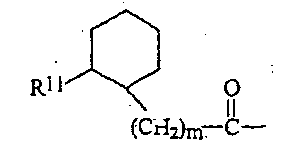 and R¹¹ is selected from the group consisting of

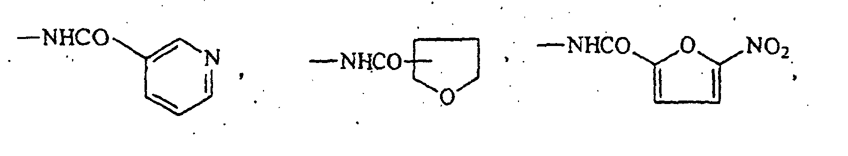

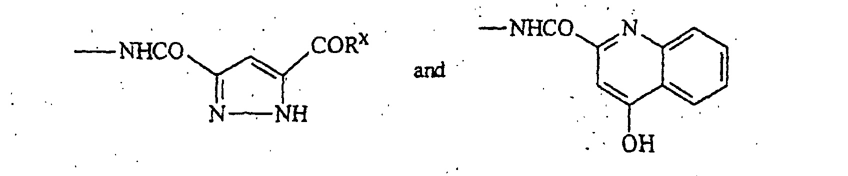

11. The compound according to claim 7, wherein R^{a} is

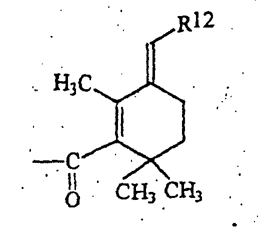

12. The compound according to claim 7, wherein R^{a} is 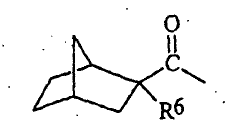 and R⁶ is selected from the group consisting of:

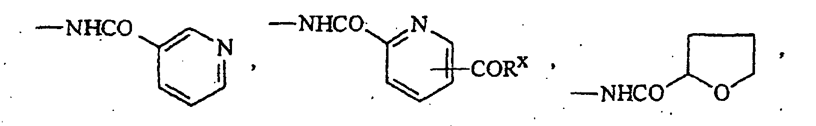

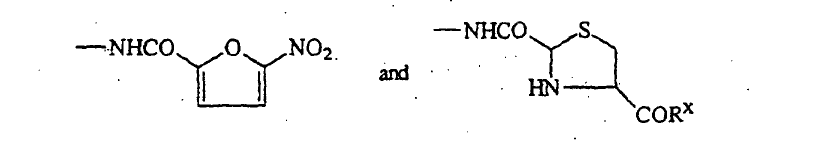

13. (1S-trans)-N-[(3-Carboxy-2,2-dimethylcyclobutyl)carbonyl]-4-[(2,6-dichlorobenzoyl)amino]-L-phenylalanine,
N-[[(3E)-3-Carboxymethylene-2,6,6-trimethyl-1-cyclohexen-1-yl)carbonyl]-4-[(2,6-dichlorobenzoyl)amino]-L-phenylalanine
(1S-*trans*)-N-[(3-Acetyl-2,2-dimethylcyclobutyl)carbonyl]-4-[(2,6-dichlorobenzoyl)amino]-L-phenylalanine,
(1S-*cis*)-N-[(3-Acetyl-2,2-dimethylcyclobutyl)carbonyl]-4-[(2,6-dichlorobenzoyl)amino]-L-phenylalanine
(1S-*cis*)-N-[(3-garboxy-2,2-dimethylcyclobutyl)carbonyl]-4-[(2,6-dichlorobenzoyl)amino]-L-phenylalanine,
N-[[[1-(1,4-Dioxlopentyl)amino]cyclobutyl]carbonyl]-4-[(2,6-dichlorobenzoyl)amino]-L-phenylalanine,
(*cis*)-N-[[2-[[(5-Carboxy-3-pyrazolyl)carbonyl]-amino]cyclohexyl]carbonyl]-4-[(2,6-dichlorobenzoyl)amino]-L-phenylalanine,
(*cis*)-N-[[2-[[(5-Nitro-2-furyl)carbonyl]amino]cyclohexyl]-carbonyl]-4-[(2,6-dichlorobenzoyl)amino]-L-phenylalanine,
(*cis*)-N-[[[[(2-Tetrahydrofuryl)carbonyl]amino)cyclohexyl]-carbonyl]-4-[(2,6-dichlorobenzoyl)amino]-L-phenylalanine,
N-[(1E)-3-Carboxy-2,4,4-trimethyl-2-cyclohexen-1-ylideneacetyl]-4-[(2,6-dichlorobenzoyl)amino)-L-phenylalanine,
N-[[(3E)-3-Cyanomethylene-2,6,6-trimethyl-1-cyclohexen-1-yl]carbonyl]-4-[(2,6-dichlorobenzoyl)-L-phenylalanine
N-[[(3E)-3-Carboxymethylene-2,6,6-trimethyl-1-cyclohexen-1-yl]carbonyl]-4-O-[(2,6-dichlorophenylmethyl)-L-tyrosine or pharmaceutically acceptable salts or the above compounds.

14. A pharmaceutical composition comprising
a therapeutically effective amount of a compound according to any one of claims 1-13; and
a pharmaceutically acceptable carrier.

15. Use of a compound according to anyone of claims 1-13 for the preparation of a pharmaceutical composition for treating or preventing α₄β₁ adhesion mediated conditions in a human.

16. Use according to claim 15, wherein said condition is selected from the group consisting of rheumatoid arthritis, asthma, allergy conditions, allograft rejection, psoriasis, eczema, contact dermatitis and other skin inflammatory. diseases and inflammatory and immunoinflammatory conditions including ophthalmic inflammatory conditions, inflammatory bowel diseases, atherosclerosis and ulcerative colitis.

## Patentansprüche

1. Verbindung der Formel: 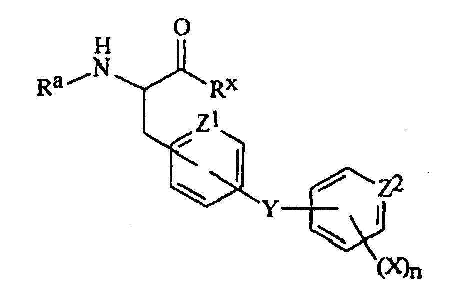 wobei
X aus einem Halogenatom, den Gruppen CF₃, NO₂ oder OH, einem C₁₋₃-Alkoxyrest, einer NH₂-Gruppe und einem C₁₋₄-Alkylrest ausgewählt ist;
Z¹ eine CH-Gruppe oder ein Stickstoffatom bedeutet;
Z² eine CH-Gruppe oder ein Stickstoffatom bedeutet;
n 1, 2 oder 3 bedeutet;
Y eine Gruppe -OCH₂- oder -NHC(=O)- bedeutet;
R^{x} eine OH-Gruppe oder einen C₁₋₆-Alkoxyrest bedeutet;
Ra aus den Resten: 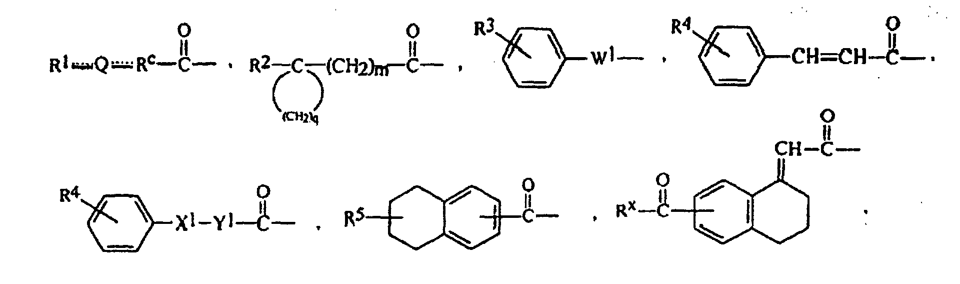 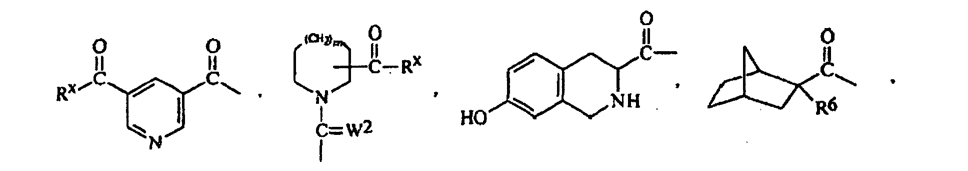 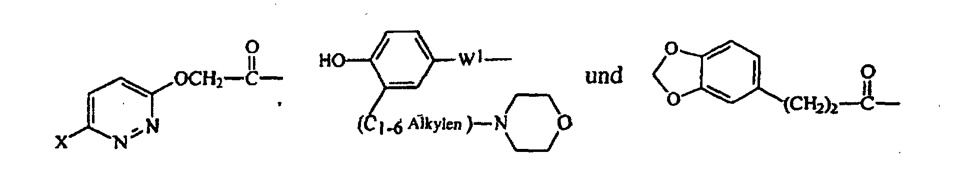 ausgewählt ist;
das Symbol - - - - - eine Einfach- oder Doppelbindung bedeutet;
W¹ aus den Gruppen -CO-, -OCH₂CO-, -OCH(CH₃)CO-, -OC(CH₃)₂CO-, - SCH₂CO-, -SCH(CH₃)CO-, -SC(CH₃)₂CO-, -CH=CHCO-, -OCH(C₆H₅)CO- und -SCH(C₆H₅)CO- ausgewählt ist;
W² ein Sauerstoff- oder Schwefelatom bedeutet;
q 5 oder 6 bedeutet;
m 0 oder 1 bedeutet;
X¹ ein Sauerstoff- oder Schwefelatom oder eine Bindung bedeutet;
Y¹ einen C₁₋₃-Alkylenrest oder eine Gruppe -CH(C₆H₅) bedeutet;
R^{c} eine Bindung oder eine Gruppe -CH₂- oder =CH- bedeutet;
Q einen C₃₋₁₀-Cycloalkan- oder C₃₋₁₀-Cycloalkenring bedeutet, wobei der Ring mit einem Rest R¹ substituiert ist und ferner mit 1 bis 3 Methylgruppen substituiert sein kann, mit der Maßgabe, dass Q keinen mit drei Methylgruppen substituierten Cyclopentanring bedeutet;
R¹ aus einem Wasserstoffatom oder den Resten -COR^{x}, -CH(OH)CH₃, -(C₂₋₇-Alkenylen)COR^{x}, -NHCO(C₁₋₆-Alkylen)COR^{x}, =CHCOR^{x}, -NHCO(C₁₋₆-Alkylen)O(C₁₋₆-alkyl), -NHCO(C₂₋₇-Alkenylen)COR^{x}, -NHCO(C₁₋₆-Alkylen)CO(C₁₋₆-alkyl), -NHCONH(C₁₋₆-Alkylen)COR^{x}, -C₂₋₇-Alkanoyl, =CHCN, -(C₁₋₆-Alkylen)COR^{x}, 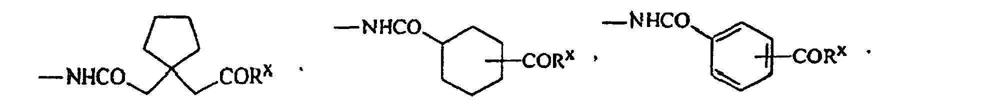 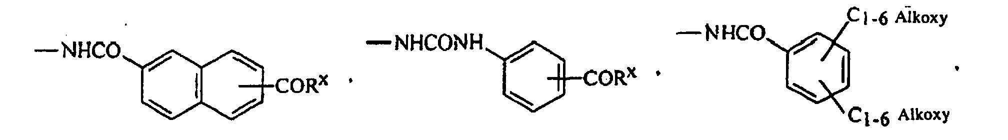 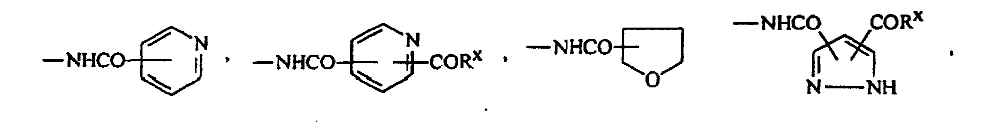 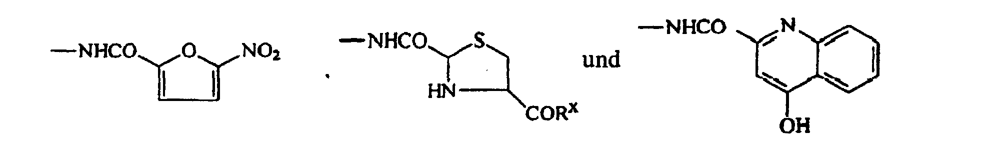 ausgewählt ist;
R² aus den Resten -CH₂COR^{x}, -NHCO(C₁₋₆-Alkylen)COR^{x}, - NHCO(C₂₋₇-Alkenylen)COR^{x}, -NHCO(C₁₋₆-Alkylen)O(C₁₋₆-alkyl), -NHCONH(C₁₋₆-Alkylen)COR^{x}, 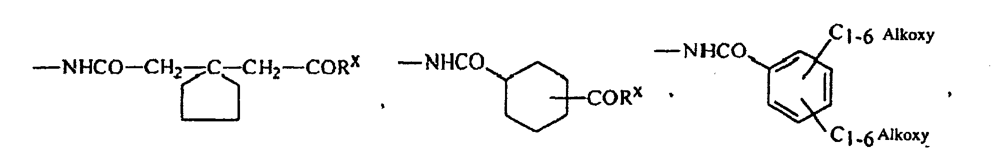 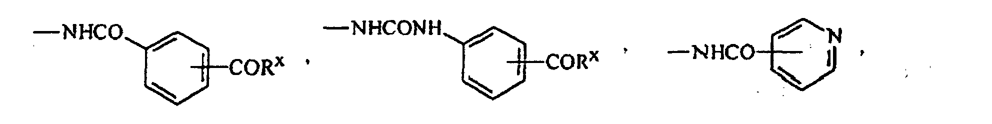 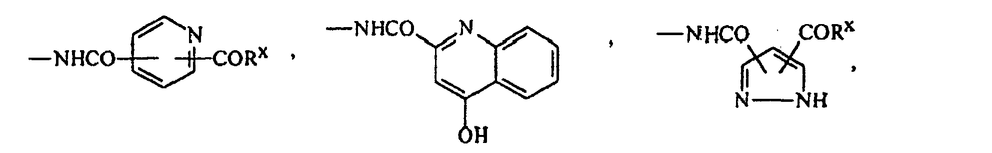 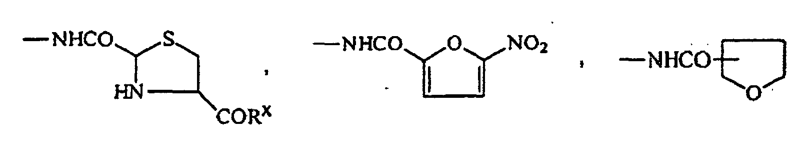 und 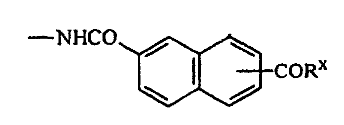 ausgewählt ist;
R³ aus den Resten -OH, -COR^{x}, -(C₂₋₇-Alkenylen)COR^{x}, -NHCO(C₁₋₆-Alkylen)COR^{x}, -NHCO(C₁₋₆-Alkylen)O(C₁₋₆-alkyl), -NHCO(C₂₋₇-Alkenylen)COR^{x}, -NHCO(C₁₋₆-Alkylen)CO(C₁₋₆-alkyl), -NHCONH(C₁₋₆-Alkylen)COR^{x}, -C₂₋₇-Alkanoyl, 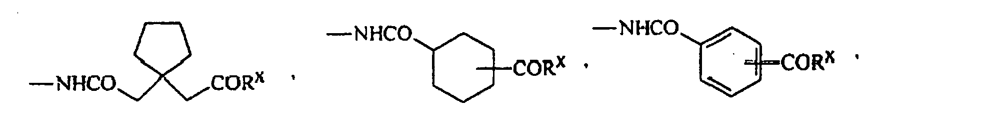 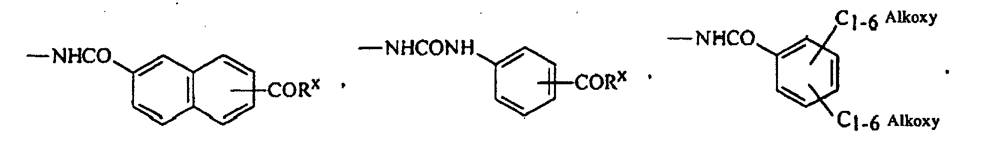 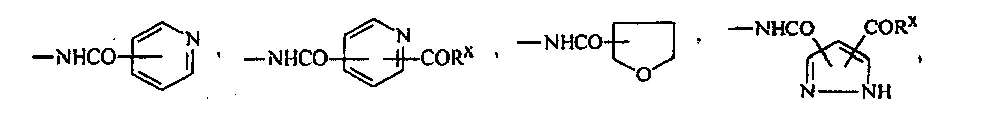 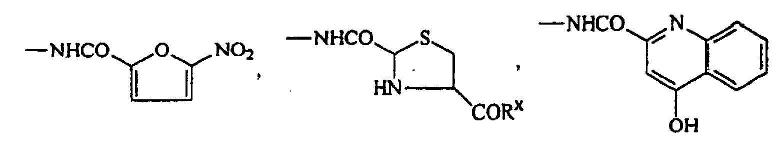 und 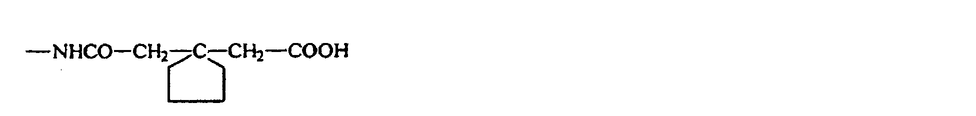 ausgewählt ist;
R⁴ aus den Gruppen -COOH oder -CH=CHCOOH und einer Methylendioxygruppe ausgewählt ist;
R⁵ einen Rest =CHCOR^{x} oder eine Gruppe =CHCN bedeutet; und
R⁶ aus den Gruppen: 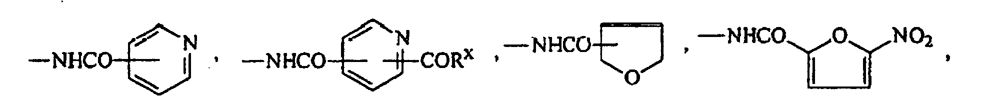 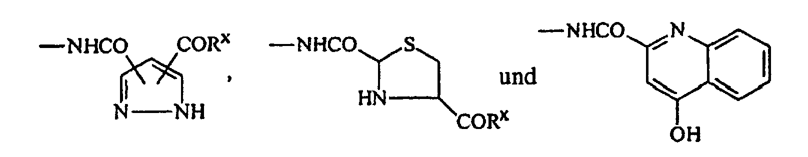 ausgewählt ist;
oder ein pharmazeutisch verträgliches Salz davon.

2. Verbindung gemäß Anspruch 1, die die Formel aufweist:

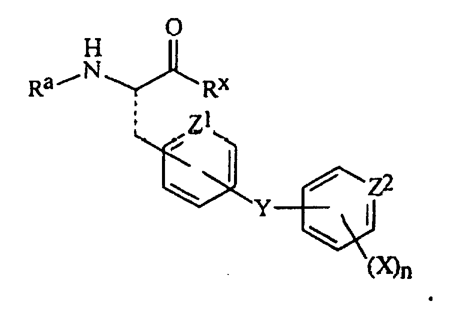

3. Verbindung gemäß Anspruch 2, wobei
X ein Halogenatom bedeutet;
Y eine Gruppe -OCH₂- oder -NHC(=O)- bedeutet;
Z¹ eine CH-Gruppe oder ein Stickstoffatom bedeutet;
Z² eine CH-Gruppe oder ein Stickstoffatom bedeutet;
R^{x} eine OH-Gruppe oder einen C₁₋₆-Alkoxyrest bedeutet;
R^{a} aus den Resten 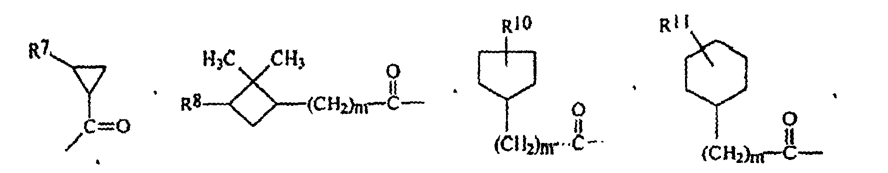 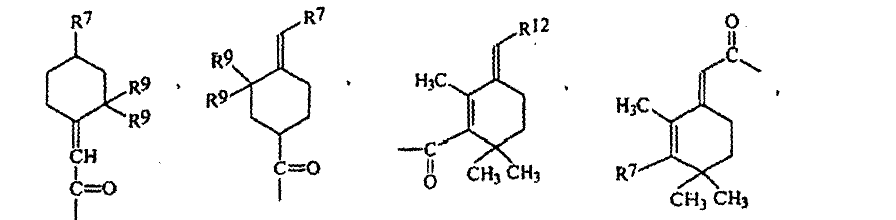 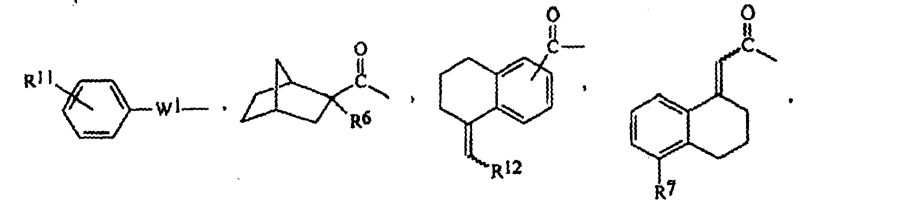 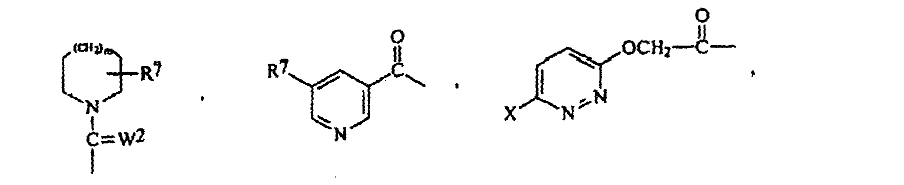 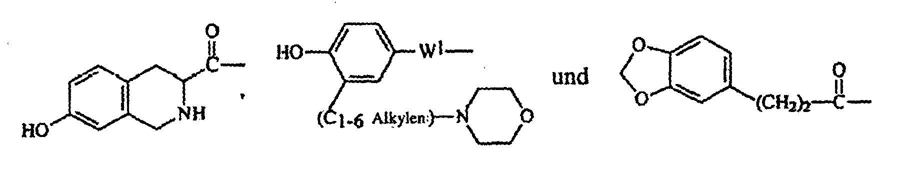 ausgewählt ist;
R⁶ aus den Resten: 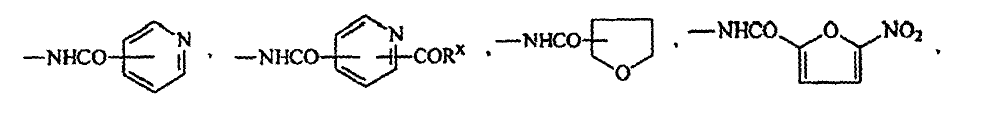 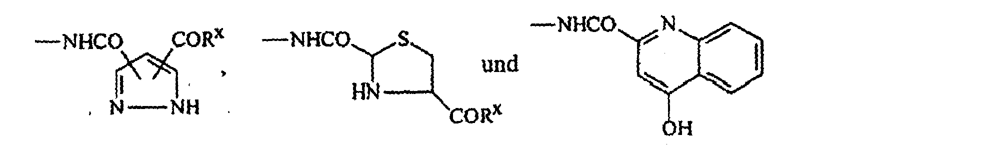 ausgewählt ist;
R⁷ einen Rest -COR^{x} bedeutet;
R⁸ aus einem Wasserstoffatom oder den Resten -COR^{x}, -COCH₃ und -CH(OH)CH₃ ausgewählt ist;
R⁹ aus einem Wasserstoffatom oder einer Methylgruppe ausgewählt ist;
R¹⁰ aus den Resten -COR^{x} oder -(C₁₋₆-Alkylen)COR^{x} ausgewählt ist;
R¹¹ aus den Resten -COR^{x}, -(C₂₋₇-Alkenylen)COR^{x}, -NHCO(C₁₋₆-Alkylen)COR^{x}, -NHCO(C₁₋₆-Alkylen)O(C₁₋₆-alkyl), -NHCO(C₂₋₇-Alkenylen)COR^{x}, -NHCO(C₁₋₆-Alkylen)CO(C₁₋₆-alkyl), -NHCONH(C₁₋₆-Alkylen)COR^{x}, -C₂₋₇-Alkanoyl, 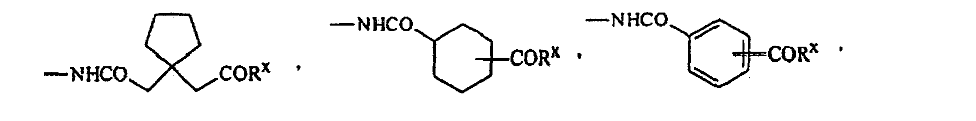 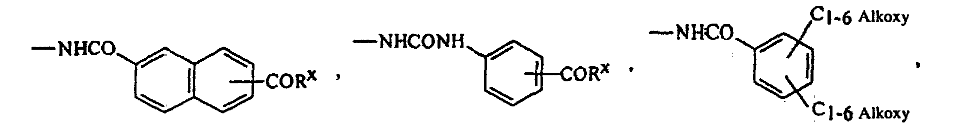 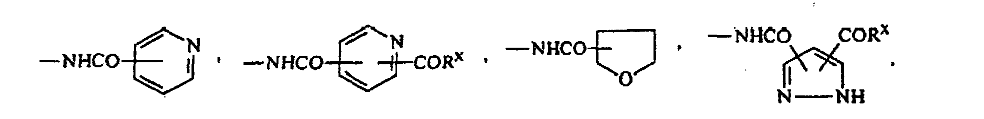 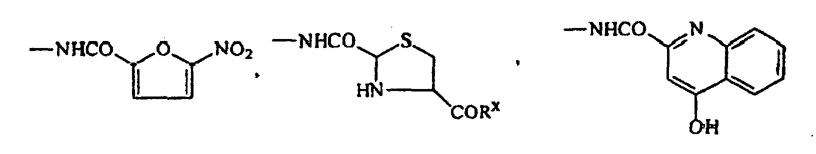 und 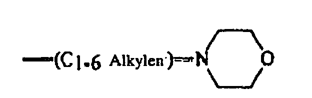 ausgewählt ist;
R¹² einen Rest -COR^{x} oder eine Gruppe -CN bedeutet;
W¹ aus den Gruppen -CO-, -OCH₂CO-, -OCH(CH₃)CO-, -OC(CH₃)₂CO-, -SCH₂CO-, -SCH(CH₃)CO-, -SC(CH₃)₂CO-, -CH=CHCO-, -OCH(C₆H₅)CO- und -SCH(C₆H₅)CO- ausgewählt ist;
W² ein Sauerstoff- oder Schwefelatom bedeutet;
n 1 oder 2 bedeutet; und
m 0 oder 1 bedeutet;
oder ein pharmazeutisch verträgliches Salz davon.

4. Verbindung gemäß Anspruch 1, wobei Y eine Gruppe -NHC(=O)- bedeutet.

5. Verbindung gemäß Anspruch 3, wobei die Formel die folgende ist:

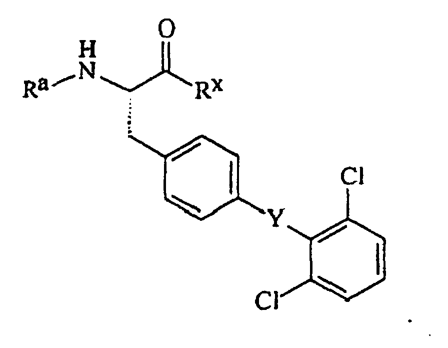

6. Verbindung gemäß Anspruch 5, wobei R^{a} aus den Resten: 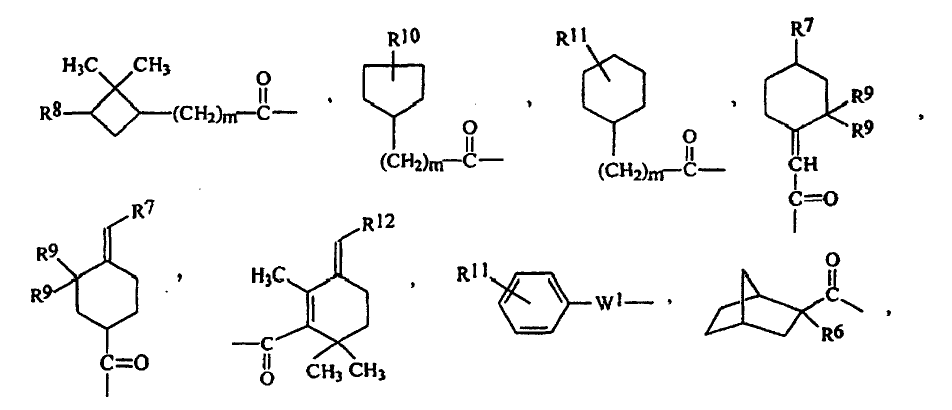 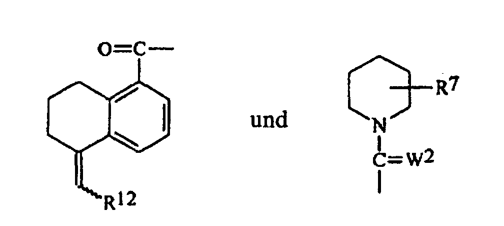 ausgewählt ist.

7. Verbindung gemäß Anspruch 6, wobei R^{a} aus den Resten: 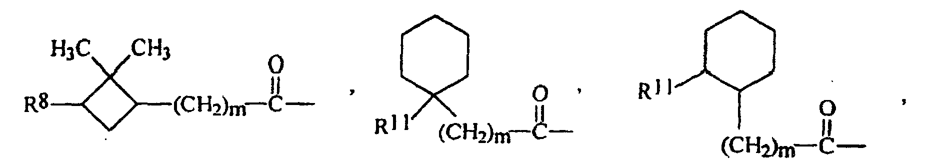 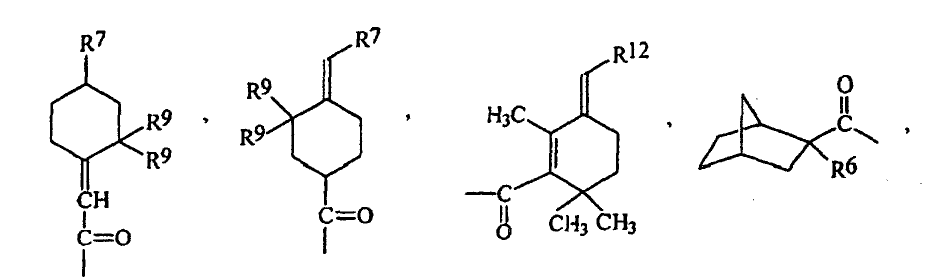 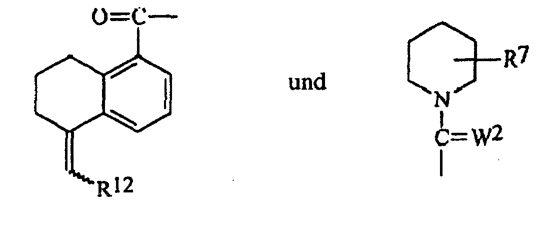 ausgewählt ist;
R⁶ aus den Resten: 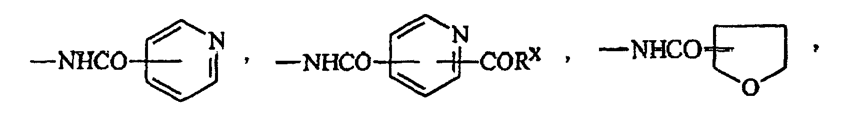 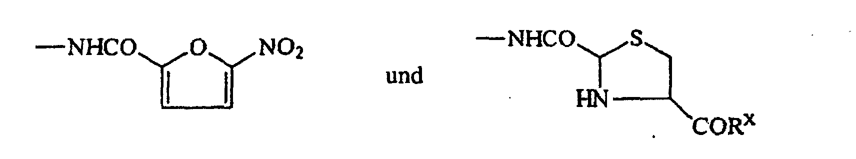 ausgewählt ist; und
R¹¹ aus den Resten -NHCO(C₂₋₇-Alkenylen)COR^{x}, -NHCO(C₁₋₆-Alkylen)CO(C₁₋₆-alkyl), 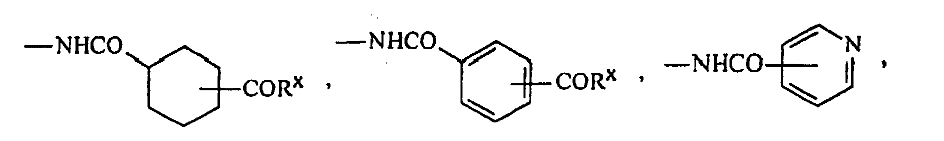 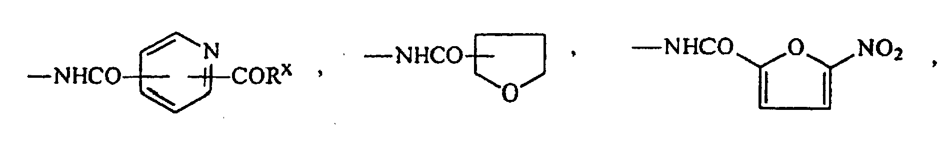 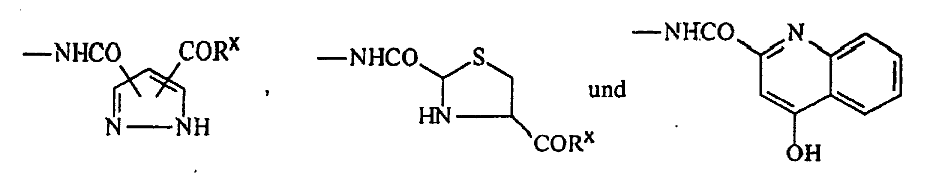 ausgewählt ist.

8. Verbindung gemäß Anspruch 7, wobei R^{a} einen Rest 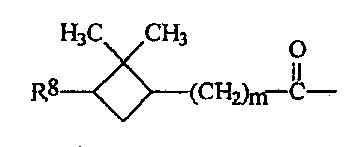 bedeutet und
R⁸ einen Rest COR^{x} oder COCH₃ bedeutet.

9. Verbindung gemäß Anspruch 7, wobei R^{a} einen Rest 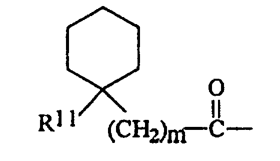 bedeutet und
R¹¹ aus den Resten -NHCO(C₁₋₆-Alkylen)CO(C₁₋₆-alkyl), 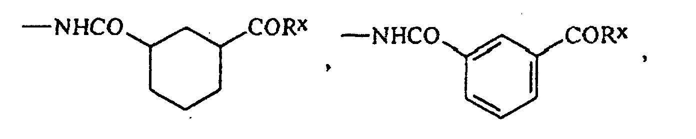 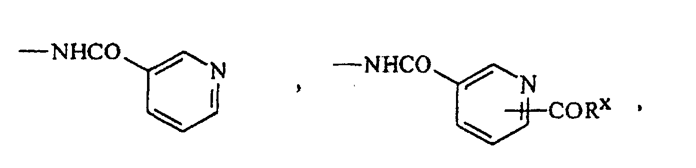 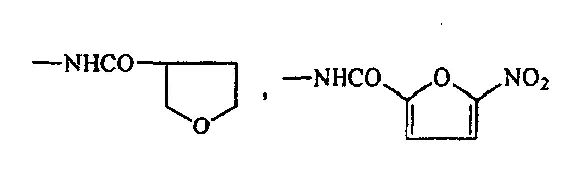 und 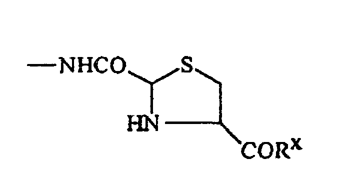 ausgewählt ist.

10. Verbindung gemäß Anspruch 7, wobei R^{a} einen Rest 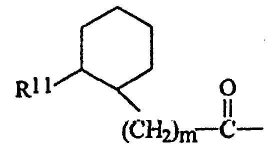 bedeutet und
R¹¹ aus den Gruppen 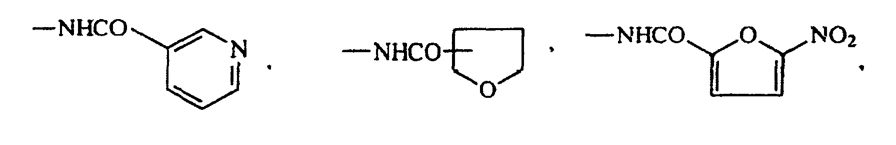 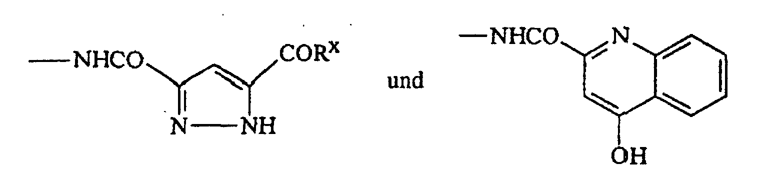 ausgewählt ist.

11. Verbindung gemäß Anspruch 7, wobei R^{a} einen Rest 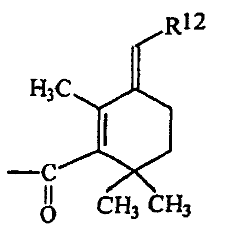 bedeutet.

12. Verbindung gemäß Anspruch 7, wobei R^{a} einen Rest 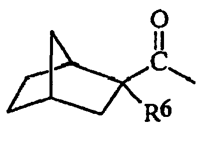 bedeutet und
R⁶ aus den Resten: 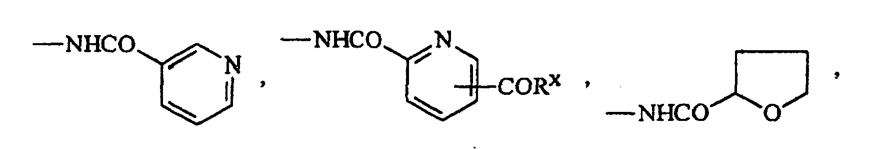 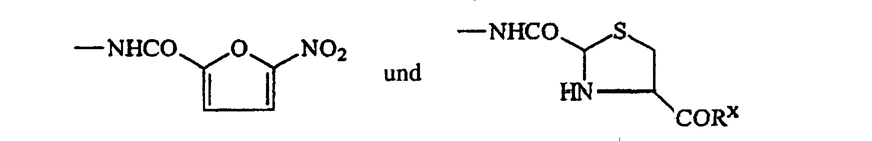 ausgewählt ist.

13. (1S-trans)-N-[(3-Carboxy-2,2-dimethylcyclobutyl)carbonyl]-4-[(2,6-dichlorbenzoyl)amino]-L-phenylalanin,
N-[[(3E)-3-Carboxymethylen-2,6,6-trimethyl-1-cyclohexen-1-yl)carbonyl]-4-[(2,6-dichlorbenzoyl)amino]-L-phenylalanin,
(1S-trans)-N-[(3-Acetyl-2,2-dimethylcyclobutyl)carbonyl]-4-[(2,6-dichlorbenzoyl)amino]-L-phenylalanin,
(1S-cis)-N-[(3-Acetyl-2,2-dimethylcyclobutyl)carbonyl]-4-[(2,6-dichlorbenzoyl)amino]-L-phenylalanin,
(1S-cis)-N-[(3-Carboxy-2,2-dimethylcyclobutyl)carbonyl]-4-[(2,6-dichlorbenzoyl)amino]-L-phenylalanin,
N-[[[1-(1,4-Dioxopentyl)amino]cyclobutyl]carbonyl]-4-[(2,6-dichlorbenzoyl)amino]-Lphenylalanin,
(cis)-N-[[2-[[(5-Carboxy-3-pyrazolyl)carbonyl]-amino]cyclohexyl]carbonyl]-4-[(2,6-dichlorbenzoyl)amino]-L-phenylalanin,
(cis)-N-[[2-[[(5-Nitro-2-furyl)carbonyl]amino]cyclohexyl]-carbonyl]-4-[(2,6-dichlorbenzoyl)amino]-L-phenylalanin,
(cis)-N-[[[[(2-Tetrahydrofuryl)carbonyl]amino]cyclohexyl]-carbonyl]-4-[(2,6-dichlorbenzoyl)amino]-L-phenylalanin,
N-[(1E)-3-Carboxy-2,4,4-trimethyl-2-cyclohexen-1-ylidenacetyl]-4-[(2,6-dichlorbenzoyl)amino]-L-phenylalanin,
N-[[(3E)-3-Cyanomethylen-2,6,6-trimethyl-1-cyclohexen-1-yl]carbonyl]-4-[(2,6-dichlorbenzoyl)-L-phenylalanin,
N-[[(3E)-3-Carboxymethylen-2,6,6-trimethyl-1-cyclohexen-1-yl]carbonyl]-4-O-[(2,6-dichlorphenylmethyl)-L-tyrosin
oder pharmazeutisch verträgliche Salze der vorgenannten Verbindungen.

14. Arzneimittel, das eine therapeutisch wirksame Menge einer Verbindung gemäß einem der Ansprüche 1 bis 13 und einen pharmazeutisch verträglichen Träger umfasst.

15. Verwendung einer Verbindung gemäß einem der Ansprüche 1 bis 13 für die Herstellung eines Arzneimittels zur Behandlung oder Vorbeugung von α₄β₁-Adhäsions-vermittelten Zuständen beim Menschen.

16. Verwendung gemäß Anspruch 15, wobei der Zustand aus rheumatoider Arthritis, Asthma, Allergiezuständen, Allotransplantatabstoßung, Psoriasis, Ekzem, Kontaktdermatitis und anderen entzündlichen Erkrankungen der Haut und entzündlichen und immunentzündlichen Zuständen inklusive entzündlichen Zuständen des Auges, entzündlichen Darmerkrankungen, Atherosklerose und Colitis ulcerosa ausgewählt ist.

## Revendications

1. Composé de formule : 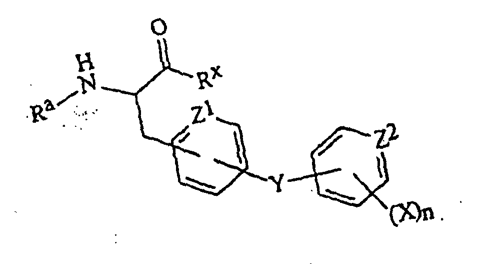 dans laquelle
X est choisi dans le groupe consistant en des groupes halogéno, CF₃, NO₂, OH, alkoxy en C₁ à C₃, NH₂ et alkyle en C₁ à C₄ ;
Z¹ représente un groupe CH ou N ;
Z² représente un groupe CH ou N ;
n est égal à 1, 2 ou 3 ;
Y représente un groupe -OCH₂- ou -NHC(=O)- ;
R^{x} représente un groupe OH ou alkoxy en C₁ à C₆ ;
R^{a} est choisi dans le groupe consistant en des groupes 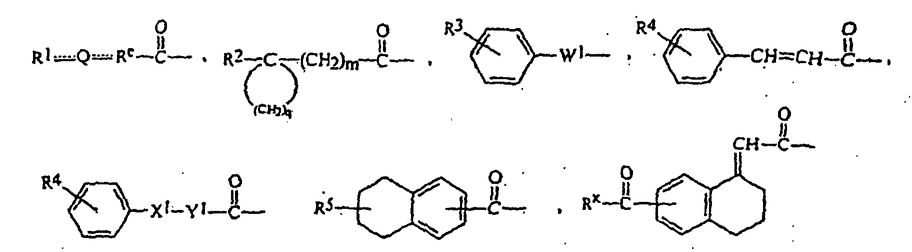 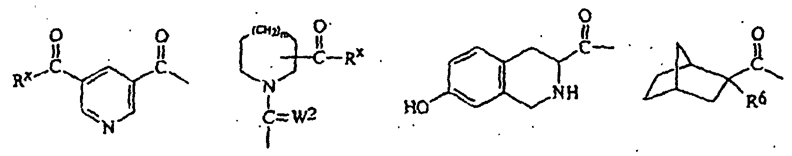 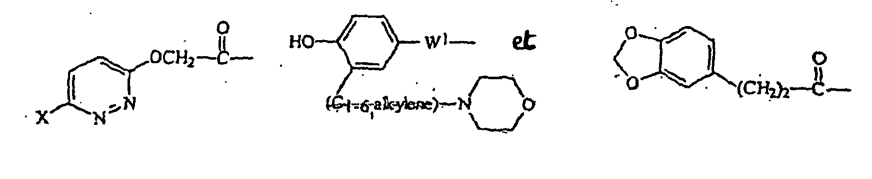 le symbole (alkylène en C₁ à C₆) représente une liaison simple ou une double liaison ;
W¹ est choisi dans le groupe consistant en -CO-, -OCH₂CO-, -OCH(CH₃)CO-, -OC(CH₃)₂CO-, - SCH₂CO-, -SCH(CH₃)CO-, -SC(CH₃)₂CO, -CH=CHCO-, -OCH(C₆H₅)CO- et -SCH(C₆H₅)CO-;
W² représente O ou S;
q est égal à 5 ou 6 ;
m est égal à 0 ou 1 ;
X¹ représente O, S ou une liaison ;
Y¹ représente un groupe alkylène en C₁ à C₃ ou -CH(C₆H₅) ;
R^{c} représente une liaison, un groupe -CH₂- ou =CH- ;
Q représente un noyau cycloalkane en C₃ à C₁₀ ou cycloalcène en C₃ à C₁₀, noyau qui est substitué avec un groupe R¹ et qui peut être en outre substitué avec 1 à 3 groupes méthyle, sous réserve que Q soit autre qu'un noyau cyclopentane qui est substitué avec trois groupes méthyle ;
R¹ est choisi dans le groupe consistant en -H, -COR^{x}, -CH(OH)CH₃, -(alcénylène en C₂ à C₇)COR^{x}, - NHCO (alkylène en C₁ à C₆)COR^{x}, =CHCOR^{x}, -NHCO(alkylène en C₁ à C₆)O(alkyle en C₁ à C₆), - NHCO (alcénylène en C₂ à C₇)COR^{x}, -NHCO(alkylène en C₁ à C₆)CO(alkyle en C₁ à C₆), -NHCONH(alkylène en C₁ à C₆)COR^{x}, -alkanoyle en C₂ à C₇, =CHCN, -(alkylène en C₁ à C₆)COR^{x}, 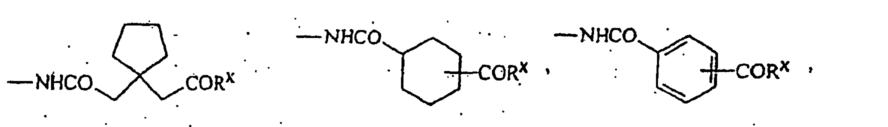 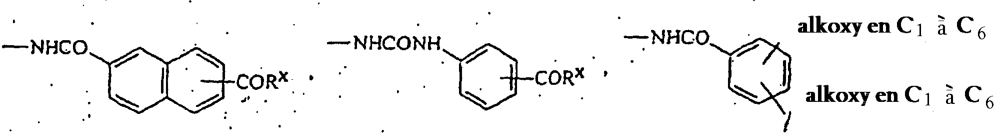 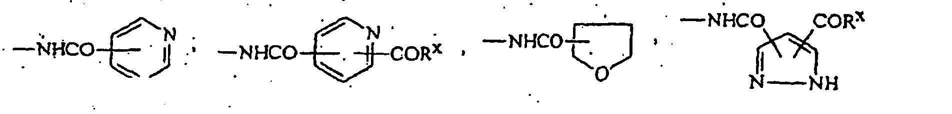 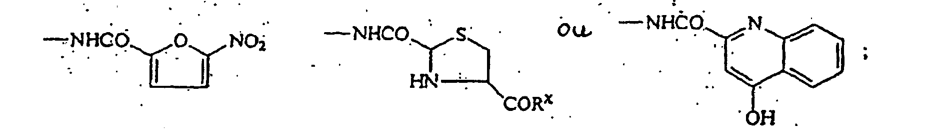
R² est choisi dans le groupe consistant en des groupes -CH₂COR^{x}, -NHCO(alkylène en C₁ à C₆) COR^{x}, -NHCO(alcénylène en C₂ à C₇)COx^{R}, -NHCO(alkylène en C₁ à C₆)O(alkyle en C₁ à C₆), - NHCONH (alkylène en C₁ à C₆)COR^{x}, 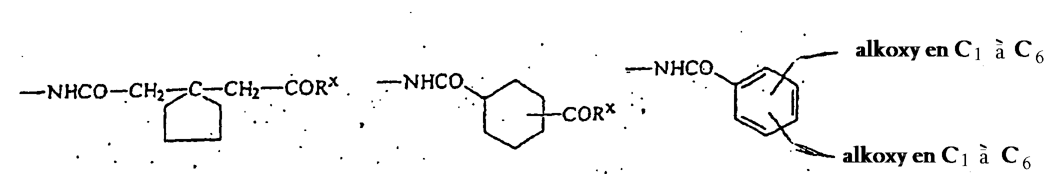 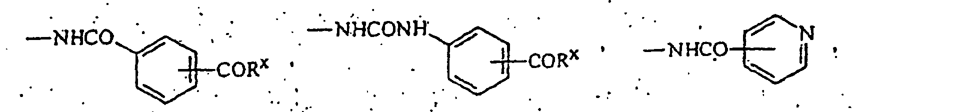 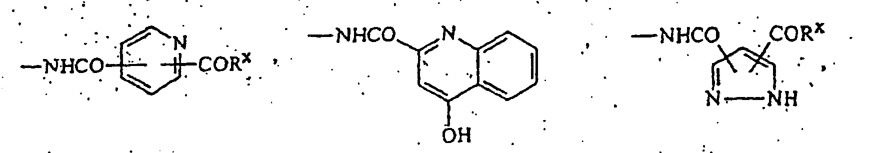 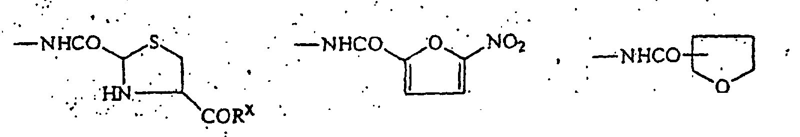 et 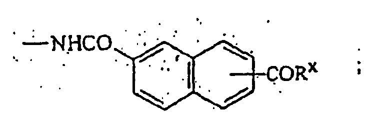
R³ est choisi dans le groupe consistant en des groupes -OH, -COR^{x}, -alcénylène en C₂ à C₇)COR^{x}, -NHCO(alkylène en C₁ à C₆)COR^{x}, -NHCO(alkylène en C₁ à C₆)O(alkyle en C₁ à C₆), - NHCO (alcénylène en C₂ à C₇)COR^{x}, -NHCO(alkylène en C₁ à C₆)CO(alkyle en C₁ à C₆), -NHCONH(alkylène en C₁ à C₆)COR^{x}, -alcanoyle en C₂ à C₇, 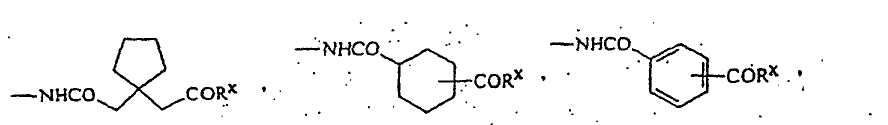 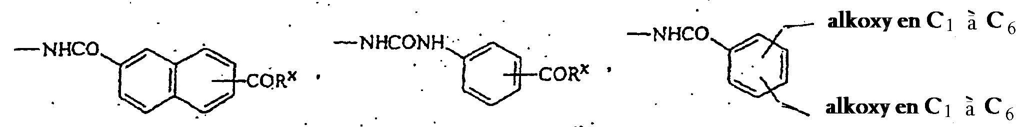 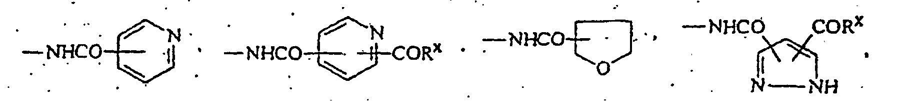 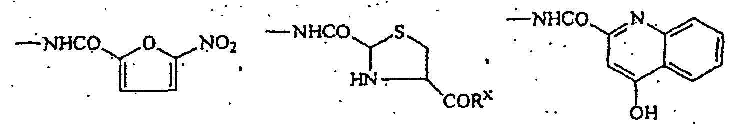 et 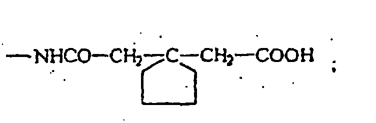
R⁴ est choisi dans le groupe consistant en des groupes -COOH, -CH=CHCOOH et méthylènedioxy ;
R⁵ représente un groupe =CHCOR^{x} ou =CHCN ; et
R⁶ est choisi dans le groupe consistant en des groupes 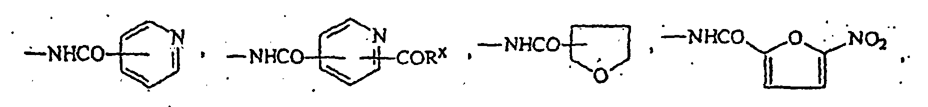 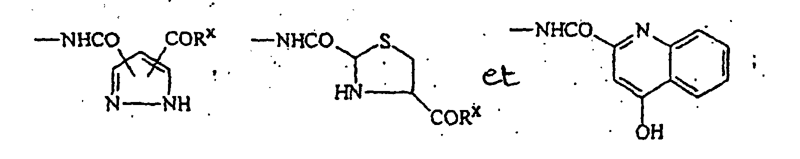
ou un de ses sels pharmaceutiquement acceptables.

2. Composé suivant la revendication 1, répondant à la formule :

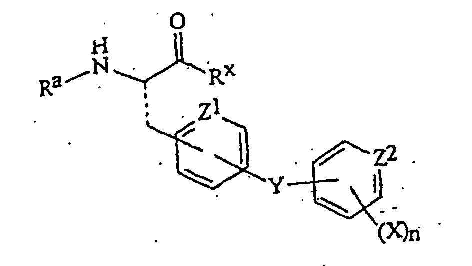

3. Composé suivant la revendication 2, dans lequel
X représente un atome d'halogène ;
Y représente un groupe -OCH₂- ou -NHC(=O)- ;
Z¹ représente un groupe CH ou N ;
Z² représente un groupe CH ou N ;
R^{x} représente un groupe OH ou alkoxy en C₁ à C₆ ;
R^{a} est choisi dans le groupe consistant en 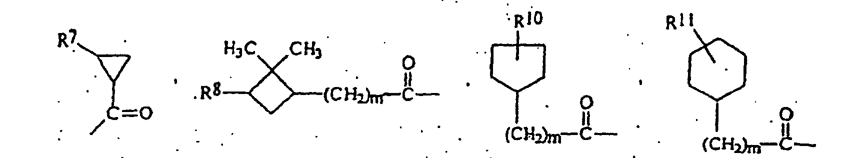 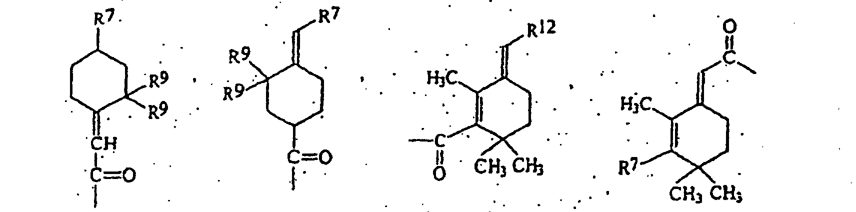 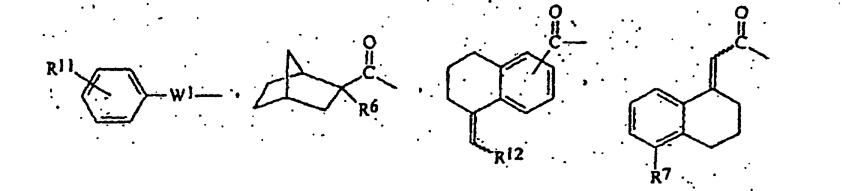 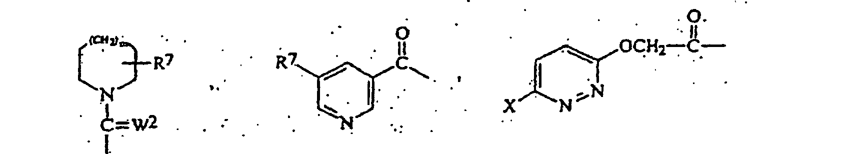 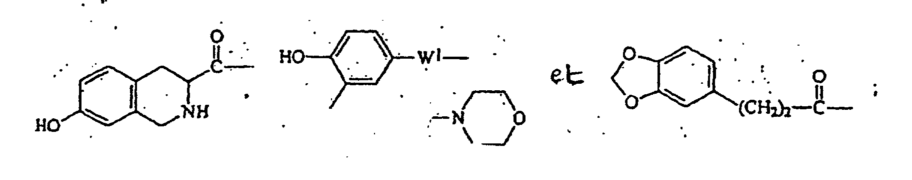
R⁶ est choisi dans le groupe consistant en : 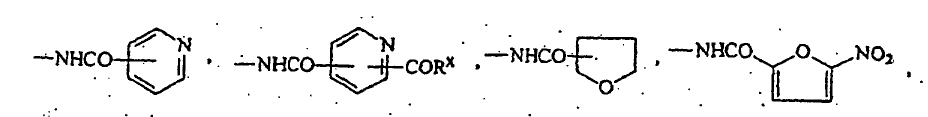 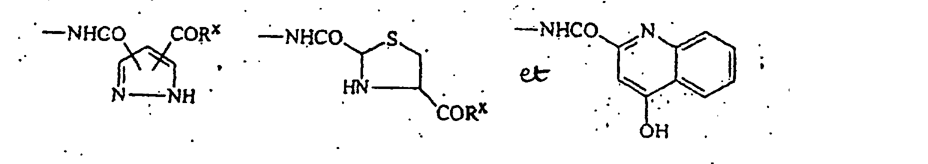
R⁷ représente un groupe -COR^{x} ;
R⁸ est choisi dans le groupe consistant en un atome d'hydrogène, des groupes -COR^{x}, -COCH₃ et -CH(OH)CH₃ ;
R⁹ représente un atom d'hydrogène ou un groupe méthyle ;
R¹⁰ représente un groupe -COR^{x} ou -(alkylène en C₁ à C₆)COR^{x} ;
R¹¹ est choisi dans le groupe consistant en des groupes -COR^{x}, -(alcénylène en C₂ à C₇)COR^{x}, -NHCO (alkylène en C₁ à C₆)COR^{x}, -NHCO (alkylène- en C₁ à C₆)O(alkyle en C₁ à C₆), -NHCO (alcénylène- en C₂ à C₇)COR^{x}, -NHCO(alkylène en C₁ à C₆)CO(alkyle en C₁ à C₆), -NHCONH(alkylène en C₁ à C₆)COR^{x}, -alcanoyle en C₂ à C₇, 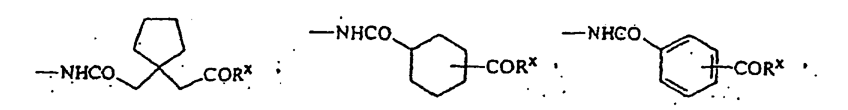 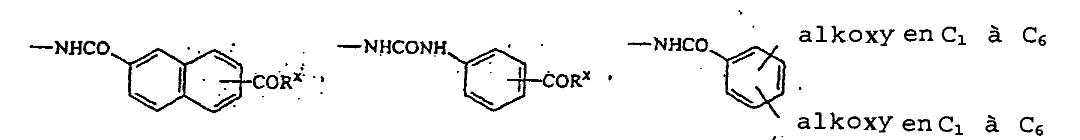 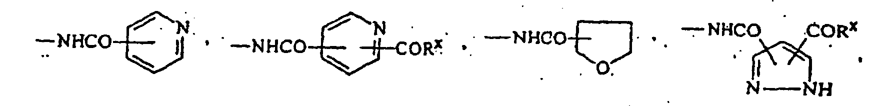 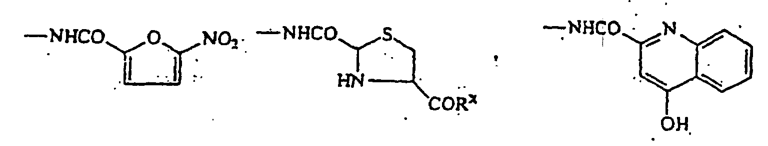 et 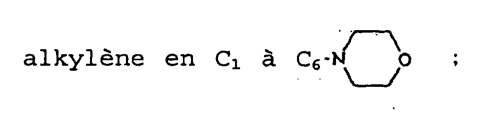
R¹² représente un groupe -COR^{x} ou -CN ;
W¹ est choisi dans le groupe consistant en des groupes -CO-, -OCH₂CO-, -OCH(CH₃)CO-, - OC(CH₃)₂CO-, -SCH₂CO-, -SCH(CH₃)CO-, -SC(CH₃)₂CO-, -CH=CHCO-, -OCH(C₆H₅)CO- et -SCH(C₆H₅)CO- ;
W² représente O ou S;
n est égal à 1 ou 2 ; et
m est égal à 0 ou 1 ;
ou un de ses sels pharmaceutiquement acceptables.

4. Composé suivant la revendication 1, dans lequel Y représente un groupe -NHC(=O)-.

5. Composé suivant la revendication 3, dont la formule est la suivante :

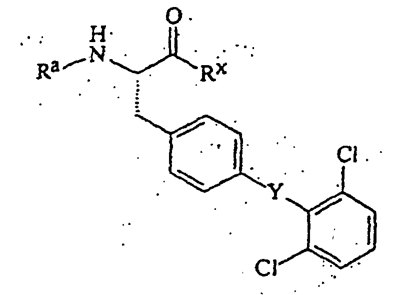

6. Composé suivant la revendication 5, dans lequel R^{a} est choisi dans le groupe consistant en :

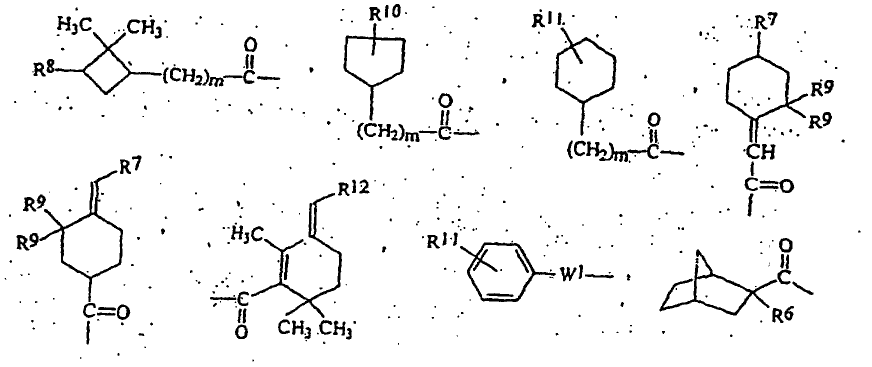

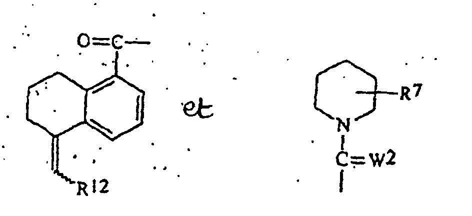

7. Composé suivant la revendication 6, dans lequel R^{a} est choisi dans le groupe consistant en : 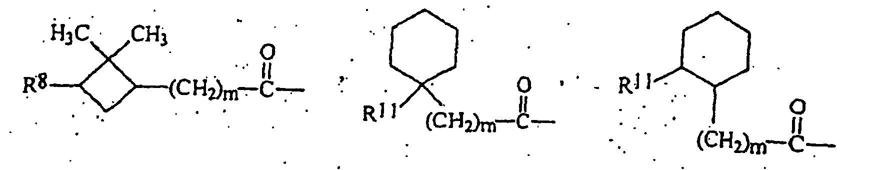 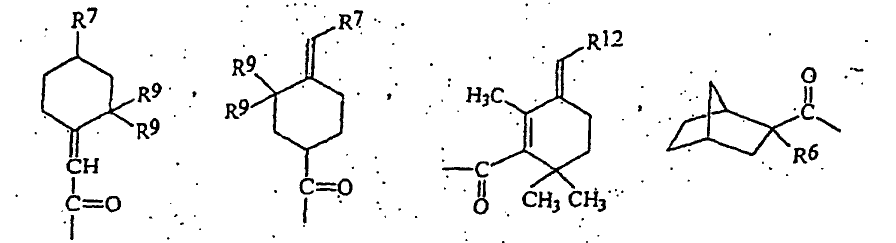 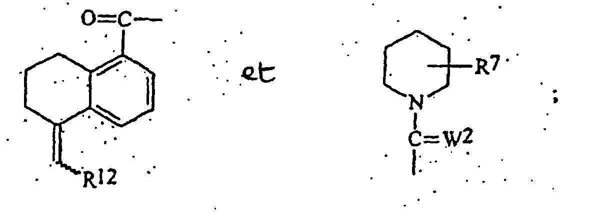
R⁶ est choisi dans le groupe consistant en : 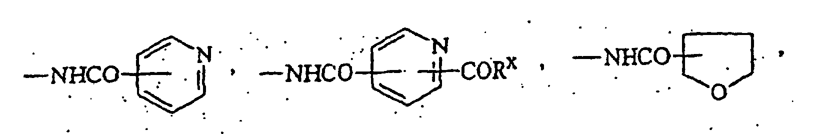 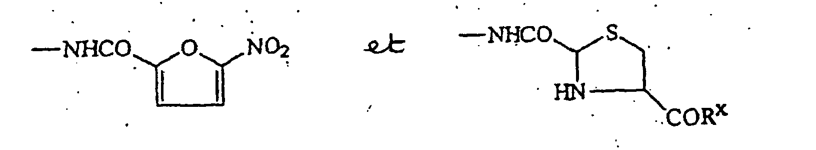 et
R¹¹ est choisi dans le groupe consistant en des groupes -NHCO(alcénylène en C₂ à C₇)COR^{x}, - NHCO (alkylène en C₁ à C₆)CO(alkyle en C₁ à C₆),

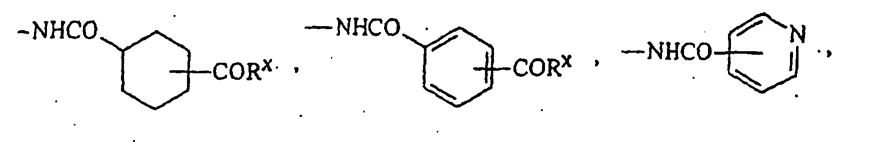

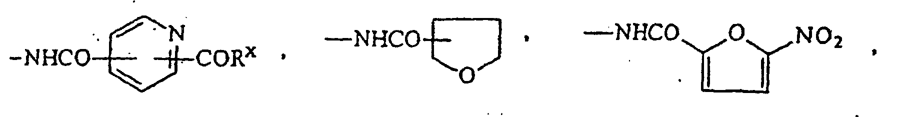

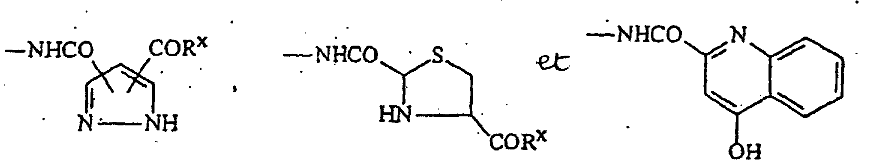

8. Composé suivant la revendication 7, dans lequel R^{a} représente un groupe 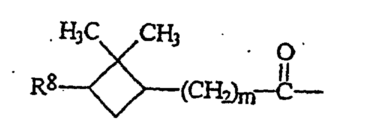 et R⁸ représente un groupe COR^{x} ou COCH₃.

9. Composé suivant la revendication 7, dans lequel R^{a} représente un groupe 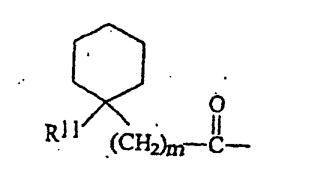 et R¹¹ est choisi dans le groupe consistant en des groupes -NHCO(alkylène en C₁ à C₆)CO(alkyle en C₁ à C₆), 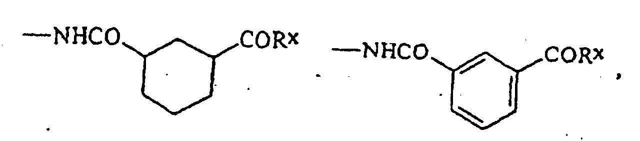 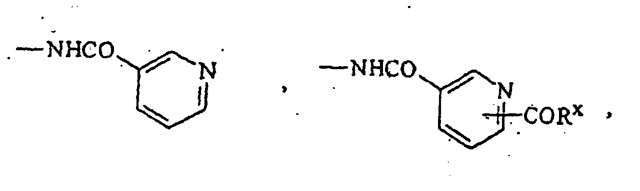 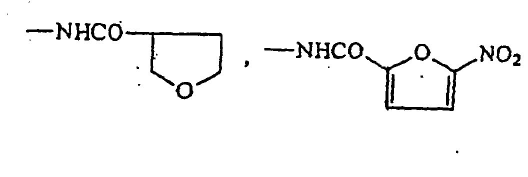 et

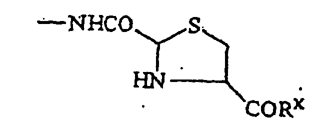

10. Composé suivant la revendication 7, dans lequel R^{a} représente un groupe 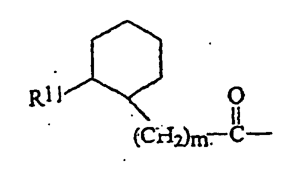 et R¹¹ est choisi dans le groupe consistant en

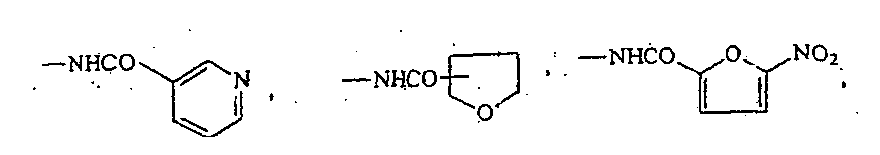

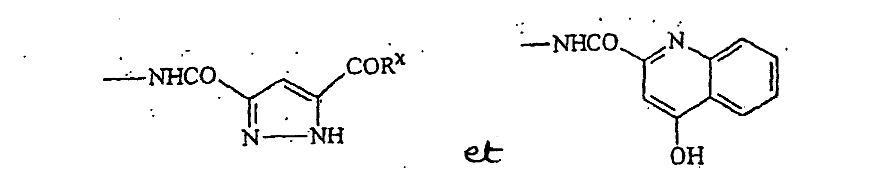

11. Composé suivant la revendication 7, dans lequel R^{a} représente un groupe

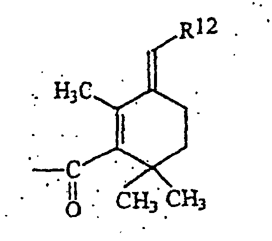

12. Composé suivant la revendication 7, dans lequel R^{a} représente un groupe 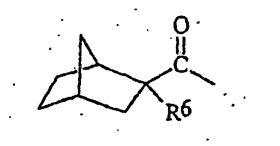 et R⁶ est choisi dans le groupe consistant en :

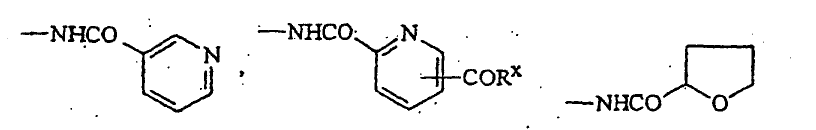

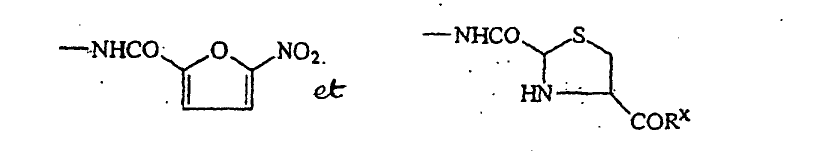

13. (1S-*trans*)-N-[(3-carboxy-2,2-diméthylcyclobutyl)-carbonyl]-4-[(2,6-dichlorobenzoyl)amino]-L-phénylalanine,
N-[[(3E)-3-carboxyméthylène-2,6,6-triméthyl-1-cyclohexén-1-yl)carbonyl]-4-[(2,6-dichlorobenzoyl)amino]-L-phénylalanine,
(1S-*trans*)-N-[(3-acétyl-2,2-diméthylcyclobutyl) carbonyl]-4-[(2,6-dichlorobenzoyl)amino]-L-phénylalanine,
(1S-*cis*)-N-[(3-acétyl-2,2-diméthylcyclobutyl) carbonyl]-4-[(2,6-dichlorobenzoyl)amino]-L-phénylalanine,
(1S-*cis*)-N-[(3-carboxy-2,2-diméthylcyclobutyl) carbonyl]-4-[(2,6-dichlorobenzoyl)amino]-L-phénylalanine,
N-[[[1-(1,4-dioxopentyl)amino]cyclobutyl]carbonyl]-4-[(2,6-dichlorobenzoyl)amino]-L-phénylalanine,
(*cis*)-N-[[2-[[(5-carboxy-3-pyrazolyl)carbonyl]-amino]cyclohexyl]carbonyl]-4-[(2,6-dichlorobenzoyl)amino]-L-phénylalanine,
(*cis*)-N-[[2-[[(5-nitro-2-furyl)carbonyl]amino) cyclohexyl]carbonyl]-4-[(2,6-dichlorobenzoyl)amino]-L-phénylalanine,
(*cis*)-N-[[[[(2-tétrahydrofuryl)carbonyl]amino) cyclohexyl]carbonyl]-4-[(2,6-dichlorobenzoyl)amino]-L-phénylalanine,
N-[(lE)-3-carboxy-2,4,4-triméthyl-2-cyclohexen-1-ylidèneacétyl]-4-[(2,6-dichlorobenzoyl)amino]-L-phénylalanine,
N-[(3E)-3-cyanométhylène-2,6,6-triméthyl-1-cyclohexen-1-yl]carbonyl]-4-[(2,6-dichlorobenzoyl) ]-L-phénylalanine,
N-[[3E)-3-carboxyméthylène-2,6,6-triméthyl-1-cyclohexen-1-yl]carbonyl]-4-O-[(2,6-dichlorophénylméthyl]-L-tyrosine,
ou les sels pharmaceutiquement acceptables des composés précités.

14. Composition pharmaceutique comprenant une quantité thérapeutiquement efficace d'un composé suivant l'une quelconque des revendications 1 à 13 ; et un support pharmaceutiquement acceptable.

15. Utilisation d'un composé suivant l'une quelconque des revendications 1 à 13 pour la préparation d'une composition pharmaceutique destinée au traitement ou à la prévention d'affections à médiation par adhésion de α₄β₁ chez l'homme.

16. Utilisation suivant la revendication 15, dans laquelle ladite affection est choisie dans le groupe consistant en la polyarthrite rhumatoïde, l'asthme, des états allergiques, le rejet d'allogreffes, le psoriasis, l'eczéma, la dermatite de contact et d'autres maladies inflammatoires cutanées et des affections inflammatoires et immuno-inflammatoires comprenant des affections inflammatoires ophtalmiques, des maladies intestinales inflammatoires, l'athérosclérose et la colite ulcérative.
